# EUROPEAN PATENT APPLICATION

(11) **EP 4 385 523 A1**
(43) Date of publication of application: **19.06.2024**
(21) Application number: 23173305.6
(22) Date of filing: 15.05.2023
(51) Int. Cl.: A61K 47/69, A61K 9/51, C12N 15/87, A61K 47/54

(54) **LIPID-BASED TOPICAL INJECTION FORMULATIONS**

(30) Priority: 14.12.2022 CN 202211599668; 20.01.2023 CN 202310098259
(71) Applicant: Beijing Jitai Pharmaceutical Technology Co., Ltd., Beijing 100192 (CN); Hangzhou Jitai Pharmaceutical Technology Co., Ltd., Hangzhou, Zhejiang 310051 (CN)
(72) Inventor: YANG, Liu, ZHEJIANG, 310051 (CN); LANG, Jiayan, BEIJING, 100192 (CN); ZHANG, Lin, BEIJING, 100192 (CN); JIANG, Tian, BEIJING, 100192 (CN); WANG, Xuhui, BEIJING, 100192 (CN); LEI, Jiani, BEIJING, 100192 (CN); LIU, Andong, ZHEJIANG, 310051 (CN); LAI, Caida, ZHEJIANG, 310051 (CN); WANG, Wenshou, ZHEJIANG, 310051 (CN)
(74) Representative: Cabinet Becker et Associés

(57) **Abstract**

The present disclosure relates to a lipid-based topical injection formulation comprising a Long-Acting SusTained delivering lipid, a structured lipid, a polymer-conjugated lipid and an ionizable lipid, and optionally a neutral phospholipid. The lipid-based topical injection formulation enables enriched delivery of drugs at an injection site and efficient expression of proteins for a prolonged period of time. The present disclosure also relates to a method of preparing the lipid-based topical injection formulation, and use of the lipid-based topical injection formulation in the delivery of biologically active substances such as nucleic acids (e.g., mRNA, miRNA, siRNA, saRNA, ASO, DNA, etc.).

## Description

### REFERENCE TO RELATED APPLICATIONS

This application claims the priority of Chinese Patent Application Nos. 202211599668.3 filed on December 14, 2022 and 202310098259.3 filed on January 20, 2023, which are incorporated herein by reference in their entirety as part of this application.

### FIELD OF THE INVENTION

The present disclosure relates to a field of pharmaceutical formulations, in particular, to a lipid-based topical injection formulation. The present disclosure also relates to a preparation method of the lipid-based topical injection formulation, and to the use of the lipid-based topical injection formulation in the delivery of biologically active substances such as nucleic acids (e.g. mRNA, miRNA, siRNA, saRNA, ASO, DNA, etc.).

### BACKGROUND OF THE INVENTION

Messenger ribonucleic acid (mRNA) is capable of encoding proteins with specific biological functions *in vivo,* thus allowing the rational design of mRNA-based therapies. In recent years, with the success of COVID-19 mRNA vaccine, mRNA drugs have developed rapidly and have received wide attention from scientific research and industry. The biggest barrier for mRNA drugs to enter the clinical stage is drug delivery, and thus choosing a suitable drug delivery system is the key to successful drug development.

At present, most mRNA drug delivery systems use lipid nanoparticles (LNPs). LNPs, including delivery systems for marketed vaccine products, typically consist of four types of lipid components: ionizable cationic lipid, phospholipid, cholesterol, and PEG lipid. Studies have shown that the acid dissociation constant (pKa) of an LNP, measured by the 2-(p-tolylamino)-6-naphthalenesulfonic acid (TNS) dye binding assay, should be in the range of 6-7 for efficient delivery of mRNA drugs. This pKa characteristic can ensure that it remains neutral at physiological pH, improving the stability in the circulatory system. But this pKa characteristic can cause it to be protonated in the acidic environment of endosomes, thereby promoting the endosome escape of mRNA.

The two marketed COVID-19 mRNA vaccines are both administered by topical (intramuscular) injection, showing the potential of LNP for this mode of administration. At present, there are nearly 1000 known monogenic diseases that can cause muscle dysfunction. For such diseases, intramuscular injection is the preferred way of administration. However, studies have found that LNP has off-target toxicity after intramuscular injection, wherein LNP is not only distributed in the injected muscle tissue, but also distributed in the whole body, and a large part is enriched in the liver. Furthermore, the associated proteins are expressed more rapidly in the liver than in the muscle.

Intratumoral injection of mRNA encoding cytokines can improve the entire tumor microenvironment, activate related APCs and T cells, and achieve the purpose of clearing tumor cells and generating immune memory. Compared with systemic drug delivery, intratumoral injection allows the drug to enter the tumor directly, significantly increasing the topical drug concentration and reducing systemic toxicity. However, cytokines expressed by mRNA will also enter the blood circulation through intratumoral blood vessels after secretion, making them also face off-target toxicity caused by systemic exposure.

In addition, although the stability of mRNA is increased after being entrapped in lipids, it is still expected in the field to further increase the action time of drugs through the optimization of LNP, so as to improve the compliance of patients, and make some drugs with short half-lives or large side effects that are difficult to be practically applied obtain ideal clinical effects. The systemic distribution and uncontrollable expression of mRNA may lead to many serious side effects, so targeting mRNA LNP formulations to specific tissues, organs and cells is a recent research hotspot.

### SUMMARY OF THE INVENTION

The present disclosure aims to solve the above problems by providing a lipid-based topical injection formulation that can realize the local enrichment delivery of a drug at the injection site and the long-term high-efficiency expression of a protein.

In order to develop an LNP formulation targeting muscles, the inventors have made various attempts and adjustments to the formulation. It has been found that a lipid nanoparticle formed by combining a Long-Acting SusTained delivering lipid such as a cationic lipid with a permanently positive form (DOTAP, EPC, etc.) and an ionizable lipid and then binding the two to mRNA sequence through electrostatic interaction can reduce the off-target effect of topical injection of mRNA-LNP drugs, significantly improve the peak concentration and bioavailability of drugs in injection site tissues, and also prolong the time to peak and half-life of drugs in injection site tissues.

To achieve the above purpose, the present disclosure provides a lipid nanoparticle for topical injection comprising a Long-Acting SusTained delivering lipid and an ionizable lipid, wherein the lipid nanoparticle is capable of acting at an injection site.

In an alternative embodiment, the site of the topical injection is a muscle or tumor tissue, alternatively muscle.

In an alternative embodiment, the lipid nanoparticle has an extended duration of action compared to a lipid nanoparticle without a Long-Acting SusTained delivering lipid.

In an alternative embodiment, the lipid nanoparticle is capable of reducing off-target effects in tissues or organs at non-injection sites.

In an alternative embodiment, the tissue or organ at a non-injection site is liver.

In an alternative embodiment, the lipid nanoparticle comprises the following components: Long-Acting SusTained delivering lipid, structured lipid, polymer-conjugated lipid and ionizable lipid, and comprises optionally neutral phospholipid.

In an alternative embodiment, the lipid nanoparticle comprises the following components: permanently cationic lipid, structured lipid, polymer-conjugated lipid and ionizable lipid, and does not comprise neutral phospholipid.

In another aspect, the present disclosure provides a lipid nanoparticle composition comprising any of the above lipid nanoparticles and a load.

In another aspect, the present disclosure provides a method of preparing the above lipid nanoparticle composition, comprising mixing the various lipid components, and then mixing them with a load.

In another aspect, the present disclosure provides a pharmaceutical composition comprising any of the above lipid nanoparticle compositions, and a pharmaceutically acceptable excipient.

In another aspect, the present disclosure provides use of any of the above lipid nanoparticle compositions, or the above pharmaceutical composition in the manufacture of a medicament for the treatment, diagnosis or prevention of diseases.

In another aspect, the present disclosure provides use of any of the above lipid nanoparticle compositions, or the above pharmaceutical composition in the manufacture of a medicament for delivery of a load, wherein the load is one or more of therapeutic agents, prophylactic agents, or diagnostic agents.

In another aspect, the present disclosure provides a method of treating, diagnosing, or preventing diseases in a subject, comprising administering to the subject any of the above lipid nanoparticle compositions, or the above pharmaceutical composition.

In another aspect, the present disclosure provides any of the above lipid nanoparticle compositions, or the above pharmaceutical composition, for use in treating, diagnosing, or preventing diseases.

In another aspect, the present disclosure provides a method of delivering a load into the body of a subject, comprising administering to the subject any of the above lipid nanoparticle compositions, or the above pharmaceutical composition.

In another aspect, the present disclosure provides any of the above lipid nanoparticle compositions, or the above pharmaceutical composition, for use in delivering a load.

In a specific embodiment, the load is one or more of therapeutic agents, prophylactic agents, or diagnostic agents; alternatively, the therapeutic agent, prophylactic agent, or diagnostic agent is a nucleic acid.

In a more specific embodiment, the nucleic acid is one or more of ASO, RNA or DNA.

In a more specific embodiment, the RNA is selected from one or more of interfering RNA (RNAi), small interfering RNA (siRNA), short hairpin RNA (shRNA), antisense RNA (aRNA), messenger RNA (mRNA), modified messenger RNA (mmRNA), long non-coding RNA (lncRNA), microRNA (miRNA), small activating RNA (saRNA), multimeric coding nucleic acid (MCNA), polymeric coding nucleic acid (PCNA), guide RNA (gRNA), CRISPRRNA (crRNA) and nucleases, alternatively mRNA, still alternatively modified mRNA.

### TERMINOLOGY

### Chemical terminology

Terminology of specific functional groups and chemical terms are described in more detail below.

When a range of values is listed, it is intended to encompass each value and sub-range within the range. For example, "C₁₋₆ alkyl" is intended to include C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅ and C₅₋₆ alkyl.

"C₁₋₃₀ alkyl" refers to a radical of a linear or branched, saturated hydrocarbon group having 1 to 30 carbon atoms. "C₆₋₃₀ alkyl" refers to a radical of a linear or branched, saturated hydrocarbon group having 6 to 30 carbon atoms. In some embodiments, C₆₋₂₈ alkyl, C₆₋₂₅ alkyl, C₆₋₂₄ alkyl, C₆₋₂₂ alkyl, C₆₋₂₀ alkyl, C₈₋₂₈ alkyl, C₈₋₂₅ alkyl, C₈₋₂₄ alkyl, C₈₋₂₂ alkyl, C₈₋₂₀ alkyl, C₁₀₋₂₈ alkyl, C₁₀₋₂₅ alkyl, C₁₀₋₂₄ alkyl, C₁₀₋₂₂ alkyl, C₁₀₋₂₀ alkyl, C₁₃₋₂₅ alkyl, C₁₃₋₂₀ alkyl, C₁₃₋₁₈ alkyl, C₁₃₋₁₇ alkyl, C₁₅₋₁₈ alkyl, C₁₅₋₂₅ alkyl, C₁₅₋₂₀ alkyl, C₄₋₂₈ alkyl, C₄₋₂₄ alkyl, C₄₋₂₀ alkyl, C₈₋₁₀ alkyl, C₂₋₈ alkyl, C₇₋₉ alkyl, C₄₋₆ alkyl, C₁₋₂₅ alkyl, C₁₋₂₀ alkyl, C₁₋₁₇ alkyl, C₁₇₋₁₈ alkyl, C₁₇ alkyl, C₁₋₁₄ alkyl, C₂₋₁₄ alkyl, C₁₋₁₃ alkyl, C₁₋₁₂ alkyl, C₁₋₁₀ alkyl, C₁₋₈ alkyl, C₁₋₇ alkyl, C₂₋₇ alkyl, C₁₋₆ alkyl, C₁₋₅ alkyl, C₅ alkyl, C₁₋₄ alkyl, C₂₋₄ alkyl, C₁₋₃ alkyl, C₂₋₃ alkyl, C₁₋₂ alkyl and Me are alternative. Examples of C₁₋₆ alkyl include methyl (C₁), ethyl (C₂), n-propyl (C₃), iso-propyl (C₃), n-butyl (C₄), tert-butyl (C₄), sec-butyl (C₄), iso-butyl (C₄), n-pentyl (C₅), 3-pentyl (C₅), pentyl (C₅), neopentyl (C₅), 3-methyl-2-butyl (C₅), tert-pentyl (C₅) and n-hexyl (C₆). The term "C₁₋₃₀ alkyl" also includes heteroalkyl, wherein one or more (e.g., 1, 2, 3 or 4) carbon atoms are substituted with heteroatoms (e.g., oxygen, sulfur, nitrogen, boron, silicon, phosphorus). Alkyl groups can be optionally substituted with one or more substituents, for example, with 1 to 5 substituents, 1 to 3 substituents or 1 substituent. Conventional abbreviations of alkyl include Me (-CH₃), Et (-CH₂CH₃), iPr (-CH(CH₃)₂), nPr (-CH₂CH₂CH₃), n-Bu (-CH₂CH₂CH₂CH₃) or i-Bu (-CH₂CH(CH₃)₂).

"C₆₋₃₀ alkenyl" refers to a radical of a linear or branched hydrocarbon group having 6 to 30 carbon atoms and at least one carbon-carbon double bond. "C₄₋₂₀ alkenyl" refers to a radical of a linear or branched hydrocarbon group having 4 to 20 carbon atoms and at least one carbon-carbon double bond. "C₂₋₁₀ alkenyl" refers to a radical of a linear or branched hydrocarbon group having 2 to 10 carbon atoms and at least one carbon-carbon double bond. In some embodiments, C₁₀₋₂₅ alkenyl, C₁₃₋₂₀ alkenyl, C₁₃₋₁₈ alkenyl, C₁₃₋₁₇ alkenyl, C₁₅₋₁₈ alkenyl, C₁₇₋₁₈ alkenyl, C₄₋₁₄ alkenyl, C₄₋₁₂ alkenyl, C₂₋₆ alkenyl, and C₂₋₄ alkenyl are alternative. Examples of C₂₋₆ alkenyl include vinyl (C₂), 1-propenyl (C₃), 2-propenyl (C₃), 1-butenyl (C₄), 2-butenyl (C₄), butadienyl (C₄), pentenyl (C₅), pentadienyl (C₅), hexenyl (C₆), etc. The term "C₂₋₆ alkenyl" also includes heteroalkenyl, wherein one or more (e.g., 1, 2, 3 or 4) carbon atoms are replaced by heteroatoms (e.g., oxygen, sulfur, nitrogen, boron, silicon, phosphorus). The alkenyl groups can be optionally substituted with one or more substituents, for example, with 1 to 5 substituents, 1 to 3 substituents or 1 substituent.

"C₆₋₃₀ alkynyl" refers to a radical of a linear or branched hydrocarbon group having 6 to 30 carbon atoms, at least one carbon-carbon triple bond and optionally one or more carbon-carbon double bonds. "C₄₋₂₀ alkynyl" refers to a radical of a linear or branched hydrocarbon group having 4 to 20 carbon atoms, at least one carbon-carbon triple bond and optionally one or more carbon-carbon double bonds. "C₂₋₁₀ alkynyl" refers to a radical of a linear or branched hydrocarbon group having 2 to 10 carbon atoms, at least one carbon-carbon triple bond and optionally one or more carbon-carbon double bonds. In some embodiments, C₁₀₋₂₅ alkynyl, C₁₃₋₂₀ alkynyl, C₁₃₋₁₈ alkynyl, C₁₃₋₁₇ alkynyl, C₁₅₋₁₈ alkynyl, C₁₇₋₁₈ alkynyl, C₄₋₁₄ alkynyl, C₄₋₁₂ alkynyl, C₂₋₆ alkynyl, and C₂₋₄ alkynyl are alternative. Examples of C₂₋₆ alkynyl include, but are not limited to, ethynyl (C₂), 1-propynyl (C₃), 2-propynyl (C₃), 1-butynyl (C₄), 2-butynyl (C₄), pentynyl (C₅), hexynyl (C₆), etc. The term "C₂₋₆ alkynyl" also includes heteroalkynyl, wherein one or more (e.g., 1, 2, 3 or 4) carbon atoms are replaced by heteroatoms (e.g., oxygen, sulfur, nitrogen, boron, silicon, phosphorus). The alkynyl groups can be substituted with one or more substituents, for example, with 1 to 5 substituents, 1 to 3 substituents or 1 substituent.

"C₁₋₂₄ alkylene" refers to a divalent group formed by removing another hydrogen of the C₁₋₂₄ alkyl, and can be substituted or unsubstituted. In some embodiments, C₄₋₂₀ alkylene, C₈₋₁₀ alkylene, C₂₋₈ alkylene, C₇₋₉ alkylene, C₄₋₆ alkylene, C₁₋₂₀ alkylene, C₁₋₁₄ alkylene, C₂₋₁₄ alkylene, C₄₋₁₄ alkylene, C₁₋₁₃ alkylene, C₁₋₁₂ alkylene, C₁₋₁₀ alkylene, C₁₋₈ alkylene, C₁₋₇ alkylene, C₂₋₇ alkylene, C₁₋₆ alkylene, C₁₋₅ alkylene, C₅ alkylene, C₁₋₄ alkylene, C₂₋₄ alkylene, C₁₋₃ alkylene, C₂₋₃ alkylene, C₁₋₂ alkylene, and methylene are alternative. The unsubstituted alkylene groups include, but are not limited to, methylene (-CH₂-), ethylene (-CH₂CH₂-), propylene (-CH₂CH₂CH₂-), butylene (-CH₂CH₂CH₂CH₂-), pentylene (-CH₂CH₂CH₂CH₂CH₂-), hexylene (-CH₂CH₂CH₂CH₂CH₂CH₂-), etc. Examples of substituted alkylene groups, such as those substituted with one or more alkyl (methyl) groups, include, but are not limited to, substituted methylene (-CH(CH₃)-, -C(CH₃)₂-), substituted ethylene (-CH(CH₃)CH₂-, -CH₂CH(CH₃)-, -C(CH₃)₂CH₂-, -CH₂C(CH₃)₂-), substituted propylene (-CH(CH₃)CH₂CH₂-, -CH₂CH(CH₃)CH₂-, -CH₂CH₂CH(CH₃)-, -C(CH₃)₂CH₂CH₂-, -CH₂C(CH₃)₂CH₂-, -CH₂CH₂C(CH₃)₂-), etc.

"C₂₋₂₄ alkenylene" refers to a C₂₋₂₄ alkenyl group wherein another hydrogen is removed to provide a divalent radical of alkenylene, which may be substituted or unsubstituted. In some embodiments, C₂₋₂₀ alkenyl, C₂₋₁₈ alkenyl, C₂₋₁₅ alkenyl, C₂₋₁₃ alkenyl, C₂₋₁₀ alkenyl, C₄₋₁₄ alkenyl, C₂₋₆ alkenyl, and C₂₋₄ alkenylene is yet alternative. Exemplary unsubstituted alkenylene groups include, but are not limited to, ethylene (-CH=CH-) and propenylene (e.g., -CH=CHCH₂-, -CH₂-CH=CH-). Exemplary substituted alkenylene groups, e.g., substituted with one or more alkyl (methyl) groups, include but are not limited to, substituted ethylene (-C(CH₃)=CH-, -CH=C(CH₃)-), substituted propylene (e.g., -C(CH₃)=CHCH₂-, -CH=C(CH₃)CH₂-, -CH=CHCH(CH₃)-, -CH=CHC(CH₃)₂-, -CH(CH₃)-CH=CH-, -C(CH₃)₂-CH=CH-, -CH₂-C(CH₃)=CH-, -CH₂-CH=C(CH₃)-), and the like.

"C₄₋₁₄ alkynylene" refers to a C₄₋₁₄ alkynyl group wherein another hydrogen is removed to provide a divalent radical of alkynylene, which may be substituted or unsubstituted. "C₂₋₁₃ alkynylene" refers to a C₂₋₁₃ alkynyl group wherein another hydrogen is removed to provide a divalent radical of alkynylene, which may be substituted or unsubstituted. In some embodiments, C₂₋₁₀ alkynylene, C₂₋₆ alkynylene, and C₂₋₄ alkynylene is yet alternative. Exemplary alkynylene groups include, but are not limited to, ethynylene (-C≡C-), substituted or unsubstituted propynylene (-C≡CCH₂-), and the like.

"C₃₋₈ cycloalkenyl" refers to a radical of a non-aromatic cyclic hydrocarbon group having from 3 to 8 ring carbon atoms and zero heteroatom and containing 1, 2 or 3 carbon-carbon double bonds.

"C₃₋₈ cycloalkenylene" refers to a C₃₋₈ cycloalkenyl group wherein another hydrogen is removed to form a divalent radical, which may be substituted or unsubstituted.

The term "variable A has a total length of x carbon atoms" means that the number of carbon atoms of the main chain in the group represented by variable A is x.

The term "variable A and variable B have a total length of x carbon atoms" means that the sum of the number of carbon atoms of the main chain in the group represented by variable A and the number of carbon atoms of the main chain in the group represented by variable B is x.

"Halo" or "halogen" refers to fluorine (F), chlorine (Cl), bromine (Br) and iodine (I).

Thus, "C₁₋₃₀ haloalkyl" refers to the above "C₁₋₃₀ alkyl" that is substituted by one or more halogens. In some embodiments, C₁₋₂₅ haloalkyl, C₁₋₂₀ haloalkyl, C₁₋₁₇ haloalkyl, C₁₋₁₀ haloalkyl, C₁₋₆ haloalkyl, C₁₋₃ haloalkyl, and C₁₋₄ haloalkyl are yet alternative, and still alternatively C₁₋₂ haloalkyl. Exemplary haloalkyl groups include, but are not limited to, -CF₃, -CH₂F, -CHF₂, -CHFCH₂F, -CH₂CHF₂, -CF₂CF₃, -CCl₃, -CH₂Cl, -CHCl₂, 2,2,2-trifluoro-1,1-dimethyl-ethyl, and the like. The haloalkyl can be substituted at any available point of attachment, for example, with 1 to 5 substituents, 1 to 3 substituents or 1 substituent.

"3- to 14-membered cycloalkyl" refers to a radical of a non-aromatic cyclic hydrocarbon group having from 3 to 14 ring carbon atoms and zero heteroatom, optionally wherein 1, 2 or 3 double or triple bonds are contained. In some embodiments, 3- to 10-membered cycloalkyl, 5- to 10-membered cycloalkyl, 3- to 8-membered cycloalkyl, 3- to 7-membered cycloalkyl, 3- to 6-membered cycloalkylare yet alternative, and still alternatively 5- to 7-membered cycloalkyl, 4- to 6-membered cycloalkyl, 5- to 6-membered cycloalkyl, 5-membered cycloalkyl, and 6-membered cycloalkyl. The cycloalkyl also includes a ring system in which the cycloalkyl ring described above is fused with one or more aryl or heteroaryl groups, wherein the point of attachment is on the cycloalkyl ring, and in such case, the number of carbon atoms continues to represent the number of carbon atoms in the cycloalkyl system. The cycloalkyl further comprises the cycloalkyl described above, in which the substituents on any non-adjacent carbon atoms are connected to form a bridged ring, together forming a polycyclic alkane sharing two or more carbon atoms. The cycloalkyl further comprises the cycloalkyl described above, in which the substituents on the same carbon atom are connected to form a ring, together forming a polycyclic alkane sharing one carbon atom. Exemplary cycloalkyl groups include, but are not limited to, cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), cycloheptyl (C₇), cycloheptenyl (C₇), cycloheptadienyl (C₇), cycloheptatrienyl (C₇), etc. The cycloalkyl can be substituted with one or more substituents, for example, with 1 to 5 substituents, 1 to 3 substituents or 1 substituent.

"3- to 8-membered cycloalkylene" refers to a divalent radical formed by removing another hydrogen of 3- to 8-membered cycloalkyl group, which may be substituted or unsubstituted. In some embodiments, C₃₋₆ cycloalkylene and C₃₋₄ cycloalkylene groups are particularly alternative, yet alternatively cyclopropylene.

"3- to 14-membered heterocyclyl" refers to a saturated or unsaturated radical of 3- to 14-membered non-aromatic ring system having ring carbon atoms and 1 to 5 ring heteroatoms, wherein each of the heteroatoms is independently selected from nitrogen, oxygen, sulfur, boron, phosphorus and silicon, optionally wherein 1, 2 or 3 double or triple bonds are contained. In the heterocyclyl containing one or more nitrogen atoms, the point of attachment can be a carbon or nitrogen atom as long as the valence permits. In some embodiments, 3- to 10-membered heterocyclyl is alternative, which is a radical of 3- to 10-membered non-aromatic ring system having ring carbon atoms and 1 to 5 ring heteroatoms; in some embodiments, 5- to 10-membered heterocyclyl is alternative, which is a radical of 5- to 10-membered non-aromatic ring system having ring carbon atoms and 1 to 5 ring heteroatoms; in some embodiments, 3- to 8-membered heterocyclyl is alternative, which is a radical of 3- to 8-membered non-aromatic ring system having ring carbon atoms and 1 to 4 ring heteroatoms; in some embodiments, 3- to 7-membered heterocyclyl is alternative, which is a radical of 3- to 7-membered non-aromatic ring system having ring carbon atoms and 1 to 4 ring heteroatoms; 5- to 7-membered heterocyclyl is alternative, which is a radical of 5- to 7-membered non-aromatic ring system having ring carbon atoms and 1 to 3 ring heteroatoms; 3- to 6-membered heterocyclyl is alternative, which is a radical of 3- to 6-membered non-aromatic ring system having ring carbon atoms and 1 to 3 ring heteroatoms; 4- to 6-membered heterocyclyl is alternative, which is a radical of 4- to 6-membered non-aromatic ring system having ring carbon atoms and 1 to 3 ring heteroatoms; 5- to 6-membered heterocyclyl is more alternative, which is a radical of 5- to 6-membered non-aromatic ring system having ring carbon atoms and 1 to 3 ring heteroatoms; 5-membered heterocyclyl is more alternative, which is a radical of 5-membered non-aromatic ring system having ring carbon atoms and 1 to 3 ring heteroatoms; 6-membered heterocyclyl is more alternative, which is a radical of 6-membered non-aromatic ring system having ring carbon atoms and 1 to 3 ring heteroatoms. The heterocyclyl also includes a ring system wherein the heterocyclyl described above is fused with one or more cycloalkyl groups, wherein the point of attachment is on the heterocyclyl ring, or the heterocyclyl described above is fused with one or more aryl or heteroaryl groups, wherein the point of attachment is on the heterocyclyl ring; and in such cases, the number of ring members continues to represent the number of ring members in the heterocyclyl ring system. The heterocyclyl further comprises the heterocyclyl described above, in which the substituents on any non-adjacent carbon or nitrogen atoms are connected to form a bridge ring, together forming a polycyclic heteroalkane sharing two or more carbon or nitrogen atoms. The heterocyclyl further comprises the heterocyclyl described above, in which the substituents on the same carbon atom are connected to form a ring, together forming a polycyclic heteroalkane sharing one carbon atom. Exemplary 3-membered heterocyclyl groups containing one heteroatom include, but are not limited to, aziridinyl, oxiranyl and thiorenyl. Exemplary 4-membered heterocyclyl groups containing one heteroatom include, but are not limited to, azetidinyl, oxetanyl and thietanyl. Exemplary 5-membered heterocyclyl groups containing one heteroatom include, but are not limited to, tetrahydrofuranyl, dihydrofuranyl, tetrahydrothiophenyl, dihydrothienyl, pyrrolidinyl, dihydropyrrolyl and pyrrolyl-2,5-dione. Exemplary 5-membered heterocyclyl groups containing two heteroatoms include, but are not limited to, pyrazolidyl, dioxolanyl, oxasulfuranyl, disulfuranyl, and oxazolidin-2-one. Exemplary 5-membered heterocyclyl groups containing three heteroatoms include, but are not limited to, triazolinyl, oxadiazolinyl, and thiadiazolinyl. Exemplary 6-membered heterocyclyl groups containing one heteroatom include, but are not limited to, piperidyl, tetrahydropyranyl, dihydropyridyl and thianyl. Exemplary 6-membered heterocyclyl groups containing two heteroatoms include, but are not limited to, piperazinyl, morpholinyl, dithianyl and dioxanyl. Exemplary 6-membered heterocyclyl groups containing three heteroatoms include, but are not limited to, triazinanyl. Exemplary 7-membered heterocyclyl groups containing one heteroatom include, but are not limited to, azepanyl, oxepanyl and thiepanyl. Exemplary 5-membered heterocyclyl groups fused with a C₆ aryl (also referred as 5,6-bicyclic heterocyclyl herein) include, but are not limited to, indolinyl, isoindolinyl, dihydrobenzofuranyl, dihydrobenzothiophenyl, benzoxazolinonyl, etc. Exemplary 6-membered heterocyclyl groups fused with a C₆ aryl (also referred as 6,6-bicyclic heterocyclyl herein) include, but are not limited to, tetrahydroquinolinyl, tetrahydroisoquinolinyl, etc. The heterocyclyl further includes the heterocyclyl described above sharing one or two atoms with a cycloalkyl, heterocyclyl, aryl or heteroaryl to form a bridged or spiro ring, as long as the valence permits, where the shared atom may be carbon or nitrogen atoms. The heterocyclyl further includes the heterocyclyl described above, which optionally can be substituted with one or more substituents, e.g., with 1 to 5 substituents, 1 to 3 substituents or 1 substituent.

"C₆₋₁₀ aryl" refers to a radical of monocyclic or polycyclic (e.g., bicyclic) 4n+2 aromatic ring system having 6-10 ring carbon atoms and zero heteroatom (e.g., having 6 or 10 shared π electrons in a cyclic array). In some embodiments, the aryl group has six ring carbon atoms ("C₆ aryl"; for example, phenyl). In some embodiments, the aryl group has ten ring carbon atoms ("C₁₀ aryl"; for example, naphthyl, e.g., 1-naphthyl and 2-naphthyl). The aryl group also includes a ring system in which the aryl ring described above is fused with one or more cycloalkyl or heterocyclyl groups, and the point of attachment is on the aryl ring, in which case the number of carbon atoms continues to represent the number of carbon atoms in the aryl ring system. The aryl can be substituted with one or more substituents, for example, with 1 to 5 substituents, 1 to 3 substituents or 1 substituent.

"5- to 14-membered heteroaryl" refers to a radical of 5- to 14-membered monocyclic or bicyclic 4n+2 aromatic ring system (e.g., having 6, 10 or 14 shared π electrons in a cyclic array) having ring carbon atoms and 1-4 ring heteroatoms, wherein each heteroatom is independently selected from nitrogen, oxygen and sulfur. In the heteroaryl group containing one or more nitrogen atoms, the point of attachment can be a carbon or nitrogen atom as long as the valence permits. heteroaryl bicyclic systems may include one or more heteroatoms in one or two rings, heteroaryl also includes ring systems wherein the heteroaryl ring described above is fused with one or more cycloalkyl or heterocyclyl groups, and the point of attachment is on the heteroaryl ring. In such case, the number the carbon atoms continues to represent the number of carbon atoms in the heteroaryl ring system. In some embodiments, 5- to 10-membered heteroaryl groups are alternative, which are radicals of 5- to 10-membered monocyclic or bicyclic 4n+2 aromatic ring systems having ring carbon atoms and 1-4 ring heteroatoms. In other embodiments, 5- to 6-membered heteroaryl groups are yet alternative, which are radicals of 5- to 6-membered monocyclic or bicyclic 4n+2 aromatic ring systems having ring carbon atoms and 1-4 ring heteroatoms. Exemplary 5-membered heteroaryl groups containing one heteroatom include, but are not limited to, pyrrolyl, furyl and thienyl. Exemplary 5-membered heteroaryl groups containing two heteroatoms include, but are not limited to, imidazolyl, pyrazolyl, oxazolyl, isoxazolyl, thiazolyl, and isothiazolyl. Exemplary 5-membered heteroaryl groups containing three heteroatoms include, but are not limited to, triazolyl, oxadiazolyl (such as, 1,2,4-oxadiazolyl), and thiadiazolyl. Exemplary 5-membered heteroaryl groups containing four heteroatoms include, but are not limited to, tetrazolyl. Exemplary 6-membered heteroaryl groups containing one heteroatom include, but are not limited to, pyridyl or pyridonyl. Exemplary 6-membered heteroaryl groups containing two heteroatoms include, but are not limited to, pyridazinyl, pyrimidinyl, and pyrazinyl. Exemplary 6-membered heteroaryl groups containing three or four heteroatoms include, but are not limited to, triazinyl and tetrazinyl, respectively. Exemplary 7-membered heteroaryl groups containing one heteroatom include, but are not limited to, azepinyl, oxepinyl, and thiepinyl. Exemplary 5,6-bicyclic heteroaryl groups include, but are not limited to, indolyl, isoindolyl, indazolyl, benzotriazolyl, benzothiophenyl, isobenzothiophenyl, benzofuranyl, benzoisofuranyl, benzimidazolyl, benzoxazolyl, benzoisoxazolyl, benzoxadiazolyl, benzothiazolyl, benzoisothiazolyl, benzothiadiazolyl, indolizinyl and purinyl. Exemplary 6,6-bicyclic heteroaryl groups include, but are not limited to, naphthyridinyl, pteridinyl, quinolyl, isoquinolyl, cinnolinyl, quinoxalinyl, phthalazinyl and quinazolinyl. The heteroaryl can be substituted with one or more substituents, for example, with 1 to 5 substituents, 1 to 3 substituents or 1 substituent.

"Hydroxyalkyl" refers to an alkyl group that is substituted with one or more hydroxyl groups.

"Alkoxy" refers to an oxyether form of a linear or branched-chain alkyl group, i.e., an -O-alkyl group. Similarly, "methoxy" refers to -O-CH₃.

"Optionally substituted with" or "optionally substituted by" means that it can be substituted with the specified substituents or unsubstituted.

The divalent groups formed by removing another hydrogen from the groups defined above such as alkyl, alkenyl, alkynyl, cycloalkyl, heterocyclyl, aryl and heteroaryl are collectively referred to as "-ylene". Ring-forming groups such as cycloalkyl, heterocyclyl, aryl and heteroaryl are collectively referred to as "cyclic groups".

The alkyl, alkenyl, alkynyl, cycloalkyl, heterocyclyl, aryl, heteroaryl groups, and the like as defined herein are optionally substituted groups.

Exemplary substituents on carbon atoms include, but are not limited to, halogen, -CN, -NO₂, -N₃, -SO₂H, -SO₃H, -OH, -OR^{aa}, -ON(R^{bb})₂, -N(R^{bb})₂, -N(R^{bb})₃⁺X⁻, -N(OR^{cc})R^{bb}, -SH, -SR^{aa}, -SSR^{cc}, -C(=O)R^{aa}, -CO₂H, -CHO, -C(OR^{cc})₂, -CO₂R^{aa}, -OC(=O)R^{aa}, -OCO₂R^{aa}, -C(=O)N(R^{bb})₂, -OC(=O)N(R^{bb})₂, -NR^{bb}C(=O)R^{aa}, -NR ^{bb}CO₂R^{aa}, -NR^{bb}C(=O)N(R^{bb})₂, -C(=NR^{bb})R^{aa}, -C(=NR^{bb})OR^{aa}, -OC(=NR^{bb})R^{aa}, -OC(=NR^{bb})OR^{aa}, -C(=NR^{bb})N(R^{bb})₂, -OC(=NR^{bb})N(R^{bb})₂, -NR^{bb}C(=NR^{bb})N(R^{bb})₂, -C(=O)NR^{bb}SO₂R^{aa}, -NR^{bb}SO₂R^{aa}, -SO₂N(R^{bb})₂, -SO₂R^{aa}, -SO₂OR^{aa}, -OSO₂R^{aa}, -S(=O)R^{aa}, -OS(=O)R^{aa}, -Si(R^{aa})₃, -OSi(R^{aa})₃, -C(=S)N(R^{bb})₂, -C(=O)SR^{aa}, -C(=S)SR^{aa}, -SC(=S)SR^{aa}, -SC(=O)SR^{aa}, -OC(=O)SR^{aa}, -SC(=O)OR^{aa}, -SC(=O)R^{aa}, -P(=O)₂R^{aa}, -OP(=O)₂R^{aa}, -P(=O)(R^{aa})₂, -OP(=O)(R^{aa})₂, -OP(=O)(OR^{cc})₂, -P(=O)₂N(R^{bb})₂, -OP(=O)₂N(R^{bb})₂, -P(=O)(NR^{bb})₂, -OP(=O)(NR^{bb})₂, -NR^{bb}P(=O)(OR^{cc})₂, -NR^{bb}P(=O)(NR^{bb})₂, -P(R^{cc})₂, -P(R^{cc})₃, -OP(R^{cc})₂, -OP(R^{cc})₃, -B(R^{aa})₂, -B(OR^{cc})₂, -BR^{aa}(OR^{cc}), alkyl, haloalkyl, alkenyl, alkynyl, cycloalkyl, heterocyclyl, aryl and heteroaryl, wherein each of the alkyl, alkenyl, alkynyl, cycloalkyl, heterocyclyl, aryl and heteroaryl is independently substituted with 0, 1, 2, 3, 4 or 5 R^{dd} groups;
or two geminal hydrogens on a carbon atom are replaced with =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb} or =NOR^{cc} groups;
each of the R^{aa} is independently selected from alkyl, haloalkyl, alkenyl, alkynyl, cycloalkyl, heterocyclyl, aryl and heteroaryl, or two of the R^{aa} groups are combined to form a heterocyclyl or heteroaryl ring, wherein each of the alkyl, alkenyl, alkynyl, cycloalkyl, heterocyclyl, aryl and heteroaryl is independently substituted with 0, 1, 2, 3, 4 or 5 R^{dd} groups;
each of the R^{bb} is independently selected from hydrogen, -OH, -OR^{aa}, -N(R^{cc})₂, -CN, -C(=O)R^{aa}, -C(=O)N(R^{cc})₂, -CO₂R^{aa}, -SO₂R^{aa}, -C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, alkyl, haloalkyl, alkenyl, alkynyl, cycloalkyl, heterocyclyl, aryl and heteroaryl, or two R^{bb} groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 R^{dd} groups;
each of the R^{cc} is independently selected from hydrogen, alkyl, haloalkyl, alkenyl, alkynyl, cycloalkyl, heterocyclyl, aryl and heteroaryl, or two R^{cc} groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 R^{dd} groups;
each of the R^{dd} is independently selected from halogen, -CN, -NO₂, -N₃, -SO₂H, -SO₃H, -OH, -OR^{ee}, -ON(R^{ff})₂, -N(R^{ff})₂, -N(R^{ff})₃⁺X⁻, -N(OR^{ee})R^{ff}, -SH, -SR^{ee}, -SSR^{ee}, -C(=O)R^{ee}, -CO₂H, -CO₂R^{ee}, -OC(=O)R^{ee}, -OCO₂R^{ee}, -C(=O)N(R^{ff})₂, -OC(=O)N(R^{ff})₂, -NR^{ff}C(=O)R^{ee}, -NR^{ff}CO₂R^{ee}, -NR^{ff}C(=O)N(R^{ff})₂, -C(=NR^{ff})OR^{ee}, -OC(=NR^{ff})R^{ee}, -OC(=NR^{ff})OR^{ee}, -C(=NR^{ff})N(R^{ff})₂, -OC(=NR^{ff})N(R^{ff})₂, -NR^{ff}C(=NR^{ff})N(R^{ff})₂, -NR^{ff}SO₂R^{ee}, -SO₂N(R^{ff})₂, -SO₂R^{ee}, -SO₂OR^{ee}, -OSO₂R^{ee}, -S(=O)R^{ee}, -Si(R^{ee})₃, -OSi(R^{ee})₃, -C(=S)N(R^{ff})₂, -C(=O)SR^{ee}, -C(=S)SR^{ee}, -SC(=S)SR^{ee}, -P(=O)₂R^{ee}, -P(=O)(R^{ee})₂, -OP(=O)(R^{ee})₂, -OP(=O)(OR^{ee})₂, alkyl, haloalkyl, alkenyl, alkynyl, cycloalkyl, heterocyclyl, aryl, heteroaryl, wherein each of the alkyl, alkenyl, alkynyl, cycloalkyl, heterocyclyl, aryl and heteroaryl is independently substituted with 0, 1, 2, 3, 4 or 5 R^{gg} groups, or two geminal R^{dd} substituents can be combined to form=O or =S;
each of the R^{ee} is independently selected from alkyl, haloalkyl, alkenyl, alkynyl, cycloalkyl, aryl, heterocyclyl, and heteroaryl, wherein each of the alkyl, alkenyl, alkynyl, cycloalkyl, heterocyclyl, aryl and heteroaryl is independently substituted with 0, 1, 2, 3, 4 or 5 R^{gg} groups;
each of the R^{ff} is independently selected from hydrogen, alkyl, haloalkyl, alkenyl, alkynyl, cycloalkyl, heterocyclyl, aryl and heteroaryl, or two R^{ff} groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 R^{gg} groups;
each of the R^{gg} is independently selected from halogen, -CN, -NO₂, -N₃, -SO₂H, -SO₃H, -OH, -OC₁₋₆ alkyl, -ON(C₁₋₆ alkyl)₂, -N(C₁₋₆ alkyl)₂, -N(C₁₋₆ alkyl)₃⁺X⁻, -NH(C₁₋₆ alkyl)₂⁺X⁻, -NH₂(C₁₋₆ alkyl)⁺X⁻, -NH₃⁺X⁻, -N(OC₁₋₆ alkyl)(C₁₋₆ alkyl), -N(OH)(C₁₋₆ alkyl), -NH(OH), -SH, -SC₁₋₆ alkyl, -SS(C₁₋₆ alkyl), -C(=O)(C₁₋₆ alkyl), -CO₂H, -CO₂(C₁₋₆ alkyl), -OC(=O)(C₁₋₆ alkyl), -OCO₂(C₁₋₆ alkyl), -C(=O)NH₂, -C(=O)N(C₁₋₆ alkyl)₂, -OC(=O)NH(C₁₋₆ alkyl), -NHC(=O)(C₁₋₆ alkyl), -N(C₁₋₆ alkyl)C(=O)(C₁₋₆ alkyl), -NHCO₂(C₁₋₆ alkyl), -NHC(=O)N(C₁₋₆ alkyl)₂, -NHC(=O)NH(C₁₋₆ alkyl), -NHC(=O)NH₂, -C(=NH)O(C₁₋₆ alkyl), -OC(=NH)(C₁₋₆ alkyl), -OC(=NH)OC₁₋₆ alkyl, -C(=NH)N(C₁₋₆ alkyl)₂, -C(=NH)NH(C₁₋₆ alkyl), -C(=NH)NH₂, -OC(=NH)N(C₁₋₆ alkyl)₂, -OC(NH)NH(C₁₋₆ alkyl), -OC(NH)NH₂, -NHC(NH)N(C₁₋₆ alkyl)₂, -NHC(=NH)NH₂, -NHSO₂(C₁₋₆ alkyl), -SO₂N(C₁₋₆ alkyl)₂, -SO₂NH(C₁₋₆ alkyl), -SO₂NH₂, -SO₂C₁₋₆ alkyl, -SO₂OC₁₋₆ alkyl, -OSO₂C₁₋₆ alkyl, -SOC₁₋₆ alkyl, -Si(C₁₋₆ alkyl)₃, -OSi(C₁₋₆ alkyl)₃, -C(=S)N(C₁₋₆ alkyl)₂, C(=S)NH(C₁₋₆ alkyl), C(=S)NH₂, -C(=O)S(C₁₋₆ alkyl), -C(=S)SC₁₋₆ alkyl, -SC(=S)SC₁₋₆ alkyl, -P(=O)₂(C₁₋₆ alkyl), -P(=O)(C₁₋₆ alkyl)₂, -OP(=O)(C₁₋₆ alkyl)₂, -OP(=O)(OC₁₋₆ alkyl)₂, C₁₋₆ alkyl, C₁₋₆ haloalkyl, C₂-C₆ alkenyl, C₂-C₆ alkynyl, C₃-C₇ cycloalkyl, C₆-C₁₀ aryl, C₃-C₇ heterocyclyl, C₅-C₁₀ heteroaryl; or two geminal R^{gg} substituents may combine to form =O or =S; wherein X⁻ is a counter-ion.

Exemplary substituents on nitrogen atoms include, but are not limited to, hydrogen, -OH, -OR^{aa}, -N(R^{cc})₂, -CN, -C(=O)R^{aa}, -C(=O)N(R^{cc})₂, -CO₂R^{aa}, -SO₂R^{aa}, -C(=NR^{bb})R^{aa}, -C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, alkyl, haloalkyl, alkenyl, alkynyl, cycloalkyl, heterocyclyl, aryl and heteroaryl, or two R^{cc} groups attached to a nitrogen atom combine to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 R^{dd} groups, and wherein R^{aa}, R^{bb}, R^{cc} and R^{dd} are as described herein.

"Nucleic acids" refers to single- or double-stranded deoxyribonucleic acid (DNA) or ribonucleic acid (RNA) molecules and their heterozygous molecules. Examples of nucleic acid molecules include, but are not limited to, messenger RNA (mRNA), microRNA (miRNA), small interfering RNA (siRNA), self-amplified RNA (saRNA), and antisense oligonucleotides (ASO), etc. Nucleic acids may be further chemically modified, and the chemical modifier selected from one of, or a combination of: pseudouridine, Nl-methyl-pseudouridine, 5-methoxyuridine, and 5-methylcytosine. mRNA molecules contain protein coding regions and may further contain expression regulatory sequences. Typical expression regulatory sequences include, but are not limited to, 5' cap, 5' untranslated region (5' UTR), 3' untranslated region (3' UTR), polyadenylate sequence (PolyA), miRNA binding sites.

mRNA includes both modified RNA and unmodified RNA. The term "modified mRNA" relates to mRNA comprising at least one chemically modified nucleotide. mRNA may contain one or more coding and non-coding regions. mRNA may be purified from natural sources, generated using recombinant expression systems, and optionally purified, chemically synthesized, etc. mRNA may comprise nucleoside analogs, such as analogs having chemically modified bases or saccharides, or backbone modifications, etc, where appropriate, for example in the case of chemically synthesized molecules. In some embodiments, the mRNA is or comprises natural nucleosides (e.g., adenosine, guanosine, cytidine, uridine); nucleoside analogs (e.g., pseudouridine, N1-methyl pseudouridine, N1-ethyl pseudouridine, 2-thiouridine, 4'-thiouridine, 5-methylcytidine, 5-methyluridine, 2-thio-1-methyl-1-deaza-pseudouridine, 2-thio-T-methyl-pseudouridine, 2-thio-5-aza-uridine, 2-thio-dihydropseudouridine, 2-thio-dihydrouridine, 2-thio-pseudouridine, 4-methoxy-2-thio-pseudouridine, 4-methoxy-pseudouridine, 4-thio-1 -methyl-pseudouridine, 4-thio-pseudouridine, 5-aza-uridine, dihydropseudouridine, 5-methoxyuridine, and 2'-O-methyluridine); chemically modified bases; biologically modified bases (e.g., methylated bases); inserted bases; modified saccharides (e.g., 2'-fluororibose, ribose, 2'-deoxyribose, arabinose, and hexose); and/or modified phosphate groups (e.g., phosphorothioate and 5'-N-phosphoramidite bonds).

"Lipid" refers to a group of organic compounds including, but not limited to, esters of fatty acids, and is characterized by being insoluble in water but soluble in many organic solvents. They are typically divided into at least three categories: (1) "simple lipids", which include fats, oils and waxes; (2) "complex lipids", which include phospholipids and glycolipids; and (3) "derived lipids" such as steroids.

"LNP" denotes particles made of lipids (e.g., cationic lipids, ionizable lipids, and conjugated lipids that prevent aggregation of particles) and nucleic acids, wherein the nucleic acids are encapsulated in the lipids. When present in the lipid particles of the present disclosure, the nucleic acid is resistant to degradation by nucleases in aqueous solution.

The lipid particles of the present disclosure typically have an average diameter of about 40 nm to about 250 nm, about 50 nm to about 250 nm, about 60 nm to about 230 nm, about 70 nm to about 230 nm, about 70 nm to about 200 nm, about 70 nm to about 180 nm, or about 70 nm to about 165 nm, and are substantially non-toxic.

"Long-Acting SusTained delivering lipid (LASTing lipid)" refers to a lipid that can reduce the off-target effect of topically injected mRNA-LNP drugs, increase the peak concentration and bioavailability of drugs in injection site tissues, and prolong the time to peak and half-life of drugs in injection site tissues after injection.

"Cationic lipid" refers to a lipid that has a net positive charge at approximately physiological pH. A cationic lipid may be an amino lipid with a positively charged head group (hydrophilic group) attached to one or more hydrophobic groups, wherein the head group is typically a long chain amine group having one or more amino groups.

"Permanently cationic lipid" refers to a cationic lipid that is continuously maintained in a net positively charged form in the *in vivo* environment involved in the entire process of the delivery of a drug by an LNP, such as a cationic lipid with no pKa or pKa > 8, alternatively such as a cationic lipid with pKa > 10 or a cationic lipid containing a quaternary ammonium structure.

An ionizable cationic lipid can be present in a positively charged form or in a neutral form depending on the pH value. The ionization of an ionizable cationic lipid affects the surface charge of lipid nanoparticles at different pH conditions.

"Neutral phospholipid" refers to a phospholipid lipid molecule that is not charged at a particular pH, such as physiological pH conditions. Examples of neutral phospholipids include, but are not limited to 1,2-distearoyl-sn-glycero-3-phosphocholine (DSPC), 1,2-dimyristoyl-sn-glycero-3-phosphocholine (DMPC), 1,2-dioleoyl-sn-glycero-3-phosphocholine (DOPC), 1,2-dipalmitoyl-sn-glycero-3-phosphocholine (DPPC), 1-palmitoyl-2-oleoyl-sn-glycero-3-phosphocholine (POPC), 1,2-dioleoyl-sn-glycero-3-phosphoethanolamine (DOPE), 1,2-dimyristoyl-sn-glycero-3-phosphoethanolamine (DMPE), 1-palmitoyl-2-oleoyl-sn-glycero-3-phosphoethanolamine (POPE), and 1,2-dipalmitoyl-sn-glycero-3-phosphoethanolamine (DPPE).

"Structured lipid" refers to a lipid that enhances the stability of nanoparticles by filling the gaps between lipids, commonly such as steroids. The steroid is a compound having a perhydrocyclopentanophenanthrene carbon framework. In an alternative embodiment, the steroid is cholesterol, sitosterol, coprosterol, fucosterol, brassicasterol, ergosterol, tomatine, ursolic acid, α-tocopherol, stigmasterol, avenasterol, ergocalciferol or campesterol.

"Polymer-conjugated lipid" refers to a molecule containing a polymer moiety and a lipid moiety. In some embodiments, the polymer lipid is a polyethylene glycol (PEG) lipid. A PEG lipid refers to any complex of polyethylene glycol (PEG) with a lipid. A PEG lipid is not particularly limited as long as it has the effect of inhibiting the aggregation of the lipid nanoparticles of the present disclosure. Other lipids that can reduce aggregation, such as products of compounds having uncharged, hydrophilic, space-barrier moieties coupled with a lipid may also be used.

"Lipid nanoparticle" refers to a particle containing a lipid component and having nanoscale dimensions.

"Biodegradable group" refers to a functional group that contains a biodegradable bond, such as esters, disulfide bonds and amides, etc. Biodegradation can affect the process of removing compounds from the body. The biodegradable groups of the present disclosure are oriented from the head to the tail in ionizable lipid molecules. Exemplary biodegradable groups include, but are not limited to: -C(O)O-, -O-, -SC(O)O-, -OC(O)NR-, -NRC(O)NR-, -OC(O)S-, -OC(O)O-, -NRC(O)O-, -OC(O)-, -SC(O)-, -C(O)S-, -NR-, -C(O)NR-, -NRC(O)-, -NRC(O)S-, -SC(O)NR-, -C(O)-, -OC(S)-, -C(S)O-, -OC(S)NR-, -NRC(S)O-, -S-S-, or -S(O)₀₋₂-, wherein R is H or C₁₋₂₀ alkyl.

"Hydrophobic chain" refers to a chain hydrocarbon consisting of carbon and hydrogen, which may be saturated or unsaturated, such as chain alkanes, chain alkenes, chain alkynes, etc. Some common such groups include octyl, nonyl, decyl, lauryl, myristyl, palmityl, stearyl, alpha-linoleyl, stearate, linoleyl, gamma-linolenyl, arachidonyl and oleyl.

"Steroid group" refers to a compound and a derivative thereof having a cyclopentane-fused polyhydrophenanthrene-like carbon skeleton that can be attached to a functional group (e.g., the above biodegradable group), such as cholesterol and derivatives thereof, phytosterols such as sitosterol and analogues thereof, for example:

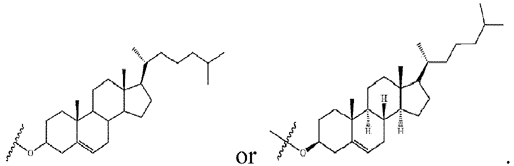

### OTHER TERMINOLOGY

The term "treating" as used herein relates to reversing, alleviating or inhibiting the progression or prevention of the disorders or conditions to which the term applies, or of one or more symptoms of such disorders or conditions. The noun "treatment" as used herein relates to the action of treating, which is a verb, and the latter is as just defined.

The term "pharmaceutically acceptable salt" as used herein refers to those carboxylate and amino acid addition salts of the compounds of the present disclosure, which are suitable for the contact with patients' tissues within a reliable medical judgment, and do not produce inappropriate toxicity, irritation, allergy, etc. They are commensurate with a reasonable benefit/risk ratio, and are effective for their intended use. The term includes, if possible, the zwitterionic form of the compounds of the disclosure.

The pharmaceutically acceptable base addition salts are formed with metals or amines, such as alkali metal and alkaline earth metal hydroxides or organic amines. Examples of the metals used as cations include sodium, potassium, magnesium, calcium, etc. Examples of suitable amines are N,N'-dibenzylethylenediamine, chloroprocaine, choline, diethanolamine, ethylenediamine, N-methylglucamine and procaine.

The base addition salt of the acidic compound can be prepared by contacting the free acid form with a sufficient amount of the required base to form a salt in a conventional manner. The free acid can be regenerated by contacting the salt form with an acid in a conventional manner and then isolating the free acid. The free acid forms are somewhat different from their respective salt forms in their physical properties, such as solubility in polar solvents. But for the purposes of the present disclosure, the salts are still equivalent to their respective free acids.

The salts can be prepared from the inorganic acids, which include sulfates, pyrosulfates, bisulfates, sulfites, bisulfites, nitrates, phosphates, monohydrogen phosphates, dihydrogen phosphates, metaphosphates, pyrophosphates, chlorides, bromides and iodides. Examples of the acids include hydrochloric acid, nitric acid, sulfuric acid, hydrobromic acid, hydroiodic acid, phosphoric acid, etc. The representative salts include hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthalate, methanesulfonate, glucoheptanate, lactobionate, lauryl sulfonate, isethionate, etc. The salts can also be prepared from the organic acids, which include aliphatic monocarboxylic and dicarboxylic acids, phenyl-substituted alkanoic acids, hydroxyalkanoic acids, alkanedioic acid, aromatic acids, aliphatic and aromatic sulfonic acids, etc. The representative salts include acetate, propionate, octanoate, isobutyrate, oxalate, malonate, succinate, suberate, sebacate, fumarate, maleate, mandelate, benzoate, chlorobenzoate, methyl benzoate, dinitrobenzoate, naphthoate, besylate, tosylate, phenylacetate, citrate, lactate, maleate, tartrate, methanesulfonate, etc. The pharmaceutically acceptable salts can include cations based on alkali metals and alkaline earth metals, such as sodium, lithium, potassium, calcium, magnesium, etc., as well as non-toxic ammonium, quaternary ammonium, and amine cations including, but not limited to, ammonium, tetramethylammonium, tetraethylammonium, methylamine, dimethylamine, trimethylamine, triethylamine, ethylamine, etc. Salts of amino acids are also included, such as arginine salts, gluconates, galacturonates, etc. (for example, see Berge S. M. et al., "Pharmaceutical Salts," J. Pharm. Sci., 1977; 66: 1- 19 for reference).

"Subjects" to which administration is contemplated include, but are not limited to, humans (e.g., males or females of any age group, e.g., paediatric subjects (e.g., infants, children, adolescents) or adult subjects (e.g., young adults, middle-aged adults or older adults) and/or non-human animals, such as mammals, e.g., primates (e.g., cynomolgus monkeys, rhesus monkeys), cattle, pigs, horses, sheep, goats, rodents, cats and/or dogs. In some embodiments, the subject is a human. In some embodiments, the subject is a non-human animal. The terms "human", "patient" and "subject" can be used interchangeably herein.

"Disease", "disorder", and "condition" can be used interchangeably herein.

Unless otherwise indicated, the term "treatment" as used herein includes the effect on a subject who is suffering from a particular disease, disorder, or condition, which reduces the severity of the disease, disorder, or condition, or delays or slows the progression of the disease, disorder or condition ("therapeutic treatment"). The term also includes the effect that occurs before the subject begins to suffer from a specific disease, disorder or condition ("prophylactic treatment").

Generally, the "effective amount" of a pharmaceutical composition refers to an amount sufficient to elicit a target biological response. As understood by those skilled in the art, the effective amount of the pharmaceutical composition of the disclosure can vary depending on the following factors, such as the desired biological endpoint, the pharmacokinetics of the pharmaceutical composition, the diseases being treated, the mode of administration, and the age, health status and symptoms of the subjects. The effective amount includes therapeutically effective amount and prophylactically effective amount.

Unless otherwise indicated, the "therapeutically effective amount" of the pharmaceutical composition as used herein is an amount sufficient to provide therapeutic benefits in the course of treating a disease, disorder or condition, or to delay or minimize one or more symptoms associated with the disease, disorder or condition. The therapeutically effective amount of a pharmaceutical composition refers to the amount of the therapeutic agent that, when used alone or in combination with other therapies, provides a therapeutic benefit in the treatment of a disease, disorder or condition. The term "therapeutically effective amount" can include an amount that improves the overall treatment, reduces or avoids the symptoms or causes of the disease or condition, or enhances the therapeutic effect of other therapeutic agents.

Unless otherwise indicated, the "prophylactically effective amount" of the pharmaceutical composition as used herein is an amount sufficient to prevent a disease, disorder or condition, or an amount sufficient to prevent one or more symptoms associated with a disease, disorder or condition, or an amount sufficient to prevent the recurrence of a disease, disorder or condition. The prophylactically effective amount of a pharmaceutical composition refers to the amount of a therapeutic agent that, when used alone or in combination with other agents, provides a prophylactic benefit in the prevention of a disease, disorder or condition. The term "prophylactically effective amount" can include an amount that improves the overall prevention, or an amount that enhances the prophylactic effect of other preventive agents.

"Topical injection" refers to local application to any area of the body using a needle. In the method or use of the present disclosure, the drug is administered into the muscle or tumor, alternatively by injection.

"Combination" and related terms refer to simultaneous or sequential administration of the pharmaceutical composition of the present disclosure and other therapeutic agent. For example, the pharmaceutical composition of the present disclosure can be administered simultaneously or sequentially with other therapeutic agent (s) in separate unit dosages, or simultaneously with other therapeutic agent (s) in a single unit dosage.

Beneficial effects of the present disclosure:
(1) The present disclosure uses a combination of Long-Acting SusTained delivering lipid (LASTing) and ionizable lipid, which can significantly improve the hepatic off-target effect of drugs. Experiments have shown that the combination can significantly reduce the amount of drug off-target to the liver, and has a good local (e.g., muscle) targeting effect, wherein the expression level of drugs at the injection site is significantly higher than that in the liver. This combination is expected to be applied to targeted local (e.g., muscle) injection formulations.
(2) The formulation of the present disclosure also has the effect of prolonging the expression time of drugs at an injection site. The formulation is highly expressed within 72h and still expressed at 120h, which can reduce the dosage of drugs and achieve better therapeutic effect.

### BRIEF DESCRIPTION OF THE DRAWINGS

FIG. 1 shows the results of fluorescence intensity at the liver and muscle sites of mice at different time points after injection of formulation 1.
FIG. 2 shows the results of fluorescence intensity at the muscle site at different time points after addition of different proportions of DOTAP in formulation 3.
FIG. 3 shows the results of comparing the fluorescence intensity respectively at the liver and muscle sites at different time points between formulation 5-1 and formulation 5-2.
FIG. 4 shows the results of comparing the fluorescence intensity respectively at the liver and muscle sites at different time points between formulation 6-1 and formulation 6-2.
FIG. 5 shows the results of comparing the fluorescence intensity respectively at the liver and muscle sites at different time points between formulation 7-1 and formulation 7-2.
FIG. 6 shows the results of comparing the fluorescence intensity respectively at the liver and muscle sites at different time points between formulation 8-1 and formulation 8-2.
FIG. 7 shows the results of comparing the fluorescence intensity respectively at the liver and muscle sites at different time points between formulation 8-1 and formulation 8-3.
FIG. 8 shows the results of comparing the fluorescence intensity respectively at the liver and muscle sites at different time points between formulation 8-1 and formulation 8-4.
FIG. 9 shows the results of comparing the fluorescence intensity respectively at the liver and muscle sites at different time points between formulation 8-1 and formulation 8-5.
FIG. 10 shows the results of comparing the fluorescence intensity respectively at the liver and muscle sites at different time points between formulation 9-1 and formulation 9-2.
FIG. 11 shows the results of comparing the fluorescence intensity respectively at the liver and muscle sites at different time points between formulation 10-1 and formulation 10-2.

### DETAILED DESCRIPTION OF THE INVENTION

As used herein, "compound of the present disclosure" refers to the following compound of formula (I), formula (II), formula (III), (IV'), formula (V'), formula (VI'), formula (VII'), and the like, or a pharmaceutically acceptable salt, isotopic variant, tautomer or stereoisomer thereof.

In the present disclosure, compounds are named using standard nomenclature. For compounds having an asymmetric center, it should be understood, unless otherwise stated, that all optical isomers and mixtures thereof are included. Furthermore, unless otherwise specified, all isomer compounds and carbon-carbon double bonds included in the present disclosure may occur in the form of Z and E. Compounds which exist in different tautomeric forms, one of which is not limited to any particular tautomer, but is intended to cover all tautomeric forms.

The present disclosure provides a lipid nanoparticle for topical injection comprising a Long-Acting SusTained delivering lipid and an ionizable lipid, wherein the lipid nanoparticle is capable of acting at the injection site.

In a more specific embodiment, the site of the topical injection is muscle or tumor tissue, alternatively muscle.

In a more specific embodiment, the lipid nanoparticles have an extended duration of action compared to lipid nanoparticles without Long-Acting SusTained delivering lipid.

In a more specific embodiment, the lipid nanoparticle is capable of reducing off-target effects in tissues or organs at non-injection sites.

In a more specific embodiment, the tissue or organ at a non-injection site is liver.

In a more specific embodiment, the present disclosure provides a lipid nanoparticle for topical injection comprising the following components: Long-Acting SusTained delivering lipid, structured lipid, polymer-conjugated lipid and ionizable lipid, and optionally comprising neutral phospholipid.

In a more specific embodiment, the present disclosure provides the above lipid nanoparticles, wherein the Long-Acting SusTained delivering lipid is a permanently cationic lipid, alternatively a permanently cationic lipid with pKa > 10, or a permanently cationic lipid containing a quaternary ammonium structure.

In a more specific embodiment, the present disclosure provides the above lipid nanoparticles, wherein the permanently cationic lipid is selected from a pharmaceutically acceptable salt of the compound of formula (I): 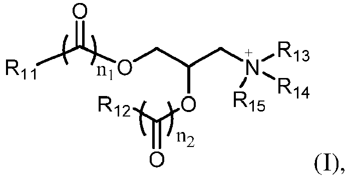 wherein
R₁₁ and R₁₂ are independently selected from C₆₋₃₀ alkyl, C₆₋₃₀ alkenyl and C₆₋₃₀ alkynyl, alternatively selected from C₁₀₋₂₅ alkyl, C₁₀₋₂₅ alkenyl and C₁₀₋₂₅ alkynyl, alternatively selected from C₁₃₋₂₀ alkyl, C₁₃₋₂₀ alkenyl and C₁₃₋₂₀ alkynyl, alternatively selected from C₁₃₋₁₈ alkyl, C₁₃₋₁₈ alkenyl and C₁₃₋₁₈ alkynyl, alternatively selected from C₁₅₋₁₈ alkyl, C₁₅₋₁₈ alkenyl and C₁₅₋₁₈ alkynyl, such as C₁₇₋₁₈ alkyl, C₁₇₋₁₈ alkenyl and C₁₇₋₁₈ alkynyl; alternatively C₁₃₋₂₀ alkyl and C₁₃₋₂₀ alkenyl, alternatively C₁₃₋₁₈ alkyl and C₁₃₋₁₈ alkenyl, alternatively C₁₅₋₁₈ alkyl and C₁₅₋₁₈ alkenyl, such as C₁₇₋₁₈ alkyl and C₁₇₋₁₈ alkenyl; alternatively C₁₃₋₂₀ alkenyl, alternatively C₁₃₋₁₈ alkenyl, alternatively C₁₅₋₁₈ alkenyl, such as C₁₇₋₁₈ alkenyl; R₁₁ and R₁₂ are optionally substituted with 1, 2, 3, 4 or 5 substituents selected from: -OH, halogen, cyano, C₁₋₃₀ alkyl, C₁₋₃₀ haloalkyl, -O-C₁₋₃₀ alkyl, -S-C₁₋₃₀ alkyl, amino, -NH-C₁₋₃₀ alkyl and -N(C₁₋₃₀ alkyl)₂, alternatively optionally substituted with 1, 2, 3, 4 or 5 substituents selected from: -OH, halogen, cyano, C₁₋₂₅ alkyl, C₁₋₂₅ haloalkyl, -O-C₁₋₂₅ alkyl, -S-C₁₋₂₅ alkyl, amino, -NH-C₁₋₂₅ alkyl and -N(C₁₋₂₅ alkyl)₂, alternatively optionally substituted with 1, 2, 3, 4 or 5 substituents selected from: -OH, halogen, cyano, C₁₋₂₀ alkyl, C₁₋₂₀ haloalkyl, -O-C₁₋₂₀ alkyl, -S-C₁₋₂₀ alkyl, amino, -NH-C₁₋₂₀ alkyl and -N(C₁₋₂₀ alkyl)₂, alternatively optionally substituted with 1, 2, 3, 4 or 5 substituents selected from: -OH, halogen, cyano, C₁₋₁₈ alkyl, C₁₋₁₈ haloalkyl, -O-C₁₋₁₈ alkyl, -S-C₁₋₁₈ alkyl, amino, -NH-C₁₋₁₈ alkyl and -N(C₁₋₁₈ alkyl)₂;
R₁₃, R₁₄ and R₁₅ are independently selected from C₁₋₆ alkyl, C₁₋₆ haloalkyl, C₂₋₆ alkenyl and C₂₋₆ alkynyl, alternatively selected from C₁₋₆ alkyl, such as Me; or any two of them and the N atom to which they are attached form 4- to 8-membered heterocycle, alternatively 5-to 6-membered heterocycle;
n₁ and n₂ are independently selected from 0 and 1.

Alternatively, n₁ is equal to n₂.

Alternatively, the pharmaceutically acceptable salt is a monovalent anionic salt, alternatively p-toluenesulfonate or halogen salt, such as a chloride or bromide salt.

In a more specific embodiment, the present disclosure provides the above lipid nanoparticles, wherein the permanently cationic lipid is selected from a pharmaceutically acceptable salt of the compound of formula (II): 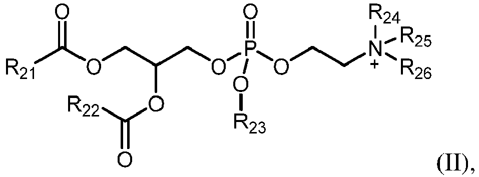 wherein
R₂₁ and R₂₂ are independently selected from C₆₋₃₀ alkyl, C₆₋₃₀ alkenyl and C₆₋₃₀ alkynyl, alternatively selected from C₁₀₋₂₅ alkyl, C₁₀₋₂₅ alkenyl and C₁₀₋₂₅ alkynyl, alternatively selected from C₁₃₋₂₀ alkyl, C₁₃₋₂₀ alkenyl and C₁₃₋₂₀ alkynyl, alternatively selected from C₁₃₋₁₇ alkyl, C₁₃₋₁₇ alkenyl and C₁₃₋₁₇ alkynyl, alternatively selected from C₁₃₋₂₀ alkyl and C₁₃₋₂₀ alkenyl, alternatively selected from C₁₃₋₁₇ alkyl and C₁₃₋₁₇ alkenyl, which are optionally substituted with 1, 2, 3, 4 or 5 substituents selected from: -OH, halogen, cyano, C₁₋₃₀ alkyl, C₁₋₃₀ haloalkyl, -O-C₁₋₃₀ alkyl, -S-C₁₋₃₀ alkyl, amino, -NH-C₁₋₃₀ alkyl and -N(C₁₋₃₀ alkyl)₂, alternatively optionally substituted with 1, 2, 3, 4 or 5 substituents selected from: -OH, halogen, cyano, C₁₋₂₅ alkyl, C₁₋₂₅ haloalkyl, -O-C₁₋₂₅ alkyl, -S-C₁₋₂₅ alkyl, amino, -NH-C₁₋₂₅ alkyl and -N(C₁₋₂₅ alkyl)₂, alternatively optionally substituted with 1, 2, 3, 4 or 5 substituents selected from: -OH, halogen, cyano, C₁₋₂₀ alkyl, C₁₋₂₀ haloalkyl, -O-C₁₋₂₀ alkyl, -S-C₁₋₂₀ alkyl, amino, -NH-C₁₋₂₀ alkyl and -N(C₁₋₂₀ alkyl)₂, alternatively optionally substituted with 1, 2, 3, 4 or 5 substituents selected from: -OH, halogen, cyano, C₁₋₁₇ alkyl, C₁₋₁₇ haloalkyl, -O-C₁₋₁₇ alkyl, -S-C₁₋₁₇ alkyl, amino, -NH-C₁₋₁₇ alkyl and -N(C₁₋₁₇ alkyl)₂;
R₂₃ is selected from C₁₋₆ alkyl, C₁₋₆ haloalkyl, C₂₋₆ alkenyl and C₂₋₆ alkynyl, alternatively selected from C₁₋₆ alkyl and C₁₋₆ haloalkyl, alternatively Me and Et, which is optionally substituted with 1, 2 or 3 R₂₃ₛ,
R₂₃ₛ is independently selected from C₁₋₆ alkyl, C₁₋₆ haloalkyl, -OC(O)R₂ₐ and -C(O)OR₂ₐ, alternatively selected from C₁₋₆ alkyl, C₁₋₆ haloalkyl and -C(O)OR₂ₐ;
R₂ₐ is independently selected from H, C₁₋₆ alkyl and C₁₋₆ haloalkyl, alternatively Et;
R₂₄, R₂₅ and R₂₆ are independently selected from C₁₋₆ alkyl, C₁₋₆ haloalkyl, C₂₋₆ alkenyl and C₂₋₆ alkynyl, alternatively selected from C₁₋₆ alkyl, such as Me; or any two of them and the N atom to which they are attached form 4- to 8-membered heterocycle, alternatively 5-to 6-membered heterocycle.

Alternatively, the pharmaceutically acceptable salt is a monovalent anionic salt, alternatively p-toluenesulfonate or halogen salt, such as a chloride or bromide salt.

In some specific embodiments of the present disclosure, the permanently cationic lipid is selected from a pharmaceutically acceptable salt of the following compounds:

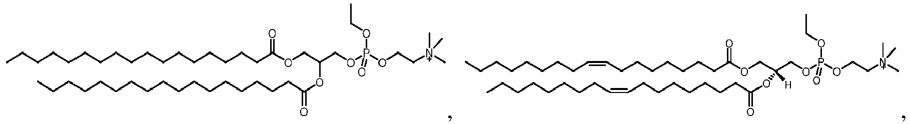

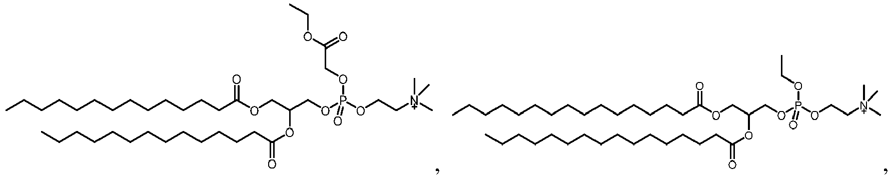

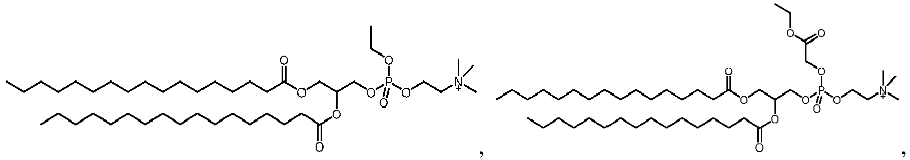

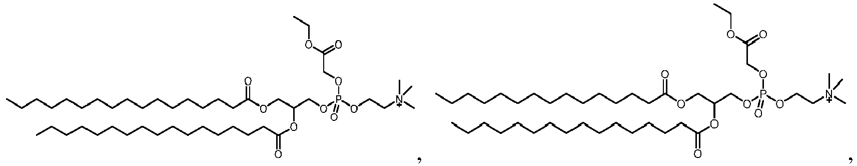

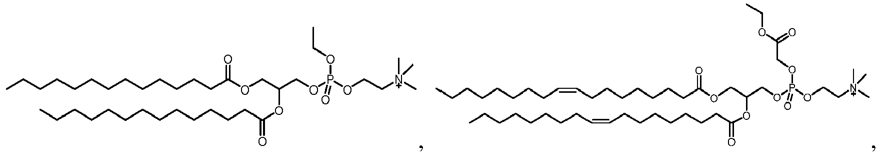

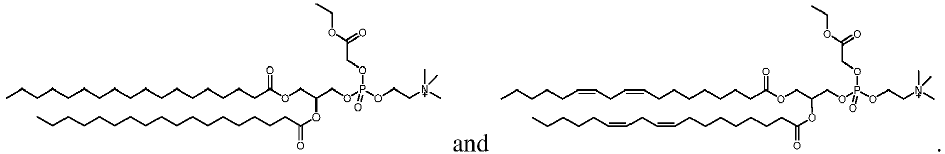

Alternatively, the pharmaceutically acceptable salt is a monovalent anionic salt, alternatively p-toluenesulfonate or halogen salt, such as a chloride or bromide salt.

In a more specific embodiment, the permanently cationic lipid may be selected from the following compounds:

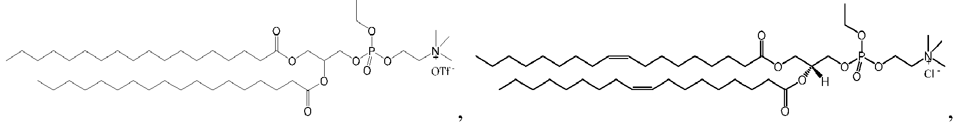

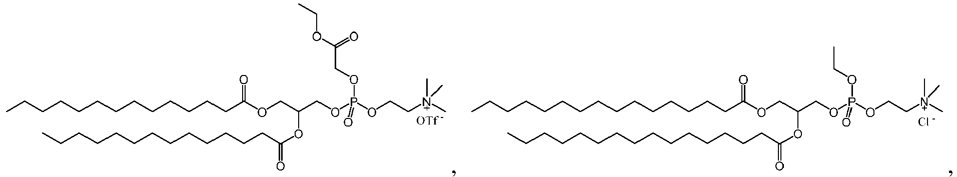

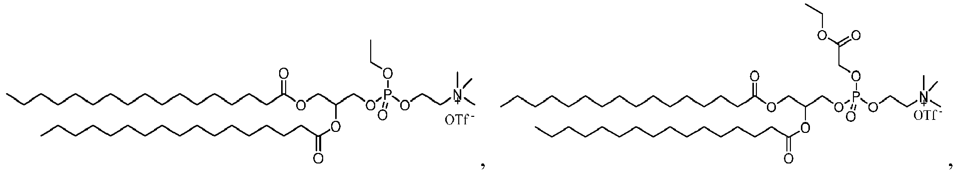

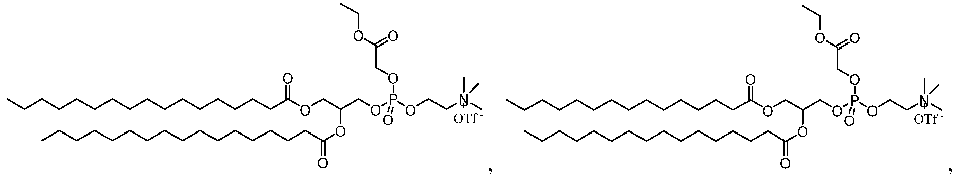

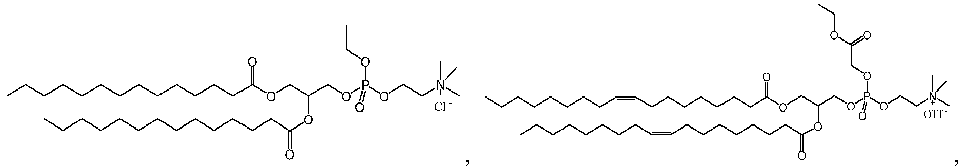

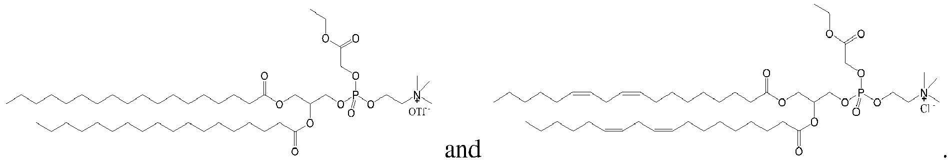

In a more specific embodiment, the present disclosure provides the above lipid nanoparticle, wherein the permanently cationic lipid is selected from one or more of: N-[1-(2,3-dioleyloxy)propyl]-N,N,N-trimethylammonium chloride (DOTMA), N-[1-(2,3-dioleoyloxy)propyl]-N,N,N-trimethylammonium chloride (DOTAP), ethylphosphatidylcholine (EPC) and derivatives thereof, N1-[2-((1S)-1-[(3-aminopropyl)amino]-4-[di(3-amino-propyl)amino]butylcarboxamido)ethyl ]-3,4-bis[oleyloxy]-benzamide (MVL5), dioctadecylamido-glycylspermine (DOGS), 3b-[N-(N',N'-dimethylaminoethyl)carbamoyl]cholesterol (DC-Chol) and dioctadecyldimethylammonium bromide (DDAB), alternatively selected from ethylphosphatidylcholine (EPC) and derivatives thereof and N-[1-(2,3-dioleoyloxy)propyl]-N,N,N-trimethylammonium chloride (DOTAP); alternatively selected from one or more of N-[1-(2,3-dioleyloxy)propyl]-N,N,N-trimethylammonium chloride (DOTMA), N-[1-(2,3-dioleoyloxy)propyl]-N,N,N-trimethylammonium chloride (DOTAP) and ethylphosphatidylcholine (EPC) and derivatives thereof; alternatively ethylphosphatidylcholine (EPC) and/or N-[1-(2,3-dioleoyloxy)propyl]-N,N,N-trimethylammonium chloride (DOTAP); still alternatively N-[1-(2,3-dioleoyloxy)propyl]-N,N,N-trimethylammonium chloride (DOTAP).

In a specific embodiment of the present disclosure, the ethylphosphatidylcholine (EPC) is 18:1EPC.

In a more specific embodiment, the present disclosure provides the above lipid nanoparticle, wherein the molar percentage content of the permanently cationic lipid is >0 mol%-30 mol%; alternatively 1.0 mol%-25 mol%; alternatively 2.5 mol%-20 mol%; alternatively 10 mol%-20 mol%; still alternatively 2.5 mol%, 5 mol%, 9.4 mol%, 10 mol% or 20 mol%.

It should be understood that all ionizable lipids in the art may be applied to the present disclosure.

In a specific embodiment, the ionizable lipid contains two non-degradable hydrophobic tails. For example, the ionizable lipid is the ionizable lipids mentioned in WO2010144740A1.

In a specific embodiment, the ionizable lipid contains two degradable hydrophobic tails. For example, the ionizable lipid is the ionizable lipids mentioned in WO2011153493A2 and WO2013086354A1.

In a specific embodiment, the ionizable lipid contains one base group and two biodegradable hydrophobic tails, wherein:
(a) the base group includes one head group and a central moiety directly bonded to the head group and two biodegradable hydrophobic tails, wherein the head group contains a protonatable group with a pKa of 4-11.
(b) the ionizable lipid has a logP value of at least 10.1.
(c) the biodegradable hydrophobic tail has the following general formula: - (hydrophobic chain I) - (biodegradable group) - (hydrophobic chain II).
wherein in at least one biodegradable hydrophobic tail:
(i) the hydrophobic chain II has a total length of 6-12 carbon atoms;
(ii) at least one biodegradable hydrophobic tail has the following general formula: -R₁₆-M₀-R₁₇;
   wherein R₁₆ is C₄-₁₄ alkylene, C₄-₁₄ alkynylene or C₄-C₁₄ alkenylene, M₀ is a biodegradable group, and R₁₇ is linear or branched C₆-₂₀ alkyl;
(iii) the total number of carbon atoms in each tail-R₁₆-M₀-R₁₇ is 15-26.

In a more specific embodiment, the ionizable lipid is selected from the compound of formula (III), or a pharmaceutically acceptable salt, isotopic variant, tautomer or stereoisomer thereof: 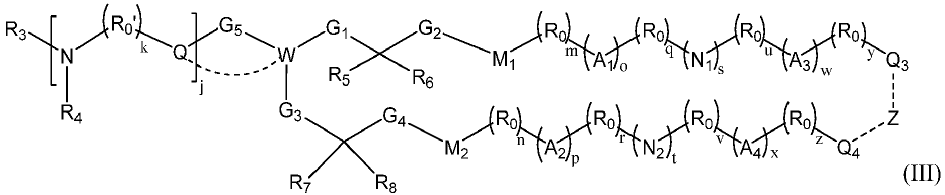 wherein
R₃ and R₄ are independently selected from C₁₋₁₀ alkyl, C₂₋₁₀ alkenyl, C₂₋₁₀ alkynyl, 3-to 14-membered cycloalkyl, -C₁₋₁₀ alkylene-3- to 14-membered cycloalkyl or 3- to 14-membered heterocyclyl, which is optionally substituted with one or more R*;
or, R₃ and R₄ are taken together with the N atom to which they are attached to form 3-to 14-membered heterocyclyl, which is optionally substituted with one or more R*;
or, R₄ is taken together with the N atom to which it is attached, one R₀' of the (R₀')ₖ, and the attached atoms between them to form 4- to 10-membered heterocycle or 5- to 10-membered heteroaromatic ring, which is optionally substituted with one or more R*;
R* is independently selected from H, halogen, cyano, C₁₋₁₀ alkyl, C₁₋₁₀ haloalkyl, -L_{b}-OR_{b}, -L_{b}-SR_{b} and -L_{b}-NR_{b}R'_{b};
R₀' is independently methylene optionally substituted with one or two R**, or the two substituents on R₀' are taken together with the C atom to which they are co-attached to form a 3- to 8-membered cycloalkylene;
R** is independently selected from H, C₁₋₈ alkyl, -L_{c}-OR_{c}, -L_{c}-SR_{c}, and -L_{c}-NR_{c}R'_{c};
k is selected from 0, 1, 2, 3, 4, 5 and 6;
j is selected from 0 and 1;
the dotted line connecting Q and W is absent or a chemical bond;
W is selected from C, CH and N;
When W is N, j is 0 and the dotted line connecting Q and W is absent;
G₅ is selected from a chemical bond, C₁-₂₄ alkylene, C₂-₂₄ alkenylene, 3- to 8-membered cycloalkylene, and C₃₋₈ cycloalkenylene, alternatively chemical bond and C₁₋₈ alkylene, which is optionally substituted with one or more R**;
G₁, G₂, G₃ and G₄ are independently selected from a chemical bond, C₁₋₁₃ alkylene, C₂₋₁₃ alkenylene and C₂₋₁₃ alkynylene, which is optionally substituted with one or more R^{s};
G₁ and G₂ have a total length of 3, 4, 5, 6, 7, 8, 9, 10, 11, 12 or 13 carbon atoms;
G₃ and G₄ have a total length of 3, 4, 5, 6, 7, 8, 9, 10, 11, 12 or 13 carbon atoms;
R^{s} is independently selected from H, C₁₋₁₄ alkyl, -L_{d}-OR_{d}, -L_{d}-SR_{d} and -L_{d}-NR_{d}R'_{d};
R₅, R₆, R₇ and R₈ are independently selected from H and C₁₋₈ alkyl, which is optionally substituted with one or more R*;
When the dotted line connecting Q and W is absent, Q is absent , or selected from: -C(O)O-, -O-, -NH-, -SC(O)O-, -OC(O)NR_{b}-, -NR_{b}C(O)NR_{b}-, -OC(O)S-, -OC(O)O-, -NR_{b}C(O)O-, -OC(O)-, -SC(O)-, -C(O)S-, -NR_{b}-, -C(O)NR_{b}-, -NR_{b}C(O)-, -NR_{b}C(O)S-, -SC(O)NR_{b}-, -C(O)-, -OC(S)-, -C(S)O-, -OC(S)NR_{b}-, -NR_{b}C(S)O-, -S-S- and -S(O)₀₋₂-;
When the dotted line connecting Q and W is a chemical bond, G5 is absent, and Q is taken together with W to form a 5- to 10-membered monocyclic or bicyclic ring, which is optionally substituted with one or more R**;
M₁ and M₂ are independently absent, or selected from -C(O)O-, -O-, -SC(O)O-, -OC(O)NRₐ-, -NRₐC(O)NRₐ-, -OC(O)S-, -OC(O)O-, -NRₐC(O)O-, -OC(O)-, -SC(O)-, -C(O)S-, -NRₐ-, -C(O)NRₐ-, -NRₐC(O)-, -NRₐC(O)S-, -SC(O)NRₐ-, -C(O)-, -OC(S)-, -C(S)O-, -OC(S)NRₐ-, -NRₐC(S)O-, -S-S- and -S(O)₀₋₂-;
R₀ is independently -(CRR')-;
R and R' are independently selected from H, C₁₋₂₀ alkyl, -Lₐ-ORₐ, -Lₐ-SRₐ and -Lₐ-NRₐR'ₐ, alternatively R' is H;
or, R and R' are taken together with the atom to which they are attached to form 3- to 8-membered cycloalkylene;
in each chain attached to W, no more than three R₀ or R₀' are cycloalkylene;
Q₃ and Q₄ are independently H, -(CRR')-, C₆₋₁₀ aryl or a steroid group, alternatively H or -(CRR')-;
A₁, A₂, A₃ and A₄ are independently -(CR₁₈R₁₈-CR₁₈=CR₁₈)- or -(CR₁₈R₁₈-C≡C)-;
R₁₈ is independently H or C₁₋₂₀ alkyl;
N₁ and N₂ are independently a biodegradable group;
Z is absent, C₁₋₁₀ alkylene or -O-P(O)(OH)-O-;
the dotted line attached to Z is absent or a chemical bond, and when Z is absent, Q₃ and Q₄ are not directly connected;
m, n, q, r, u, v, y and z are each independently selected from 0, 1, 2, 3, 4, 5, 6, 7, 8, 9 and 10;
o, p, w and x are each independently selected from 0, 1 and 2;
s and t are each independently selected from 0 and 1;
the total length of chain segments from G₁ to Q₃ or from G₃ to Q₄ is 8-30 atoms, alternatively 10-25 atoms;
Lₐ and Lₑ are independently selected from a chemical bond and C₁₋₂₀ alkylene;
L_{b} and L_{f} are independently selected from a chemical bond and C₁₋₁₀ alkylene;
L_{c} is independently selected from a chemical bond and C₁₋₈ alkylene;
L_{d} is independently selected from a chemical bond and C₁₋₁₄ alkylene;
Rₐ and R'ₐ are independently selected from H, C₁₋₂₀ alkyl, 3- to 14-membered cycloalkyl, and 3- to 14-membered heterocyclyl, which is optionally substituted with one or more substituents as follows: H, C₁₋₂₀ alkyl, -Lₑ-ORₑ, -Lₑ-SRₑ or -Lₑ-NRₑR'ₑ;
R_{b} and R'_{b} are independently selected from H, C₁₋₁₀ alkyl, 3- to 14-membered cycloalkyl, and 3- to 14-membered heterocyclyl, which is optionally substituted with one or more substituents as follows: H, C₁₋₁₀ alkyl, -L_{f}-OR_{f}, -L_{f}-SR_{f} or -L_{f}-NR_{f}R'_{f};
R_{c} and R'_{c} are independently selected from H and C₁₋₈ alkyl;
R_{d} and R'_{d} are independently selected from H and C₁₋₁₄ alkyl;
Rₑ and R'ₑ are independently selected from H and C₁₋₂₀ alkyl;
R_{f} and R'_{f} are independently selected from H and C₁₋₁₀ alkyl.

In a more specific embodiment, the ionizable lipid relates to a compound of formula (IV'), or a pharmaceutically acceptable salt, isotopic variant, tautomer or stereoisomer thereof: 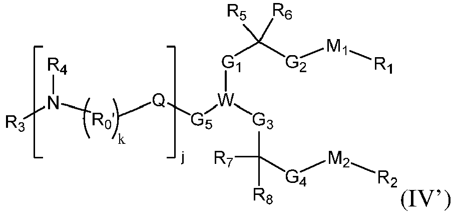 wherein
j is selected from 0 and 1;
W is CH or N; when W is N, j is 0;
R₀' is independently methylene optionally substituted with one or two R**, or the two substituents on R₀' are taken together with the C atom to which they are co-attached to form a 3- to 8-membered cycloalkylene;
k is selected from 0, 1, 2, 3, 4, 5, 6, 7 and 8, alternatively 0, 1, 2, 3, 4, 5 and 6, alternatively selected from 0, 1, 2, 3, 4 and 5, alternatively selected from 1, 2, 3, 4 and 5, alternatively selected from 3, 4 and 5, still alternatively 3 and 4;
M₁ and M₂ are independently selected from -C(O)O-, -O-, -SC(O)O-, -OC(O)NRₐ-, -NRₐC(O)NRₐ-, -OC(O)S-, -OC(O)O-, -NRₐC(O)O-, -OC(O)-, -SC(O)-, -C(O)S-, -NRₐ-, -C(O)NRₐ-, -NRₐC(O)-, -NRₐC(O)S-, -SC(O)NRₐ-, -C(O)-, -OC(S)-, -C(S)O-, -OC(S)NRₐ-, -NRₐC(S)O-, -S-S- and -S(O)₀₋₂-;
Q is selected from a chemical bond, -C(O)O-, -O-, -SC(O)O-, -OC(O)NR_{b}-, -NR_{b}C(O)NR_{b}-, -OC(O)S-, -OC(O)O-, -NR_{b}C(O)O-, -OC(O)-, -SC(O)-, -C(O)S-, -NR_{b}-, -C(O)NR_{b}-, -NR_{b}C(O)-, -NR_{b}C(O)S-, -SC(O)NR_{b}-, -C(O)-, -OC(S)-, -C(S)O-, -OC(S)NR_{b}-, -NR_{b}C(S)O-, -S-S-, -S(O)₀₋₂-, phenylene and pyridylene, wherein the phenylene and pyridylene are optionally substituted with one or more R*;
G₅ is selected from a chemical bond and C₁₋₈ alkylene, which is optionally substituted with one or more R**;
R** is independently selected from H, C₁₋₈ alkyl, -L_{c}-OR_{c}, -L_{c}-SR_{c} and -L_{c}-NR_{c}R'_{c};
G₁, G₂, G₃ and G₄ are independently selected from a chemical bond, C₁₋₁₃ alkylene, C₂₋₁₃ alkenylene and C₂₋₁₃ alkynylene, which is optionally substituted with one or more R^{s};
G₁ and G₂ have a total length of 3, 4, 5, 6, 7, 8, 9, 10, 11, 12 or 13 carbon atoms;
G₃ and G₄ have a total length of 3, 4, 5, 6, 7, 8, 9, 10, 11, 12 or 13 carbon atoms;
R₃ and R₄ are independently selected from H, C₁₋₁₀ alkyl, C₁₋₁₀ haloalkyl, C₂₋₁₀ alkenyl, C₂₋₁₀ alkynyl, 3- to 14-membered cycloalkyl, 3- to 14-membered heterocyclyl, C₆-₁₀ aryl and 5-to 14-membered heteroaryl, which is optionally substituted with one or more R*;
or, R₃ and R₄ are taken together with the N atom to which they are attached to form 3-to 14-membered heterocyclyl, which is optionally substituted with one or more R*;
or, R₄ is taken together with the N atom to which it is attached, one R₀' of the (R₀')ₖ, and the attached atoms between them to form 3- to 14-membered heterocyclyl or 5- to 14-membered heteroaryl, which is optionally substituted with one or more R*;
R* is independently selected from H, halogen, cyano, C₁₋₁₀ alkyl, C₁₋₁₀ haloalkyl, -L_{b}-OR_{b}, -L_{b}-SR_{b} and -L_{b}-NR_{b}R'_{b};
R₅, R₆, R₇ and R₈ are independently selected from H and C₁₋₈ alkyl, wherein the alkyl is optionally substituted with one or more R*;
R₁ and R₂ are independently selected from C₄₋₂₀ alkyl, C₄₋₂₀ alkenyl and C₄₋₂₀ alkynyl, which is optionally substituted with one or more R, wherein one or more methylene units are optionally and independently replaced by -NR"-;
R^{s} is independently selected from H, C₁₋₁₄ alkyl, -L_{d}-OR_{d}, -L_{d}-SR_{d} and -L_{d}-NR_{d}R'_{d};
R is independently selected from H, C₁₋₂₀ alkyl, -Lₐ-ORₐ, -Lₐ-SRₐ and -Lₐ-NRₐR'ₐ;
R" is independently selected from H and C₁₋₂₀ alkyl;
Lₐ and Lₑ are independently selected from a chemical bond and C₁₋₂₀ alkylene;
L_{b} and L_{f} are independently selected from a chemical bond and C₁₋₁₀ alkylene;
L_{c} is independently selected from a chemical bond and C₁₋₈ alkylene;
L_{d} is independently selected from a chemical bond and C₁₋₁₄ alkylene;
Rₐ and R'ₐ are independently selected from H, C₁₋₂₀ alkyl, 3- to 14-membered cycloalkyl, and 3- to 14-membered heterocyclyl, which is optionally substituted with one or more substituents as follows: H, C₁₋₂₀ alkyl, -Lₑ-ORₑ, -Lₑ-SRₑ or -Lₑ-NRₑR'ₑ;
R_{b} and R'_{b} are independently selected from H, C₁₋₁₀ alkyl, 3- to 14-membered cycloalkyl, and 3- to 14-membered heterocyclyl, which is optionally substituted with one or more substituents as follows: H, C₁₋₁₀ alkyl, -L_{f}-OR_{f}, -L_{f}-SR_{f} or -L_{f}-NR_{f}R'_{f};
R_{c} and R'_{c} are independently selected from H and C₁₋₈ alkyl;
R_{d} and R'_{d} are independently selected from H and C₁₋₁₄ alkyl;
Rₑ and R'ₑ are independently selected from H and C₁₋₂₀ alkyl;
R_{f} and R'_{f} are independently selected from H and C₁₋₁₀ alkyl.

### j

In one embodiment, j is 0; in another embodiment, j is 1.

### W

In one embodiment, W is CH; in another embodiment, W is N.

In a more specific embodiment, when W is N, j is 0; in another more specific embodiment, when W is CH, j is 1.

### R₀'

In one embodiment, R₀' is methylene; in another specific embodiment, R₀' is optionally substituted with one or two R**; in another specific embodiment, the two substituents on R₀' are taken together with the C atom to which they are co-attached to form a 3- to 8-membered cycloalkylene.

### k

In one embodiment, k is 0; in another embodiment, k is 1; in another embodiment, k is 2; in another embodiment, k is 3; in another embodiment, k is 4; in another embodiment, k is 5; in another embodiment, k is 6; in another embodiment, k is 7; in another embodiment, k is 8.

In one specific embodiment, k is selected from 0, 1, 2, 3, 4, 5 and 6; in another specific embodiment, k is selected from 0, 1, 2, 3, 4 and 5; in another specific embodiment, k is selected from 1, 2, 3, 4 and 5; in another specific embodiment, k is selected from 3, 4 and 5; in another specific embodiment, k is 3 or 4.

### M₁ and M₂

In one embodiment, M₁ is -C(O)O-; in another embodiment, M₁ is -O-; in another embodiment, M₁ is -SC(O)O-; in another embodiment, M₁ is -OC(O)NRₐ-; in another embodiment, M₁ is -NRₐC(O)NRₐ-; in another embodiment, M₁ is -OC(O)S-; in another embodiment, M₁ is -OC(O)O-; in another embodiment, M₁ is -NRₐC(O)O-; in another embodiment, M₁ is -OC(O)-; in another embodiment, M₁ is -SC(O)-; in another embodiment, M₁ is -C(O)S-; in another embodiment, M₁ is -NRₐ-; in another embodiment, M₁ is -C(O)NRₐ-; in another embodiment, M₁ is -NRₐC(O)-; in another embodiment, M₁ is -NRₐC(O)S-; in another embodiment, M₁ is -SC(O)NRₐ-; in another embodiment, M₁ is -C(O)-; in another embodiment, M₁ is -OC(S)-; in another embodiment, M₁ is -C(S)O-; in another embodiment, M₁ is -OC(S)NRₐ-; in another embodiment, M₁ is -NRₐC(S)O-; in another embodiment, M₁ is -S-S-; in another embodiment, M₁ is -S(O)₀₋₂-.

In one embodiment, M₂ is -C(O)O-; in another embodiment, M₂ is -O-; in another embodiment, M₂ is -SC(O)O-; in another embodiment, M₂ is -OC(O)NRₐ-; in another embodiment, M₂ is -NRₐC(O)NRₐ-; in another embodiment, M₂ is -OC(O)S-; in another embodiment, M₂ is -OC(O)O-; in another embodiment, M₂ is -NRₐC(O)O-; in another embodiment, M₂ is -OC(O)-; in another embodiment, M₂ is -SC(O)-; in another embodiment, M₂ is -C(O)S-; in another embodiment, M₂ is -NRₐ-; in another embodiment, M₂ is -C(O)NRₐ-; in another embodiment, M₂ is -NRₐC(O)-; in another embodiment, M₂ is -NRₐC(O)S-; in another embodiment, M₂ is -SC(O)NRₐ-; in another embodiment, M₂ is -C(O)-; in another embodiment, M₂ is -OC(S)-; in another embodiment, M₂ is -C(S)O-; in another embodiment, M₂ is -OC(S)NRₐ-; in another embodiment, M₂ is -NRₐC(S)O-; in another embodiment, M₂ is -S-S-; in another embodiment, M₂ is -S(O)₀₋₂-.

In a more specific embodiment, M₁ and M₂ are independently selected from -C(O)O-, -O-, -SC(O)O-, -OC(O)NRₐ-, -NRₐC(O)NRₐ-, -OC(O)S-, -OC(O)O-, -NRₐC(O)O-, -OC(O)-, -SC(O)-, -C(O)S-, -NRₐ-, -C(O)NRₐ-, -NRₐC(O)-, -NRₐC(O)S-, -SC(O)NRₐ-, -C(O)-, -OC(S)-, -C(S)O-, -OC(S)NRₐ-, -NRₐC(S)O-, -S-S- and -S(O)₀₋₂-; in another more specific embodiment, M₁ and M₂ are independently selected from -C(O)O-, -SC(O)O-, -OC(O)NRₐ-, -NRₐC(O)NRₐ-, -OC(O)S-, -OC(O)O-, -NRₐC(O)O-, -OC(O)-, -SC(O)-, -C(O)S-, -C(O)NRₐ-, -NRₐC(O)S-, -SC(O)NRₐ-, -OC(S)-, -C(S)O-, -OC(S)NRₐ- and -NRₐC(S)O-; in another more specific embodiment, M₁ and M₂ are independently selected from -C(O)O-, -OC(O)-, -C(O)S-, -SC(O)-, -C(O)NRₐ- and -NRₐC(O)-; in another more specific embodiment, M₁ and M₂ are independently selected from -C(O)O-, -OC(O)-, -C(O)S- and -C(O)NRₐ-; in another more specific embodiment, M₁ and M₂ are independently -C(O)O- or -OC(O)-; in another more specific embodiment, M₁ and M₂ are independently -C(O)O- or -C(O)S-.

### Q

In one embodiment, Q is a chemical bond; in another embodiment, Q is -C(O)O-; in another embodiment, Q is -O-; in another embodiment, Q is -SC(O)O-; in another embodiment, Q is -OC(O)NR_{b}-; in another embodiment, Q is -NR_{b}C(O)NR_{b}-; in another embodiment, Q is -OC(O)S-; in another embodiment, Q is -OC(O)O-; in another embodiment, Q is -NR_{b}C(O)O-; in another embodiment, Q is -OC(O)-; in another embodiment, Q is -SC(O)-; in another embodiment, Q is -C(O)S-; in another embodiment, Q is -NR_{b}-; in another embodiment, Q is -C(O)NR_{b}-; in another embodiment, Q is -NR_{b}C(O)-; in another embodiment, Q is -NR_{b}C(O)S-; in another embodiment, Q is -SC(O)NR_{b}-; in another embodiment, Q is -C(O)-; in another embodiment, Q is -OC(S)-; in another embodiment, Q is -C(S)O-; in another embodiment, Q is -OC(S)NR_{b}-; in another embodiment, Q is -NR_{b}C(S)O-; in another embodiment, Q is -S-S-; in another embodiment, Q is -S(O)₀₋₂-; in another embodiment, Q is phenylene; in another embodiment, Q is pyridylene; in another embodiment, the phenylene or pyridylene are optionally substituted with one or more R*.

In a more specific embodiment, Q is selected from a chemical bond, -C(O)O-, -O-, -SC(O)O-, -OC(O)NR_{b}-, -NR_{b}C(O)NR_{b}-, -OC(O)S-, -OC(O)O-, -NR_{b}C(O)O-, -OC(O)-, -SC(O)-, -C(O)S-, -NR_{b}-, -C(O)NR_{b}-, -NR_{b}C(O)-, -NR_{b}C(O)S-, -SC(O)NR_{b}-, -C(O)-, -OC(S)-, -C(S)O-, -OC(S)NR_{b}-, -NR_{b}C(S)O-, -S-S-, and -S(O)₀₋₂-; in another more specific embodiment, Q is selected from -C(O)O-, -O-, -SC(O)O-, -OC(O)NH-, -NHC(O)NH-, -OC(O)S-, -OC(O)O-, -NHC(O)O-, -OC(O)-, -SC(O)-, -C(O)S-, -NH-, -C(O)NH-, -NHC(O)-, -NHC(O)S-, -SC(O)NH-, -C(O)-, -OC(S)-, -C(S)O-, -OC(S)NH- and -NHC(S)O-; in another more specific embodiment, Q is selected from -C(O)O-, -O-, -SC(O)O-, -OC(O)NH-, -NHC(O)NH-, -OC(O)S-, -OC(O)O- and -NHC(O)O-; in another more specific embodiment, Q is -C(O)O-.

### G₅

In one embodiment, G₅ is a chemical bond; in another embodiment, G₅ is C₁₋₈ alkylene; in another embodiment, G₅ is optionally substituted with one or more R**.

### R**

In one embodiment, R** is H; in another embodiment, R** is C₁₋₈ alkyl, alternatively C₁₋₆ alkyl; in another embodiment, R** is -L_{c}-OR_{c}; in another embodiment, R** is -L_{c}-SR_{c}; in another embodiment, R** is -L_{c-}NR_{c}R'_{c}; in another embodiment, two R** are taken together with the C atom to which they are co-attached to form 3- to 8-membered cycloalkylene.

In a more specific embodiment, R** is selected from H, C₁₋₆ alkyl, -L_{c}-OR_{c} and -L_{c-}NR_{c}R'_{c}; in another more specific embodiment, R** is H, -L_{c}-OR_{c} or C₁₋₆ alkyl; in another more specific embodiment, R** is H or -L_{c}-OR_{c}; in another more specific embodiment, R** is H or C₁₋₆ alkyl; in another more specific embodiment, R** is H.

### R₃ and R₄

In one embodiment, R₃ is H; in another embodiment, R₃ is C₁₋₁₀ alkyl; in another embodiment, R₃ is C₁₋₁₀ haloalkyl; in another embodiment, R₃ is C₂₋₁₀ alkenyl; in another embodiment, R₃ is C₂₋₁₀ alkynyl; in another embodiment, R₃ is 3- to 14-membered cycloalkyl; in another embodiment, R₃ is 3- to 14-membered heterocyclyl; in another embodiment, R₃ is C₆₋₁₀ aryl; in another embodiment, R₃ is 5- to 14-membered heteroaryl; in another embodiment, R₃ is C₁₋₆ alkyl; in another embodiment, R₃ is C₁₋₆ haloalkyl; in another embodiment, R₃ is 3- to 10-membered cycloalkyl; in another embodiment, R₃ is 3- to 10-membered heterocyclyl; in another embodiment, R₃ is 3- to 7-membered cycloalkyl; in another embodiment, R₃ is 3- to 7-membered heterocyclyl; in another embodiment, R₃ is Me; in another embodiment, R₃ is -CH₂CH₃; in another embodiment, R₃ is -CH₂CH₂OH; in another embodiment, R₃ is -CH₂CH₂CH₂CH₂OH; in another embodiment, R₃ is -CH(CH₃)₂; in another embodiment, R₃ is substituted with one or more R*; in another embodiment, R₃ is optionally substituted with 1, 2, 3, 4 or 5 R*.

In one embodiment, R₄ is H; in another embodiment, R₄ is C₁₋₁₀ alkyl; in another embodiment, R₄ is C₁₋₁₀ haloalkyl; in another embodiment, R₄ is C₂₋₁₀ alkenyl; in another embodiment, R₄ is C₂₋₁₀ alkynyl; in another embodiment, R₄ is 3- to 14-membered cycloalkyl; in another embodiment, R₄ is 3- to 14-membered heterocyclyl; in another embodiment, R₄ is C₆-₁₀ aryl; in another embodiment, R₄ is 5- to 14-membered heteroaryl; in another embodiment, R₄ is C₁₋₆ alkyl; in another embodiment, R₄ is C₁₋₆ haloalkyl; in another embodiment, R₄ is 3- to 10-membered cycloalkyl; in another embodiment, R₄ is 3- to 10-membered heterocyclyl; in another embodiment, R₄ is 3- to 7-membered cycloalkyl; in another embodiment, R₄ is 3- to 7-membered heterocyclyl; in another embodiment, R₄ is Me; in another embodiment, R₄ is substituted with one or more R*; in another embodiment, R₄ is optionally substituted with 1, 2, 3, 4 or 5 R*.

In one embodiment, R₃ and R₄ are taken together with the N atom to which they are attached to form 3- to 14-membered heterocyclyl; in another embodiment, R₃ and R₄ are taken together with the N atom to which they are attached to form 3- to 10-membered heterocyclyl; in another embodiment, R₃ and R₄ are taken together with the N atom to which they are attached to form 3- to 7-membered heterocyclyl; in another embodiment, R₃ and R₄ are taken together with the N atom to which they are attached to form 5- to 7-membered heterocyclyl; in another embodiment, R₃ and R₄ are taken together with the N atom to which they are attached to form 4- to 6-membered heterocyclyl; in another embodiment, R₃ and R₄ are taken together with the N atom to which they are attached to form 5-membered heterocyclyl; in another embodiment, R₃ and R₄ are taken together with the N atom to which they are attached to form 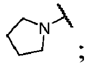 in another embodiment, R₃ and R₄ are taken together with the N atom to which they are attached to form 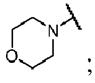 in another embodiment, R₃ and R₄ are taken together with the N atom to which they are attached to form 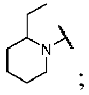 in another embodiment, R₃ and R₄ are taken together with the N atom to which they are attached to form 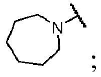 in another embodiment, the heterocyclyl formed by R₃, R₄ and the N atom to which they are attached is optionally substituted with one or more R*; in another embodiment, the heterocyclyl formed by R₃, R₄ and the N atom to which they are attached is optionally substituted with 1, 2, 3, 4 or 5 R*.

In one embodiment, R₄ is taken together with the N atom to which it is attached, one R₀' of the (R₀')ₖ, and the attached atoms between them to form 3- to 14-membered heterocyclyl; in another embodiment, R₄ is taken together with the N atom to which it is attached, one R₀' of the (R₀')ₖ, and the attached atoms between them to form 5- to 14-membered heteroaryl; in another embodiment, R₄ is taken together with the N atom to which it is attached, one R₀' of the (R₀')ₖ, and the attached atoms between them to form 3- to 10-membered heterocyclyl; in another embodiment, R₄ is taken together with the N atom to which it is attached, one R₀' of the (R₀')ₖ, and the attached atoms between them to form 3- to 7-membered heterocyclyl; in another embodiment, R₄ is taken together with the N atom to which it is attached, one R₀' of the (R₀')ₖ, and the attached atoms between them to form 6-membered heterocyclyl; in another embodiment, R₄ is taken together with the N atom to which it is attached, one R₀' of the (R₀')ₖ, and the attached atoms between them to form 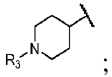 in another embodiment, the heterocyclyl formed by R₄ the N atom to which it is attached, one R₀' of the (R₀')ₖ, and the attached atoms between them is optionally substituted with one or more R*; in another embodiment, the heterocyclyl formed by R₄, the N atom to which it is attached, one R₀' of the (R₀')ₖ, and the attached atoms between them is optionally substituted with 1, 2, 3, 4 or 5 R*.

In a more specific embodiment, R₃ and R₄ are independently selected from H, C₁₋₆ alkyl, C₁₋₆ haloalkyl, 3- to 10-membered cycloalkyl and 3- to 10-membered heterocyclyl, which is optionally substituted with 1, 2, 3, 4 or 5 R*;
or R₃ and R₄ are taken together with the N atom to which they are attached to form 3-to 10-membered heterocyclyl, which is optionally substituted with 1, 2, 3, 4 or 5 R*;
or R₄ is taken together with the N atom to which it is attached, one R₀' of the (R₀')ₖ, and the attached atoms between them to form 3- to 10-membered heterocyclyl or 5- to 10-membered heteroaromatic ring, alternatively form 3- to 10-membered heterocyclyl, which is optionally substituted with 1, 2, 3 or 4 R* .

In another more specific embodiment, R₃ and R₄ are independently selected from H, C₁₋₆ alkyl, C₁₋₆ haloalkyl, 3- to 7-membered cycloalkyl and 3- to 7-membered heterocyclyl, which is optionally substituted with 1, 2, 3, 4 or 5 R*;
or R₃ and R₄ are taken together with the N atom to which they are attached to form 3-to 7-membered heterocyclyl, which is optionally substituted with 1, 2, 3, 4 or 5 R*;
or R₄ is taken together with the N atom to which it is attached, one R₀' of the (R₀')ₖ, and the attached atoms between them to form 3- to 7-membered heterocyclyl, which is optionally substituted with 1, 2, 3, 4 or 5 R*.

In a more specific embodiment, R₃ and R₄ are independently selected from C₁₋₆ alkyl, which is optionally substituted with 1, 2, 3, 4 or 5 R*;
or R₃ and R₄ are taken together with the N atom to which they are attached to form 5-to 7-membered heterocyclyl, alternatively form 4- to 6-membered heterocyclyl, still alternatively form 5-membered heterocyclyl, which is optionally substituted with 1, 2, 3, 4 or 5 R*;
or R₄ is taken together with the N atom to which it is attached, one R₀' of the (R₀')ₖ, and the attached atoms between them to form 6-membered heterocyclyl, which is optionally substituted with 1, 2, 3, 4 or 5 R*.

In a more specific embodiment, R₃ is Me, -CH₂CH₃, -CH₂CH₂OH, -CH₂CH₂CH₂CH₂OH or -CH(CH₃)₂, alternatively -CH₂CH₂OH or -CH₂CH₂CH₂CH₂OH, or alternatively Me, -CH₂CH₃ or -CH(CH₃)₂, still alternatively Me or -CH₂CH₃; R₄ is Me;
or R₃ and R₄ are taken together with the N atom to which they are attached to form 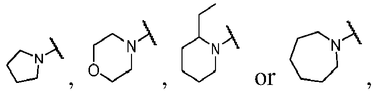 alternatively form 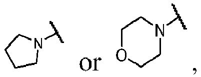 still alternatively form 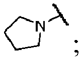
or R₄ is taken together with the N atom to which it is attached, one R₀' of the (R₀')ₖ, and the attached atoms between them to form

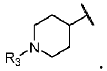

### R*

In one embodiment, R* is H; in another embodiment, R* is halogen; in another embodiment, R* is cyano; in another embodiment, R* is C₁₋₁₀ alkyl; in another embodiment, R* is C₁₋₁₀ haloalkyl; in another embodiment, R* is -L_{b}-OR_{b}; in another embodiment, R* is -L_{b}-SR_{b}, alternatively SR_{b}; in another embodiment, R* is -L_{b}-NR_{b}R'_{b}, alternatively NR_{b}R'_{b}; in another embodiment, R* is C₁₋₆ alkyl; in another embodiment, R* is C₁₋₆ haloalkyl; in another embodiment, R* is -OR_{b}.

In a more specific embodiment, R* is independently selected from H, halogen, cyano, C₁₋₆ alkyl, C₁₋₆ haloalkyl, -L_{b}-OR_{b} and -L_{b}-NR_{b}R'_{b}; in another more specific embodiment, R* is independently selected from H, C₁₋₆ alkyl, C₁₋₆ haloalkyl and -OR_{b}; in another more specific embodiment, R* is independently selected from H, halogen, C₁₋₆ alkyl and C₁₋₆ haloalkyl; in another more specific embodiment, R* is independently selected from H, C₁₋₆ alkyl and C₁₋₆ haloalkyl; in another more specific embodiment, R* is H, Me or OH; in another more specific embodiment, R* is H or Me.

### G₁, G₂, G₃ and G₄

In one embodiment, G₁ is a chemical bond; in another embodiment, G₁ is C₁₋₁₃ alkylene; in another embodiment, G₁ is C₂₋₁₃ alkenylene; in another embodiment, G₁ is C₂₋₁₃ alkynylene; in another embodiment, G₁ is optionally substituted with one or more R^{s}.

In one embodiment, G₂ is a chemical bond; in another embodiment, G₂ is C₁₋₁₃ alkylene; in another embodiment, G₂ is C₂₋₁₃ alkenylene; in another embodiment, G₂ is C₂₋₁₃ alkynylene; in another embodiment, G₂ is optionally substituted with one or more R^{s}.

In one embodiment, G₁ and G₂ have a total length of 3 carbon atoms; in another embodiment, G₁ and G₂ have a total length of 4 carbon atoms; in another embodiment, G₁ and G₂ have a total length of 5 carbon atoms; in another embodiment, G₁ and G₂ have a total length of 6 carbon atoms; in another embodiment, G₁ and G₂ have a total length of 7 carbon atoms; in another embodiment, G₁ and G₂ have a total length of 8 carbon atoms; in another embodiment, G₁ and G₂ have a total length of 9 carbon atoms; in another embodiment, G₁ and G₂ have a total length of 10 carbon atoms; in another embodiment, G₁ and G₂ have a total length of 11 carbon atoms; in another embodiment, G₁ and G₂ have a total length of 12 carbon atoms; in another embodiment, G₁ and G₂ have a total length of 13 carbon atoms.

In one embodiment, G₃ is a chemical bond; in another embodiment, G₃ is C₁₋₁₃ alkylene; in another embodiment, G₃ is C₂₋₁₃ alkenylene; in another embodiment, G₃ is C₂₋₁₃ alkynylene; in another embodiment, G₃ is optionally substituted with one or more R^{s}.

In one embodiment, G₄ is a chemical bond; in another embodiment, G₄ is C₁₋₁₃ alkylene; in another embodiment, G₄ is C₂₋₁₃ alkenylene; in another embodiment, G₄ is C₂₋₁₃ alkynylene; in another embodiment, G₄ is optionally substituted with one or more R^{s}.

In one embodiment, G₃ and G₄ have a total length of 3 carbon atoms; in another embodiment, G₃ and G₄ have a total length of 4 carbon atoms; in another embodiment, G₃ and G₄ have a total length of 5 carbon atoms; in another embodiment, G₃ and G₄ have a total length of 6 carbon atoms; in another embodiment, G₃ and G₄ have a total length of 7 carbon atoms; in another embodiment, G₃ and G₄ have a total length of 8 carbon atoms; in another embodiment, G₃ and G₄ have a total length of 9 carbon atoms; in another embodiment, G₃ and G₄ have a total length of 10 carbon atoms; in another embodiment, G₃ and G₄ have a total length of 11 carbon atoms; in another embodiment, G₃ and G₄ have a total length of 12 carbon atoms; in another embodiment, G₃ and G₄ have a total length of 13 carbon atoms.

In a more specific embodiment, -G₁-C(R₅R₆)-G₂- is

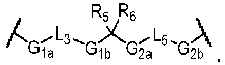

In one embodiment, G₁ₐ is a chemical bond; in another embodiment, G₁ₐ is C₁₋₇ alkylene; in another embodiment, G₁ₐ is -CH₂-; in another embodiment, G₁ₐ is -(CH₂)₂-; in another embodiment, G₁ₐ is -(CH₂)₃-; in another embodiment, G₁ₐ is -(CH₂)₄-; in another embodiment, G₁ₐ is -(CH₂)₅-; in another embodiment, G₁ₐ is -(CH₂)₆-; in another embodiment, G₁ₐ is optionally substituted with 1, 2, 3, 4 or 5 R^{s}.

In one embodiment, G_{1b} is a chemical bond; in another embodiment, G_{1b} is C₁₋₇ alkylene; in another embodiment, G_{1b} is C₁₋₃ alkylene; in another embodiment, G_{1b} is -CH₂-; in another embodiment, G_{1b} is -(CH₂)₂-; in another embodiment, G_{1b} is -(CH₂)₃-; in another embodiment, G_{1b} is optionally substituted with 1, 2, 3, 4 or 5 R^{s}.

In one embodiment, G₂ₐ is a chemical bond; in another embodiment, G₂ₐ is C₁₋₇ alkylene; in another embodiment, G₂ₐ is C₁₋₃ alkylene; in another embodiment, G₂ₐ is optionally substituted with 1, 2, 3, 4 or 5 R^{s}.

In one embodiment, G_{2b} is a chemical bond; in another embodiment, G_{2b} is C₁₋₇ alkylene; in another embodiment, G_{2b} is C₁₋₄ alkylene; in another embodiment, G_{2b} is -CH₂-; in another embodiment, G_{2b} is -(CH₂)₂-; in another embodiment, G_{2b} is -(CH₂)₃-; in another embodiment, G_{2b} is optionally substituted with 1, 2, 3, 4 or 5 R^{s}.

In a more specific embodiment, G₁ₐ, G₁₆, G₂ₐ and G_{2b} have a total length of 1, 2, 3, 4, 5, 6 or 7 carbon atoms; in another more specific embodiment, G₁ₐ, G_{1b}, G₂ₐ and G_{2b} have a total length of 1, 2, 3, 4, 5 or 6 carbon atoms.

In one embodiment, one of L₃ and L₅ is -(CR^{s}R^{s}')₂-, and the other is a chemical bond; in another embodiment, one of L₃ and L₅ is -(CHR^{s})₂-, and the other is a chemical bond; in another embodiment, one of L₃ and L₅ is -CH=CH-, and the other is a chemical bond; in another embodiment, one of L₃ and L₅ is -C=C-, and the other is a chemical bond.

In a more specific embodiment, -G₃-C(R₇R₈)-G₄- is

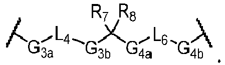

In one embodiment, G₃ₐ is a chemical bond; in another embodiment, G₃ₐ is C₁₋₇ alkylene; in another embodiment, G₃ₐ is -CH₂-; in another embodiment, G₃ₐ is -(CH₂)₂-; in another embodiment, G₃ₐ is -(CH₂)₃-; in another embodiment, G₃ₐ is -(CH₂)₄-; in another embodiment, G₃ₐ is -(CH₂)₅-; in another embodiment, G₃ₐ is -(CH₂)₆-; in another embodiment, G₃ₐ is optionally substituted with 1, 2, 3, 4 or 5 R^{s}.

In one embodiment, G_{3b} is a chemical bond; in another embodiment, G_{3b} is C₁₋₇ alkylene; in another embodiment, G_{3b} is C₁₋₃ alkylene; in another embodiment, G_{3b} is -CH₂-; in another embodiment, G_{3b} is -(CH₂)₂-; in another embodiment, G_{3b} is -(CH₂)₃-; in another embodiment, G_{3b} is optionally substituted with 1, 2, 3, 4 or 5 R^{s};

In one embodiment, G₄ₐ is a chemical bond; in another embodiment, G₄ₐ is C₁₋₇ alkylene; in another embodiment, G₄ₐ is C₁₋₃ alkylene; in another embodiment, G₄ₐ is optionally substituted with 1, 2, 3, 4 or 5 R^{s}.

In one embodiment, G_{4b} is a chemical bond; in another embodiment, G_{4b} is C₁₋₇ alkylene; in another embodiment, G_{4b} is C₁₋₄ alkylene; in another embodiment, G_{4b} is -CH₂-; in another embodiment, G_{4b} is -(CH₂)₂-; in another embodiment, G_{4b} is -(CH₂)₃-; in another embodiment, G_{4b} is optionally substituted with 1, 2, 3, 4 or 5 R^{s}.

In a more specific embodiment, G₃ₐ, G_{3b}, G₄ₐ and G_{4b} have a total length of 1, 2, 3, 4, 5, 6 or 7 carbon atoms; in another more specific embodiment, G₃ₐ, G_{3b}, G₄ₐ and G_{4b} have a total length of 1, 2, 3, 4, 5 or 6 carbon atoms.

In one embodiment, one of L₄ and L₆ is -(CR^{s}R^{s}')₂-, and the other is a chemical bond; in another embodiment, one of L₄ and L₆ is -(CHR^{s})₂-, and the other is a chemical bond; in another embodiment, one of L₄ and L₆ is -CH=CH-, and the other is a chemical bond; in another embodiment, one of L₄ and L₆ is -C=C-, and the other is a chemical bond.

In a more specific embodiment, one of L₃ and L₅, or one of L₄ and L₆ is selected from -(CHR^{s})₂-, -CH=CH- and -C=C-, and the other is a chemical bond;
G₁ₐ and G₃ₐ are independently selected from a chemical bond and C₁₋₇ alkylene;
G₁₆ and G_{3b} are independently selected from a chemical bond and C₁₋₃ alkylene;
G₂ₐ and G₄ₐ are independently selected from a chemical bond and C₁₋₃ alkylene;
G_{2b} and G_{4b} are independently selected from a chemical bond and C₁₋₄ alkylene;
G₁ₐ, G_{1b}, G₂ₐ and G_{2b} have a total length of 1, 2, 3, 4, 5, 6 or 7 carbon atoms;
G₃ₐ, G_{3b}, G₄ₐ and G_{4b} have a total length of 1, 2, 3, 4, 5, 6 or 7 carbon atoms.

In another more specific embodiment, one of L₃ and L₅, or one of L₄ and L₆ is selected from -(CH₂)₂-, -CH=CH- and -C=C-, and the other is a chemical bond;
G₁ₐ and G₃ₐ are independently selected from a chemical bond, -CH₂-, -(CH₂)₂-, -(CH₂)₃-, -(CH₂)₄-, -(CH₂)₅- and -(CH₂)₆-;
G_{1b} and G_{3b} is a chemical bond;
G₂ₐ and G₄ₐ is a chemical bond;
G_{2b} and G_{4b} are independently selected from a chemical bond, -CH₂-, -(CH₂)₂- and -(CH₂)₃-;
G₁ₐ, G_{1b}, G₂ₐ and G_{2b} have a total length of 1, 2, 3, 4, 5 or 6 carbon atoms;
G₃ₐ, G_{3b}, G₄ₐ and G_{4b} have a total length of 1, 2, 3, 4, 5 or 6 carbon atoms.

Optionally, R^{s} and R^{s}' are independently selected from H, C₁₋₁₀ alkyl, -L_{d}-OR_{d} and -L_{d}-NR_{d}R'_{d}; alternatively independently selected from H and C₁₋₆ alkyl; still alternatively H.

In a more specific embodiment, -G₁ₐ-L₃-G_{1b}- or -G₃ₐ-L₄-G_{3b}-- is independently selected from: -(CH₂)₂-, -(CH₂)₃-, -(CH₂)₄-, -(CH₂)₅-, -(CH₂)₆-, -(CH₂)₇-, -(CH₂)₈-, -(CH₂)₃-CH=CH-, -(CH₂)₃-C≡C-, -(CH₂)₂-CH=CH- and -(CH₂)₂-C≡C-.

In a more specific embodiment, -G₂ₐ-L₅-G_{2b}- or -G₄ₐ-L₆-G_{4b}- is independently selected from: a chemical bond, -CH₂-, -(CH₂)₂-, -(CH₂)₃-, -CH=CH-CH₂-, -C=C- and -C≡C-CH₂-.

In a more specific embodiment, 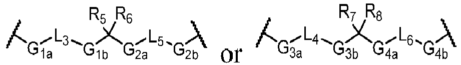 has a total length of 4, 5, 6, 7, 8 or 9 carbon atoms.

In a more specific embodiment, 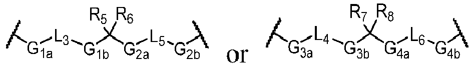 is independently selected from: -(CH₂)₃-C(CH₃)₂-, -(CH₂)₄-C(CH₃)₂-, -(CH₂)₅-C(CH₃)₂-, -(CH₂)₆-C(CH₃)₂-, -(CH₂)₇-C(CH₃)₂-, -(CH₂)₈-C(CH₃)₂-, -(CH₂)₃-CH=CH-C(CH₃)₂-, -(CH₂)₃-C≡C-C(CH₃)₂-, -(CH₂)₄-C(CH₃)₂-CH₂-, -(CH₂)₃-C(CH₃)₂-(CH₂)₂-, -(CH₂)₂-C(CH₃)₂-(CH₂)₃-, -(CH₂)₂-CH=CH-C(CH₃)₂-CH₂-, -(CH₂)₂-C(CH₃)₂-C≡C-CH₂-, -(CH₂)₂-C(CH₃)₂-CH=CH-CH₂-, -(CH₂)₂-C≡C-C(CH₃)₂-CH₂- and -(CH₂)₃-C(CH₃)₂-C≡C-; in another more specific embodiment, 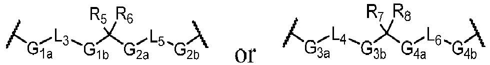 is independently selected from -(CH₂)₄-C(CH₃)₂-, -(CH₂)₅-C(CH₃)₂- and -(CH₂)₆-C(CH₃)₂-; in another more specific embodiment, 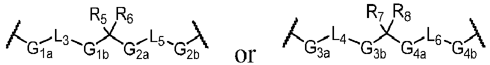 is -(CH₂)₅-C(CH₃)₂-.

### R₅, R₆, R₇ and R₈

In one embodiment, R₅ is H; in another embodiment, R₅ is C₁₋₈ alkyl; in another embodiment, R₅ is C₁₋₆ alkyl; in another embodiment, R₅ is C₁₋₃ alkyl; in another embodiment, R₅ is Me; in another embodiment, R₅ is optionally substituted with one or more R*; in another embodiment, R₅ is optionally substituted with 1, 2, 3, 4 or 5 R*.

In one embodiment, R₆ is H; in another embodiment, R₆ is C₁₋₈ alkyl; in another embodiment, R₆ is C₁₋₆ alkyl; in another embodiment, R₆ is C₁₋₃ alkyl; in another embodiment, R₆ is Me; in another embodiment, R₆ is optionally substituted with one or more R*; in another embodiment, R₆ is optionally substituted with 1, 2, 3, 4 or 5 R*.

In one embodiment, R₇ is H; in another embodiment, R₇ is C₁₋₈ alkyl; in another embodiment, R₇ is C₁₋₆ alkyl; in another embodiment, R₇ is C₁₋₃ alkyl; in another embodiment, R₇ is Me; in another embodiment, R₇ is optionally substituted with one or more R*; in another embodiment, R₇ is optionally substituted with 1, 2, 3, 4 or 5 R*.

In one embodiment, R₈ is H; in another embodiment, R₈ is C₁₋₈ alkyl; in another embodiment, R₈ is C₁₋₆ alkyl; in another embodiment, R₈ is C₁₋₃ alkyl; in another embodiment, R₈ is Me; in another embodiment, R₈ is optionally substituted with one or more R*; in another embodiment, R₈ is optionally substituted with 1, 2, 3, 4 or 5 R*.

In a more specific embodiment, R₅, R₆, R₇ and R₈ are independently selected from H and C₁₋₈ alkyl; in another more specific embodiment, R₅, R₆, R₇ and R₈ are independently selected from H and C₁₋₆ alkyl; in another more specific embodiment, R₅, R₆, R₇ and R₈ are independently selected from H and C₁₋₃ alkyl.

### R₁ and R₂

In one embodiment, R₁ is C₄₋₂₀ alkyl; in another embodiment, R₁ is C₄₋₂₀ alkenyl; in another embodiment, R₁ is C₄₋₂₀ alkynyl; in another embodiment, R₁ is optionally substituted with one or more R; in another embodiment, one or more methylene units in R₁ are optionally and independently replaced by -NR"-.

In a more specific embodiment, R₁ is -G₇-L₁-G₈-H.

In one embodiment, G₇ is a chemical bond; in another embodiment, G₇ is C₁₋₁₂ alkylene; in another embodiment, G₇ is C₁₋₆ alkylene; in another embodiment, G₇ is C₁₋₅ alkylene; in another embodiment, G₇ is linear C₁₋₅ alkylene; in another embodiment, G₇ is -CH₂-; in another embodiment, G₇ is -(CH₂)₂-; in another embodiment, G₇ is -(CH₂)₄-; in another embodiment, G₇ is -(CH₂)₅-; in another embodiment, G₇ is optionally substituted with 1, 2, 3, 4, 5 or 6 R; in another embodiment, 1, 2 or 3 methylenes in G₇ are optionally and independently substituted with 1 R; in another embodiment, 1 or 2 methylenes in G₇ are optionally and independently substituted with 1 R; in another embodiment, the methylene attached to M₁ in G₇ is not substituted with R.

In one embodiment, G₈ is a chemical bond; in another embodiment, G₈ is C₁₋₁₂ alkylene; in another embodiment, G₈ is C₁₋₁₀ alkylene; in another embodiment, G₈ is C₁₋₈ alkylene; in another embodiment, G₈ is linear C₁₋₈ alkylene; in another embodiment, G₈ is -(CH₂)₂-; in another embodiment, G₈ is -(CH₂)₄-; in another embodiment, G₈ is -(CH₂)₆-; in another embodiment, G₈ is -(CH₂)₇-; in another embodiment, G₈ is -(CH₂)₈-; in another embodiment, G₈ is optionally substituted with 1, 2, 3, 4, 5 or 6 R; in another embodiment, 1, 2 or 3 methylenes in G₈ are optionally and independently substituted with 1 R; in another embodiment, 1 or 2 methylenes in G₈ are optionally and independently substituted with 1 R.

In one embodiment, G₇ and G₈ have a total length of 4 carbon atoms; in another embodiment, G₇ and G₈ have a total length of 5 carbon atoms; in another embodiment, G₇ and G₈ have a total length of 6 carbon atoms; in another embodiment, G₇ and G₈ have a total length of 7 carbon atoms; in another embodiment, G₇ and G₈ have a total length of 8 carbon atoms; in another embodiment, G₇ and G₈ have a total length of 9 carbon atoms; in another embodiment, G₇ and G₈ have a total length of 10 carbon atoms; in another embodiment, G₇ and G₈ have a total length of 11 carbon atoms; in another embodiment, G₇ and G₈ have a total length of 12 carbon atoms.

In a more specific embodiment, G₇ and G₈ have a total length of 6, 7, 8, 9 or 10 carbon atoms.

In a more specific embodiment, G₇ and G₈ have a total length of 6, 7 or 8 carbon atoms.

In one embodiment, L₁ is -(CRR')₂-; in another embodiment, L₁ is -CH=CH-; in another embodiment, L₁ is -C=C-; in another embodiment, L₁ is -NR"-; in another embodiment, L₁ is -(CHR)₂-.

In one embodiment, R₂ is C₄₋₂₀ alkyl; in another embodiment, R₂ is C₄₋₂₀ alkenyl; in another embodiment, R₂ is C₄₋₂₀ alkynyl; in another embodiment, R₂ is optionally substituted with one or more R; in another embodiment, one or more methylene units in R₂ are optionally and independently replaced by -NR"-.

In a more specific embodiment, R₂ is -G₉-L₂-G₁₀-H.

In one embodiment, G₉ is a chemical bond; in another embodiment, G₉ is C₁₋₁₂ alkylene; in another embodiment, G₉ is C₁₋₆ alkylene; in another embodiment, G₉ is C₁₋₅ alkylene; in another embodiment, G₉ is linear C₁₋₅ alkylene; in another embodiment, G₉ is -CH₂-; in another embodiment, G₉ is -(CH₂)₂-; in another embodiment, G₉ is -(CH₂)₄-; in another embodiment, G₉ is -(CH₂)₅-; in another embodiment, G₉ is optionally substituted with 1, 2, 3, 4, 5 or 6 R; in another embodiment, 1, 2 or 3 methylenes in G₉ are optionally and independently substituted with 1 R; in another embodiment, 1 or 2 methylenes in G₉ are optionally and independently substituted with 1 R; in another embodiment, the methylene attached to M₂ in G₉ is not substituted with R.

In one embodiment, G₁₀ is a chemical bond; in another embodiment, G₁₀ is C₁₋₁₂ alkylene; in another embodiment, G₁₀ is C₁₋₁₀ alkylene; in another embodiment, G₁₀ is C₁₋₈ alkylene; in another embodiment, G₁₀ is linear C₁₋₈ alkylene; in another embodiment, G₁₀ is -(CH₂)₂-; in another embodiment, G₁₀ is -(CH₂)₄-; in another embodiment, G₁₀ is -(CH₂)₆-; in another embodiment, G₁₀ is -(CH₂)₇-; in another embodiment, G₁₀ is -(CH₂)₈-; in another embodiment, G₁₀ is optionally substituted with 1, 2, 3, 4, 5 or 6 R; in another embodiment, 1, 2 or 3 methylenes in G₁₀ are optionally and independently substituted with 1 R; in another embodiment, 1 or 2 methylenes in G₁₀ are optionally and independently substituted with 1 R.

In one embodiment, G₉ and G₁₀ have a total length of 4 carbon atoms; in another embodiment, G₉ and G₁₀ have a total length of 5 carbon atoms; in another embodiment, G₉ and G₁₀ have a total length of 6 carbon atoms; in another embodiment, G₉ and G₁₀ have a total length of 7 carbon atoms; in one embodiment, G₉ and G₁₀ have a total length of 8 carbon atoms; in one embodiment, G₉ and G₁₀ have a total length of 9 carbon atoms; in one embodiment, G₉ and G₁₀ have a total length of 10 carbon atoms; in one embodiment, G₉ and G₁₀ have a total length of 11 carbon atoms; in one embodiment, G₉ and G₁₀ have a total length of 12 carbon atoms.

In a more specific embodiment, G₉ and G₁₀ have a total length of 6, 7, 8, 9 or 10 carbon atoms.

In a more specific embodiment, G₉ and G₁₀ have a total length of 6, 7 or 8 carbon atoms.

In one embodiment, L₂ is -(CRR')₂-; in another embodiment, L₂ is -CH=CH-; in another embodiment, L₂ is -C=C-; in another embodiment, L₂ is -NR"-; in another embodiment, L₁ is -(CHR)₂-.

In a more specific embodiment, L₁ and L₂ are independently selected from -(CRR')₂-, -CH=CH-, -C=C- and -NR"-;
G₇, G₈, G₉ and G₁₀ are independently selected from a chemical bond and C₁₋₁₂ alkylene, which is optionally substituted with 1, 2, 3, 4, 5 or 6 R;
G₇ and G₈ have a total length of 4, 5, 6, 7, 8, 9, 10, 11 or 12 carbon atoms;
G₉ and G₁₀ have a total length of 4, 5, 6, 7, 8, 9, 10, 11 or 12 carbon atoms;
R' is independently selected from H, C₁₋₂₀ alkyl, -Lₐ-ORₐ and -Lₐ-NRₐR'ₐ.

In another more specific embodiment, L₁ and L₂ are independently selected from -(CHR)₂-, -CH=CH-, -C=C- and -NR"-;
G₇ and G₉ are independently selected from a chemical bond and C₁₋₆ alkylene;
G₈ and G₁₀ are independently selected from C₁₋₁₀ alkylene;
G₇ and G₈ have a total length of 4, 5, 6, 7, 8, 9 or 10 carbon atoms;
G₉ and G₁₀ have a total length of 4, 5, 6, 7, 8, 9 or 10 carbon atoms;
1, 2 or 3 methylenes in G₇, G₈, G₉ or G₁₀ are optionally and independently substituted with 1 R.

In another more specific embodiment, L₁ and L₂ are independently selected from -(CHR)₂-, -CH=CH-, -C=C- and -NR"-;
G₇ and G₉ are independently selected from a chemical bond and C₁₋₅ alkylene, alternatively selected from a chemical bond and linear C₁₋₅ alkylene;
G₈ and G₁₀ are independently selected from C₁₋₈ alkylene, alternatively selected from linear C₁₋₈ alkylene;
G₇ and G₈ have a total length of 4, 5, 6, 7, 8, 9, or 10 carbon atoms, alternatively 6, 7, 8, 9 or 10 carbon atoms;
G₉ and G₁₀ have a total length of 4, 5, 6, 7, 8, 9, or 10 carbon atoms, alternatively 6, 7, 8, 9 or 10 carbon atoms;
1 or 2 methylenes in G₇, G₈, G₉ or G₁₀ are optionally and independently substituted with 1 R.

Optionally, the methylenes attached to M₁ and M₂ are not substituted with R.

In another more specific embodiment, L₁ and L₂ are independently selected from -(CHR)₂-, -CH=CH-, -C=C- and -NR"-;
G₇ and G₉ are independently selected from a chemical bond, -CH₂-, -(CH₂)₂-, -(CH₂)₄- and -(CH₂)₅-;
G₈ and G₁₀ are independently selected from -(CH₂)₂-, -(CH₂)₄-, -(CH₂)₆-, -(CH₂)₇- and -(CH₂)₈-;
G₇ and G₈ have a total length of 4, 5, 6, 7, 8, 9, or 10 carbon atoms, alternatively 6, 7, 8, 9 or 10 carbon atoms;
G₉ and G₁₀ have a total length of 4, 5, 6, 7, 8, 9, or 10 carbon atoms, alternatively 6, 7, 8, 9 or 10 carbon atoms;
1 or 2 methylenes in G₇, G₈, G₉ or G₁₀ are optionally and independently substituted with 1 R.

Optionally, the methylenes attached to M₁ and M₂ are not substituted with R.

In another more specific embodiment, -G₇-L₁-G₈-H or -G₉-L₂-G₁₀-H is independently selected from: -(CH₂)₅CH₃, -(CH₂)₆CH₃, -(CH₂)₇CH₃, -(CH₂)₈CH₃, -(CH₂)₉CH₃, -(CH₂)₁₀CH₃, -(CH₂)₁₁CH₃, -CH₂-C≡C-(CH₂)₅CH₃, -CH₂-C≡C-(CH₂)₆CH₃, -(CH₂)₂-C≡C-(CH₂)₅CH₃, -(CH₂)₄-C≡C-(CH₂)₃CH₃, -CH₂-CH=CH-(CH₂)₅CH₃, -CH₂-CH=CH-(CH₂)₆CH₃, -(CH₂)₂-CH=CH-(CH₂)₅CH₃, -(CH₂)₄-CH=CH-(CH₂)₃CH₃, -(CH₂)₅-CH=CH-CH₂CH₃,

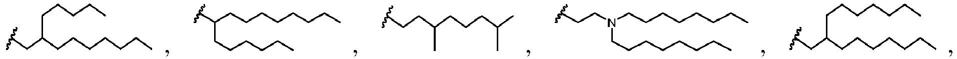

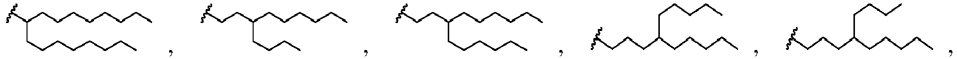

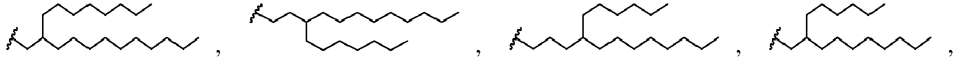

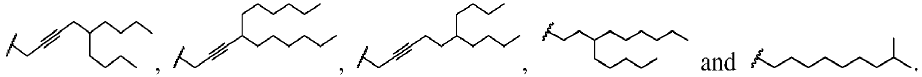

### R^{s}

In one embodiment, R^{s} is H; in another embodiment, R^{s} is C₁₋₁₄ alkyl; in another embodiment, R^{s} is -L_{d}-OR_{d}; in another embodiment, R^{s} is -L_{d}-SR_{d}; in another embodiment, R^{s} is -L_{d}-NR_{d}R'_{d}; in another embodiment, R^{s} is C₁₋₁₀ alkyl; in another embodiment, R^{s} is C₁₋₆ alkyl.

In a more specific embodiment, R^{s} is selected from H, C₁₋₁₀ alkyl, -L_{d}-OR_{d} and -L_{d}-NR_{d}R'_{d}; in another more specific embodiment, R^{s} is H or C₁₋₆ alkyl.

### R

In one embodiment, R is H; in another embodiment, R is C₁₋₂₀ alkyl; in another embodiment, R is -Lₐ-ORₐ; in another embodiment, R is -Lₐ-SRₐ; in another embodiment, R is -Lₐ-NRₐR'ₐ; in another embodiment, R is C₁₋₁₀ alkyl; in another embodiment, R is C₁₋₈ alkyl; in another embodiment, R is linear C₁₋₈ alkyl.

In a more specific embodiment, R is selected from H, Me, -(CH₂)₃CH₃, -(CH₂)₄CH₃, -(CH₂)₅CH₃, -(CH₂)₆CH₃ and -(CH₂)₇CH₃.

### R"

In one embodiment, R" is H; in another embodiment, R" is C₁₋₂₀ alkyl; in another embodiment, R" is C₁₋₁₄ alkyl; in another embodiment, R" is C₁₋₁₀ alkyl; in another embodiment, R" is C₇₋₉ alkyl; in another embodiment, R" is -(CH₂)₇CH₃.

### Lₐ and Lₑ

In one embodiment, Lₐ and Lₑ are independently a chemical bond; in another embodiment, Lₐ and Lₑ are independently C₁₋₂₀ alkylene; in another embodiment, Lₐ and Lₑ are independently C₁₋₁₄ alkylene; in another embodiment, Lₐ and Lₑ are independently C₁₋₁₀ alkylene.

### L_{b} and L_{f}

In one embodiment, L_{b} and L_{f} are independently a chemical bond; in another embodiment, L_{b} and L_{f} are independently C₁₋₁₀ alkylene; in another embodiment, L_{b} and L_{f} are independently C₁₋₆ alkylene.

### L_{c}

In one embodiment, L_{c} is a chemical bond; in another embodiment, L_{c} is C₁₋₈ alkylene; in another embodiment, L_{c} is C₁₋₆ alkylene.

### L_{d}

In one embodiment, L_{d} is a chemical bond; in another embodiment, L_{d} is C₁₋₁₄ alkylene; in another embodiment, L_{d} is C₁₋₁₀ alkylene.

### Rₐ and R'ₐ

In one embodiment, Rₐ is H; in another embodiment, Rₐ is C₁₋₂₀ alkyl; in another embodiment, Rₐ is 3- to 14-membered cycloalkyl; in another embodiment, Rₐ is 3- to 14-membered heterocyclyl; in another embodiment, Rₐ is C₁₋₁₄ alkyl; in another embodiment, Rₐ is C₁₋₁₀ alkyl; in another embodiment, Rₐ is C₈₋₁₀ alkyl; in another embodiment, Rₐ is linear C₈₋₁₀ alkyl; in another embodiment, Rₐ is -(CH₂)₈CH₃; in another embodiment, Rₐ is optionally substituted with one or more substituents as follows: H, C₁₋₂₀ alkyl, -Lₑ-ORₑ, -Lₑ-SRₑ or -Lₑ-NRₑR'ₑ.

In one embodiment, R'ₐ is H; in another embodiment, R'ₐ is C₁₋₂₀ alkyl; in another embodiment, R'ₐ is 3- to 14-membered cycloalkyl; in another embodiment, R'ₐ is 3- to 14-membered heterocyclyl; in another embodiment, R'ₐ is C₁₋₁₄ alkyl; in another embodiment, R'ₐ is C₁₋₁₀ alkyl; in another embodiment, R'ₐ is C₈₋₁₀ alkyl; in another embodiment, R'ₐ is linear C₈₋₁₀ alkyl; in another embodiment, R'ₐ is -(CH₂)₈CH₃; in another embodiment, R'ₐ is optionally substituted with one or more substituents as follows: H, C₁₋₂₀ alkyl, -Lₑ-ORₑ, -Lₑ-SRₑ or -Lₑ₋NRₑR'ₑ.

### R_{b} and R'_{b}

In one embodiment, R_{b} is H; in another embodiment, R_{b} is C₁₋₁₀ alkyl; in another embodiment, R_{b} is 3- to 14-membered cycloalkyl; in another embodiment, R_{b} is 3- to 14-membered heterocyclyl; in another embodiment, R_{b} is C₁₋₆ alkyl; in another embodiment, R_{b} is 3- to 10-membered cycloalkyl; in another embodiment, R_{b} is 3- to 10-membered heterocyclyl; in another embodiment, R_{b} is optionally substituted with one or more substituents as follows: H, C₁₋₁₀ alkyl, -L_{f}-OR_{f}, -L_{f}-SR_{f} or -L_{f}-NR_{f}R'_{f}.

In one embodiment, R'_{b} is H; in another embodiment, R'_{b} is C₁₋₁₀ alkyl; in another embodiment, R'_{b} is 3- to 14-membered cycloalkyl; in another embodiment, R'_{b} is 3- to 14-membered heterocyclyl; in another embodiment, R'_{b} is C₁₋₆ alkyl; in another embodiment, R'_{b} is 3- to 10-membered cycloalkyl; in another embodiment, R'_{b} is 3- to 10-membered heterocyclyl; in another embodiment, R'_{b} is optionally substituted with one or more substituents as follows: H, C₁₋₁₀ alkyl, -L_{f}-OR_{f}, -L_{f}-SR_{f} or -L_{f}-NR_{f}R'_{f}.

### R_{c} and R'_{c}

In one embodiment, R_{c} is H; in another embodiment, R_{c} is C₁₋₈ alkyl; in another embodiment, R_{c} is C₁₋₆ alkyl.

In one embodiment, R'_{c} is H; in another embodiment, R'_{c} is C₁₋₈ alkyl; in another embodiment, R'_{c} is C₁₋₆ alkyl.

### R_{d} and R'_{d}

In one embodiment, R_{d} is H; in another embodiment, R_{d} is C₁₋₁₄ alkyl; in another embodiment, R_{d} is C₁₋₁₀ alkyl.

In one embodiment, R'_{d} is H; in another embodiment, R'_{d} is C₁₋₁₄ alkyl; in another embodiment, R'_{d} is C₁₋₁₀ alkyl.

### Rₑ and R'ₑ

In one embodiment, Rₑ is H; in another embodiment, Rₑ is C₁₋₂₀ alkyl.

In one embodiment, R'ₑ is H; in another embodiment, R'ₑ is C₁₋₂₀ alkyl.

### R_{f} and R'_{f}

In one embodiment, R_{f} is H; in another embodiment, R_{f} is C₁₋₁₀ alkyl.

In one embodiment, R'_{f} is H; in another embodiment, R'_{f} is C₁₋₁₀ alkyl.

Any technical solution in the above specific embodiments or any combination thereof may be combined with any technical solution in other specific embodiments or any combination thereof. For example, any technical solution of Q, or any combination thereof, may be combined with any technical solution of j, W, R₀', k, M₁, M₂, G₅, R₀', R**, G₁, G₂, G₃, G₄, R₁, R₂, R₃, R₄, R₅, R₆, R₇, R₈, R, R", R^{s}, Ar₂, Lₐ, Lₑ, L_{b}, L_{f}, L_{c}, L_{d}, Rₐ, R'ₐ, R_{b}, R'_{b}, R_{c}, R'_{c}, R_{d}, R'_{d}, Rₑ, R'ₑ, R_{f} and R'_{f} or any combination thereof. The present disclosure is intended to include all of these combinations of technical solutions, which are not listed one by one due to space limitation.

In a more specific embodiment, the present disclosure provides the above lipid nanoparticle, wherein the compound of formula (IV') has the following structural formula: 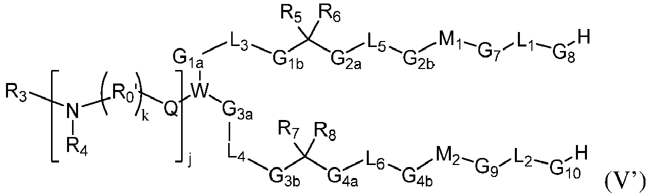 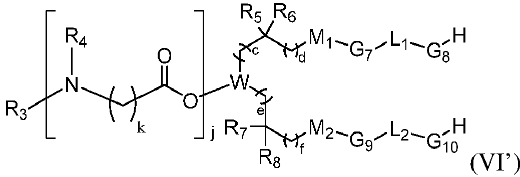 or 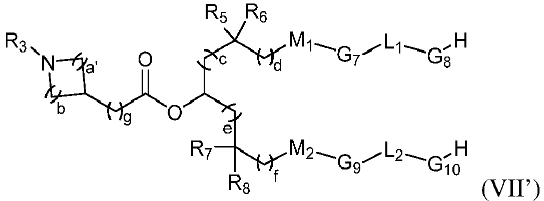 wherein
k is selected from 0, 1, 2, 3, 4, 5 and 6, alternatively selected from 0, 1, 2, 3, 4 and 5, alternatively selected from 1, 2, 3, 4 and 5, alternatively selected from 3, 4 and 5, still alternatively 3 and 4;
a' and b are independently selected from 0, 1, 2, 3, 4 and 5, alternatively selected from 0, 1, 2, 3 and 4, alternatively 2, and a' and b are not 0 at the same time;
g is selected from 0, 1, 2, 3, 4 and 5, alternatively 0, 1 and 2, alternatively 0 and 1;
a'+g is equal to 0, 1, 2, 3, 4 or 5, alternatively a'+g is equal to 2, 3 or 4, alternatively a'+g is equal to 2 or 3;
c, d, e and f are each independently selected from 0, 1, 2, 3, 4, 5, 6 and 7;
c+d is equal to 2, 3, 4, 5, 6, 7, 8 or 9, alternatively c+d is equal to 4, 5, 6 or 7, alternatively c+d is equal to 4, 5 or 6, alternatively c+d is equal to 5 or 6;
e+f is equal to 2, 3, 4, 5, 6, 7, 8 or 9, alternatively e+f is equal to 4, 5, 6 or 7, alternatively c+d is equal to 4, 5 or 6, alternatively e+f is equal to 5 or 6;
j is 0 or 1;
the methylene in 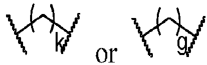 is optionally substituted with 1, 2, 3, 4 or 5 C₁₋₆ alkyl;
W is CH or N;
when W is N, j is 0;
the rest of the groups are as defined above.

Alternatively, when W is N, in formula (III) or formula (IV') or formula (V') or formula (VI'): R₃ is C₁₋₈ alkyl or -C₁₋₈ alkylene-OH, alternatively C₁₋₆ alkyl or -C₁₋₆ alkylene-OH, still alternatively -C₁₋₆ alkylene-OH.

In a more specific embodiment, the present disclosure provides the above lipid nanoparticle, wherein in the compound of formula (V'),
Q is selected from -C(O)O-, -O-, -SC(O)O-, -OC(O)NR_{b}-, -NR_{b}C(O)NR_{b}-, -OC(O)S-, -OC(O)O-, -NR_{b}C(O)O-, -OC(O)-, -SC(O)-, -C(O)S-, -NR_{b}-, -C(O)NR_{b}-, -NR_{b}C(O)-, -NR_{b}C(O)S-, -SC(O)NR_{b}-, -C(O)-, -OC(S)-, -C(S)O-, -OC(S)NR_{b}-, -NR_{b}C(S)O-, -S-S- and -S(O)₀₋₂-;
R₀' is independently methylene optionally substituted with 1 or 2 R**;
k is selected from 1, 2, 3, 4 and 5;
R** is independently selected from H, C₁₋₆ alkyl, -L_{c}-OR_{c} and -L_{c}-NR_{c}R'_{c};
j is 0 or 1;
W is CH or N; when W is N, j is 0;
one of L₃ and L₅, or one of L₄ and L₆ is selected from -(CR^{s}R^{s}')₂-, -CH=CH-, and -C=C-, and the other is a chemical bond;
G₁ₐ, G_{1b}, G₂ₐ, G_{2b}, G₃ₐ, G_{3b}, G₄ₐ and G_{4b} are independently a chemical bond or C₁₋₇ alkylene, which is optionally substituted with 1, 2, 3, 4 or 5 R^{s};
G₁ₐ, G_{1b}, G₂ₐ and G_{2b} have a total length of 1, 2, 3, 4, 5, 6 or 7 carbon atoms;
G₃ₐ, G_{3b}, G₄ₐ and G_{4b} have a total length of 1, 2, 3, 4, 5, 6 or 7 carbon atoms;
R₃ and R₄ are independently selected from H, C₁₋₆ alkyl, C₁₋₆ haloalkyl, 3- to 10-membered cycloalkyl and 3- to 10-membered heterocyclyl, which is optionally substituted with 1, 2, 3, 4 or 5 R*;
or R₃ and R₄ are taken together with the N atom to which they are attached to form 3-to 10-membered heterocyclyl, which is optionally substituted with 1, 2, 3, 4 or 5 R*;
or R₄ is taken together with the N atom to which it is attached, one R₀' of the (R₀')ₖ, and the attached atoms between them to form 3- to 10-membered heterocyclyl, which is optionally substituted with 1, 2, 3 or 4 R* ;
R* is independently selected from H, halogen, cyano, C₁₋₆ alkyl, C₁₋₆ haloalkyl, -L_{b}-OR_{b} and -L_{b}-NR_{b}R'_{b};
R₅, R₆, R₇ and R₈ are independently selected from H and C₁₋₆ alkyl, wherein the alkyl is optionally substituted with 1, 2, 3, 4 or 5 R*;
M₁ and M₂ are independently selected from -C(O)O-, -OC(O)-, -C(O)NRₐ-, -NRₐC(O)-, -SC(O)- and -C(O)S-;
L₁ and L₂ are independently selected from -(CRR')₂-, -CH=CH-, -C=C- and -NR"-;
G₇, G₈, G₉ and G₁₀ are independently selected from a chemical bond and C₁₋₁₂ alkylene, which is optionally substituted with 1, 2, 3, 4, 5 or 6 R;
G₇ and G₈ have a total length of 4, 5, 6, 7, 8, 9, 10, 11 or 12 carbon atoms;
G₉ and G₁₀ have a total length of 4, 5, 6, 7, 8, 9, 10, 11 or 12 carbon atoms;
R^{s} and R^{s}' are independently selected from H, C₁₋₁₀ alkyl, -L_{d}-OR_{d} and -L_{d}-NR_{d}R'_{d};
R and R' are independently selected from H, C₁₋₁₄ alkyl, -Lₐ-ORₐ and -Lₐ-NRₐR'ₐ;
R" is independently selected from H and C₁₋₁₄ alkyl;
Lₐ is independently selected from a chemical bond and C₁₋₁₄ alkylene;
L_{b} is independently selected from a chemical bond and C₁₋₆ alkylene;
L_{c} is independently selected from a chemical bond and C₁₋₆ alkylene;
L_{d} is independently selected from a chemical bond and C₁₋₁₀ alkylene;
Rₐ and R'ₐ are independently selected from H, C₁₋₁₄ alkyl, 3- to 10-membered cycloalkyl and 3- to 10-membered heterocyclyl;
R_{b} and R'_{b} are independently selected from H, C₁₋₆ alkyl, 3- to 10-membered cycloalkyl and 3- to 10-membered heterocyclyl;
R_{c} and R'_{c} are independently selected from H and C₁₋₆ alkyl;
R_{d} and R'_{d} are independently selected from H and C₁₋₁₀ alkyl.

In a more specific embodiment, the present disclosure provides the above lipid nanoparticle, wherein in the compound of formula (V'),
Q is selected from -C(O)O-, -O-, -SC(O)O-, -OC(O)NH-, -NHC(O)NH-, -OC(O)S-, -OC(O)O-, -NHC(O)O-, -OC(O)-, -SC(O)-, -C(O)S-, -NH-, -C(O)NH-, -NHC(O)-, -NHC(O)S-, -SC(O)NH-, -C(O)-, -OC(S)-, -C(S)O-, -OC(S)NH- and -NHC(S)O-;
R₀' is independently methylene optionally substituted with 1 or 2 R**;
k is selected from 1, 2, 3, 4 and 5;
R** is independently selected from H and C₁₋₆ alkyl;
j is 0 or 1;
W is CH or N; when W is N, j is 0;
one of L₃ and L₅, or one of L₄ and L₆ is selected from -(CHR^{s})₂-, -CH=CH- and -C=C-, and the other is a chemical bond;
G₁ₐ and G₃ₐ are independently selected from a chemical bond and C₁₋₇ alkylene;
G_{1b} and G_{3b} are independently selected from a chemical bond and C₁₋₃ alkylene;
G₂ₐ and G₄ₐ are independently selected from a chemical bond and C₁₋₃ alkylene;
G_{2b} and G_{4b} are independently selected from a chemical bond and C₁₋₄ alkylene;
G₁ₐ, G_{1b}, G₂ₐ and G_{2b} have a total length of 1, 2, 3, 4, 5, 6 or 7 carbon atoms;
G₃ₐ, G_{3b}, G₄ₐ and G_{4b} have a total length of 1, 2, 3, 4, 5, 6 or 7 carbon atoms;
R₃ and R₄ are independently selected from H, C₁₋₆ alkyl, C₁₋₆ haloalkyl, 3- to 7-membered cycloalkyl and 3- to 7-membered heterocyclyl, which is optionally substituted with 1, 2, 3, 4 or 5 R*;
or R₃ and R₄ are taken together with the N atom to which they are attached to form 3-to 7-membered heterocyclyl, which is optionally substituted with 1, 2, 3, 4 or 5 R*;
or R₄ is taken together with the N atom to which it is attached, one R₀' of the (R₀')ₖ, and the attached atoms between them to form 3- to 7-membered heterocyclyl, which is optionally substituted with 1, 2, 3, 4 or 5 R*;
R* is independently selected from H, halogen, cyano, C₁₋₆ alkyl, C₁₋₆ haloalkyl, -L_{b}-OR_{b} and -L_{b}-NR_{b}R'_{b};
R₅, R₆, R₇ and R₈ are independently selected from H and C₁₋₆ alkyl;
M₁ and M₂ are independently selected from -C(O)O-, -OC(O)-, -C(O)NRₐ-, -NRₐC(O)-, -SC(O)- and -C(O)S-;
L₁ and L₂ are independently selected from -(CHR)₂-, -CH=CH-, -C=C- and -NR"-;
G₇ and G₉ are independently selected from a chemical bond and C₁₋₆ alkylene;
G₈ and G₁₀ are independently selected from C₁₋₁₀ alkylene;
G₇ and G₈ have a total length of 4, 5, 6, 7, 8, 9 or 10 carbon atoms;
G₉ and G₁₀ have a total length of 4, 5, 6, 7, 8, 9 or 10 carbon atoms;
1, 2 or 3 methylenes in G₇, G₈, G₉ or G₁₀ are optionally and independently substituted with 1 R;
R^{s} is independently selected from H and C₁₋₆ alkyl;
R is independently selected from H and C₁₋₁₀ alkyl;
R" is independently selected from H and C₁₋₁₀ alkyl;
L_{b} is independently selected from a chemical bond and C₁₋₆ alkylene;
Rₐ is independently selected from H and C₁₋₁₀ alkyl;
R_{b} and R'_{b} are independently selected from H and C₁₋₆ alkyl.

In a more specific embodiment, the present disclosure provides the above lipid nanoparticle, wherein in the compound of formula (V'),
Q is selected from -C(O)O-, -O-, -SC(O)O-, -OC(O)NH-, -NHC(O)NH-, -OC(O)S-, -OC(O)O- and -NHC(O)O-;
R₀' is methylene;
k is selected from 1, 2, 3, 4 and 5, alternatively selected from 3, 4 and 5, still alternatively 3 and 4;
j is 0 or 1;
W is CH or N; when W is N, j is 0; alternatively, when W is CH, j is 1;
one of L₃ and L₅, or one of L₄ and L₆ is selected from -(CH₂)₂-, -CH=CH-, and -C=C-, and the other is a chemical bond;
G₁ₐ and G₃ₐ are independently selected from a chemical bond, -CH₂-, -(CH₂)₂-, -(CH₂)₃-, -(CH₂)₄-, -(CH₂)₅- and -(CH₂)₆-;
G_{1b} and G_{3b} is a chemical bond;
G₂ₐ and G₄ₐ is a chemical bond;
G_{2b} and G_{4b} are independently selected from a chemical bond, -CH₂-, -(CH₂)₂- and -(CH₂)₃-;
G₁ₐ, G_{1b}, G₂ₐ and G_{2b} have a total length of 1, 2, 3, 4, 5 or 6 carbon atoms;
G₃ₐ, G_{3b}, G₄ₐ and G_{4b} have a total length of 1, 2, 3, 4, 5 or 6 carbon atoms;
R₃ and R₄ are independently selected from C₁₋₆ alkyl, which is optionally substituted with 1, 2 or 3 R*;
or R₃ and R₄ are taken together with the N atom to which they are attached to form 5-to 7-membered heterocyclyl, which is optionally substituted with 1, 2, 3, 4 or 5 R*;
or R₄ is taken together with the N atom to which it is attached, one R₀' of the (R₀')ₖ, and the attached atoms between them to form 6-membered heterocyclyl, which is optionally substituted with 1, 2, 3, 4 or 5 R*;
R* is independently selected from H, C₁₋₆ alkyl, C₁₋₆ haloalkyl and -OR_{b};
R₅, R₆, R₇ and R₈ are independently selected from H and C₁₋₃ alkyl;
M₁ and M₂ are independently selected from -C(O)O-, -OC(O)-, -C(O)NRₐ-, -NRₐC(O)-, -SC(O)- and -C(O)S-, alternatively -C(O)O-, -OC(O)-, -SC(O)- and -C(O)S-;
L₁ and L₂ are independently selected from -(CHR)₂-, -CH=CH-, -C=C- and -NR"-;
G₇ and G₉ are independently selected from a chemical bond and C₁₋₅ alkylene, alternatively selected from a chemical bond and linear C₁₋₅ alkylene;
G₈ and G₁₀ are independently selected from C₁₋₈ alkylene, alternatively selected from linear C₁₋₈ alkylene;
G₇ and G₈ have a total length of 4, 5, 6, 7, 8, 9, or 10 carbon atoms;
G₉ and G₁₀ have a total length of 4, 5, 6, 7, 8, 9, or 10 carbon atoms;
1 or 2 methylenes in G₇, G₈, G₉ or G₁₀ are optionally and independently substituted with 1 R;
R is independently selected from H and C₁₋₈ alkyl, alternatively selected from H and linear C₁₋₈ alkyl;
R" is independently selected from H and C₇₋₉ alkyl, alternatively selected from H and linear C₇₋₉ alkyl;
Rₐ is independently selected from H and C₈₋₁₀ alkyl, alternatively selected from H and linear C₈₋₁₀ alkyl;
R_{b} is independently selected from H and C₁₋₆ alkyl, alternatively H.

In a more specific embodiment, the present disclosure provides the above lipid nanoparticle, wherein in the compound of formula (V'),
Q is selected from -C(O)O-, -O-, -SC(O)O-, -OC(O)NH-, -NHC(O)NH-, -OC(O)S-, -OC(O)O- and -NHC(O)O-;
R₀' is methylene;
k is selected from 1, 2, 3, 4 and 5, alternatively selected from 3, 4 and 5, still alternatively 3 and 4;
j is 0 or 1;
W is CH or N; when W is N, j is 0; when W is CH, j is 1;
-G₁ₐ₋L₃-G_{1b}- or -G₃ₐ-L₄-G_{3b}- is independently selected from the following groups: -(CH₂)₂-, -(CH₂)₃-, -(CH₂)₄-, -(CH₂)₅-, -(CH₂)₆-, -(CH₂)₇-, -(CH₂)₈-, -(CH₂)₃-CH=CH-, -(CH₂)₃-C≡C-, -(CH₂)₂-CH=CH- and -(CH₂)₂-C≡C-;
-G₂ₐ-L₃-G_{2b}- or -G₄ₐ-L₆-G_{4b}- is independently selected from the following groups: a chemical bond, -CH₂-, -(CH₂)₂-, -(CH₂)₃-, -CH=CH-CH₂-, -C=C- and -C=C-CH₂-; 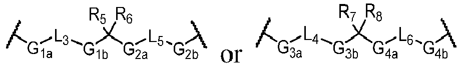
has a total length of 4, 5, 6, 7, 8 or 9 carbon atoms;
R₃ is Me, -CH₂CH₃, -CH₂CH₂OH, -CH₂CH₂CH₂CH₂OH or -CH(CH₃)₂;
R₄ is Me;
or R₃ and R₄ are taken together with the N atom to which they are attached to form 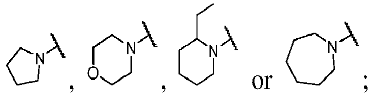
or R₄ is taken together with the N atom to which it is attached, one R₀' of the (R₀')ₖ, and the attached atoms between them to form 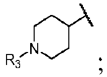
R₅, R₆, R₇ and R₈ is H or Me;
M₁ and M₂ are independently selected from -C(O)O-, -OC(O)-, -C(O)NRₐ-, -NRₐC(O)-, -SC(O)- and -C(O)S-, alternatively -C(O)O-, -OC(O)-, -SC(O)- and -C(O)S-;
L₁ and L₂ are independently selected from -(CHR)₂-, -CH=CH-, -C=C- and -NR"-;
G₇ and G₉ are independently selected from a chemical bond, -CH₂-, -(CH₂)₂-, -(CH₂)₄- and -(CH₂)₅-;
G₈ and G₁₀ are independently selected from -(CH₂)₂-, -(CH₂)₄-, -(CH₂)₆-, -(CH₂)₇- and -(CH₂)₈-;
G₇ and G₈ have a total length of 4, 5, 6, 7, 8, 9, or 10 carbon atoms;
G₉ and G₁₀ have a total length of 4, 5, 6, 7, 8, 9, or 10 carbon atoms;
1 or 2 methylenes in G₇, G₈, G₉ or G₁₀ are optionally and independently substituted with 1 R;
R is independently selected from H, Me, -(CH₂)₃CH₃, -(CH₂)₄CH₃, -(CH₂)₅CH₃, -(CH₂)₆CH₃ and -(CH₂)₇CH₃;
R" is -(CH₂)₇CH₃;
Rₐ is independently selected from H and -(CH₂)₈CH₃.

Optionally, 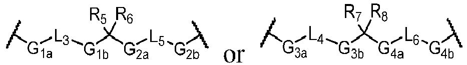 is independently selected from the following groups:
-(CH₂)₃-C(CH₃)₂-, -(CH₂)₄-C(CH₃)₂-, -(CH₂)₅-C(CH₃)₂-, -(CH₂)₆-C(CH₃)₂-, -(CH₂)₇-C(CH₃)₂-, -(CH₂)₈-C(CH₃)₂-, -(CH₂)₃-CH=CH-C(CH₃)₂-, -(CH₂)₃-C≡C-C(CH₃)₂-, -(CH₂)₄-C(CH₃)₂-CH₂-, -(CH₂)₃-C(CH₃)₂-(CH₂)₂-, -(CH₂)₂-C(CH₃)₂-(CH₂)₃-, -(CH₂)₂-CH=CH-C(CH₃)₂-CH₂-, -(CH₂)₂-C(CH₃)₂-C≡C-CH₂-, -(CH₂)₂-C(CH₃)₂-CH=CH-CH₂-, -(CH₂)₂-C≡C-C(CH₃)₂-CH₂- and -(CH₂)₃-C(CH₃)₂-C≡C-;
-G₇-L₁-G₈-H or -G₉-L₂-G₁₀-H is independently selected from the following groups: -(CH₂)₅CH₃, -(CH₂)₆CH₃, -(CH₂)₇CH₃, -(CH₂)₈CH₃, -(CH₂)₉CH₃, -(CH₂)₁₀CH₃, -(CH₂)₁₁CH₃, -CH₂-C≡C-(CH₂)₅CH₃, -CH₂-C≡C-(CH₂)₆CH₃, -(CH₂)₂-C≡C-(CH₂)₅CH₃, -(CH₂)₄-C≡C-(CH₂)₃CH₃, -CH₂-CH=CH-(CH₂)₅CH₃, -CH₂-CH=CH-(CH₂)₆CH₃, -(CH₂)₂-CH=CH-(CH₂)₅CH₃, -(CH₂)₄-CH=CH-(CH₂)₃CH₃, -(CH₂)₅-CH=CH-CH₂CH₃,

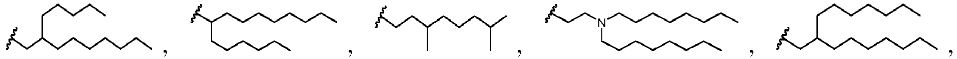

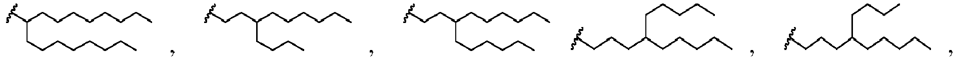

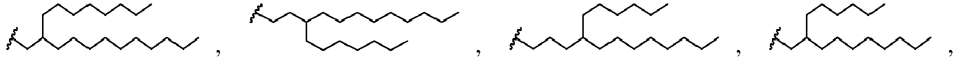

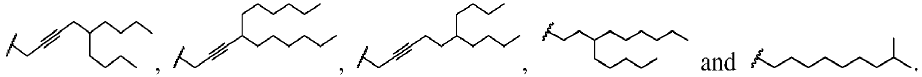

In a more specific embodiment, the present disclosure provides the above lipid nanoparticle, wherein in the compound of formula (VI') or formula (VII'):
k is selected from 0, 1, 2, 3, 4 and 5, alternatively selected from 1, 2, 3, 4 and 5, alternatively selected from 3, 4 and 5, still alternatively 3 and 4;
a' and b are independently selected from 0, 1, 2, 3, 4 and 5, alternatively selected from 0, 1, 2, 3 and 4, alternatively 2, and a' and b are not 0 at the same time;
g is selected from 0, 1, 2, 3, 4 and 5, alternatively 0, 1 and 2, alternatively 0 and 1;
a'+g is equal to 0, 1, 2, 3, 4 or 5, alternatively a'+g is equal to 2, 3 or 4, alternatively a'+g is equal to 2 or 3;
the methylene in 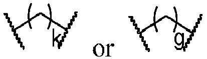 is optionally substituted with 1, 2, 3, 4 or 5 C₁₋₆ alkyl;
W is CH or N;

(i) when W is N, in the formula (VI'):
   j is 0;
   c, d, e and f are each independently selected from 0, 1, 2, 3, 4, 5, 6 and 7;
   c+d is equal to 4, 5, 6 or 7, alternatively c+d is equal to 5 or 6; e+f is equal to 4, 5, 6 or 7, alternatively e+f is equal to 5 or 6;
   R₃ is C₁₋₈ alkyl or -C₁₋₈ alkylene-OH, alternatively C₁₋₆ alkyl or -C₁₋₆ alkylene-OH, still alternatively -C₁₋₆ alkylene-OH;
   R₅, R₆, R₇ and R₈ are independently H or C₁₋₆ alkyl;
   M₁ and M₂ are independently selected from -C(O)O-, -OC(O)-, -SC(O)- and -C(O)S-, alternatively -C(O)O- and -OC(O)-;
   L₁ and L₂ are independently selected from -(CHR)₂-, -CH=CH- and -C=C-, alternatively -(CHR)₂-;
   G₇ and G₉ are independently selected from C₁₋₄ alkylene;
   G₈ and G₁₀ are independently selected from C₂₋₈ alkylene;
   G₇ and G₈ have a total length of 6, 7, 8 or 9 carbon atoms, alternatively 7, 8 or 9 carbon atoms;
   G₉ and G₁₀ have a total length of 6, 7, 8 or 9 carbon atoms, alternatively 7, 8 or 9 carbon atoms;
   1, 2, 3 or 4 methylenes in G₇, G₈, G₉ or G₁₀ are optionally and independently substituted with 1 R;
   R is independently selected from H and C₁₋₁₀ alkyl, alternatively H and C₁₋₈ alkyl;
(ii) in the formula (VII'), and in the formula (VI') wherein W is CH:
   j is 1;
   c, d, e and f are each independently selected from 0, 1, 2, 3, 4, 5 and 6;
   c+d is equal to 4, 5 or 6, and e+f is equal to 4, 5 or 6;
   R₃ and R₄ are independently selected from C₁₋₆ alkyl, which is optionally substituted with 1, 2 or 3 R*;
   R* is independently selected from H, C₁₋₆ alkyl and C₁₋₆ haloalkyl;
   R₅, R₆, R₇ and R₈ is independently H or C₁₋₆ alkyl, alternatively C₁₋₆ alkyl;
   M₁ and M₂ are independently selected from -C(O)O-, -OC(O)-, -SC(O)- and -C(O)S-, alternatively -C(O)O- and -C(O)S-;
   L₁ and L₂ are independently selected from -(CHR)₂-, -CH=CH- and -C=C-;
   G₇ and G₉ are independently selected from C₁₋₄ alkylene;
   G₈ and G₁₀ are independently selected from C₂₋₇ alkylene;
   G₇ and G₈ have a total length of 6, 7 or 8 carbon atoms;
   G₉ and G₁₀ have a total length of 6, 7 or 8 carbon atoms;
   1, 2 or 3 methylenes in G₇, G₈, G₉ or G₁₀ are optionally and independently substituted with 1 R;
   R is independently selected from H and C₁₋₁₀ alkyl, alternatively selected from H and C₁₋₇ alkyl;
   provided that, when L₁ is -C≡C-, G₇ is C₁₋₂ alkylene, and when L₂ is -C≡C-, G₉ is C₁₋₂ alkylene;
   alternatively, in the formula (VII'), L₁ and L₂ is -(CHR)₂-.

In a more specific embodiment, the present disclosure provides the above lipid nanoparticle, wherein the compound of formula (III) is selected from:

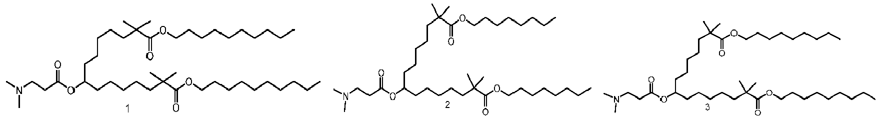

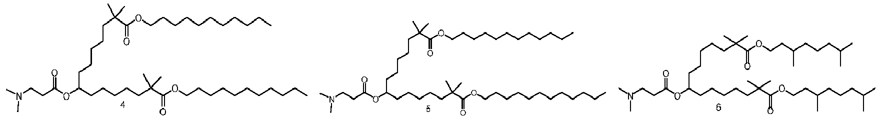

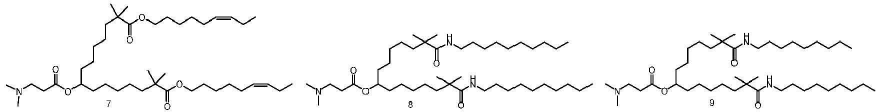

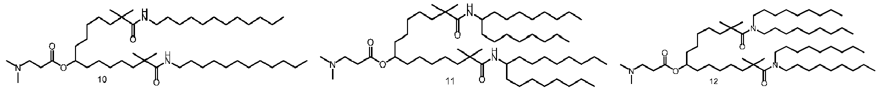

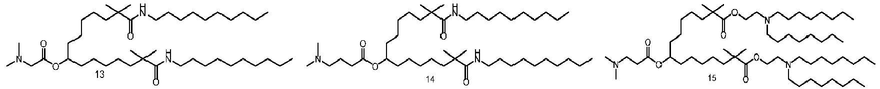

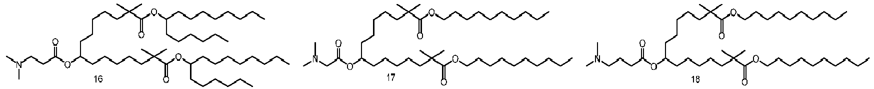

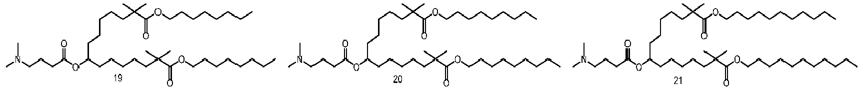

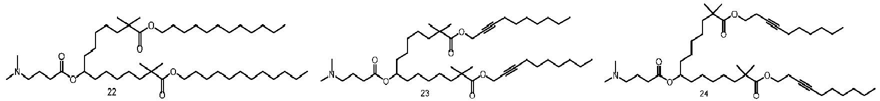

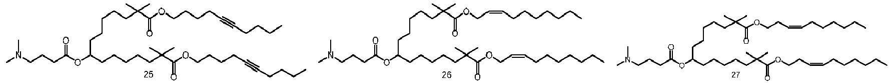

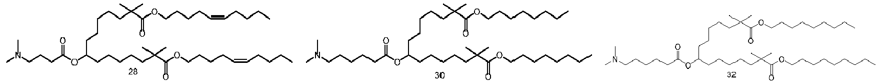

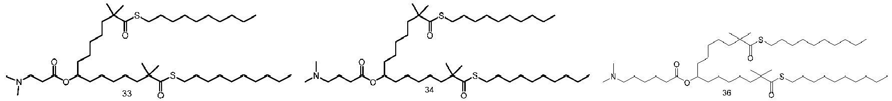

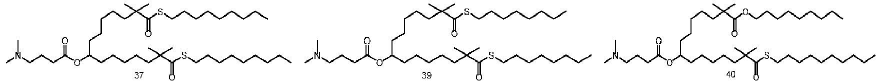

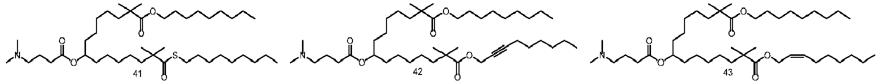

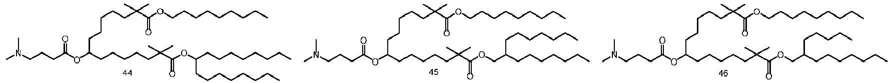

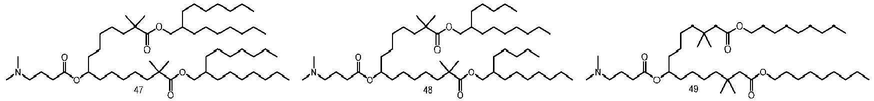

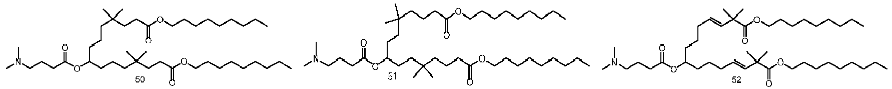

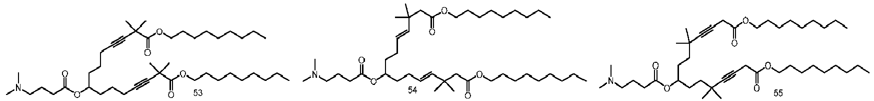

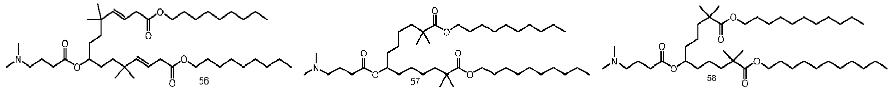

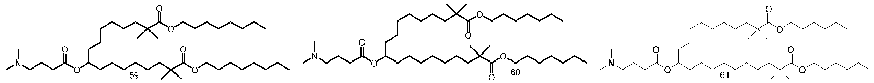

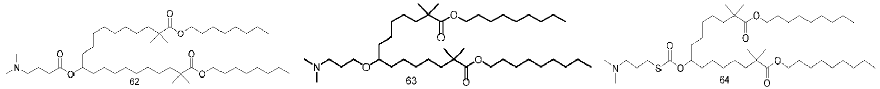

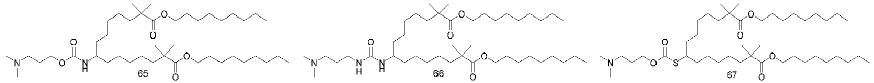

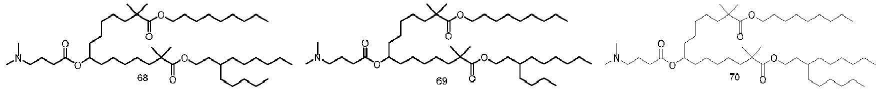

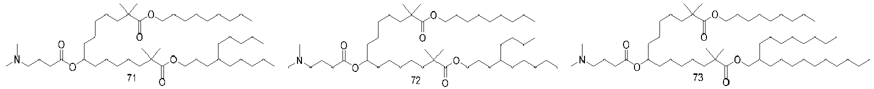

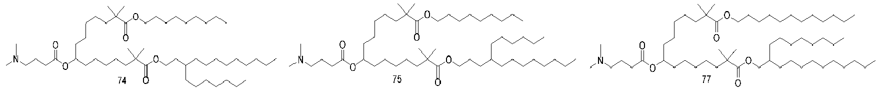

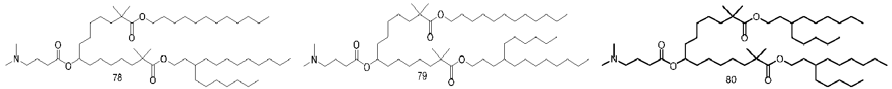

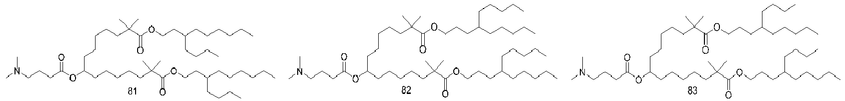

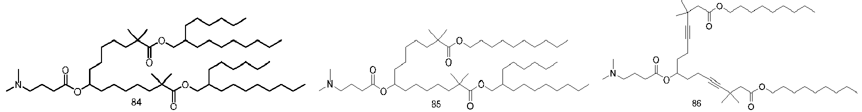

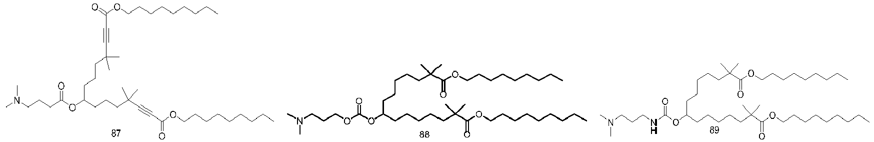

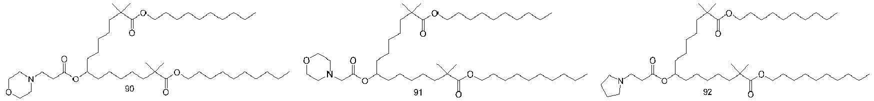

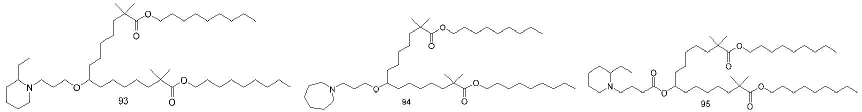

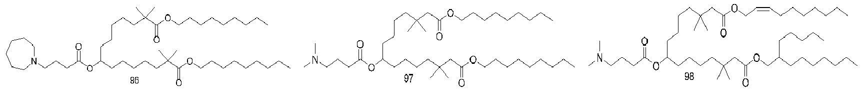

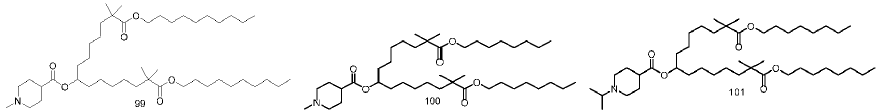

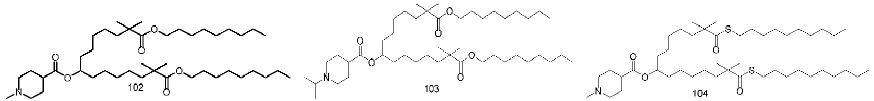

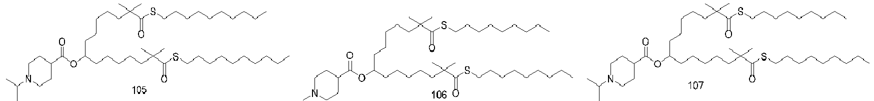

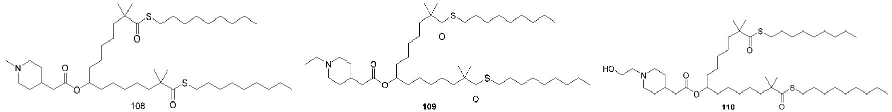

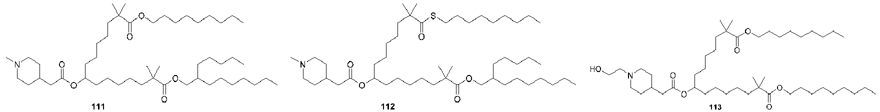

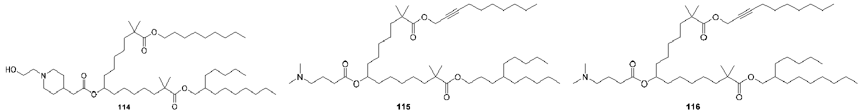

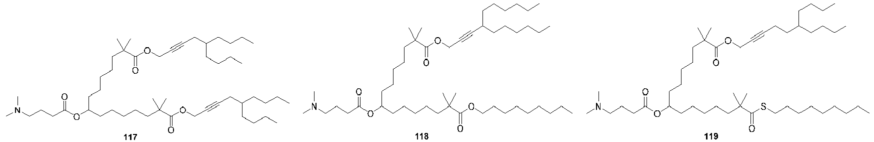

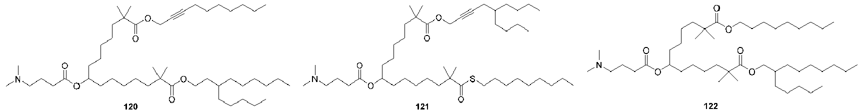

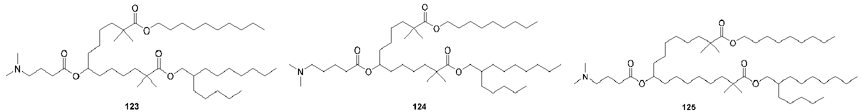

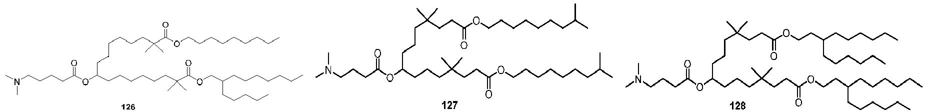

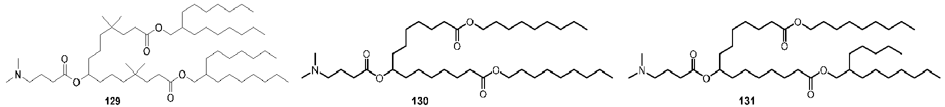

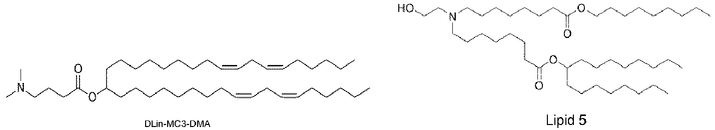

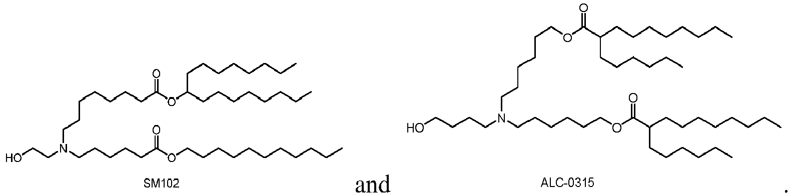

Alternatively, the compound of formula (IV) is selected from the following compounds:

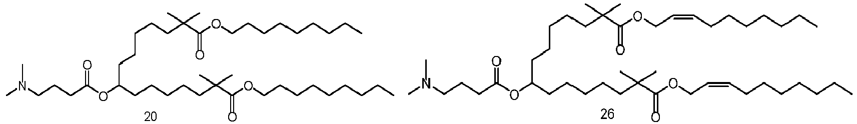

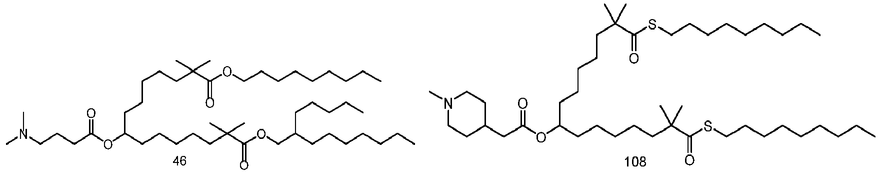

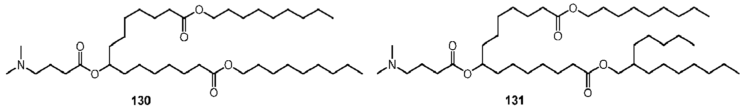

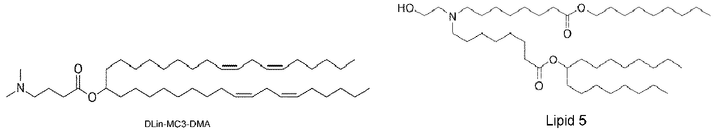

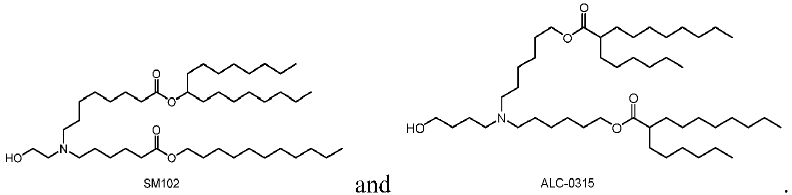

In a more specific embodiment, the present disclosure provides the above lipid nanoparticle, wherein the ionizable lipid is selected from one or more of: 1,2-dioleoyloxy-3-dimethylaminopropane (DODAP), 1,2-dioleyloxy-3-dimethylaminopropane (DODMA), 1,2-dilinoleyloxy-3-dimethylaminopropane (DLinDMA), 2,2-dilinoleoyl-4-(2-dimethylaminoethyl)-[1,3]-dioxolane (DLin-KC2-DMA), (dilinoleyl)methyl 4-(N,N-dimethylamino)butanoate (DLin-MC3-DMA), heptadecan-9-yl 8-[(2-hydroxyethyl)(6-oxo-6-undecyloxyhexyl)amino]octanoate (SM-102) and [(4-hydroxybutyl)azanediyl]bis(hexane-6,1-diyl)bis(2-hexyldecanoate) (ALC-0315); alternatively selected from (dilinoleyl)methyl 4-(N,N-dimethylamino)butanoate (DLin-MC3-DMA) and/or [(4-hydroxybutyl)azanediyl]bis(hexane-6,1-diyl)bis(2-hexyldecanoate) (ALC-0315).

In a more specific embodiment, the present disclosure provides the above lipid nanoparticle, wherein the molar percentage of the ionizable lipid is 30 mol%-80 mol%; alternatively 30 mol%-65 mol%; alternatively 35 mol%-65 mol%; alternatively 40 mol%-50 mol%; still alternatively 40 mol%, 46.3 mol%, 47.5 mol%, 49.5 mol% or 50 mol%.

In a more specific embodiment, the present disclosure provides the above lipid nanoparticle, wherein the structured lipid is selected from one or more of: cholesterol, sitosterol, coprosterol, fucosterol, brassicasterol, ergosterol, tomatine, ursolic acid, α-tocopherol, stigmasterol, avenasterol, ergocalciferol and campestero, alternatively selected from cholesterol and/or β-sitosterol, still alternatively cholesterol.

In a more specific embodiment, the present disclosure provides the above lipid nanoparticle, wherein the molar percentage of the structured lipid is 30 mol%-70 mol%; alternatively 30 mol%-65 mol%; alternatively 30 mol%-60 mol%; alternatively 38.5 mol%-53.5 mol%; alternatively 38.5 mol%-48.5 mol%; still alternatively 38.5 mol%, 40.5 mol%, 42.7 mol%, 46.5 mol%, 48.5 mol% or 53.5 mol%.

In a more specific embodiment, the present disclosure provides the above lipid nanoparticle, wherein the molar ratio of the structured lipid to the permanently cationic lipid is 1:1-20:1; alternatively 1.5:1-19:1.

In a more specific embodiment, the present disclosure provides the above lipid nanoparticle, wherein the polymer-conjugated lipid is pegylated lipid.

Optionally, the pegylated lipid is selected from one or more of: PEG modified phosphatidylethanolamine, PEG modified phosphatidic acid, PEG modified ceramide, PEG modified dialkyl amine, PEG modified diacylglycerol, and PEG modified dialkylglycerol;

Alternatively, the pegylated lipid contains a PEG moiety of about 1000 Da to about 20 kDa, alternatively a PEG moiety of about 1000 Da to about 5000 Da;

Alternatively, the pegylated lipid is selected from one or more of: DMPE-PEG1000, DPPE-PEG1000, DSPE-PEG1000, DOPE-PEG1000, DMG-PEG2000, Ceramide-PEG2000, DMPE-PEG2000, DPPE-PEG2000, DSPE-PEG2000, Azido-PEG2000, DSPE-PEG2000-Mannose, Ceramide-PEG5000, DSPE-PEG5000, DSPE-PEG2000 amine and ALC-0159, alternatively DMG-PEG2000 and/or ALC-0159.

In a more specific embodiment, the present disclosure provides the above lipid nanoparticle, wherein the molar percentage of the polymer-conjugated lipid is >0 mol%-5 mol%; alternatively 0.5 mol%-3 mol%; alternatively 1.5 mol%-2 mol%; still alternatively 1.5 mol%, 1.6 mol% or 2 mol%.

In a more specific embodiment, the present disclosure provides the above lipid nanoparticle, wherein the neutral phospholipid is selected from phosphatidylcholine and/or phosphatidylethanolamine.

Optionally, the phosphatidylcholine is selected from one or more of: 1,2-distearoyl-sn-glycero-3-phosphocholine (DSPC), 1,2-dipalmitoyl-sn-glycero-3-phosphocholine (DPPC), 1,2-dimyristoyl-sn-glycero-3-phosphocholine (DMPC), 1-palmitoyl-2-oleoyl-sn-glycero-3-phosphocholine (POPC) and 1,2-dioleoyl-sn-glycero-3-phosphocholine (DOPC).

Optionally, the phosphatidylethanolamine is selected from one or more of: 1,2-dioleoyl-sn-glycero-3-phosphoethanolamine (DOPE), 2-((2,3-bis(oleoyloxy)propyl)dimethylammonio)ethyl hydrogen phosphate (DOCP), dimyristoylphosphatidylethanolamine (DMPE), 1-palmitoyl-2-oleoylphosphatidylethanolamine (POPE) and dipalmitoylphosphatidylethanolamine (DPPE).

In a more specific embodiment, the present disclosure provides the above lipid nanoparticle, wherein the molar percentage content of the neutral phospholipid is 3 mol%-30 mol%; alternatively 5 mol%-20 mol%; alternatively 10 mol%-20 mol%.

In a more specific embodiment, the present disclosure provides the above lipid nanoparticle, which does not contain neutral phospholipid.

In a more specific embodiment, the present disclosure provides a lipid nanoparticle without neutral phospholipid, comprising the following components: permanently cationic lipid, structured lipid, polymer-conjugated lipid and ionizable lipid.

In a more specific embodiment, the present disclosure provides the above lipid nanoparticle, wherein the permanently cationic lipid is as described above.

In a more specific embodiment, the present disclosure provides the above lipid nanoparticle, wherein the ionizable lipid is as described above.

In a more specific embodiment, the present disclosure provides the above lipid nanoparticle, wherein the structured lipid is as described above.

In a more specific embodiment, the present disclosure provides the above lipid nanoparticle, wherein the polymer-conjugated lipid is as described above.

In a more specific embodiment, the present disclosure provides the above lipid nanoparticle, comprising the following components in molar percentages:
30 mol%-80 mol%, alternatively 30% mol%-65 mol% of ionizable lipid;
30 mol%-70 mol%, alternatively 30% mol%-65 mol% of structured lipid;
>0 mol%-30 mol% of permanently cationic lipid;
>0 mol%-5 mol% of polymer-conjugated lipid;
alternatively, comprising the following components in molar percentages:
   35 mol%-65 mol% of ionizable lipid;
   30 mol%-60 mol% of structured lipid;
   1 mol%-25 mol% of permanently cationic lipid;
   0.5 mol%-3 mol% of polymer-conjugated lipid;
alternatively, comprising the following components in molar percentages:
   40 mol%-50 mol% of ionizable lipid;
   38.5 mol%-53.5 mol% of structured lipid;
   2.5 mol%-20 mol% of permanently cationic lipid;
   1.5 mol%-2 mol% of polymer-conjugated lipid.

Alternatively, the content of the permanently cationic lipid is 10 mol%-20 mol%.

Alternatively, the content of the structured lipid is 38.5 mol%-48.5 mol%.

In some specific embodiments, the molar ratio of ionizable lipid:structured lipid:permanently cationic lipid:polymer-conjugated lipid is: 40:53.5:5:1.5, 40:48.5:10:1.5, 40:38.5:20:1.5, 47.5:40.5:10:2, 49.5:46.5:2.5:1.5, 50:38.5:10:1.5, or 46.3:42.7:9.4:1.6.

In a more specific embodiment, the present disclosure provides a lipid nanoparticle composition, comprising any of the above lipid nanoparticles and a load.

In a more specific embodiment, the present disclosure provides the above lipid nanoparticle composition, wherein the load is selected from one or more of therapeutic agent, prophylactic agent and diagnostic agent.

Optionally, the therapeutic, prophylactic or diagnostic agent is a nucleic acid.

Optionally, the nucleic acid is selected from one or more of ASO, RNA and DNA.

Optionally, the RNA is selected from one or more of: interfering RNA (RNAi), small interfering RNA (siRNA), short hairpin RNA (shRNA), antisense RNA (aRNA), messenger RNA (mRNA), modified messenger RNA (mmRNA), long non-coding RNA (lncRNA), microRNA (miRNA), small activating RNA (saRNA), multimeric coding nucleic acid (MCNA), polymeric coding nucleic acid (PCNA), guide RNA (gRNA), CRISPRRNA (crRNA) and nucleases, alternatively mRNA, still alternatively, modified mRNA.

In a more specific embodiment, the present disclosure provides the above lipid nanoparticle composition, wherein the molar ratio N:P of total N atom in the permanently cationic lipid and ionizable lipid to P atom in the nucleic acid molecules is 1-15:1, alternatively 3-12:1, alternatively 4-7:1, still alternatively 4, 6 or 7.

In a more specific embodiment, the present disclosure provides the above lipid nanoparticle composition, wherein the lipid nanoparticles have an average particle size of 60-230 nm, alternatively 70-200 nm, still alternatively 70-165 nm.

In a more specific embodiment, the present disclosure provides a method of preparing the above lipid nanoparticle compositions, comprising: mixing the various lipid components, and then mixing the various lipid components with a load.

Alternatively, the method comprises mixing a solution containing the various lipid components with a solution containing a load.

Alternatively, a solution containing the various lipid components is mixed with a solution containing a load by means of microfluidic or impingement jets;

Alternatively, in the solution containing lipid components, the solvent is an organic solvent, alternatively an alcoholic solvent, alternatively ethanol;

Alternatively, the load is a nucleic acid, which is dissolved using sodium acetate solution, alternatively 20-30 mmol/L sodium acetate solution.

In a more specific embodiment, the present disclosure provides the above preparation method, further comprising a step of removing impurities; alternatively removing impurities by ultrafiltration; alternatively using a 30 kDa ultrafiltration tube for ultrafiltration.

In a more specific embodiment, the present disclosure provides the above preparation method, further comprising a sterilization step; alternatively, filter sterilization using a sterile filter membrane; alternatively, using a sterile filter membrane with a pore size of 0.2 µm.

In a more specific embodiment, the present disclosure provides a pharmaceutical composition, comprising any one of the above lipid nanoparticle compositions, and pharmaceutically acceptable excipient(s).

In a more specific embodiment, the present disclosure provides the use of any one of the above lipid nanoparticle compositions or the above pharmaceutical composition in the manufacture of a medicament for the treatment, diagnosis or prevention of a disease.

In a more specific embodiment, the present disclosure provides the use of any one of the above lipid nanoparticle compositions or the above pharmaceutical composition in the manufacture of a medicament for deliverying a load, wherein the load is selected from one or more of therapeutic agent, prophylactic agent and diagnostic agent.

Optionally, the therapeutic, prophylactic, or diagnostic agent is a nucleic acid.

Alternatively, the use is a use in the manufacture of a medicament for locally deliverying a load.

Alternatively, the use is a use in the manufacture of a medicament for deliverying a load in muscle or tumor.

Still alternatively, the use is a use in the manufacture of a medicament for deliverying a load in muscle.

In a more specific embodiment, the present disclosure provides a method of treating, diagnosing, or preventing a disease in a subject, comprising administering to the subject any one of the above lipid nanoparticle compositions, or the above pharmaceutical composition.

In a more specific embodiment, the present disclosure provides any one of the above lipid nanoparticle compositions, or the above pharmaceutical composition, for use in treating, diagnosing, or preventing a disease.

In a more specific embodiment, the present disclosure provides a method of delivering a load in the body of a subject, comprising administering to the subject any one of the above lipid nanoparticle compositions, or the above pharmaceutical composition;
wherein the load is selected from one or more of therapeutic agent, prophylactic agent and diagnostic agent.

Alternatively, the method is to locally deliver a load to the body of a subject.

Alternatively, the method is to deliver a load into the subject's muscle or tumor.

Still alternatively, the method is to deliver a load into the subject's muscle.

In a more specific embodiment, the present disclosure provides any one of the above lipid nanoparticle compositions, or the above pharmaceutical composition, for use in delivering a load;
wherein the load is selected from one or more of therapeutic agent, prophylactic agent and diagnostic agent.

The compounds of the present disclosure may include one or more asymmetric centers, and thus may exist in a variety of stereoisomeric forms, for example, enantiomers and/or diastereomers. For example, the compounds of the present disclosure may be in the form of an individual enantiomer, diastereomer or geometric isomer (e.g., cis- and trans-isomers), or may be in the form of a mixture of stereoisomers, including racemic mixture and a mixture enriched in one or more stereoisomers. The isomers can be separated from the mixture by the methods known to those skilled in the art, including chiral high pressure liquid chromatography (HPLC) and the formation and crystallization of chiral salts; or alternative isomers can be prepared by asymmetric synthesis.

The compounds of the present disclosure may exist in tautomer forms. The tautomer is a functional group isomer resulting from the rapid shift of an atom between two positions in a molecule. The tautomer is a special functional group isomer, wherein a pair of tautomers can convert between each other, but usually exist in a relatively stable isomer as its main form. The most important examples are the enol and keto tautomers.

The present disclosure also comprises compounds that are labeled with isotopes (isotope variants), which are equivalent to those described in formula (IV), but one or more atoms are replaced by atoms having an atom mass or mass number that are different from that of atoms that are common in nature. Examples of isotopes which may be introduced into the compounds of the disclosure include isotopes of hydrogen, carbon, nitrogen, oxygen, phosphorus, sulfur, fluorine and chlorine, such as ²H, ³H, ¹³C, ¹¹C, ¹⁴C, ¹⁵N, ¹⁸O, ¹⁷O, ³¹P, ³²P, ³⁵S, ¹⁸F and ³⁶Cl, respectively. Compounds of the present disclosure that comprise the above isotopes and/or other isotopes of other atoms, prodrugs thereof and pharmaceutically acceptable salts of the compounds or prodrugs all are within the scope of the present disclosure. Certain isotope-labeled compounds of the present disclosure, such as those incorporating radioactive isotopes (e.g., ³H and ¹⁴C), can be used for the measurement of the distribution of drug and/or substrate in tissue. Tritium, which is ³H and carbon-14, which is ¹⁴C isotope, are yet alternative, because they are easy to prepare and detect. Furthermore, replaced by heavier isotopes, such as deuterium, which is ²H, may provide therapeutic benefits due to the higher metabolic stability, such as prolonging the half-life *in vivo* or decreasing the dosage requirements, and thus may be alternative in some cases. Isotope-labeled compounds of formula (I) of the present disclosure and prodrugs thereof can be prepared generally by using readily available isotope-labeled reagents to replace non-isotope-labeled reagents in the following schemes and/or the procedures disclosed in the examples and preparation examples.

The present disclosure also provides a pharmaceutical formulation comprising a therapeutically effective amount of a compound of formula (VI), or therapeutically acceptable salts thereof, and pharmaceutically acceptable carriers, diluents or excipients thereof. All of these forms belong to the present disclosure.

### Pharmaceutical compositions and kits

In another aspect, the present disclosure provides a pharmaceutical composition comprising nanoparticle compositions of the present disclosure and pharmaceutically acceptable excipient(s), the nanoparticle composition comprises the compounds of the present disclosure.

A pharmaceutically acceptable excipient for use in the present disclosure refers to a non-toxic carrier, adjuvant or vehicle which does not destroy the pharmacological activity of the compound formulated together. Pharmaceutically acceptable carriers, adjuvants, or vehicles that may be used in the compositions of the present disclosure include, but are not limited to, ion exchangers, alumina, aluminum stearate, lecithin, serum proteins (e.g., human serum albumin), buffer substances (such as phosphate), glycine, sorbic acid, potassium sorbate, a mixture of partial glycerides of saturated plant fatty acids, water, salt or electrolyte (such as protamine sulfate), disodium hydrogen phosphate, potassium hydrogen phosphate, sodium chloride, zinc salt, silica gel, magnesium trisilicate, polyvinyl pyrrolidone, cellulose-based materials, polyethylene glycol, sodium carboxymethyl cellulose, polyacrylate, wax, polyethylene-polyoxypropylene block polymers, polyethylene glycol and lanolin.

The present disclosure also includes kits (e.g., pharmaceutical packs). Kits provided may include a nanoparticle composition of the present disclosure and other therapeutic, or diagnostic, or prophylactic agents, and a first and a second containers (e.g., vials, ampoules, bottles, syringes, and/or dispersible packages or other materials) containing the nanoparticle composition of the present disclosure or other therapeutic, or diagnostic, or prophylactic agents. In some embodiments, kits provided can also optionally include a third container containing a pharmaceutically acceptable excipient for diluting or suspending the nanoparticle composition of the present disclosure and/or other therapeutic, or diagnostic, or prophylactic agent. In some embodiments, the nanoparticle composition of the present disclosure provided in the first container and the other therapeutic, or diagnostic, or prophylactic agents provided in the second container is combined to form a unit dosage form.

### Administration

The pharmaceutical composition provided by the present disclosure can be administered by a variety of routes including, but not limited to, oral administration, parenteral administration, inhalation administration, topical administration, rectal administration, nasal administration, oral administration, vaginal administration, administration by implant or other means of administration. For example, parenteral administration as used herein includes subcutaneous administration, intradermal administration, intravenous administration, intramuscular administration, intra-articular administration, intraarterial administration, intrasynovial administration, intrasternal administration, intracerebroventricular administration, intralesional administration, and intracranial injection or infusion techniques.

Generally, the pharmaceutical compositions provided herein are administered in an effective amount. The amount of the pharmaceutical composition actually administered will typically be determined by a physician, in the light of the relevant circumstances, including the condition to be treated or prevented, the chosen route of administration, the actual pharmaceutical composition administered, the age, weight, and response of the individual patient, the severity of the patient's symptoms, and the like.

When used to prevent the disorder of the present disclosure, the pharmaceutical compositions provided herein will be administered to a subject at risk for developing the condition, typically on the advice and under the supervision of a physician, at the dosage levels described above. Subjects at risk for developing a particular condition generally include those that have a family history of the condition, or those who have been identified by genetic testing or screening to be particularly susceptible to developing the condition.

The pharmaceutical compositions provided herein can also be administered chronically ("chronic administration"). Chronic administration refers to administration of a compound or pharmaceutical composition thereof over an extended period of time, e.g., for example, over 3 months, 6 months, 1 year, 2 years, 3 years, 5 years, *etc*., or may be continued indefinitely, for example, for the rest of the subject's life. In certain embodiments, the chronic administration is intended to provide a constant level of the compound in the blood, e.g., within the therapeutic window over the extended period of time.

The pharmaceutical compositions of the present disclosure may be further delivered using a variety of dosing methods. For example, in certain embodiments, the pharmaceutical composition may be given as a bolus, e.g., in order to raise the concentration of the compound in the blood to an effective level. The placement of the bolus dose depends on the systemic levels of the active ingredient desired throughout the body, e.g., an intramuscular or subcutaneous bolus dose allows a slow release of the active ingredient, while a bolus delivered directly to the veins (e.g., through an IV drip) allows a much faster delivery which quickly raises the concentration of the active ingredient in the blood to an effective level.

The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the active substance is usually a minor component (from about 0.1 to about 50% by weight or alternatively from about 1 to about 40% by weight) with the remainder being various vehicles or excipients and processing aids helpful for forming the desired dosing form.

With oral dosing, one to five and especially two to four and typically three oral doses per day are representative regimens. Using these dosing patterns, each dose provides from about 0.001mg/kg to about 10 mg/kg of the therapeutic, or diagnostic, or prophylactic agents, with alternative doses each providing from about 0.1 mg/kg to about 10 mg/kg, and especially about 1 to about 5 mg/kg.

Injectable compositions are typically based upon injectable sterile saline or phosphate-buffered saline or other injectable excipients known in the art. As before, the active compound in such compositions is typically a minor component, often being from about 0.05 to 10% by weight with the remainder being the injectable excipient and the like.

### Examples

In order to make the technical solutions of the present disclosure clearer and more explicit, the present disclosure is further elaborated through the following examples. The following examples are used only to illustrate specific embodiments of the present disclosure so that a person skilled in the art can understand the present disclosure, but are not intended to limit the scope of protection of the present disclosure. The technical means or methods, etc. not specifically described in the specific embodiments of the present disclosure are conventional technical means or methods, etc. in the art. The materials, reagents, etc. used in examples are commercially available if not otherwise specified.

**Table 1**

| **Abbreviation** | **Full name** |
|---|---|
| THF | Tetrahydrofuran |
| DCM | dichloromethane |
| MeOH | methanol |
| DMF | N,N-Dimethylformamide |
| DMSO | Dimethyl sulfoxide |
| DCE | 1,2-Dichloroethane |
| CDCl₃ | Deuterated chloroform |
| TBAI | Tetrabutylammonium iodide |
| TsCH₂CN | 4-Toluenesulfonylacetonitrile |
| TMSOK | Potassium trimethylsiloxide |
| TBDMSCl | tert-Butyldimethylsilyl chloride |
| LDA | Lithium diisopropylamide |
| DMAP | 4-Dimethylaminopyridine |
| (COCl)₂ | Oxalyl chloride |
| SOCl₂ | Thionyl dichloride |
| NaBH₄ | Sodium borohydride |
| NaH | Sodium hydride |
| K₂CO₃ | Potassium carbonate |
| EDCI | 1-(3-Dimethylaminopropyl)-3-ethylcarbodiimide |
| DIPEA | N,N-Diisopropylethylamine |
| Et₃N | Triethylamine |
| AcOH | Acetic acid |
| NaBH₃CN | Sodium cyanoborohydride |
| Imidazole | Imidazole |
| NMO | 4-Methylmorpholine N-oxide |
| BDMEP | 2,6-di-tert-Butylpyridine |

### Example 1: Synthesis of compound 1

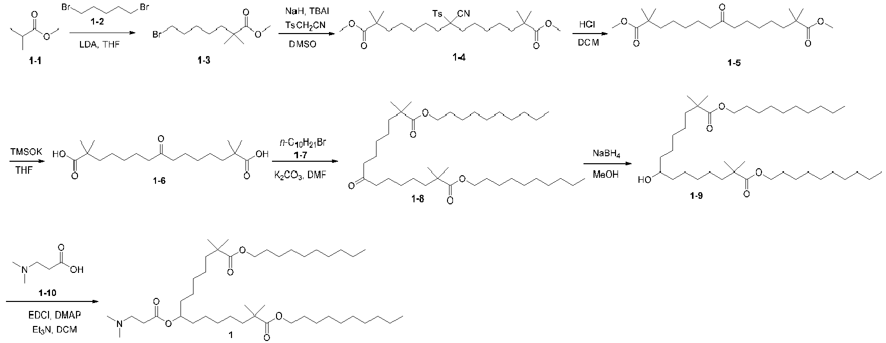

A solution of compound 1-1 (100 g, 979 mmol) in tetrahydrofuran (800 mL) was cooled to -40°C. LDA (2 M, 490 mL) was added slowly dropwise to the solution and the mixture was stirred for another 1 h after completion of the dropwise addition. A solution of 1-2 (315 g, 1.37 mol) in tetrahydrofuran (100 mL) was added dropwise to the reaction system at the same temperature and the reaction system was stirred overnight. The reaction system was quenched with saturated aqueous ammonium chloride, and extracted with ethyl acetate. The organic phases were combined, dried over anhydrous sodium sulfate, and filtered. The filtrate was concentrated to dryness to give a crude product. The crude product was purified by silica gel column to give compound 1-3 (115 g). ¹H NMR (400 MHz, CDCl₃): δ ppm 1.06-1.11 (m, 6 H), 1.13-1.22 (m, 2 H), 1.29-1.39 (m, 2 H), 1.42-1.49 (m, 2 H), 1.73-1.82 (m, 2 H), 3.28-3.40 (m, 2 H), 3.55-3.66 (m, 3 H).

A solution of compound 1-3 (100 g, 398 mmol), TsCH₂CN (38.9 g, 199 mmol) and TBAI (14.7 g, 39.8 mmol) in dimethyl sulfoxide (800 mL) was cooled to 0 °C, and sodium hydride (20.7 g, 517 mmol) was added slowly in batches. The mixture was reacted at room temperature overnight. The reaction system was quenched with saturated aqueous sodium chloride solution and extracted with ethyl acetate. The organic phases were combined, dried over anhydrous sodium sulfate, and filtered. The filtrate was concentrated to dryness to give 115 g of crude compound 1-4, which was used directly in the next reaction without isolation and purification.

To a solution of compound 1-4 crude (110 g, 205 mmol) in dichloromethane (880 mL) was added 330 mL of concentrated hydrochloric acid, and the mixture was reacted at room temperature for 2 h. The complete reaction of the substrate was monitored by TLC. The reaction system was quenched with saturated aqueous ammonium chloride solution and extracted with ethyl acetate. The organic phases were combined, dried over anhydrous sodium sulfate, and filtered. The filtrate was concentrated to dryness to give a crude product. The crude product was purified by silica gel column to give compound 1-5 (30.0 g, 80.9 mmol, yield 39.4%).

TMSOK (11.0 g, 86.4 mmol) was added to a solution of compound 1-5 (8.0 g, 21.6 mmol) in tetrahydrofuran (35.0 mL) at room temperature, and the reaction system was heated to 70°C with stirring. The complete consumption of reaction materials was monitored by TLC. The reaction solution was cooled to room temperature, and the organic solvent was removed by rotary evaporation. The crude product was added to 20 mL of water and extracted with dichloromethane. The aqueous layer was collected, and the solution was adjusted to a pH of <5 with 1 M hydrochloric acid. The solution was extracted with dichloromethane. The organic phases were combined, dried over anhydrous sodium sulfate, and filtered. The filtrate was collected and concentrated to give compound 1-6 (7.0 g). ¹H NMR (400 MHz, CDCl₃): δ ppm 1.03 (s, 12H), 1.08-1.17 (m, 8H), 1.34-1.45 (m, 8H), 2.21 (t, *J=* 7.2 Hz, 4H).

Potassium carbonate (482 mg, 3.48 mmol) was added to a solution of compound 1-6 (294 mg, 0.87 mmol) and 1-7 (771 mg, 3.48 mmol) in DMF, then the reaction was warmed up to 60 °C for 6 h. The complete disappearance of reactant 1-6 was monitored. The mixture was cooled to room temperature. The reaction system was quenched with saturated aqueous sodium chloride solution and extracted with ethyl acetate. The organic phases were combined, dried over anhydrous sodium sulfate, and filtered. The filtrate was concentrated to dryness to give the crude product. The crude was purified by silica gel column to give compound 1-8 (325 mg).

Compound 1-8 (325 mg) was dissolved in 4.0 mL of methanol and sodium borohydride (30 mg, 0.84 mmol) was added to the reaction system. The mixture was reacted at room temperature. The complete disappearance of the reactants was monitored by TLC. The reaction system was quenched with saturated aqueous sodium chloride solution and extracted with dichloromethane. The organic phases were combined, dried over anhydrous sodium sulfate, and filtered. The filtrate was concentrated to dryness to give crude compound 1-9 (260 mg), which was used directly in the next reaction without purification.

Crude compound 1-9 (260 mg, 0.42 mmol), 1-10 (73.1 mg, 0.63 mmol), EDCI (238 mg, 1.26 mmol), triethylamine (0.17 mL, 1.26 mmol) and DMAP (51 mg, 0.42 mmol) were dissolved in 5.0 mL of dichloromethane, and the reaction solution was stirred to react at room temperature for 12 h. The reaction solution was quenched with saturated aqueous sodium chloride and extracted with dichloromethane. The organic phases were combined, dried over anhydrous sodium sulfate, and filtered. The organic phase was collected and the organic solvent was removed using a rotary-evaporator to give the crude product, which was purified by preparative high performance liquid chromatography to give compound 1 (130 mg).

¹H NMR (400 MHz, CDCl₃): δ ppm 0.89 (t, *J* = 7.2 Hz, 6H), 1.15 (s, 12H), 1.27 (m, 40H), 1.49 (m, 8H), 1.61 (m, 4H), 2.26 (s, 6H), 2.44-2.52 (t, *J* = 7.2 Hz, 2H), 2.63 (t, *J* = 7.2 Hz, 2H), 4.04 (t, *J* = 6.8 Hz, 4H), 4.86 (m, 1H); ESI-MS m/z: 724.7 [M+H]⁺.

### Example 2: Synthesis of compound 2

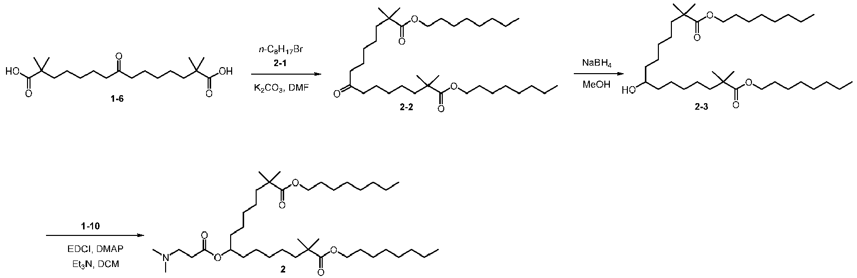

Referring to the method of Example 1, compound 2 was prepared as an oily product: 25.7 mg.

¹H NMR (400 MHz, CDCl₃): δ ppm 0.89 (t, *J* = 6.8 Hz, 6H), 1.15 (s, 12H), 1.29 (m, 32H), 1.49 (m, 8H), 1.60 (m, 4H), 2.24 (s, 6H), 2.46 (t, *J* = 7.2 Hz, 2H), 2.61(t, *J* = 7.2 Hz, 2H), 4.04 (t, *J=* 6.8 Hz, 4H), 4.86 (m, 1H); ESI-MS m/z: 668.6 [M+H]⁺.

### Example 3: Synthesis of compound 3

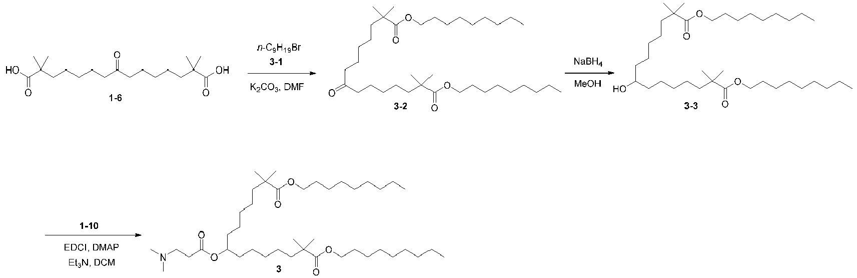

Referring to the method of Example 1, compound 3 was prepared as an oily product: 31.2 mg.

¹H NMR (400 MHz, CDCl₃): δ ppm 0.89 (t, *J* = 6.8 Hz, 6H), 1.16 (s, 12H), 1.28 (m, 36H), 1.49 (m, 8H), 1.62 (m, 4H), 2.25 (s, 6H), 2.47 (t, *J* = 7.2 Hz, 2H), 2.62(t, *J* = 7.2 Hz, 2H), 4.05 (t, *J* = 6.8 Hz, 4H), 4.88 (m, 1H); ESI-MS m/z: 696.6 [M+H]⁺.

### Example 4: Synthesis of compound 4

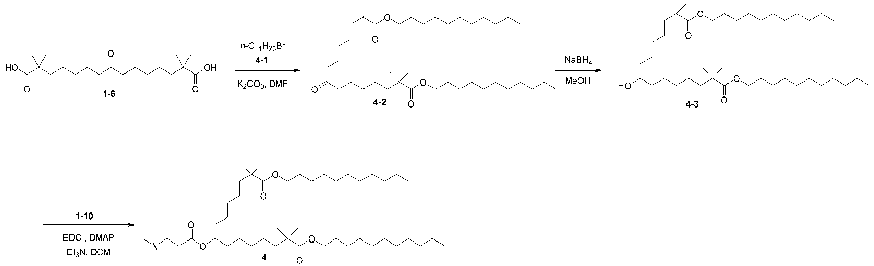

Referring to the method of Example 1, compound 4 was prepared as an oily product: 32 mg.

¹H NMR (400 MHz, CDCl₃): δ ppm 0.89 (t, *J* = 6.8 Hz, 6H), 1.16 (s, 12H), 1.28 (m, 44H), 1.49 (m, 8H), 1.52 (m, 4H), 2.51 (s, 6H), 2.53 (t, *J* = 7.2 Hz, 2H), 3.12 (t, *J* = 7.2 Hz, 2H), 3.91 (t, *J* = 6.8 Hz, 4H), 4.82 (m, 1H); ESI-MS m/z: 752.7 [M+H]⁺.

### Example 5: Synthesis of compound 5

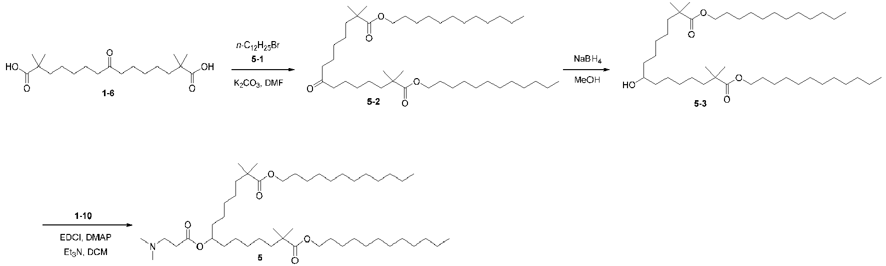

Referring to the method of Example 1, compound 5 was prepared as an oily product: 31.4 mg.

¹H NMR (400 MHz, CDCl₃): δ ppm 0.88 (t, *J* = 6.8 Hz, 6H), 1.15 (s, 12H), 1.25 (m, 48H), 1.49 (m, 8H), 1.52 (m, 4H), 2.46 (s, 6H), 2.63 (m, 2H), 2.86 (m, 2H), 4.03 (t, *J* = 6.8 Hz, 4H), 4.84 (m, 1H); ESI-MS m/z: 780.7 [M+H]⁺.

### Example 6: Synthesis of compound 6

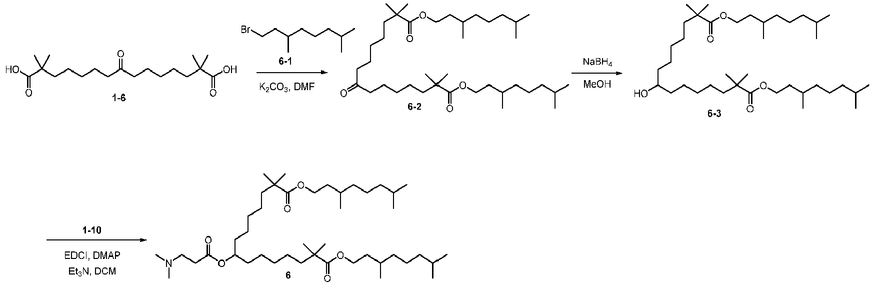

Referring to the method of Example 1, compound 6 was prepared as an oily product: 30.7 mg.

¹H NMR (400 MHz, CDCl₃): δ ppm 0.83 (t, *J* = 6.8 Hz, 18H), 1.00-1.28 (m, 34H), 1.31-1.62 (m, 18H), 2.21 (s, 6H), 2.36-2.46 (m, 2H), 2.51-2.62 (m, 2H), 4.02 (t, *J* = 6.8 Hz, 4H), 4.71-4.85 (m, 1H); ESI-MS m/z: 724.6 [M+H]⁺.

### Example 7: Synthesis of compound 7

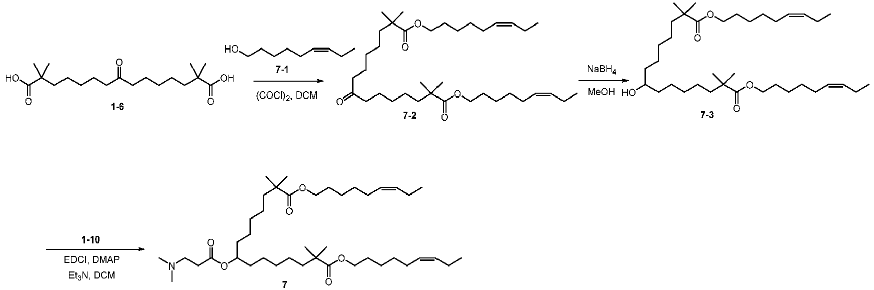

Compound 1-6 (548 mg, 1.5 mmol) was dissolved in 5.0 mL of dichloromethane, and the reaction system was cooled to 0 °C in an ice bath. DMF (12 µL, 0.15 mmol) was added and oxalyl chloride (0.47 mL, 6.0 mmol) was added dropwise to the reaction solution. The ice bath was removed and the mixture was stirred for 1 h at room temperature. The solvent was removed using a rotary-evaporator to give acyl chloride crude product (458 mg) as an oil, which was used directly in the next reaction step.

The above obtained acyl chloride crude product (458 mg) was dissolved in 3.0 mL of 1,2-dichloroethane, and then compound 7-1 (429 mg, 3.0 mmol) was added to the reaction solution. The mixture was stirred at room temperature until the substrate was reacted completely. The solvent was removed using a rotary-evaporator to give the crude product, which was purified by silica gel column to give compound 7-2 (540 mg).

Then referring to the method of Example 1, compound 7 was prepared as an oily product: 33.2 mg.

¹H NMR (400 MHz, CDCl₃): δ ppm 0.89 (t, *J* = 6.8 Hz, 6H), 1.23 (s, 12H), 1.29-1.51 (m, 32H), 1.95 (m, 8H), 2.18 (s, 6H), 2.41 (m, 2H), 2.53 (m, 2H), 3.91 (t, *J* = 6.8 Hz, 4H), 4.78 (m, 1H), 5.25 (m, 4H); ESI-MS m/z: 692.6 [M+H]⁺.

### Example 8: Synthesis of compound 8

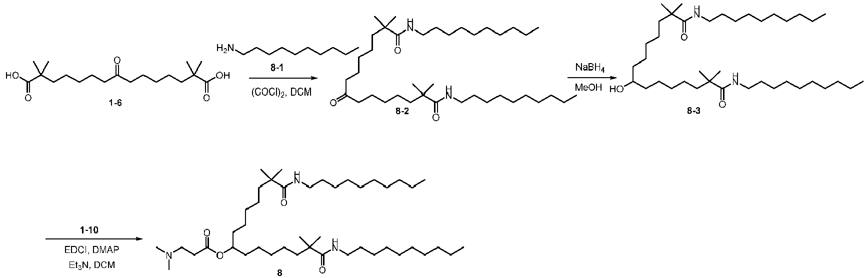

Compound 1-6 (548 mg, 1.5 mmol) was dissolved in 5.0 mL of dichloromethane, and the reaction system was cooled in an ice bath. DMF (12 µL, 0.15 mmol) was added and oxalyl chloride (0.47 mL, 6.0 mmol) was added dropwise to the reaction solution. The ice bath was removed and the mixture was stirred for 1 h at room temperature. The solvent was removed using a rotary-evaporator to give acyl chloride crude product (458 mg) as an oil, which was used directly in the next reaction step.

The above obtained 458 mg of acyl chloride crude product was dissolved in 3.0 mL of 1,2-dichloroethane, and then compound 8-1 (472 mg, 3.0 mmol) was added to the reaction solution. The mixture was stirred at room temperature until the substrate was reacted completely. The solvent was removed using a rotary-evaporator to give crude product, which was purified by silica gel column to give compound 8-2 (518 mg).

518 mg of compound 8-2 was dissolved in 5.0 mL of methanol, and sodium borohydride (48 mg, 1.25 mmol) was added to the reaction system. The mixture was reacted at room temperature. The complete disappearance of the reactants was monitored by TLC. The reaction system was quenched with saturated aqueous sodium chloride solution and extracted with dichloromethane. The organic phases were combined, dried over anhydrous sodium sulfate, and filtered. The filtrate was concentrated to dryness to give 473 mg of crude compound 8-3, which was used directly in the next reaction without purification.

Crude compound 8-3 (270 mg, 0.43 mmol), 1-10 (76.1 mg, 0.65 mmol), EDCI (248 mg, 1.3 mmol), triethylamine (0.18 mL, 1.3 mmol) and DMAP (53 mg, 0.43 mmol) were dissolved in 5.0 mL of dichloromethane, and the reaction solution was stirred to react at room temperature for 12 h. The reaction system was quenched with saturated aqueous sodium chloride and extracted with dichloromethane. The organic phases were combined, dried over anhydrous sodium sulfate, and filtered. The organic phase was collected and the organic solvent was removed using a rotary-evaporator to give the crude product, which was purified by preparative high performance liquid chromatography to give compound 8 (39 mg).

¹H NMR (400 MHz, CDCl₃): δ ppm 0.89 (t, *J* = 6.8 Hz, 6H), 1.14 (s, 12H), 1.15-1.31 (m, 40H), 1.40-1.52 (m, 12H), 2.25 (s, 6H), 2.45 (m, 2H), 2.60 (m, 2H), 3.15 (t, *J* = 6.8 Hz, 4H), 4.77-4.89 (m, 1H), 5.51-5.67 (m, 2H); ESI-MS m/z: 722.7 [M+H]⁺.

### Example 9: Synthesis of compound 9

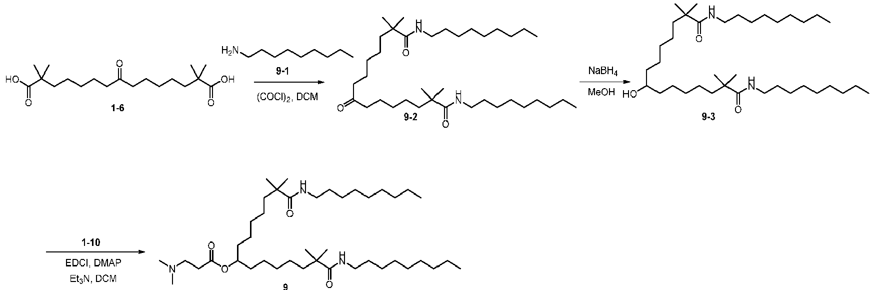

Referring to the method of Example 8, compound 9 (73 mg) was prepared.

¹H NMR (400 MHz, CDCl₃): δ ppm 0.89 (t, *J* = 6.8 Hz, 6H), 1.15 (s, 12H), 1.27-1.49 (m, 48H), 2.25 (s, 6H), 2.46 (t, *J* = 7.2 Hz, 2H), 2.62 (t, *J* = 7.2 Hz, 2H), 3.24 (m, 4H), 4.85 (m, 1H), 5.58 (m, 2H); ESI-MS m/z: 694.6 [M+H]⁺.

### Example 10: Synthesis of compound 10

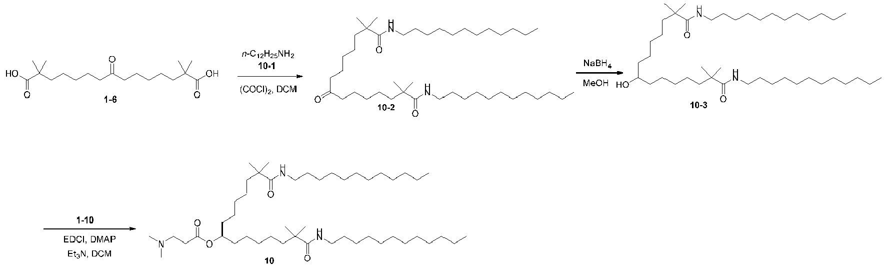

Referring to the method of Example 8, compound 10 (31.2 mg) was prepared.

¹H NMR (400 MHz, CDCl₃): δ ppm 0.79 (t, *J* = 7.2 Hz, 6H), 1.07 (s, 12H), 1.27-1.49 (m, 48H), 1.41 (m, 12H), 2.18 (s, 6H), 2.41 (t, *J* = 7.2 Hz, 2H), 2.55 (t, *J* = 7.2 Hz, 2H), 3.16 (m, 4H), 4.78 (m, 1H), 5.51 (m, 2H); ESI-MS m/z: 778.8 [M+H]⁺.

### Example 11: Synthesis of compound 11

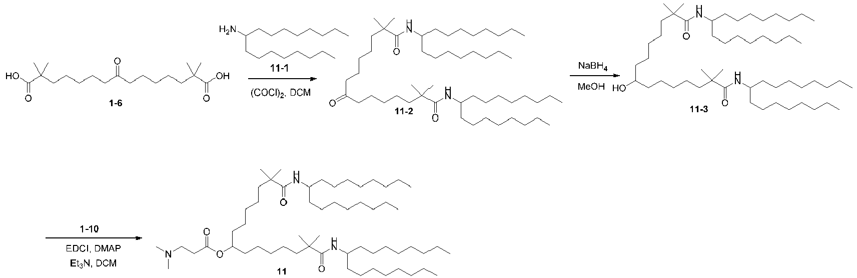

Referring to the method of Example 8, compound 11 (48.1 mg) was prepared.

¹H NMR (400 MHz, CDCl₃): δ ppm 0.78 (t, *J* = 7.2 Hz, 12H), 1.07 (s, 12H), 1.14-1.19 (m, 60H), 1.40 (m, 16H), 2.18 (s, 6H), 2.36-2.47 (m, 2H), 2.49-2.68 (m, 2H), 3.76-3.88 (m, 2H), 4.74-4.83 (m, 1H), 5.10-5.19 (m, 2H); ESI-MS m/z: 918.9 [M+H]⁺.

### Example 12: Synthesis of compound 12

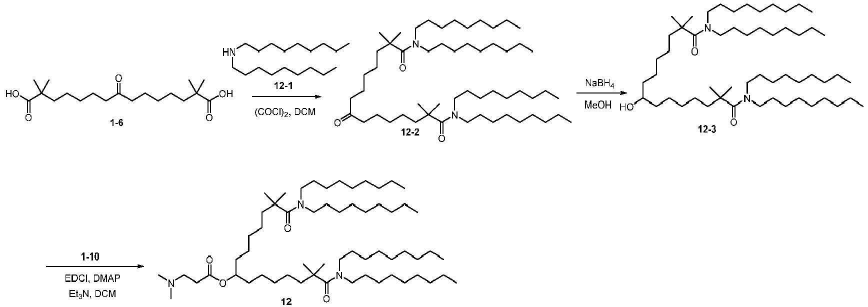

Referring to the method of Example 8, compound 12 (52 mg) was prepared.

¹H NMR (400 MHz, CDCl₃): δ ppm 0.82 (t, *J* = 6.8 Hz, 12H), 1.15-1.32 (m, 72H), 1.54 (m, 16H), 2.31 (s, 6H), 2.51 (t, *J* = 7.2 Hz, 2H), 2.60 (t, *J* = 7.2 Hz, 2H), 3.14-3.33 (m, 8H), 4.75-4.83 (m, 1H); ESI-MS m/z: 946.9 [M+H]⁺.

### Example 13: Synthesis of compound 13

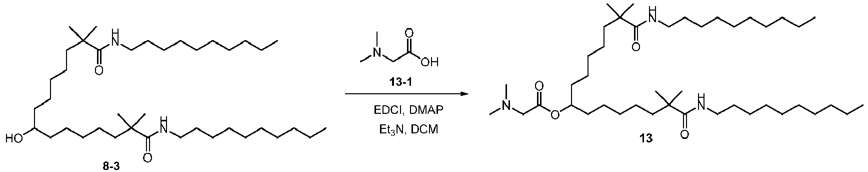

Referring to the method of Example 8, compound 13 (32 mg) was prepared.

¹H NMR (400 MHz, CDCl₃): δ ppm 0.81 (t, *J* = 7.2 Hz, 6H), 1.19 (m, 52H), 1.41 (m, 12H), 2.26 (s, 6H), 3.05 (s, 2H), 3.16 (m, 4H), 4.83 (m, 1H), 5.51 (m, 2H); ESI-MS m/z: 708.7 [M+H]⁺.

### Example 14: Synthesis of compound 14

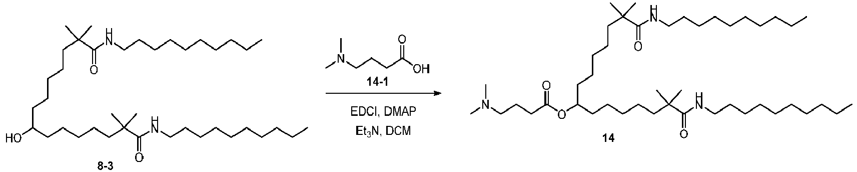

Referring to the method of Example 8, compound 14 (18 mg) was prepared.

¹H NMR (400 MHz, CDCl₃): δ ppm 0.81 (t, *J* = 7.2 Hz, 6H), 1.07 (s, 12H), 1.08-1.31 (m, 44H), 1.35-1.47 (m, 8H), 1.71-1.84 (m, 2H), 2.09-2.38 (m, 10H), 3.12-3.27 (m, 4H), 4.70-4.82 (m, 1H), 5.49-5.63 (m, 2H); ESI-MS m/z: 736.7 [M+H]⁺.

### Example 15: Synthesis of compound 15

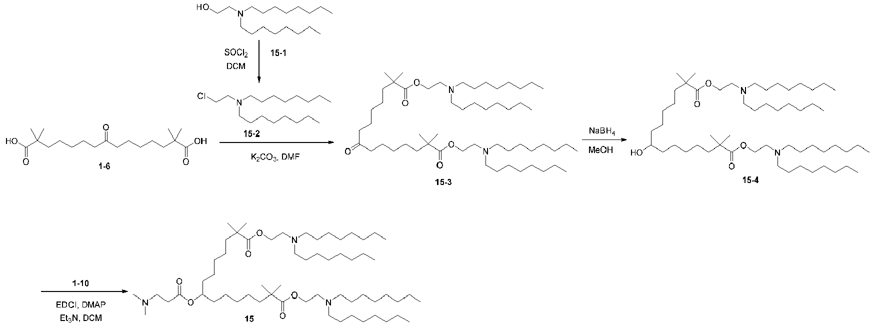

A solution of compound 15-1 (400 mg, 1.4 mmol) in dichloromethane (3.0 mL) was cooled to 0 °C, then a solution of SOCl₂ (0.12 mL, 1.68 mmol) in dichloromethane (2.0 mL) was added dropwise. After the dropwise addition was completed, the mixture was stirred at 0 °C for another 1 h. After the reaction was completed, the reaction was quenched by adding saturated sodium bicarbonate solution to the reaction system, and the reaction system was extracted with dichloromethane. The organic phases were combined and the organic solvent was removed to give crude compound 15-2, which was used directly in the next reaction without purification.

Compound 1-6 (223, 0.65 mmol), 15-2 (496 mg, 1.63 mmol) and potassium carbonate (361 mg, 2.6 mmol) were dissolved in 5.0 mL of DMF and the reaction solution was heated to 70°C to react for 6 hours. The reaction solution was cooled to room temperature, then the reaction was quenched by adding saturated sodium chloride solution to the reaction system, and the reaction system was extracted with dichloromethane. The organic phases were combined and the organic solvent was removed to give the crude product. The crude was purified by silica gel column to give compound 15-3.

Then referring to the method of Example 1, compound 15 (40 mg) was prepared.

¹H NMR (400 MHz, CDCl₃): δ ppm 0.89 (t, *J* = 7.2 Hz, 12H), 1.08 (s, 12H), 1.12-1.35 (m, 46H), 1.38-1.58 (m, 22H), 2.35 (s, 6H), 2.41-2.52 (m, 10H), 2.57-2.65 (m, 2H), 2.62 (m, 4H), 4.10 (t, *J* = 6.4 Hz, 4H), 4.86 (m, 1H); ESI-MS m/z: 978.9 [M+H]⁺.

### Example 16: Synthesis of compound 16

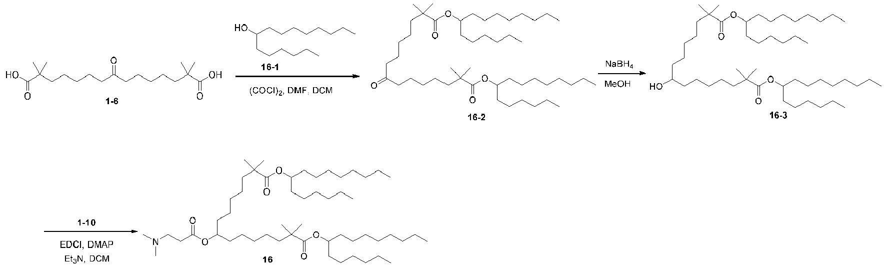

DMF (11 µL, 0.14 mmol) was added to a solution of compound 1-6 (460 mg, 1.34 mmol) in dichloromethane (5.0 mL) under ice bath conditions, and oxalyl chloride (0.47 mL, 5.37 mmol) was then added dropwise to the reaction solution. The ice bath was removed, and the mixture was stirred for 1 h at room temperature. The solvent was removed using a rotary-evaporator to give 255 mg of acyl chloride crude product as an oil, which was used directly in the next reaction step.

The above obtained acyl chloride crude product (255 mg, 0.67 mmol) was dissolved in 3.0 mL of 1,2-dichloroethane, and then compound 16-1 (384 mg, 1.68 mmol) was added to the reaction solution. The mixture was stirred at room temperature until the substrate was reacted completely. The solvent was removed using a rotary-evaporator to give the crude product, which was purified by silica gel column to give 300 mg of compound 16-2. ¹H NMR (400 MHz, CDCl₃): δ ppm 0.78-0.83 (m, 12H), 1.07 (s, 12H), 1.13-1.22 (m, 48H), 1.49 (br s, 16H), 2.29 (t, *J* = 7.50 Hz, 4H), 4.76 (m, 2H).

Compound 16-2 (300 mg, 0.39 mmol) was dissolved in 4.0 mL of methanol. Then NaBH₄ (45 mg, 1.17 mmol) was slowly added to the reaction solution and the mixture was stirred at room temperature for 2 h. The reaction solution was quenched with saturated ammonium chloride solution, extracted with ethyl acetate. The organic phases were combined and the organic solvent was removed to give 300 mg of crude compound 16-3, which was used directly in the next reaction without purification.

Crude compound 16-3 (300 mg, 0.39 mmol) was dissolved in 2.0 mL DMF, and then 1-10 (69 mg, 0.59 mmol), EDCI (225 mg, 1.17 mmol), triethylamine (119 mg, 1.17 mmol) and DMAP (48 mg, 0.39 mmol) were added. The mixture was stirred at room temperature until the reactants was reacted completely. The reaction solution was quenched with saturated sodium chloride solution and extracted with ethyl acetate. The organic phases were combined, dried over anhydrous sodium sulfate, and filtered. The filtrate was concentrated to dryness to give a crude product. The crude product was purified by preparative high performance liquid chromatography to give compound 16 (32.5 mg).

¹H NMR (400 MHz, CDCl₃): δ ppm 0.79 (t, *J* = 7.2 Hz, 12H), 1.07 (s, 12H), 1.19 (m, 52H), 1.40-1.46 (m, 16H), 2.15 (s, 6H), 2.34-2.58 (m, 4H), 4.74-4.81 (m, 3H); ESI-MS m/z: 864.8 [M+H]⁺.

### Example 17: Synthesis of compound 17

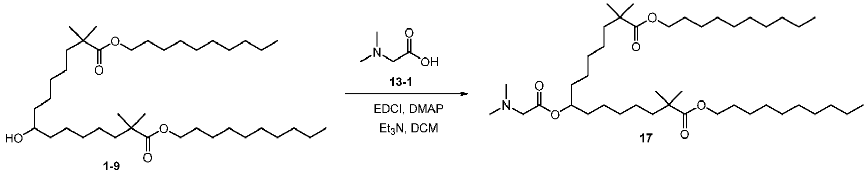

Referring to the method of Example 1, compound 17 was prepared as an oily product: 41.3 mg.

¹H NMR (400 MHz, CDCl₃): δ ppm 0.82 (t, *J* = 7.2 Hz, 6H), 1.08 (s, 12H), 1.14-1.20 (m, 36H), 1.40-1.64 (m, 16H), 2.32 (s, 6H), 3.08-3.21 (m, 2H), 3.97 (t, *J* = 7.2 Hz, 4H), 4.83-4.92 (m, 1H); ESI-MS m/z: 710.6 [M+H]⁺.

### Example 18: Synthesis of compound 18

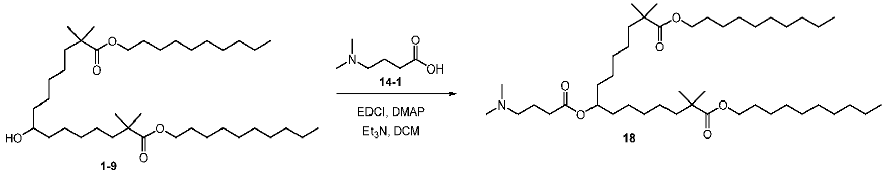

Referring to the method of Example 1, compound 18 was prepared as an oily product: 35.4 mg.

¹H NMR (400 MHz, CDCl₃): δ ppm 0.79 (t, *J* = 7.2 Hz, 6H), 1.08 (s, 12H), 1.13-1.25 (m, 36H), 1.28-1.47 (m, 10H), 1.47-1.62 (m, 6H), 1.68-1.79 (m, 2H), 2.15 (s, 6H), 2.21-2.31 (m, 4H), 3.97 (t, *J* = 7.2 Hz, 4H), 4.73-4.82 (m, 1H); ESI-MS m/z: 738.7 [M+H]⁺.

### Example 19: Synthesis of compound 19

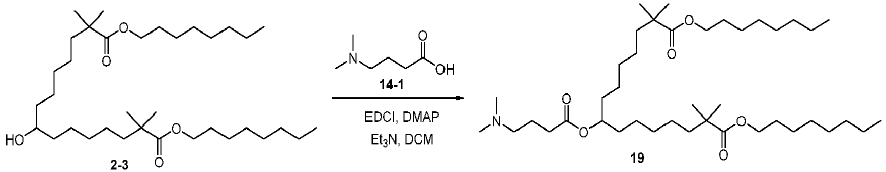

Referring to the method of Example 1, compound 19 was prepared as an oily product: 33.1 mg.

¹H NMR (400 MHz, CDCl₃): δ ppm 0.89 (t, *J* = 7.2 Hz, 6H), 1.15 (s, 12H), 1.29 (m, 30H), 1.50 (m, 8H), 1.60 (m, 6H), 1.64 (m, 2H), 2.23 (s, 6H), 2.33 (m, 4H), 4.05 (t, *J* = 6.8 Hz, 4H), 4.86 (m, 1H); ESI-MS m/z: 682.6 [M+H]⁺.

### Example 20: Synthesis of compound 20

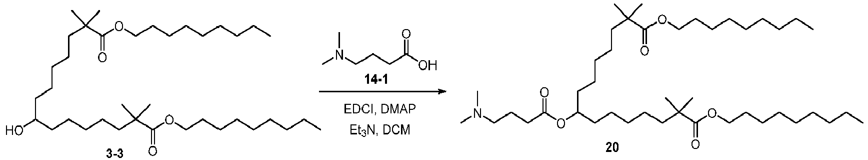

Referring to the method of Example 1, compound 20 was prepared as an oily product: 302 mg.

¹H NMR (400 MHz, CDCl₃): δ ppm 0.89 (t, *J* = 7.2 Hz, 6H), 1.15 (s, 12H), 1.27 (m, 34H), 1.47 (m, 8H), 1.51 (m, 6H), 1.79 (m, 2H), 2.23 (s, 6H), 2.33 (m, 4H), 4.04 (t, *J* = 6.8 Hz, 4H), 4.85 (m, 1H); ESI-MS m/z: 710.7 [M+H]⁺.

### Example 21: Synthesis of compound 21

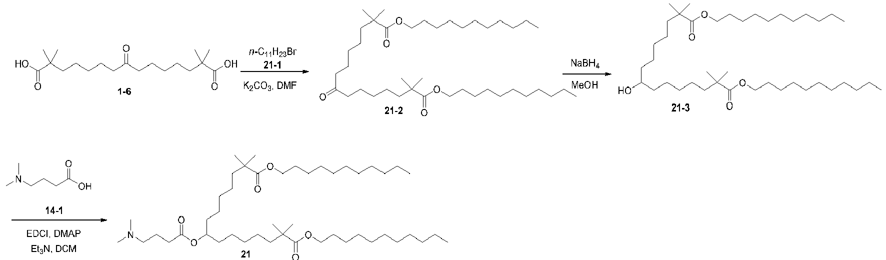

Referring to the method of Example 1, compound 21 was prepared as an oily product: 31.2 mg.

¹H NMR (400 MHz, CDCl₃): δ ppm 0.79 (t, *J* = 7.2 Hz, 6H), 1.08 (s, 12H), 1.25 (m, 44H), 1.39 (m, 8H), 1.51 (m, 4H), 1.82 (m, 2H), 2.25 (t, *J* = 7.2 Hz, 2H), 2.32 (s, 6H), 2.41 (m, 2H), 3.96 (t, *J* = 6.8 Hz, 4H), 4.75 (m, 1H); ESI-MS m/z: 766.7 [M+H]⁺.

### Example 22: Synthesis of compound 22

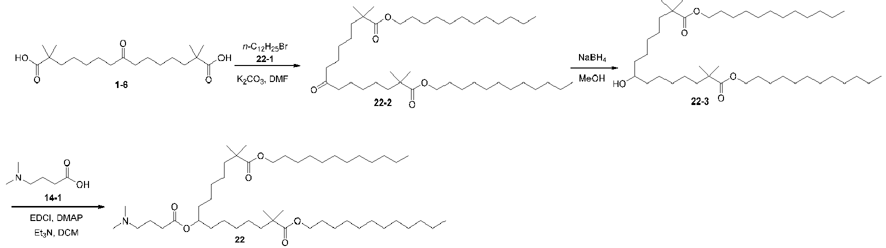

Referring to the method of Example 1, compound 22 was prepared as an oily product: 31.8 mg.

¹H NMR (400 MHz, CDCl₃): δ ppm 0.79 (t, *J* = 7.2 Hz, 6H), 1.07 (s, 12H), 1.28 (m, 48H), 1.40 (m, 8H), 1.53 (m, 4H), 1.84 (m, 2H), 2.26 (t, *J* = 7.2 Hz, 2H), 2.35 (s, 6H), 2.48 (m, 2H), 3.98 (t, *J* = 6.8 Hz, 4H), 4.75 (m, 1H); ESI-MS m/z: 794.7 [M+H]⁺.

### Example 23: Synthesis of compound 23

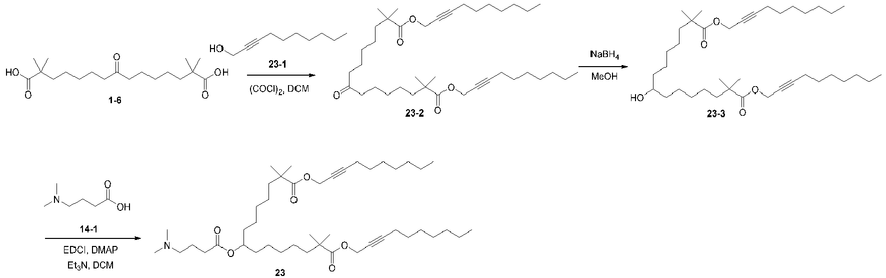

Referring to the method of Example 7, compound 23 was prepared as an oily product: 31.0 mg.

¹H NMR (400 MHz, CDCl₃): δ ppm 0.87 (t, *J* = 7.2 Hz, 6H), 1.16 (s, 12H), 1.20-1.39 (m, 28H), 1.45-1.54 (m, 12H), 1.74-1.82 (m, 2H), 2.12-2.35 (m, 14), 4.63 (t, *J* = 2.4 Hz, 4H), 4.79-4.88 (m, 1H); ESI-MS m/z: 730.6 [M+H]⁺.

### Example 24: Synthesis of compound 24

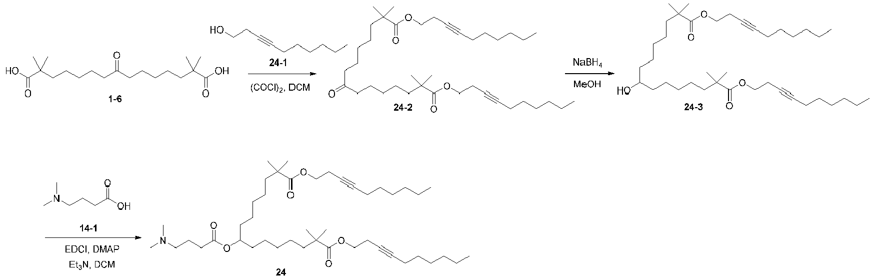

Referring to the method of Example 7, compound 24 was prepared as an oily product: 31.0 mg.

¹H NMR (400 MHz, CDCl₃): δ ppm 0.88 (t, *J* = 7.2 Hz, 6H), 1.15 (s, 12H), 1.20-1.38 (m, 24H), 1.43-1.52 (m, 12H), 1.76-1.84 (m, 2H), 2.09-2.14 (m, 4H), 2.23 (s, 6H), 2.28-2.36 (m, 4H), 2.43-2.49 (m, 4H), 4.10 (t, *J* = 7.2 Hz, 4H), 4.80-4.88 (m, 1H); ESI-MS m/z: 730.6 [M+H]⁺.

### Example 25: Synthesis of compound 25

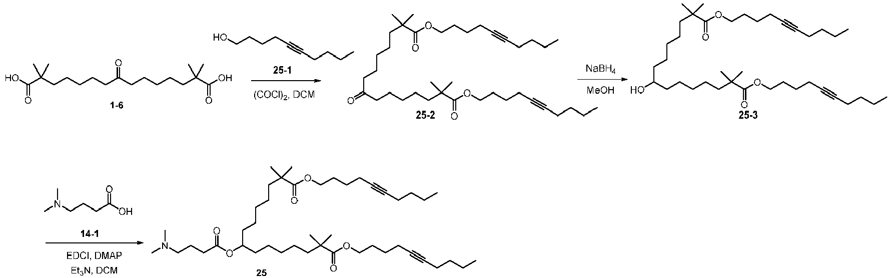

Referring to the method of Example 7, compound 25 was prepared as an oily product: 32.1 mg.

¹H NMR (400 MHz, CDCl₃): δ ppm 0.84 (t, *J* = 7.2 Hz, 6H), 1.08 (s, 12H), 1.02-1.21 (m, 12H), 1.38-1.47 (m, 22 H), 1.59-1.78 (m, 6H), 2.02-2.17 (m, 14 H), 2.19-2.30 (m, 4 H), 4.01 (t, *J* = 6.8 Hz, 4H), 4.71-4.83 (m, 1H); ESI-MS m/z: 730.6 [M+H]⁺.

### Example 26: Synthesis of compound 26

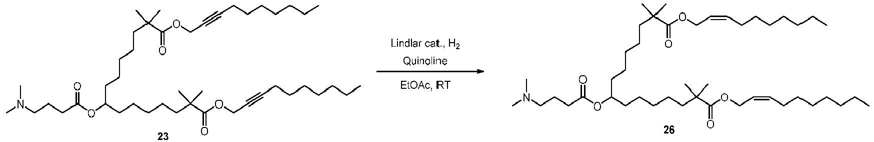

Compound 23 (300 mg, 0.41 mmol) and quinoline (106 mg, 0.82 mmol) were dissolved in 3.0 mL of ethyl acetate, and the air in the reaction system was replaced with nitrogen for 2-3 min at room temperature, then lindlar catalyst (16.9 mg) was added. Hydrogen gas was introduced to the reaction solution and the air was replaced with hydrogen for 2-3 min. The reaction system was kept under hydrogen atmosphere (15 psi) at room temperature for 30 min. The complete disappearance of the reactants was monitored by LC-MS. The reaction solution was filtered, and the filter cake was rinsed with ethyl acetate 3-4 times. The combined ethyl acetate was collected and the organic solvent was removed using a rotary-evaporator to give the crude product, which was purified by preparative high performance liquid chromatography to give compound 26 (31.3 mg).

¹H NMR (400 MHz, CDCl₃): δ ppm 0.81 (t, *J* = 7.2 Hz, 6H), 1.08 (s, 12H), 1.15-1.28 (m, 32H), 1.38-1.44 (m, 8H), 1.70-1.79 (m, 2H), 2.01 (m, 4H), 2.15 (s, 6H), 2.16-2.28 (m, 4H), 4.54 (d, *J* = 12.0 Hz, 4H), 4.75 (m, 1H), 5.39-5.59 (m, 4H); ESI-MS m/z: 734.6 [M+H]⁺.

### Example 27: Synthesis of compound 27

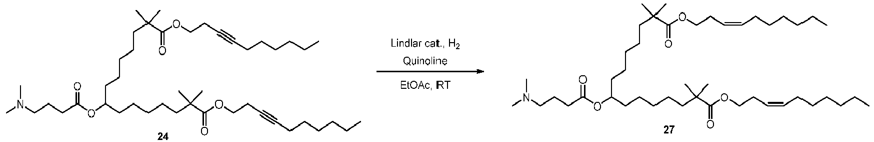

Referring to the method of Example 26, compound 27 was prepared as an oily product: 35.0 mg.

¹H NMR (400 MHz, CDCl₃): δ ppm 0.82 (m, 6H), 1.08 (s, 12H), 1.14-1.31 (m, 28H), 1.37-1.45 (m, 8H), 1.70-1.79 (m, 2H), 1.96 (m, 4H), 2.06-2.36 (m, 14H), 3.98 (t, *J* = 7.2 Hz, 4H), 4.74-4.82 (m, 1H), 5.22-5.31 (m, 2H), 5.37-5.48 (m, 2H); ESI-MS m/z: 734.7 [M+H]⁺.

### Example 28: Synthesis of compound 28

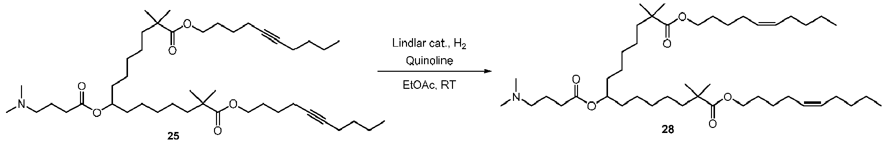

Referring to the method of Example 26, compound 28 was prepared as an oily product: 31.8 mg.

¹H NMR (400 MHz, CDCl₃): δ ppm 0.92 (t, *J* = 6.8 Hz, 6H), 1.18 (s, 12H), 1.21-1.39 (m, 22H), 1.40-1.59 (m, 12H), 1.60-1.72 (m, 4 H), 1.89-2.01 (m, 2 H), 2.02-2.15 (m, 8 H), 2.34-2.69 (m, 8 H), 4.08 (t, *J* = 6.4 Hz, 4 H), 4.82-4.92 (m, 1 H), 5.30-5.48 (m, 4 H); ESI-MS m/z: 734.6 [M+H]⁺.

### Example 30: Synthesis of compound 30

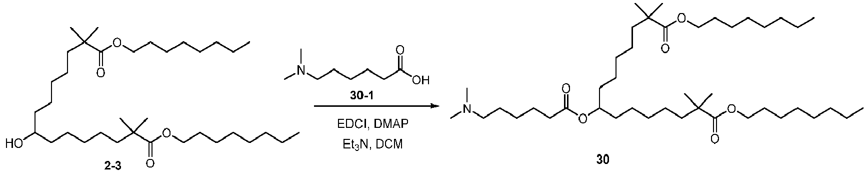

Referring to the method of Example 1, compound 30 was prepared as an oily product: 33.0 mg.

¹H NMR (400 MHz, CDCl₃): δ ppm 0.92 (t, *J* = 6.8 Hz, 6 H), 1.18 (s, 12 H), 1.19-1.37 (m, 36 H), 1.45-1.57 (m, 8 H), 1.58-1.74 (m, 8 H), 2.27-2.50 (m, 8 H), 4.07 (t, *J* = 6.8 Hz, 4 H), 4.83-4.90 (m, 1 H); ESI-MS m/z: 710.6 [M+H]⁺.

### Example 32: Synthesis of compound 32

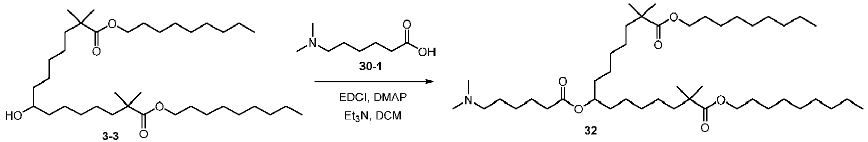

Referring to the method of Example 1, compound 32 was prepared as an oily product: 31.1 mg.

¹H NMR (400 MHz, CDCl₃): δ ppm 0.80 (t, *J* = 6.8 Hz, 6H), 1.08 (s, 12H), 1.20-1.27 (m, 34H), 1.34-1.47 (m, 12H), 1.48-1.62 (m, 8H), 2.15 (s, 6H), 2.19-2.24 (m, 4H), 3.97 (t, *J* = 6.8 Hz, 4H), 4.74-4.80 (m, 1H); ESI-MS m/z: 738.6 [M+H]⁺.

### Example 33: Synthesis of compound 33

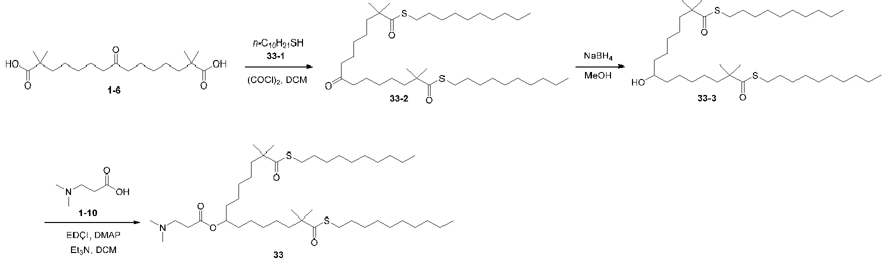

Compound 1-6 (448 mg, 1.3 mmol) was dissolved in 5.0 mL of dichloromethane, and the reaction system was cooled to 0 °C in an ice bath. DMF (10 µL, 0.13 mmol) was added, and oxalyl chloride (0.44 mL, 5.2 mmol) was then added dropwise to the reaction solution. The ice bath was removed after the dropwise addition was completed and the mixture was stirred for 1 h at room temperature. The solvent was removed using a rotary-evaporator to give acyl chloride crude product (330 mg) as an oil, which was used directly in the next reaction step.

1-Decanethiol 33-1 (455 mg, 2.61 mmol) was added to a solution of crude acyl chloride (330 mg, 0.87 mmol) in DCE (3.0 mL), and the reaction was heated to 70 °C to react overnight. The reaction solution was cooled to room temperature and the solvent was removed using a rotary-evaporator to give the crude product, which was purified by silica gel column to give compound 33-2 (400 mg). ¹H NMR (400 MHz, CDCl₃): δ ppm 0.84-0.87 (m, 6H), 1.14-1.18 (m, 12H), 1.20-1.28 (m, 36H), 1.48-1.55 (m, 12H), 2.33 (t, *J* = 7.2 Hz, 4H), 2.79 (t, *J* = 7.2 Hz, 4H).

Compound 33-2 (300 mg, 0.46 mmol) was dissolved in 3.0 mL of methanol and NaBH₄ (52.5 mg, 1.38 mmol) was added in batches. The reaction solution was stirred under nitrogen atmosphere at room temperature for 2 h. The complete disappearance of the reaction material was monitored by TLC. The reaction solution was quenched by adding saturated ammonium chloride solution, and extracted with ethyl acetate. The organic phases were combined, dried over anhydrous sodium sulfate, and filtered. The filtrate was collected and concentrated to give 300 mg of crude compound 33-3, which was directly used in the next reaction step without further purification.

Crude compound 33-3 (150 mg, 0.23 mmol) was dissolved in 3.0 mL of dichloromethane, and 1-10 (80.2 mg, 0.69 mmol), EDCI (131 mg, 0.69 mmol), triethylamine (0.1 mL, 0.69 mmol) and DMAP (28 mg, 0.23 mmol) were added to the reaction system. The reaction solution was stirred at room temperature for 12 h. The reaction solution was then quenched by adding saturated ammonium chloride solution, and extracted with dichloromethane. The organic phases were combined, dried over anhydrous sodium sulfate, and filtered. The filtrate was collected and concentrated to give the crude product, which was passed through preparative high performance liquid chromatography to give compound 33 (28.6 mg).

¹H NMR (400 MHz, CDCl₃): δ ppm 0.81 (t, *J* = 7.2 Hz, 6H), 1.15 (s, 12H), 1.31 (m, 40H), 1.48 (m, 12H), 2.23 (s, 6H), 2.42 (m, 4H), 2.80 (t, *J* = 7.2 Hz, 4H), 4.82 (m, 1H); ESI-MS m/z: 756.6 [M+H]⁺.

### Example 34: Synthesis of compound 34

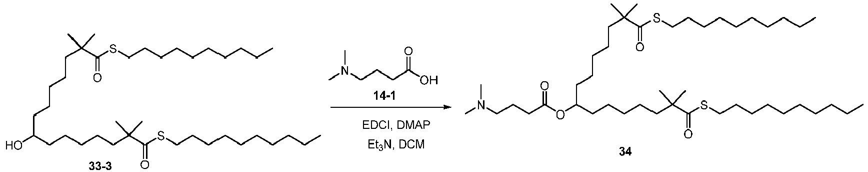

Referring to the method of Example 33, compound 34 was prepared as an oily product: 105.2 mg.

¹H NMR (400 MHz, CDCl₃): δ ppm 0.85 (t, *J* = 7.2 Hz, 6H), 1.15 (s, 12H), 1.6-1.32 (m, 40H), 1.37-1.53 (m, 14H), 1.75 (m, 2H), 2.24-2.34 (m, 8H), 2.80 (t, *J* = 7.2 Hz, 4H), 4.72-4.82 (m, 1H); ESI-MS m/z: 770.6 [M+H]⁺.

### Example 36: Synthesis of compound 36

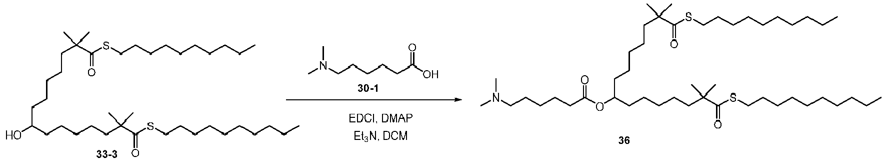

Referring to the method of Example 33, compound 36 was prepared as an oily product: 33.4 mg.

¹H NMR (400 MHz, CDCl₃): δ ppm 0.86 (t, *J* = 6.8 Hz, 6H), 1.16 (s, 12H), 1.18-1.38 (m, 40H), 1.41-1.59 (m, 16H), 1.61-1.67 (m, 2H), 2.19-2.33 (m, 10H), 2.82 (t, *J* = 7.2 Hz, 4H), 4.83 (m, H); ESI-MS m/z: 798.6 [M+H]⁺.

### Example 37: Synthesis of compound 37

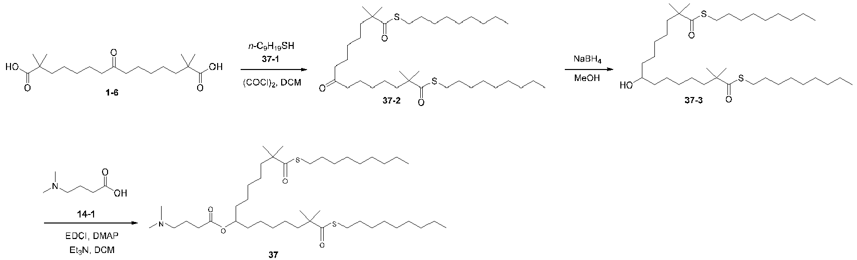

Referring to the method of Example 33, compound 37 was prepared as an oily product: 33.2 mg.

¹H NMR (400 MHz, CDCl₃): δ ppm 0.87 (t, *J* = 6.8 Hz, 6H), 1.20 (s, 12H), 1.19-1.37 (m, 36H), 1.39-1.56 (m, 12H), 1.75-1.84 (m, 2H), 2.24 (s, 6H), 2.28-2.34 (m, 4H), 2.81 (t, *J* = 7.2 Hz, 4H), 4.79-4.87 (m, 1H); ESI-MS m/z: 742.6 [M+H]⁺.

### Example 39: Synthesis of compound 39

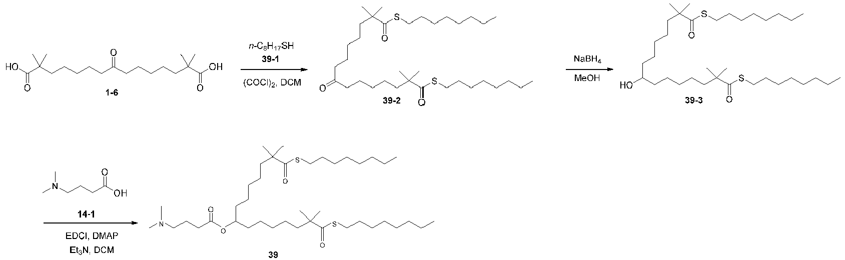

Referring to the method of Example 33, compound 39 was prepared as an oily product: 30.7 mg.

¹H NMR (400 MHz, CDCl₃): δ ppm 0.91 (t, *J* = 7.2 Hz, 6H), 1.22 (s, 12H), 1.17-1.38 (m, 32H), 1.47-1.58 (m, 12H), 1.78-1.87 (m, 2H), 2.28 (s, 6H), 2.34-2.37 (m, 4H), 2.85 (t, *J* = 7.2 Hz, 4H), 4.81-4.90 (m, 1H); ESI-MS m/z: 714.6 [M+H]⁺.

### Example 40: Synthesis of compound 40

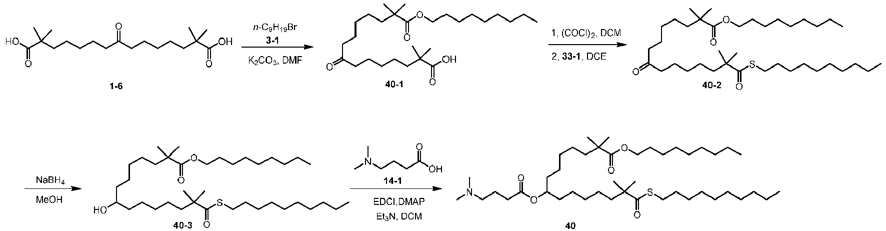

Potassium carbonate (1.55 g, 11.2 mmol, 4.0 eq.) was added to a solution of compound 1-6 (959 mg, 2.8 mmol, 1.0 eq.) and 3-1 (638 mg, 3.08 mmol, 1.1 eq.) in DMF. Then the reaction was warmed up to 60 °C for 4 h. The reaction was cooled to room temperature. The reaction system was quenched with saturated aqueous sodium chloride solution and extracted with ethyl acetate. The organic phases were combined, dried over anhydrous sodium sulfate, and filtered. The filtrate was concentrated to dryness to give the crude product, which was purified by silica gel column to give compound 40-1 (682 mg).

Compound 40-1 (324 mg, 0.69 mmol, 1.0 eq.) was dissolved in 5.0 mL of dichloromethane, and the reaction system was cooled to 0 °C in an ice bath. 2 drops of DMF were added and oxalyl chloride (0.24 mL, 2.8 mmol, 4.0 eq.) was then added dropwise to the reaction solution. The ice bath was removed after the dropwise addition was completed and the mixture was stirred for 1 h at room temperature. The solvent was removed using a rotary-evaporator to give acyl chloride crude product (309 mg) as an oil, which was used directly in the next reaction step.

1-Decanethiol 33-1 (331 mg, 1.9 mmol, 3.0 eq) was added to a solution of crude acyl chloride (309 mg) in DCE (3.0 mL), and the reaction was heated to 70 °C to react overnight. The reaction solution was cooled to room temperature and the solvent was removed using a rotary-evaporator to give the crude product, which was purified by silica gel column to give compound 40-2 (274 mg).

Then referring to the method of Example 1, compound 40 was prepared as an oily product: 34.2 mg.

¹H NMR (400 MHz, CDCl₃): δ ppm 0.81 (t, *J* = 7.2 Hz, 6H), 1.05 (s, 6H), 1.12 (s, 6H), 1.08-1.28 (m, 36H), 1.37-1.57 (m, 14H), 1.71-1.76 (m, 2H), 2.22 (s, 6H), 2.25-2.31 (m, 4H), 2.75 (t, *J* = 7.2 Hz, 2H), 3.97 (t, *J* = 7.2 Hz, 2H), 4.75-4.84 (m, 1H); ESI-MS m/z: 740.6 [M+H]⁺.

### Example 41: Synthesis of compound 41

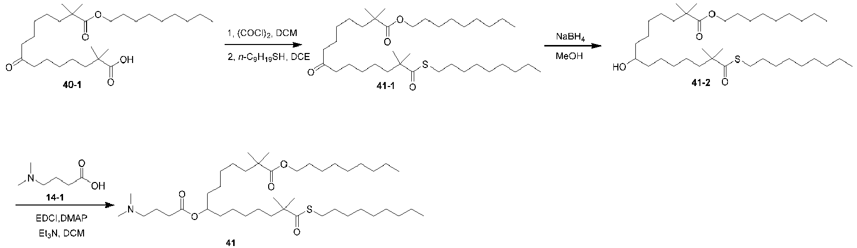

Referring to the method of Example 40, compound 41 was prepared as an oily product: 31.1 mg.

¹H NMR (400 MHz, CDCl₃): δ ppm 0.86-0.89 (m, 6H), 1.10 (s, 6H), 1.15 (s, 6H), 1.08-1.31 (m, 34H), 1.41-1.61 (m, 14H), 1.74-1.82 (m, 2H), 2.17-2.35 (m, 10H), 2.85 (t, *J* = 7.2 Hz, 2H), 4.03 (t, *J* = 7.2 Hz, 2H), 4.82-4.87 (m, 1H); ESI-MS m/z: 726.6 [M+H]⁺.

### Example 42: Synthesis of compound 42

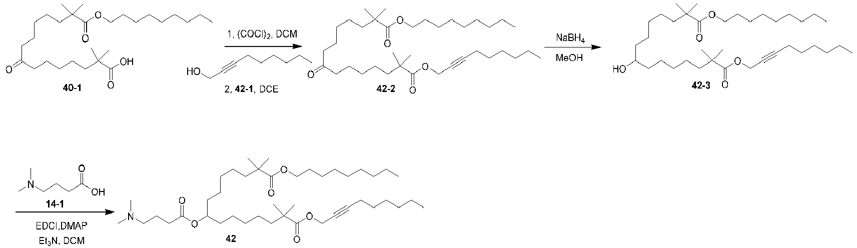

Referring to the method of Example 40, compound 42 was prepared as an oily product: 30.9 mg.

¹H NMR (400 MHz, CDCl₃): δ ppm 0.77-0.82 (m, 6H), 1.05 (s, 6H), 1.10 (s, 6H), 1.11-1.28 (m, 31H), 1.33-1.42 (m, 9H), 1.47-1.59 (m, 2H), 1.73-1.81 (m, 2H), 2.08-2.14 (m, 2H), 2.21-2.33 (m, 10H), 3.97 (t, *J* = 7.2 Hz, 2H), 4.55 (m, 2H), 4.72-4.81 (m, 1H); ESI-MS m/z: 706.6 [M+H]⁺.

### Example 43: Synthesis of compound 43

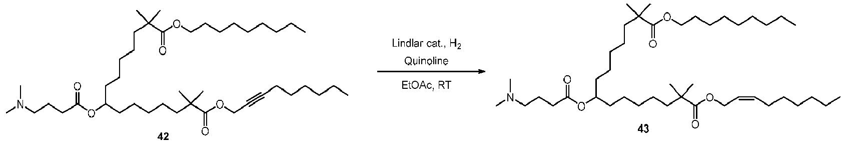

Referring to the method of Example 26, compound 43 was prepared as an oily product: 31.3 mg.

¹H NMR (400 MHz, CDCl₃): δ ppm 0.80 (m, 6H), 1.05 (s, 12H), 1.08-1.28 (m, 34H), 1.36-1.47 (m, 8H), 1.49-1.58 (m, 2H), 1.73-1.82 (m, 2H), 1.98-2.07 (m, 2H), 2.21-2.38 (m, 8H), 3.97 (t, *J* = 7.2 Hz, 2H), 4.53 (d, *J* = 7.2 Hz, 2H), 4.72-4.78 (m, 1H), 5.41-5.59 (m, 2H); ESI-MS m/z: 708.6 [M+H]⁺.

### Example 44: Synthesis of compound 44

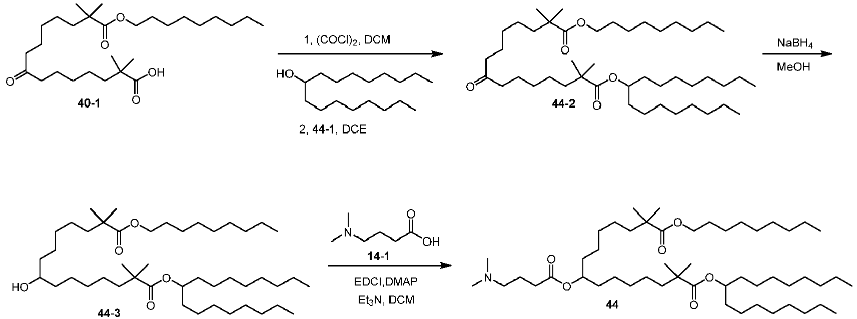

Referring to the method of Example 40, compound 44 was prepared as an oily product: 33.1 mg.

¹H NMR (400 MHz, CDCl₃): δ ppm 0.85 (m, 9H), 1.13 (s, 12H), 1.14-1.33 (m, 46H), 1.37-1.59 (m, 16H), 1.78-1.87 (m, 2H), 2.17-2.35 (m, 10H), 4.03 (t, *J* = 6.8 Hz, 2H), 4.79-4.88 (m, 2H); ESI-MS m/z: 822.8 [M+H]⁺.

### Example 45: Synthesis of compound 45

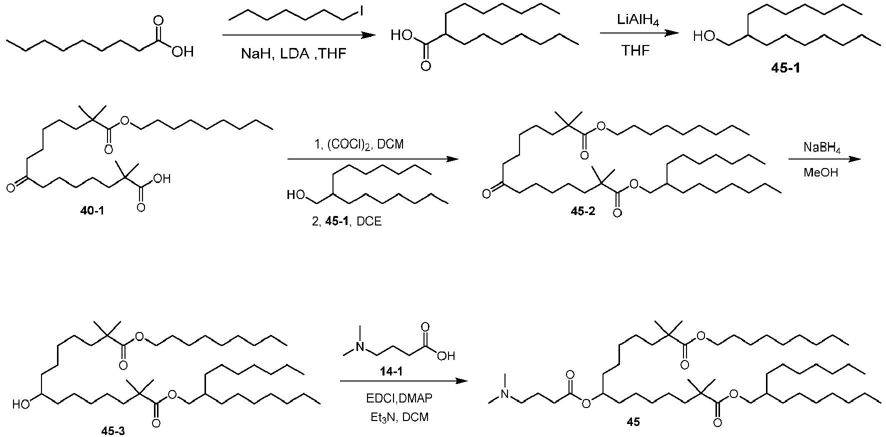

n-Nonanoic acid (3.0 g, 19 mmol) was added to 50 mL of anhydrous tetrahydrofuran and the reaction solution was cooled to 0°C in an ice bath. Sodium hydride (836 mg, 20.9 mmol) and LDA (49.4 mL, 24.7 mmol) were added to the reaction solution, and the reaction solution was stirred at 0°C for 1 hour. Then 1-iodoheptane was added dropwise to the reaction system. The ice bath was removed, then the mixture was reacted at room temperature for 12 h. The reaction solution was quenched by pouring the reaction solution into saturated ammonium chloride solution, and extracted with ethyl acetate. The organic phase was collected, dried over anhydrous sodium sulfate, and filtered. The filtrate was collected, and concentrated to remove the solvent to give the crude product, which was purified by silica gel column to give 2.0 g of compound 2-heptylnonanoic acid.

The 2-heptylnonanoic acid (2.0 g, 7.8 mmo) obtained in the previous step was dissolved in 30 mL of anhydrous tetrahydrofuran, and lithium tetrahydroaluminum (593 mg, 15.6 mmol) was added to the reaction solution. The reaction system was heated to 80°C to react for 2 hours. The reaction solution was cooled to room temperature, quenched by pouring the reaction solution into saturated ammonium chloride solution, and extracted with ethyl acetate. The organic phase was collected, dried over anhydrous sodium sulfate, and filtered. The filtrate was collected, and concentrated to remove the solvent to give the crude product, which was purified by silica gel column to give 1.3 g of compound 45-1.

Then referring to the method of Example 40, compound 45 was prepared as an oily product: 31.6 mg.

¹H NMR (400 MHz, CDCl₃): δ ppm 0.88 (t, *J* = 6.8 Hz, 9H), 1.14 (s, 12H), 1.15-1.26 (m, 47H), 1.47-1.50 (m, 8H), 1.57-1.62 (m, 4H), 1.79-1.81 (m, 2H), 2.25 (s, 6H), 2.32 (t, *J* = 7.2 Hz, 4H), 3.93 (d, *J* = 5.6 Hz, 2H), 4.03 (t, *J* = 7.2 Hz, 2H), 4.81-4.87 (m, 1H); ESI-MS m/z: 808.7 [M+H]⁺.

### Example 46: Synthesis of compound 46

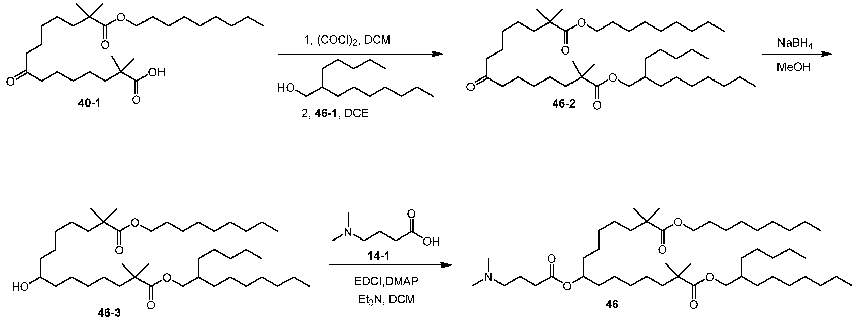

Referring to the method of Example 40, compound 46 was prepared as an oily product: 32.6 mg.

¹H NMR (400 MHz, CDCl₃): δ ppm 0.88 (t, *J* = 7.2 Hz, 9H), 1.14 (s, 12H), 1.15-1.28 (m, 37H), 1.47-1.59 (m, 18H), 1.75-1.84 (m, 2H), 2.24-2.35 (m, 10H), 3.95 (d, *J* = 5.6 Hz, 2H), 4.03 (t, *J* = 6.8 Hz, 2H), 4.80-4.87 (m, 1H); ESI-MS m/z: 780.7 [M+H]⁺.

### Example 47: Synthesis of compound 47

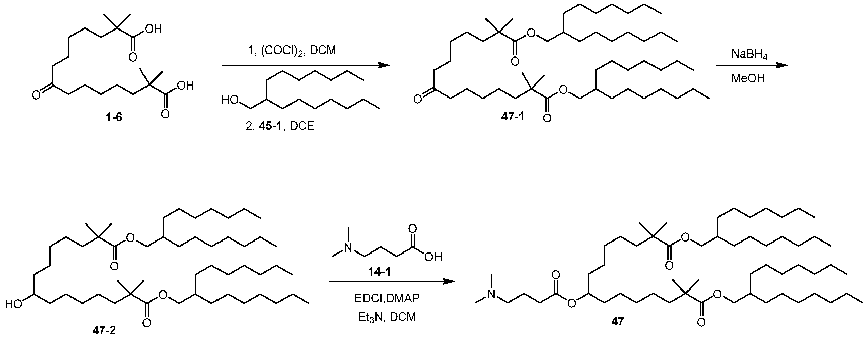

Referring to the method of Example 7, compound 47 was prepared as an oily product: 33.1 mg.

¹H NMR (400 MHz, CDCl₃): δ ppm 0.81 (t, *J* = 6.8 Hz, 12H), 1.08 (s, 12H), 1.09-1.24 (m, 56H), 1.40-1.61 (m, 14H), 1.67-1.72 (m, 2H), 2.17 (s, 6H), 2.19-2.28 (m, 4H), 3.88 (d, *J* = 5.6 Hz, 4H), 4.74-4.80 (m, 1H); ESI-MS m/z: 906.8 [M+H]⁺.

### Example 48: Synthesis of compound 48

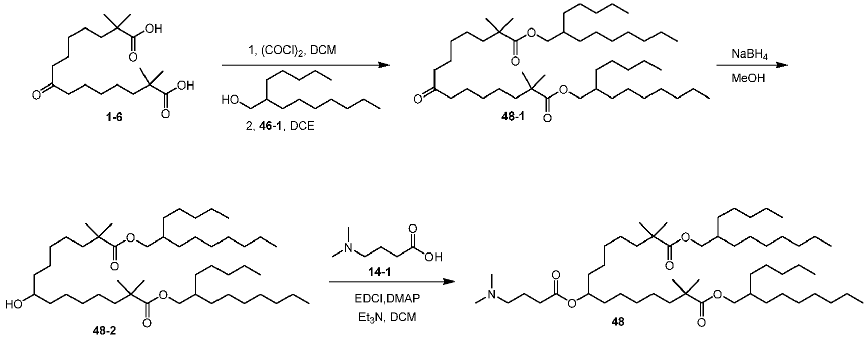

Referring to the method of Example 7, compound 48 was prepared as an oily product: 34.8 mg.

¹H NMR (400 MHz, CDCl₃): δ ppm 0.81 (t, *J* = 7.2 Hz, 12H), 1.08 (s, 12H), 1.09-1.23(m, 48H), 1.37-1.64 (m, 14H), 1.67-1.73 (m, 2H), 2.15 (s, 6H), 2.20-2.37 (m, 4H), 3.88 (d, *J* = 5.6 Hz, 4H), 4.74-4.89 (m, 1H); ESI-MS m/z: 850.8 [M+H]⁺.

The compounds of Table 2 were synthesized using the methods of the above examples, or similar methods using the corresponding intermediates.

**Table 2**

| | |
|---|---|
| 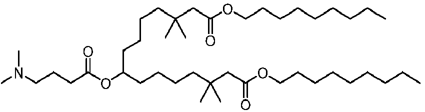 Example 49: compound 49 | 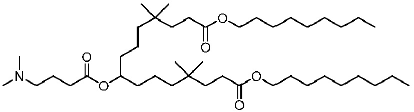 Example 50: compound 50 |
| [M+H]⁺: 710.6 | [M+H]⁺: 710.6 |
| 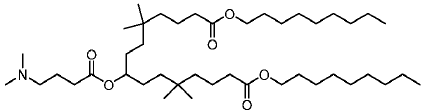 Example 51: compound 51 | 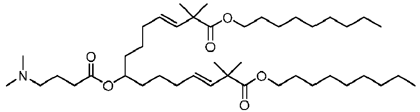 Example 52: compound 52 |
| [M+H]⁺: 710.6 | [M+H]⁺: 706.6 |
| 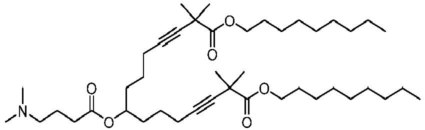 Example 53: compound 53 | 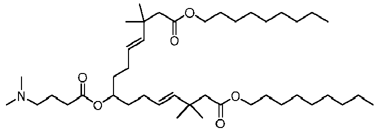 Example 54: compound 54 |
| [M+H]⁺: 702.6 | [M+H]⁺: 706.6 |
| 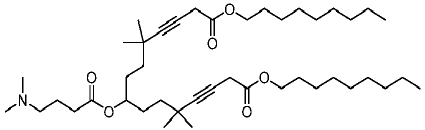 Example 55: compound 55 | 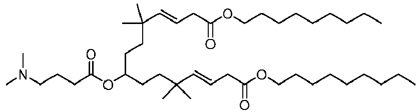 Example 56: compound 56 |
| [M+H]⁺: 702.6 | [M+H]⁺: 706.6 |
| 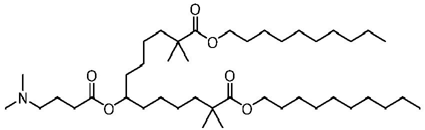 Example 57: compound 57 | 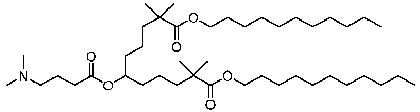 Example 58: compound 58 |
| [M+H]⁺: 710.6 | [M+H]⁺: 710.6 |
| 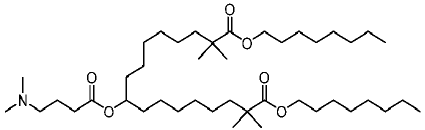 Example 59: compound 59 | 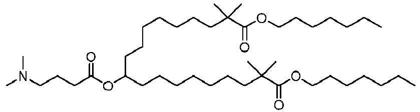 Example 60: compound 60 |
| [M+H]⁺: 710.6 | [M+H]⁺: 710.6 |
| 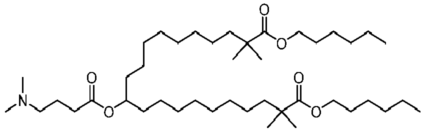 Example 61: compound 61 | 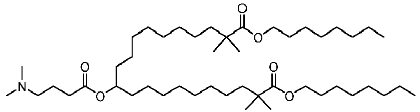 Example 62: compound 62 |
| [M+H]⁺: 710.6 | [M+H]⁺: 766.7 |
| 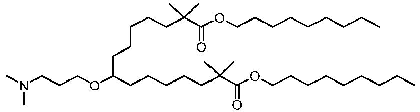 Example 63: compound 63 | 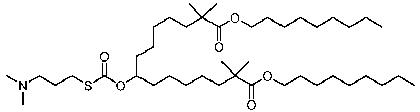 Example 64: compound 64 |
| [M+H]⁺: 682.6 | [M+H]⁺: 742.6 |
| 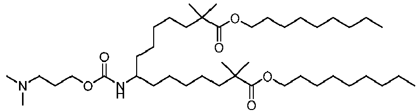 Example 65: compound 65 | 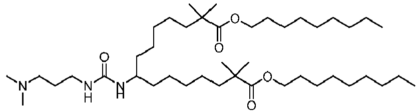 Example 66: compound 66 |
| M+H⁺: 725.6 | M+H⁺: 724.6 |
| 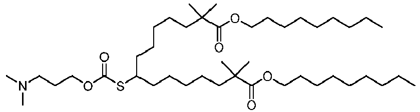 Example 67: compound 67 | 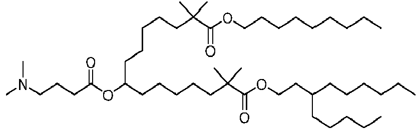 Example 68: compound 68 |
| [M+H]⁺: 742.6 | [M+H]⁺: 780.7 |
| 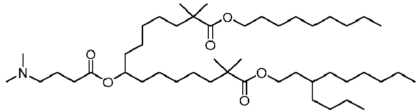 Example 69: compound 69 | 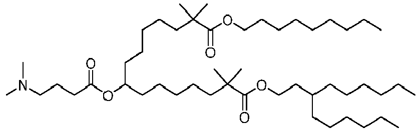 Example 70: compound 70 |
| [M+H]⁺: 766.7 | [M+H]⁺: 794.7 |
| 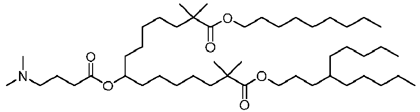 Example 71: compound 71 | 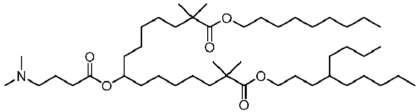 Example 72: compound 72 |
| [M+H]⁺: 780.7 | [M+H]⁺: 766.7 |
| 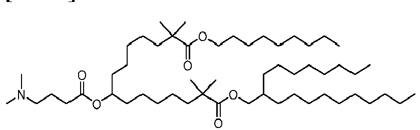 Example 73: compound 73 | 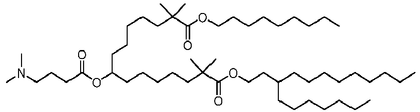 Example 74: compound 74 |
| [M+H]⁺: 864.8 | [M+H]⁺: 850.8 |
| 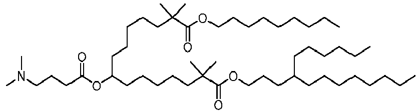 Example 75: compound 75 | |
| [M+H]⁺: 836.8 | |
| 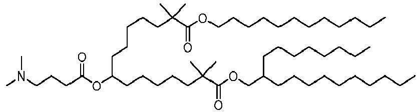 Example 77: compound 77 | 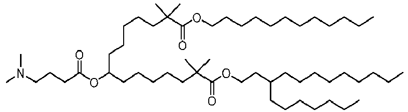 Example 78: compound 78 |
| [M+H]⁺: 906.8 | M+H⁺: 892.8 |
| 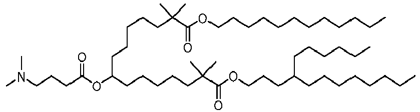 Example 79: compound 79 | 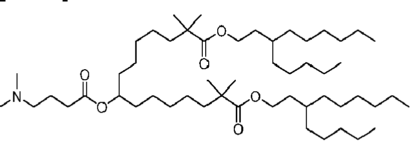 Example 80: compound 80 |
| [M+H]+: 878.8 | [M+H]⁺: 850.8 |
| 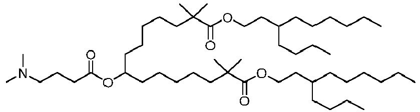 Example 81: compound 81 | 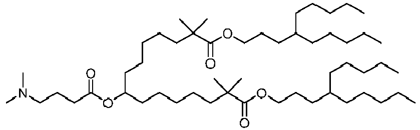 Example 82: compound 82 |
| [M+H]⁺: 822.8 | [M+H]+: 850.8 |
| 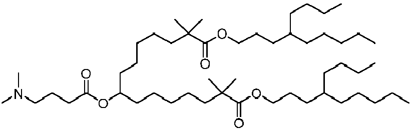 Example 83: compound 83 | 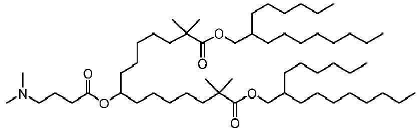 Example 84: compound 84 |
| [M+H]+: 822.8 | [M+H]⁺: 906.8 |
| 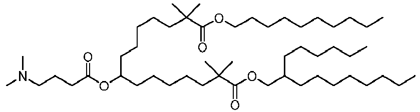 Example 85: compound 85 | 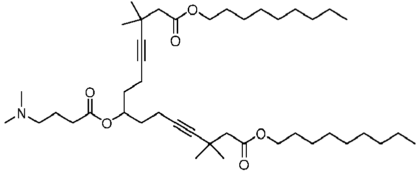 Example 86: compound 86 |
| [M+H]⁺: 822.8 | |
| | [M+H]⁺: 702.6 |
| 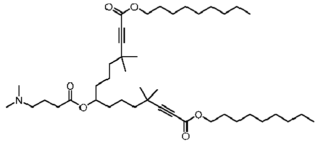 | 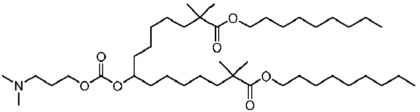 Example 88: compound 88 |
| Example 87: compound 87 | [M+H]⁺: 726.6 |
| [M+H]⁺: 702.6 | |
| 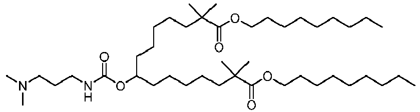 Example 89: compound 89 | |
| [M+H]⁺: 725.6 | |

### Example 90: Synthesis of compound 90

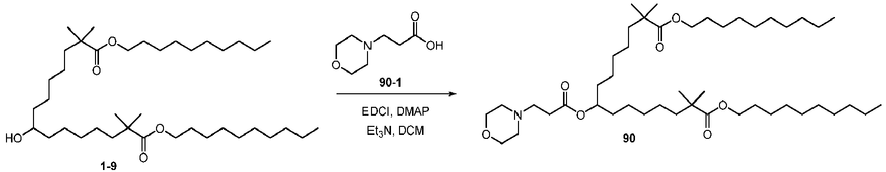

Referring to the method of Example 1, compound 90 was prepared as an oily product: 40.5 mg.

¹H NMR (400 MHz, CDCl₃): δ ppm 0.81 (t, *J* = 6.8 Hz, 6H), 1.08 (s, 12H), 1.10-1.28 (m, 36H), 1.38-1.47 (m, 12H), 1.50-1.58 (m, 4H), 2.40 (m, 6H), 2.58 (t, *J* = 6.8 Hz, 2H), 3.59-3.65 (m, 4H), 3.97 (t, *J* = 6.8 Hz, 4H), 4.75-4.83 (m, 1H); ESI-MS m/z: 766.7 [M+H]⁺.

### Example 91: Synthesis of compound 91

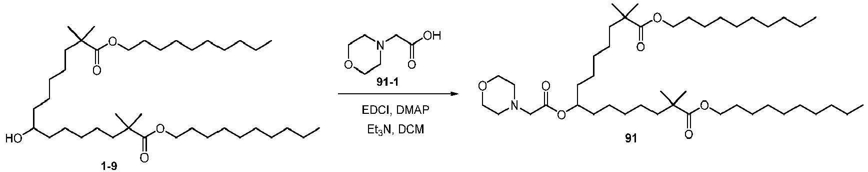

Referring to the method of Example 1, compound 91 was prepared as an oily product: 32.2 mg.

¹H NMR (400 MHz, CDCl₃): δ ppm 0.88 (t, *J* = 6.8 Hz, 6H), 1.15 (s, 12H), 1.16-1.38 (m, 40H), 1.46 (m, 8H), 1.60 (m, 4H), 2.59 (m, 4H), 3.19 (s, 2H), 3.76 (t, *J* = 4.8 Hz, 4H), 4.04 (t, *J* = 6.8 Hz, 4H), 4.91 (m, 1H); ESI-MS m/z: 752.7 [M+H]⁺.

### Example 92: Synthesis of compound 92

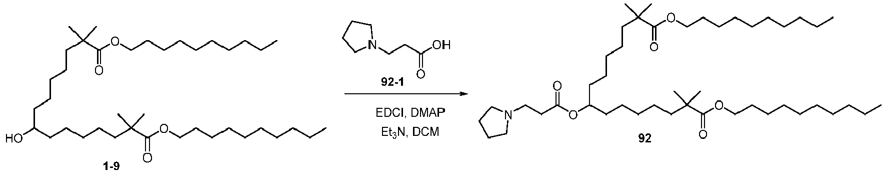

Referring to the method of Example 1, compound 92 was prepared as an oily product: 32 mg.

¹H NMR (400 MHz, CDCl₃): δ ppm 0.87 (t, *J* = 6.8 Hz, 6H), 1.13 (s, 12H), 1.28 (m, 42H), 1.46 (m, 8H), 1.45 (m, 4H), 1.76 (m, 4H), 2.50 (m, 4H), 2.76 (m, 2H), 4.01 (t, *J* = 6.8 Hz, 4H), 4.84 (m, 1H); ESI-MS m/z: 750.9 [M+H]⁺.

### Example 93: Synthesis of compound 93

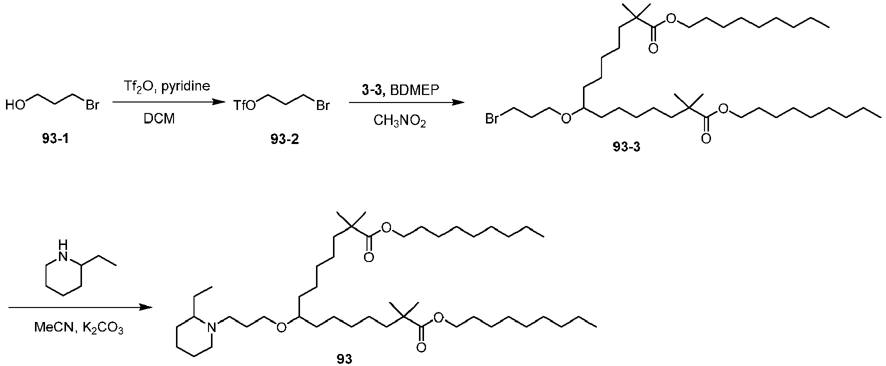

3-Bromopropanol (20 g, 144 mmol), trifluoromethanesulfonic anhydride (26.6 mL, 158 mmol) and pyridine (14.0 mL, 173 mmol) were added to a round bottom flask containing 500 mL of dichloromethane. The mixture was stirred at room temperature until the reaction materials were completely consumed by TLC monitoring. The reaction solution was quenched with 1 M hydrochloric acid solution, and extracted with dichloromethane. The organic phase were combined, dried over anhydrous sodium sulfate, and filtered to remove the sodium sulfate. The filtrate was collected. The solvent was removed using a rotary-evaporator to give 25 g of crude compound 93-2, which was used directly for subsequent reactions without further purification.

3-3 (6.0 g, 10 mmol) and crude compound 93-2 (3.0 g, 11 mmol) were added to a round bottom flask containing 50 mL of nitromethane, then 2,6-di-tert-butylpyridine (3.37 mL, 15 mmol) was added to the reaction solution. The reaction solution was warmed up to 95°C to react overnight. The reaction solution was cooled to room temperature. The solvent was removed using a rotary-evaporator to give a crude product. The crude product was then dissolved in dichloromethane, extracted after adding saturated aqueous ammonium chloride. The organic phase were collected and combined, dried over anhydrous sodium sulfate, and filtered to remove the sodium sulfate. The filtrate was collected. The solvent was removed using a rotary-evaporator and then purified by silica gel column to give compound 93-3 (2.3 g).

Compound 93-3 (251 mg, 0.35 mmol) and 2-ethylpiperidine (71 µL, 0.53 mmol) were dissolved in 3.0 mL of anhydrous acetonitrile and anhydrous potassium carbonate (73 mg, 0.53 mmol) was added to the reaction solution. The mixture was warmed up to 80 °C to react for 6 hours. The reaction solution was cooled to room temperature, quenched by adding saturated aqueous ammonium chloride, and extracted with dichloromethane. The organic phase were collected and combined, dried over anhydrous sodium sulfate, and filtered to remove the sodium sulfate. The filtrate was collected. The solvent was removed using a rotary-evaporator and then purified by preparative high performance liquid chromatography to give compound 93 (82 mg).

¹H NMR (400 MHz, CDCl₃): δ ppm 0.72-0.91 (m, 9H), 1.08 (s, 12H), 1.11-1.75 (m, 60H), 1.87-2.25 (m, 3H), 2.57-2.93 (m, 4H), 3.04-3.15 (m, 2H), 3.32-3.45 (m, 2H), 3.98 (d, *J* = 6.8 Hz, 4H); ESI-MS m/z: 750.6 [M+H]⁺.

### Example 94: Synthesis of compound 94

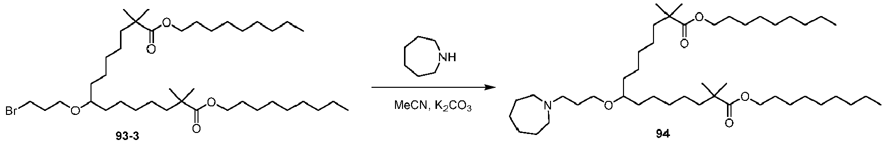

Referring to the method of Example 93, compound 94 was prepared as an oily product: 79.2 mg.

¹H NMR (400 MHz, CDCl₃): δ ppm 0.82 (t, *J* = 7.2 Hz, 6H), 1.09 (s, 12H), 1.11-1.34 (m, 44H), 1.52 (m, 14H), 2.45-2.74 (m, 6H), 3.07 (m, 1H), 3.38 (m, 2H), 3.94 (t, *J* = 6.8 Hz, 4H); ESI-MS m/z: 736.6 [M+H]⁺.

The compounds of Table 3 were synthesized using the methods of the above examples, or similar methods using the corresponding intermediates.

**Table 3**

| | |
|---|---|
| 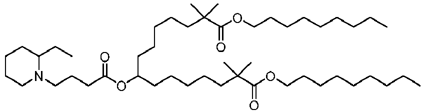 Example 95: compound 95 | 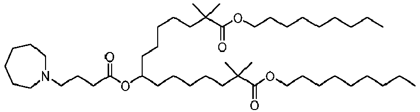 Example 96: compound 96 |
| [M+H]⁺: 778.7 | [M+H]⁺: 764.7 |

### Example 97: Synthesis of compound 97

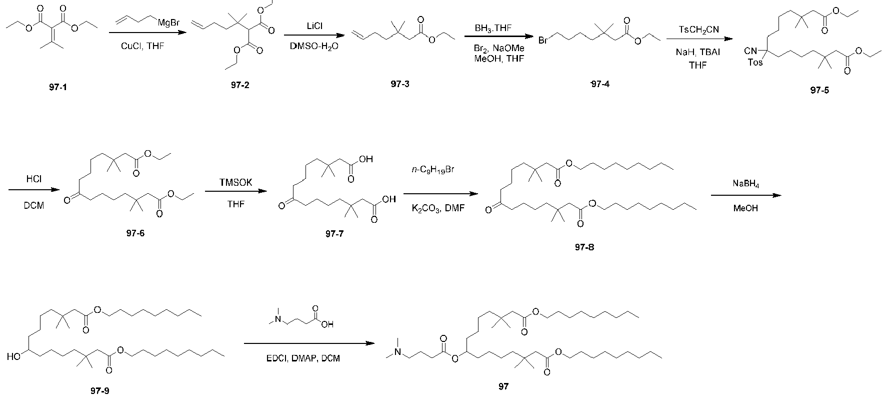

To a round bottom flask were added CuCl (989 mg, 9.99 mmol) and 160 mL THF, and the reaction system was cooled to -30°C. Then 3-butenylmagnesium bromide (1 M, 299 mL) was added. 160 mL of solution of compound 97-1 (40.0 g, 199 mmol) in tetrahydrofuran was slowly added to the reaction system. After the dropwise addition was completed, the reaction system was warmed up to room temperature and stirred to react for another 2 hours. After the reaction material 97-1 was reacted completely by TLC monitoring, the reaction solution was quenched with 300 mL of saturated aqueous ammonium chloride, and extracted with ethyl acetate. The organic phases were combined, dried over anhydrous sodium sulfate, and filtered. The filtrate was concentrated to dryness to give the crude product. The crude was purified by silica gel column to give compound 97-2 (45.0 g).

Compound 97-2 (42.0 g, 164 mmol) was dissolved in 400 mL of DMSO, and 4 mL of water and LiCl (27.8 g, 655 mmol) were added to the reaction solution. Then the reaction system was heated to 180 °C and stirred until the reactant 97-2 was reacted completely by TLC monitoring. The reaction system was cooled to room temperature, then poured into water and extracted with ethyl acetate. The organic phases were combined, dried over anhydrous sodium sulfate, and filtered. The filtrate was concentrated to dryness to give the crude product 97-3 (31.0 g), which was used directly in the next reaction without further purification.

Crude product 97-3 (30.0 g, 163 mmol) was dissolved in 240 mL of tetrahydrofuran and BH₃·THF (1 M, 244 mL) was added dropwise to the reaction solution in an ice bath. Then the mixture was warmed up to room temperature and stirred for 2 h. The reaction system was then cooled to 0 °C in an ice bath and methanol (13.2 mL, 325 mmol), Br₂ (8.39 mL, 163 mmol) and sodium methoxide (43.9 g, 244 mmol) were added sequentially. The mixture was warmed up to room temperature and stirred for another 1h. The reaction solution was quenched with cold saturated aqueous ammonium chloride solution and extracted with ethyl acetate. The organic phases were combined, dried over anhydrous sodium sulfate, and filtered. The filtrate was concentrated to dryness to give the crude product, which was purified by silica gel column to give compound 97-4 (14.0 g).

Then referring to the method of Example 1, compound 97 was prepared as an oily product: 31.6 mg.

¹H NMR (400 MHz, CDCl₃): δ ppm 0.84-0.90 (m, 6H), 0.93-1.01 (m, 12H), 1.20-1.31 (m, 32H), 1.45-1.62 (m, 16H), 2.17 (s, 4H), 2.19 - 2.44 (m, 8H), 3.99-4.08 (m, 4H), 4.81-4.91 (m, 1H); ESI-MS m/z: 710.7 [M+H]⁺.

### Example 98: Synthesis of compound 98

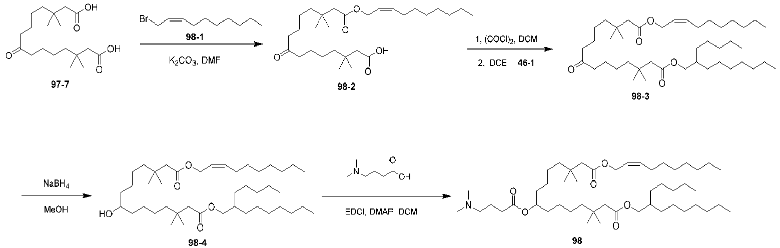

Referring to the method of Example 97, compound 98 was prepared as an oily product: 31.0 mg.

¹H NMR (400 MHz, CDCl₃): δ ppm 0.88 (t, *J* = 6.8 Hz, 9H), 0.97 (s, 12H), 1.25-1.39 (m, 38H), 1.45-1.59 (m, 10H), 1.79 (m, 2H), 2.06-2.13 (m, 2H), 2.18 (m, 4H), 2.20-2.39 (m, 9H), 3.94 (d, *J* = 5.6 Hz, 2H), 4.59 (d, *J* = 6.8 Hz, 2H), 4.82-4.87 (m, 1H), 5.48-5.53 (m, 1H), 5.60-5.64 (m, 1H); ESI-MS m/z: 792.7 [M+H]⁺.

### Example 99: Synthesis of compound 99

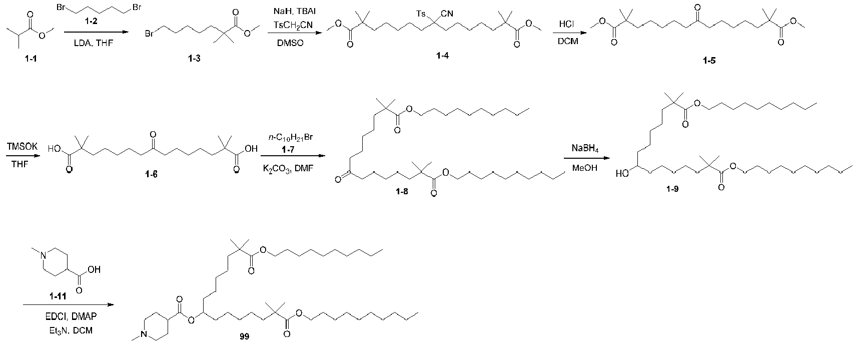

A solution of compound 1-1 (100 g, 979 mmol) in tetrahydrofuran (800 mL) was cooled to -40°C. LDA (2 M, 490 mL) was added slowly dropwise to the solution and the mixture was stirred for another 1 h after completion of the dropwise addition. A solution of 1-2 (315 g, 1.37 mol) in tetrahydrofuran (100 mL) was added dropwise to the reaction system at the same temperature and the reaction system was stirred overnight. The reaction system was quenched with saturated aqueous ammonium chloride, and extracted with ethyl acetate. The organic phases were combined, dried over anhydrous sodium sulfate, and filtered. The filtrate was concentrated to dryness to give a crude product. The crude product was purified by silica gel column to give compound 1-3 (115 g). ¹H NMR (400 MHz, CDCl₃): δ ppm 1.06-1.11 (m, 6 H), 1.13-1.22 (m, 2 H), 1.29-1.39 (m, 2 H), 1.42-1.49 (m, 2 H), 1.73-1.82 (m, 2 H), 3.28-3.40 (m, 2 H), 3.55-3.66 (m, 3 H).

A solution of compound 1-3 (100 g, 398 mmol), TsCH₂CN (38.9 g, 199 mmol) and TBAI (14.7 g, 39.8 mmol) in dimethyl sulfoxide (800 mL) was cooled to 0 °C, and sodium hydride (20.7 g, 517 mmol, 60% purity) was added slowly in batches. The mixture was reacted at room temperature overnight. The reaction system was quenched with saturated aqueous sodium chloride solution and extracted with ethyl acetate. The organic phases were combined, dried over anhydrous sodium sulfate, and filtered. The filtrate was concentrated to dryness to give 115 g of crude compound 1-4, which was used directly in the next reaction without isolation and purification.

To a solution of compound 1-4 crude (110 g, 205 mmol) in dichloromethane (880 mL) was added 330 mL of concentrated hydrochloric acid, and the mixture was reacted at room temperature for 2 h. The complete reaction of the substrate was monitored by TLC. The reaction system was quenched with saturated aqueous ammonium chloride solution and extracted with ethyl acetate. The organic phases were combined, dried over anhydrous sodium sulfate, and filtered. The filtrate was concentrated to dryness to give a crude product. The crude product was purified by silica gel column to give compound 1-5 (30.0 g, 80.9 mmol, 39.4%).

TMSOK (11.0 g, 86.4 mmol) was added to a solution of compound 1-5 (8.0 g, 21.6 mmol) in tetrahydrofuran (35.0 mL) at room temperature, and the reaction system was heated to 70°C with stirring. The complete consumption of reaction materials was monitored by TLC. The reaction solution was cooled to room temperature, and the organic solvent was removed by rotary evaporation. The crude product was added to 20 mL of water and extracted with dichloromethane. The aqueous layer was collected, and the solution was adjusted to a pH of <5 with 1 M hydrochloric acid. The solution was extracted with dichloromethane. The organic phases were combined, dried over anhydrous sodium sulfate, and filtered. The filtrate was collected and concentrated to give compound 1-6 (7.0 g). ¹H NMR (400 MHz, CDCl₃): δ ppm 1.03 (s, 12H), 1.08-1.17 (m, 8H), 1.34-1.45 (m, 8H), 2.21 (t, *J* = 7.2 Hz, 4H).

Potassium carbonate (482 mg, 3.48 mmol) was added to a solution of compound 1-6 (294 mg, 0.87 mmol) and 1-7 (771 mg, 3.48 mmol) in DMF, then the reaction was warmed up to 60 °C for 6 h. The complete disappearance of reactant 1-6 was monitored. The mixture was cooled to room temperature. The reaction system was quenched with saturated aqueous sodium chloride solution and extracted with ethyl acetate. The organic phases were combined, dried over anhydrous sodium sulfate, and filtered. The filtrate was concentrated to dryness to give the crude product. The crude was purified by silica gel column to give compound 1-8 (325 mg).

Compound 1-8 (325 mg) was dissolved in 4.0 mL of methanol and sodium borohydride (30 mg, 0.84 mmol) was added to the reaction system. The mixture was reacted at room temperature. The complete disappearance of the reactants was monitored by TLC. The reaction system was quenched with saturated aqueous sodium chloride solution and extracted with dichloromethane. The organic phases were combined, dried over anhydrous sodium sulfate, and filtered. The filtrate was concentrated to dryness to give crude compound 1-9 (260 mg), which was used directly in the next reaction without purification.

Crude compound 1-9 (250 mg, 0.40 mmol), 1-11 (35.9 mg, 0.60 mmol), EDCI (230 mg, 1.20 mmol), triethylamine (0.17 mL, 1.20 mmol) and DMAP (49 mg, 0.40 mmol) were dissolved in 5.0 mL of dichloromethane, and the reaction solution was stirred to react at room temperature for 12 h. The reaction solution was quenched with saturated aqueous sodium chloride and extracted with dichloromethane. The organic phases were combined, dried over anhydrous sodium sulfate, and filtered. The organic phase was collected and the organic solvent was removed using a rotary-evaporator to give the crude product, which was purified by preparative high performance liquid chromatography to give compound 99 (31.6 mg)

¹H NMR (400 MHz, CDCl₃): δ ppm 0.86 (t, *J* = 6.8 Hz, 6H), 1.13 (s, 12H), 1.25 (m, 43H), 1.46 (m, 8H), 1.57 (m, 4H), 1.84 (m, 4H), 2.33 (s, 3H), 2.86 (m, 2H), 4.01 (m, 4H), 4.81 (m, 1H); ESI-MS m/z: 751.0 [M+H]⁺.

### Example 100: Synthesis of compound 100

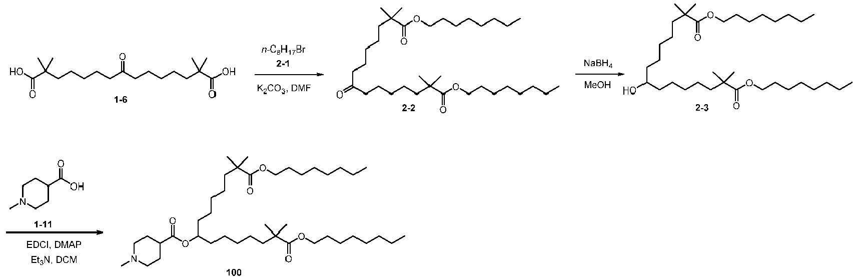

Referring to the method of Example 99, compound **100** was prepared as an oily product: 33.5 mg.

¹H NMR (400 MHz, CDCl₃): δ ppm 0.81 (t, *J* = 6.8 Hz, 6 H), 1.08 (s, 12 H), 1.11-1.31 (m, 30 H), 1.41 (m, 9 H), 1.54 (m, 5 H), 1.65-1.77 (m, 2 H), 1.78-1.98 (m, 4H), 2.20 (m, 4H), 2.74 (m, 2H), 3.97 (t, *J* = 6.8 Hz, 4 H), 4.71-4.85 (m, 1 H); ESI-MS m/z: 694.6 [M+H]⁺.

### Example 101: Synthesis of compound 101

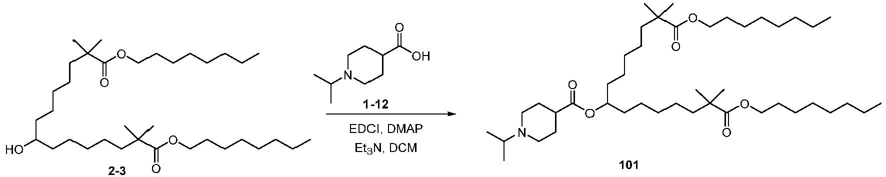

Referring to the method of Example 99, compound **101** was prepared as an oily product: 30.8 mg.

¹H NMR (400 MHz, CDCl₃): δ ppm 0.81 (t, *J* = 6.8 Hz, 6 H), 0.96 (d, *J*= 6.8 Hz, 6H), 1.08 (s, 12 H), 1.11-1.31 (m, 32 H), 1.35-1.46 (m, 8 H), 1.54 (m, 4 H), 1.59-1.74 (m, 4 H), 2.01-2.13 (m, 3H), 2.62 (m, 1H), 2.77 (m, 2H), 3.97 (t, *J* = 6.8 Hz, 4 H), 4.71-4.83 (m, 1 H); ESI-MS m/z: 722.6 [M+H]⁺.

### Example 102: Synthesis of compound 102

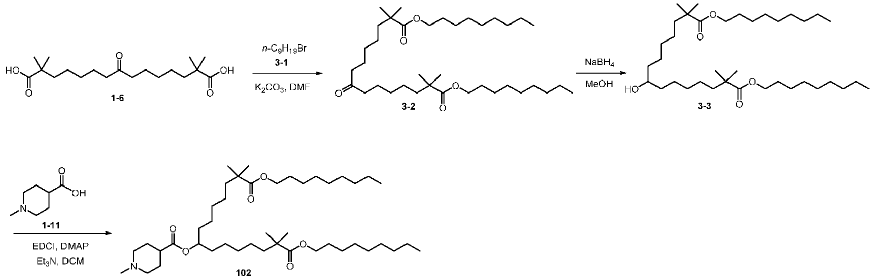

Referring to the method of Example 99, compound **102** was prepared as an oily product: 32.4 mg.

¹H NMR (400 MHz, CDCl₃): δ ppm 0.90 (t, *J* = 6.8 Hz, 6H), 1.17 (s, 12H), 1.20-1.41 (m, 34H), 1.44-1.55 (m, 8H), 1.57-1.69 (m, 5H), 1.73-1.86 (m, 3H), 1.92 (m, 2H), 2.02 (m, 2H), 2.21-2.33 (m, 4H), 2.82-2.85 (m, 2H), 4.06 (t, *J* = 6.8 Hz, 4H), 4.84-4.90 (m, 1H); ESI-MS m/z: 722.6 [M+H]⁺.

### Example 103: Synthesis of compound 103

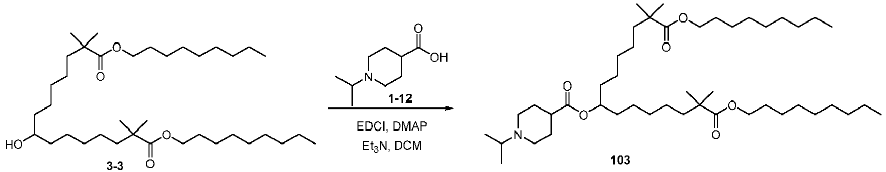

Referring to the method of Example 99, compound **103** was prepared as an oily product: 32.8 mg.

¹H NMR (400 MHz, CDCl₃): δ ppm 0.81 (t, *J* = 6.8 Hz, 6H), 0.96 (d, *J* = 6.8 Hz, 6H), 1.08 (s, 12H), 1.11-1.33 (m, 34H), 1.34-1.47 (m, 8H), 1.54 (m, 5H), 1.60-1.75 (m, 3H), 1.83 (m, 2H), 2.03-2.24 (m, 3H), 2.56-2.79 (m, 3H), 3.97 (t, *J* = 6.8 Hz, 4H), 4.74-4.81 (m, 1H); ESI-MS m/z: 750.6 [M+H]⁺.

### Example 104: Synthesis of compound 104

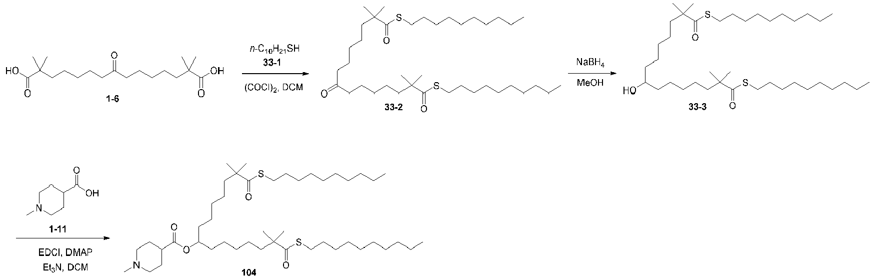

Compound **1-6** (448 mg, 1.3 mmol) was dissolved in 5.0 mL of dichloromethane, and the reaction system was cooled to 0 °C in an ice bath. DMF (10 µL, 0.13 mmol) was added and oxalyl chloride (0.44 mL, 5.2 mmol) was then added dropwise to the reaction solution. The ice bath was removed after the dropwise addition was completed and the mixture was stirred for 1 h at room temperature. The solvent was removed using a rotary-evaporator to give acyl chloride crude product (330 mg) as an oil, which was used directly in the next reaction step.

1-Decanethiol **33-1** (455 mg, 2.61 mmol) was added to a solution of crude acyl chloride (330 mg, 0.87 mmol) in DCE (3.0 mL), and the reaction was heated to 70 °C to react overnight. The reaction solution was cooled to room temperature and the solvent was removed using a rotary-evaporator to give the crude product, which was purified by silica gel column to give compound **33-2** (400 mg). ¹H NMR (400 MHz, CDCl₃): δ ppm 0.84-0.87 (m, 6H), 1.14-1.18 (m, 12H), 1.20-1.28 (m, 36H), 1.48-1.55 (m, 12H), 2.33 (t, *J* = 7.2 Hz, 4H), 2.79 (t, *J* = 7.2 Hz, 4H).

Compound **33-2** (300 mg, 0.46 mmol) was dissolved in 3.0 mL of methanol and NaBH₄ (52.5 mg, 1.38 mmol) was added in batches. The reaction solution was stirred under nitrogen atmosphere at room temperature for 2 h. The complete disappearance of the reaction material was monitored by TLC. The reaction solution was quenched by adding saturated ammonium chloride solution, and extracted with ethyl acetate. The organic phases were combined, dried over anhydrous sodium sulfate, and filtered. The filtrate was collected and concentrated to give 300 mg of crude compound **33-3,** which was directly used in the next reaction step without further purification.

Crude compound **33-3** (300 mg, 0.46 mmol), **1-11** (98.8 mg, 0.69 mmol), EDCI (264.5 mg, 1.38 mmol), triethylamine (0.19 mL, 1.38 mmol) and DMAP (56.2 mg, 0.46 mmol) were dissolved in 8.0 mL of dichloromethane, and the reaction solution was stirred at room temperature until the reaction material **33-3** was completely consumed. The reaction solution was quenched with saturated aqueous sodium chloride and extracted with dichloromethane. The organic phases were combined, dried over anhydrous sodium sulfate, and filtered. The organic phase was collected, and the organic solvent was removed using a rotary-evaporator. The crude product was purified by preparative high performance liquid chromatography to give the compound **104** (67.3 mg).

¹H NMR (400 MHz, CDCl₃): δ ppm 0.81 (t, *J*= 6.8 Hz, 6H), 1.08 (s, 12H), 1.09-1.31 (m, 42H), 1.35-1.51 (m, 14H), 1.61-2.25 (m, 8H), 2.73 (t, *J* = 7.2 Hz, 4H), 4.77 (m, 1H); ESI-MS m/z: 782.7 [M+H]⁺.

### Example 105: Synthesis of compound 105

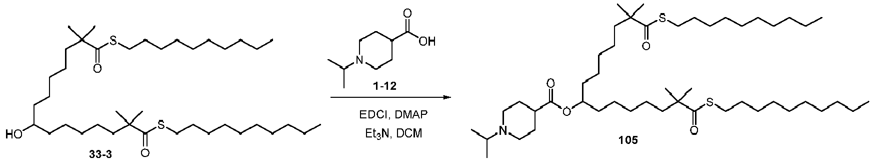

Referring to the method of Example 104, compound **105** was prepared as an oily product: 27.1 mg.

¹H NMR (400 MHz, CDCl₃): δ ppm 0.85-0.89 (m, 6H), 1.02 (br d, *J* = 6.4 Hz, 6H), 1.18 (s, 12H), 1.20-1.40 (m, 40H), 1.42-1.59 (m, 12H), 1.64-1.83 (m, 3H), 1.87-1.93 (m, 2H), 2.11 - 2.23 (m, 3H), 2.66-2.94 (m, 6H), 4.72-4.94 (m, 1H); ESI-MS m/z: 810.6 [M+H]⁺.

### Example 106: Synthesis of compound 106

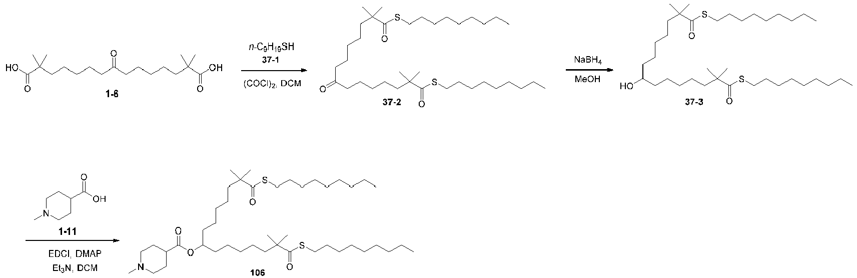

Referring to the method of Example 104, compound **106** was prepared as an oily product: 38.4 mg.

¹H NMR (400 MHz, CDCl₃): δ ppm 0.88 (t, *J* = 7.2 Hz, 6H), 1.17 (s, 12H), 1.15-1.34 (m, 36H), 1.43-1.57 (m, 15H), 1.69-2.09 (m, 5H), 2.27-2.34 (m, 3H), 2.77-2.86 (m, 5H), 4.78-4.85 (m, 1H); ESI-MS m/z: 754.6 [M+H]⁺.

### Example 107: Synthesis of compound 107

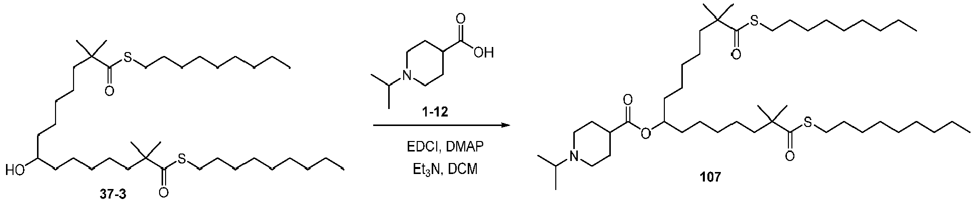

Referring to the method of Example 104, compound 107 was prepared as an oily product: 39 mg.

¹H NMR (400 MHz, CDCl₃): δ ppm 0.88 (t, *J* = 6.8 Hz, 6H), 1.05 (d, *J* = 6.8 Hz, 6H), 1.16 (s, 12H), 1.12-1.35 (m, 34H), 1.37-1.55 (m, 15H), 1.62-1.92 (m, 4H), 2.15-2.19 (m, 3H), 2.71-2.93 (m, 6H), 4.78-4.85 (m, 1H); ESI-MS m/z: 782.6 [M+H]⁺.

### Example 108: Synthesis of compound 108

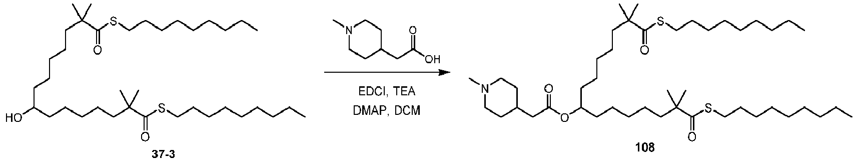

Referring to the method of Example 104, compound 108 was prepared as an oily product: 43.8 mg.

¹H NMR (400 MHz, CDCl₃): δ ppm 0.88 (t, *J* = 6.80 Hz, 6H), 1.18 (s, 12 H), 1.20-1.39 (m, 38H), 1.40-1.62 (m, 14H), 1.66-1.86 (m, 3H), 1.89-2.10 (m, 2H), 2.19-2.27 (m, 3H), 2.28 (br s, 2H), 2.79-2.83 (m, 4H), 4.79-4.88 (m, 1H); ESI-MS m/z: 768.5 [M+H]⁺.

### Example 109: Synthesis of compound 109

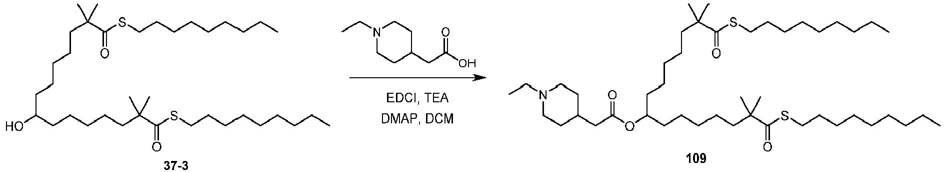

Referring to the method of Example **104,** compound **109** was prepared as an oily product: 44.8 mg.

¹H NMR (400 MHz, CDCl₃): δ ppm 0.86-0.89 (m, 6H), 1.18 (s, 15H), 1.23-1.37 (m, 36H), 1.46-1.54 (m, 14H), 1.76-1.93 (m, 4H), 2.11-2.20 (m, 1H), 2.24-2.28 (m, 2H), 2.54-2.71 (m, 2H), 2.81 (d, *J* = 7.2 Hz, 4 H), 3.08-3.24 (m, 2H), 4.79-4.88 (m, 1H); ESI-MS m/z: 782.6 [M+H]⁺.

### Example 110: Synthesis of compound 110

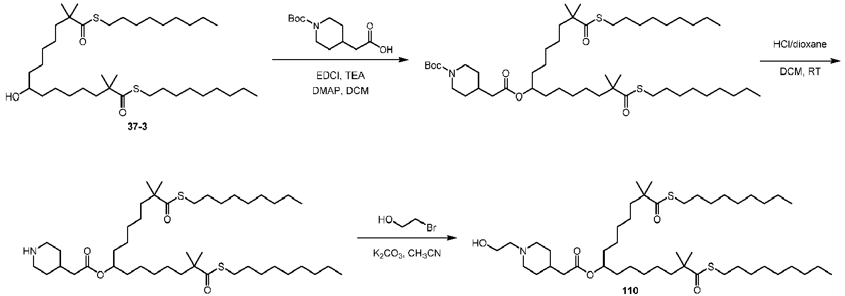

Referring to the method of Example **104,** compound **110** was prepared as an oily product: 34.4 mg.

¹H NMR (400 MHz, CDCl₃): δ ppm 0.81 (t, *J* = 7.2 Hz, 6H), 1.12 (s, 12H), 1.14-1.27 (m, 34H), 1.44-1.48 (m, 12H), 1.66-1.77 (m, 7H), 2.05-2.24 (m, 4H), 2.53 (m, 2H), 2.75 (t, *J* = 7.2 Hz, 4H), 2.90-2.92 (m, 2H), 3.57 (t, *J* = 5.2 Hz, 2H), 4.74-4.80 (m, 1H); ESI-MS m/z: 798.6 [M+H]⁺.

### Example 111: Synthesis of compound 111

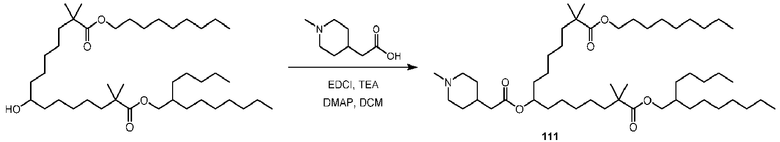

Referring to the method of Example **104,** compound **111** was prepared as an oily product: 31.4 mg.

¹H NMR (400 MHz, CDCl₃): δ ppm 0.92 (t, *J* = 6.8 Hz, 9H), 1.19 (s, 12H), 1.20-1.35 (m, 43H), 1.47-1.55 (m, 9H), 1.54-1.82 (m, 12H), 2.07-2.37 (m, 7H), 2.94-3.01 (m, 2H), 3.98 (d, *J* = 6.8 Hz, 2H), 4.07 (t, *J* = 6.8 Hz, 2H), 4.84-4.91 (m, 1H); ESI-MS m/z: 806.7 [M+H]⁺.

### Example 112: Synthesis of compound 112

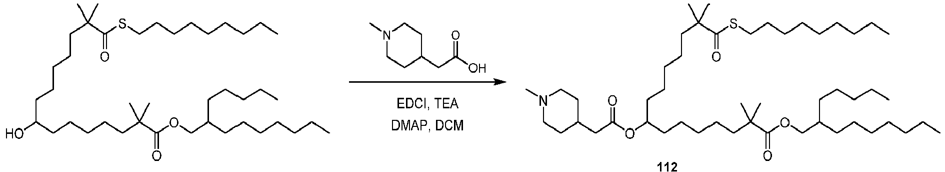

Referring to the method of Example **104,** compound **112** was prepared as an oily product: 24.4 mg.

¹H NMR (400 MHz, CDCl₃): δ ppm 0.80-0.83 (m, 9H), 1.08 (s, 12H), 1.10-1.35 (m, 28H), 1.41-1.57 (m, 28H), 1.65-1.75 (m, 4H), 1.95-2.10 (m, 2H), 2.16 (d, *J* = 6.4 Hz, 2H), 2.30 (s, 3H), 2.73-2.91 (m, 4H), 3.87 (d, *J* = 5.6 Hz, 2H), 4.75-4.79 (m, 1H); ESI-MS m/z: 822.7 [M+H]⁺.

### Example 113: Synthesis of compound 113

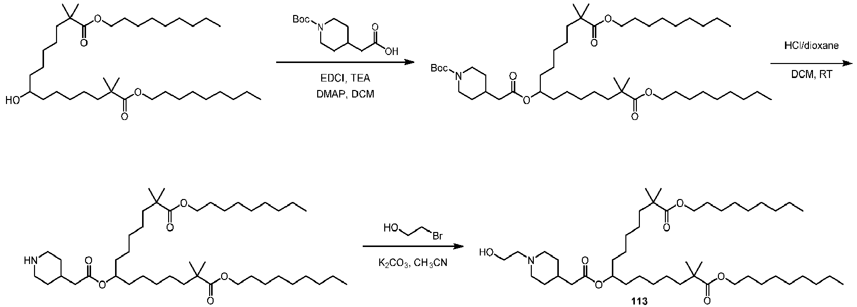

Referring to the method of Example **110,** compound **113** was prepared as an oily product: 31.1 mg.

¹H NMR (400 MHz, CDCl₃): δ ppm 0.81 (t, *J* = 7.2 Hz, 6H), 1.08 (s, 12H), 1.10-1.24 (m, 36H), 1.36-1.43 (m, 8H), 1.48-1.54 (m, 6H), 1.64-1.72 (m, 6H), 2.05 (t, *J* = 6.8 Hz, 1H), 2.15 (d, *J* = 6.8 Hz, 2H), 2.47 (t, *J* = 5.6 Hz, 2H), 2.82-2.89 (m, 2H), 3.54 (t, *J* = 5.6 Hz, 2H), 3.97 (t, *J* = 6.8 Hz, 4H), 4.73-4.79 (m, 1H); ESI-MS m/z: 766.6 [M+H]⁺.

### Example 114: Synthesis of compound 114

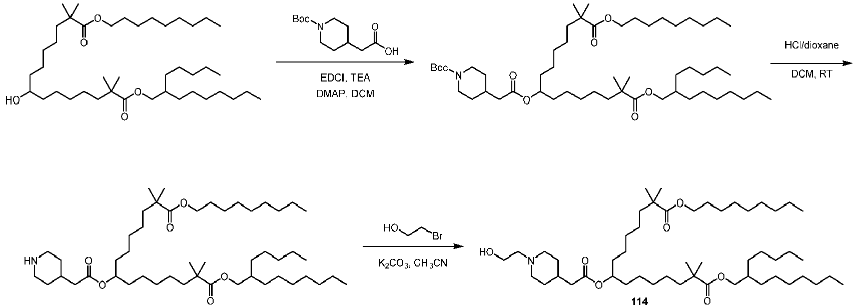

Referring to the method of Example **110,** compound **114** was prepared as an oily product: 32.7 mg.

¹H NMR (400 MHz, CDCl₃): δ ppm 0.85-0.88 (m, 9H), 1.07 (s, 12H), 1.09-1.35 (m, 46H), 1.41-1.58 (m, 13H), 1.97-2.25 (m, 3H), 2.32 (d, *J* = 5.6 Hz, 2H), 2.83-2.86 (m, 2H), 3.17-3.19 (m, 2H), 3.78-3.81 (d, *J* = 7.2 Hz, 2H), 3.92 (d, *J* = 5.6 Hz, 2H), 4.01 (t, *J* = 6.4 Hz, 2H), 4.10 (m, 1H), 4.81-4.86 (m, 1H); ESI-MS m/z: 836.7 [M+H]⁺.

### Example 115: Synthesis of compound 115

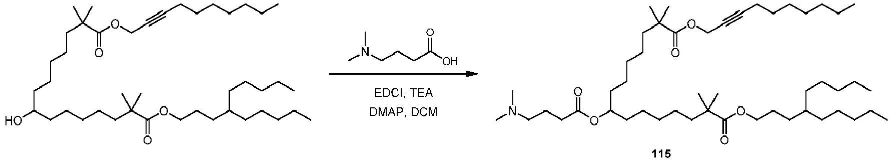

Referring to the method of Example **104,** compound **115** was prepared as an oily product: 31.0 mg.

¹H NMR (400 MHz, CDCl₃): δ ppm 0.87-0.91 (m, 9H), 1.14-1.37 (m, 51H), 1.49-1.60 (m, 12H), 1.75-1.81 (m, 2H), 2.21-2.26 (m, 2H), 2.28 (s, 6H), 2.32-2.36 (m, 4H), 4.03 (t, *J* = 6.4 Hz, 2H), 4.65 (s, 2H), 4.82-4.88 (m, 1H); ESI-MS m/z: 790.6 [M+H]⁺.

### Example 116: Synthesis of compound 116

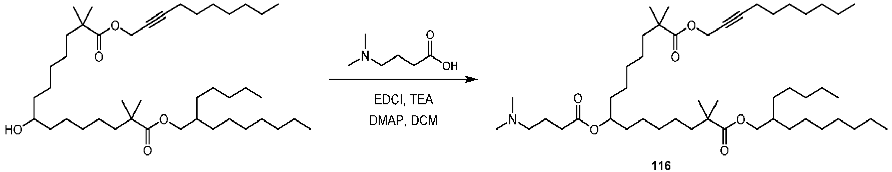

Referring to the method of Example **104,** compound **116** was prepared as an oily product: 31.3 mg.

¹H NMR (400 MHz, CDCl₃): δ ppm 0.80-0.83 (m, 9H), 1.07-1.27 (m, 51H), 1.40-1.45 (m, 12H), 1.70-1.81 (m, 2H), 2.13-2.36 (m, 12H), 3.87 (d, *J* = 5.6 Hz, 2H), 4.57 (s, 2H), 4.74-4.80 (m, 1H); ESI-MS m/z: 790.6 [M+H]⁺.

### Example 117: Synthesis of compound 117

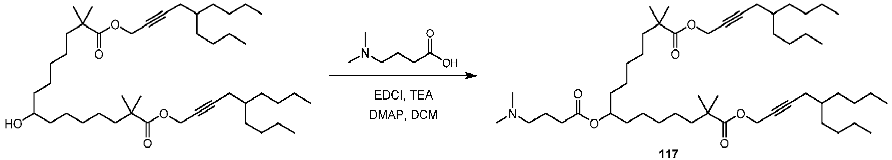

Referring to the method of Example **104,** compound **117** was prepared as an oily product: 35.9 mg.

¹H NMR (400 MHz, CDCl₃): δ ppm 0.90 (t, *J* = 6.8 Hz, 12H), 1.15 (s, 12H), 1.16-1.33 (m, 38H), 1.48-1.53 (m, 8H), 1.82-1.86 (m, 2H), 2.18-2.20 (m, 4H), 2.30-2.42 (m, 10 H), 4.65 (m, 4H), 4.82-4.88 (m, 1H); ESI-MS m/z: 814.6 [M+H]⁺.

### Example 118: Synthesis of compound 118

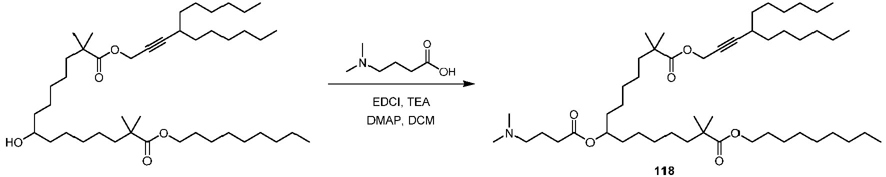

Referring to the method of Example **104,** compound **118** was prepared as an oily product: 32.0 mg.

¹H NMR (400 MHz, CDCl₃): δ ppm 0.87-0.90 (m, 9H), 1.15-1.32 (m, 50H), 1.40-1.61 (m, 16H), 1.76-1.84 (m, 2H), 2.23 (s, 6H), 2.29-2.34 (m, 6H), 4.04 (t, *J* = 6.8 Hz, 2H), 4.66 (d, *J* = 2.0 Hz, 1H), 4.83-4.87 (m, 1H); ESI-MS m/z: 804.6 [M+H]⁺.

### Example 119: Synthesis of compound 119

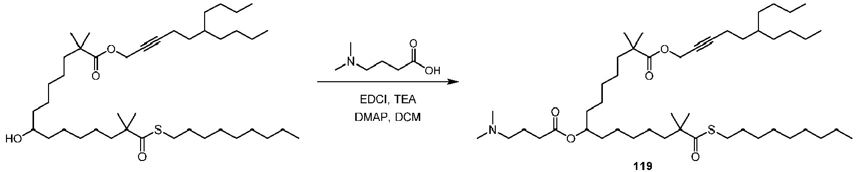

Referring to the method of Example **104,** compound **119** was prepared as an oily product: 34.8 mg.

¹H NMR (400 MHz, CDCl₃): δ ppm 0.91-0.94 (m, 9H), 1.21-1.40 (m, 48H), 1.51-1.57 (m, 12H), 1.61-1.86 (m, 5H), 2.23 (m, 2H), 2.31 (s, 6H), 2.35-2.39 (m, 2H), 2.85 (t, *J* = 7.2 Hz, 2H), 4.67 (t, *J* = 2.0 Hz, 1H), 4.84-4.90 (m, 1H); ESI-MS m/z: 792.6 [M+H]⁺.

### Example 120: Synthesis of compound 120

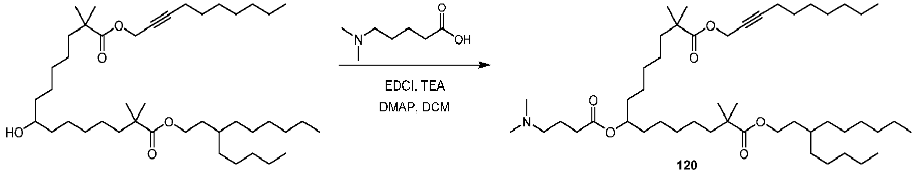

Referring to the method of Example **104,** compound **120** was prepared as an oily product: 34.5 mg.

¹H NMR (400 MHz, CDCl₃): δ ppm 0.89 (t, *J* = 6.8 Hz, 9H), 1.15-1.31 (m, 48H), 1.47-1.57 (m, 16H), 1.75-1.85 (m, 4H), 2.19-2.41 (m, 11H), 4.07 (t, *J* = 6.8 Hz, 2H), 4.65 (t, *J* = 2.0 Hz, 2H), 4.27-4.88 (m, 1H); ESI-MS m/z: 804.6 [M+H]⁺.

### Example 121: Synthesis of compound 121

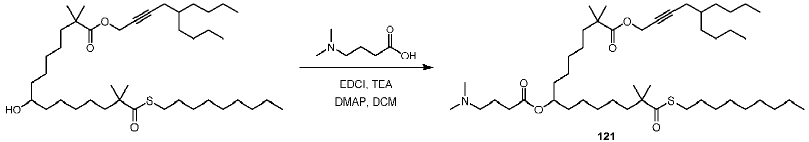

Referring to the method of Example **104,** compound **121** was prepared as an oily product: 33.8 mg.

¹H NMR (400 MHz, CDCl₃): δ ppm 0.91-0.95 (m, 9H), 1.20-1.43 (m, 46H), 1.47-1.57 (m, 10H), 1.63-1.85 (m, 6H), 2.29-2.40 (m, 12H), 2.85 (t, *J* = 7.2 Hz, 2H), 4.68 (s, 2H), 4.84-4.90 (m, 1H); ESI-MS m/z: 764.6 [M+H]⁺.

### Example 122: Synthesis of compound 122

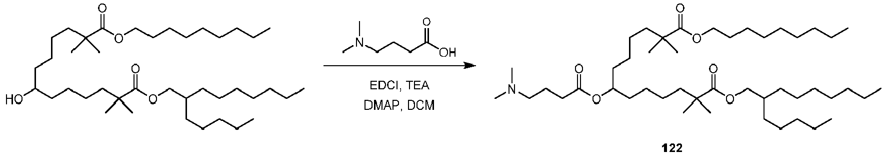

Referring to the method of Example **104,** compound **122** was prepared as an oily product: 33.7 mg.

¹H NMR (400 MHz, CDCl₃): δ ppm 0.81 (t, *J* = 6.8 Hz, 9H), 1.08-1.31 (m, 46H), 1.40-1.58 (m, 17H), 1.68-1.73 (m, 2H), 2.17 (s, 6H), 2.25 (t, *J* = 7.2 Hz, 4H), 3.87 (d, *J* = 5.6 Hz, 2H), 3.97 (t, *J*= 6.8 Hz, 2H), 4.73-4.80 (m, 1H); ESI-MS m/z: 752.7 [M+H]⁺.

### Example 123: Synthesis of compound 123

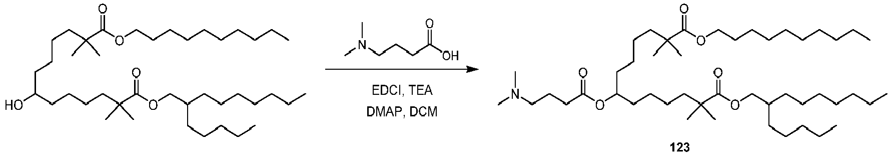

Referring to the method of Example **104,** compound **123** was prepared as an oily product: 35.2 mg.

¹H NMR (400 MHz, CDCl₃): δ ppm 0.81 (t, *J* = 6.8 Hz, 9H), 1.08 (s, 12H), 1.10-1.33 (m, 36H), 1.40-1.57 (m, 17H), 1.68-1.73 (m, 2H), 2.17 (s, 6H), 2.25 (t, *J* = 7.2 Hz, 4H), 3.87 (d, *J* = 5.6 Hz, 2H), 3.97 (t, *J* = 6.8 Hz, 2H), 4.73-4.79 (m, 1H); ESI-MS m/z: 766.7 [M+H]⁺.

### Example 124: Synthesis of compound 124

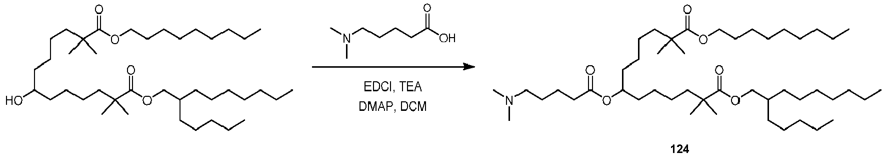

Referring to the method of Example **104,** compound **124** was prepared as an oily product: 33.4 mg.

¹H NMR (400 MHz, CDCl₃): δ ppm 0.89 (t, *J* = 6.8 Hz, 9H), 1.15 (s, 12H), 1.18-1.37 (m, 39H), 1.47-1.65 (m, 16H), 2.28 (s, 6H), 2.29-2.35 (m, 4H), 3.95 (d, *J*= 5.6 Hz, 2H), 4.04 (t, *J* = 6.8 Hz, 2H), 4.79-4.86 (m, 1H); ESI-MS m/z: 766.6 [M+H]⁺.

### Example 125: Synthesis of compound 125

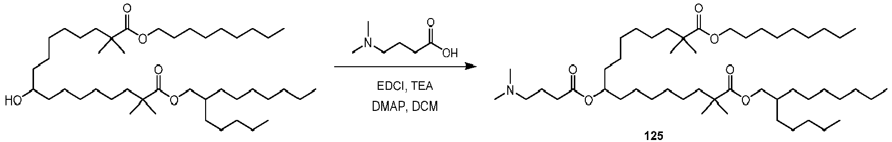

Referring to the method of Example **104,** compound **125** was prepared as an oily product: 31.1 mg.

¹H NMR (400 MHz, CDCl₃): δ ppm 0.89 (t, *J* = 6.8 Hz, 9H), 1.15 (s, 12H), 1.18-1.37 (m, 48H), 1.48-1.51 (m, 8H), 1.60-1.63 (m, 3H), 1.80-1.88 (m, 2H), 2.32-2.41 (m, 10H), 3.95 (d, *J* = 5.6 Hz, 2H), 4.04 (t, *J* = 6.8 Hz, 2H), 4.81-4.88 (m, 1H); ESI-MS m/z: 808.7 [M+H]⁺.

### Example 126: Synthesis of compound 126

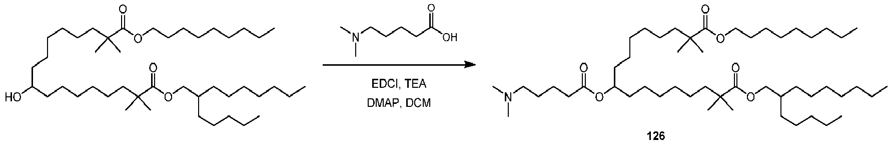

Referring to the method of Example **104,** compound **126** was prepared as an oily product: 35.1 mg.

¹H NMR (400 MHz, CDCl₃): δ ppm 0.89 (t, *J* = 6.8 Hz, 9H), 1.16 (s, 12H), 1.18-1.37 (m, 48H), 1.48-1.65 (m, 15H), 2.32-2.43 (m, 10H), 3.95 (d, *J* = 5.6 Hz, 2H), 4.05 (t, *J* = 6.8 Hz, 2H), 4.81-4.89 (m, 1H); ESI-MS m/z: 822.7 [M+H]⁺.

### Example 127: Synthesis of compound 127

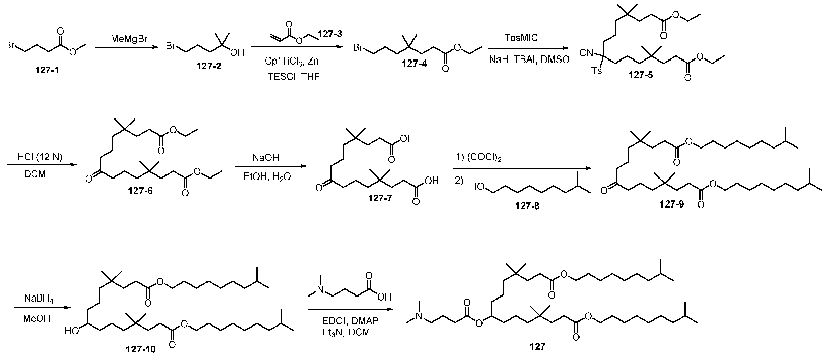

A solution of compound 127-1 (100 g, 552.4 mmol) in anhydrous ether (800 mL) was cooled to 0 °C in an ice bath, and methylmagnesium bromide (3 M in ether, 737 mL) was slowly added dropwise to the solution. After the dropwise addition was completed, the ice bath was removed and the mixture was stirred to react for 4 h at room temperature. The reaction system was quenched with saturated ammonium chloride aqueous solution, and extracted with ether. The organic phases were combined, dried over anhydrous sodium sulfate, and filtered. The filtrate was concentrated to dryness to give a crude product. The crude product was purified by silica gel column to give compound 127-2 (100 g).

Compound 127-2 (42 g, 232 mmol), compound 127-3 (30.3 mL, 278 mmol), Cp^{∗}TiCl₃ (5.09 g, 23.2 mmol), zinc powder (45.5 g, 696 mmol), and triethylchlorosilane (116.8 mL, 696 mmol) were added to a round bottom flask. Then anhydrous tetrahydrofuran (1200 mL) was added to the reaction system and the reaction was carried out under the protection of argon gas. The reaction system was heated to 60 °C and stirred to react for 1 hour. The reaction system was quenched with saturated aqueous sodium chloride solution and extracted with ethyl acetate. The organic phases were combined, dried over anhydrous sodium sulfate, and filtered. The filtrate was concentrated to dryness to give crude product 1-4, which was purified by silica gel column to give compound 127-4 (21 g).

Compound TosMIC (7.03 g, 36 mmol) was dissolved in DMSO (200 mL), and NaH (4.32 g, 60%, 108 mmol) was added to the reaction system in batches under ice bath conditions. After the addition was completed, the ice bath was removed and the mixture was reacted at room temperature for another 1 h. Compound 127-4 (21 g, 79 mmol) and TBAI (1.33 g, 3.6 mmol) were added to the reaction system, and the mixture was stirred at room temperature overnight. The reaction system was quenched with saturated aqueous sodium chloride solution and extracted with ethyl acetate. The organic phases were combined, dried over anhydrous sodium sulfate, and filtered. The filtrate was concentrated to dryness to give crude compound 127-5 (21.9 g), which was used directly in the next reaction step without purification.

To a solution of crude compound 127-5 (21.9 g, 38.8 mmol) in dichloromethane (350 mL) was added 200 mL of concentrated hydrochloric acid, and the mixture was reacted at room temperature for 2 h. The complete reaction of the substrate was monitored by TLC. The reaction system was quenched with saturated aqueous ammonium chloride solution and extracted with ethyl acetate. The organic phases were combined, dried over anhydrous sodium sulfate, and filtered. The filtrate was concentrated to dryness to give the crude product, which was purified by silica gel column to give compound 127-6 (12.5 g).

Compound 127-6 (12.5 g, 31.4 mmol) was dissolved in ethanol (20 mL)-water (40 mL), and NaOH (3.77 g, 94.2 mmol) was added to the mixed solution in batches under ice bath conditions. After the addition was completed, the ice bath was removed and the mixture was stirred at room temperature. The complete consumption of the reaction materials was monitored by TLC. The organic solvent was removed by rotary evaporation, and the residue was extracted with dichloromethane. The aqueous layer was collected, and the solution was adjusted to a pH of <5 with 1 M hydrochloric acid. The solution was extracted with dichloromethane. The organic phases were combined, dried over anhydrous sodium sulfate, and filtered. The filtrate was collected and concentrated to give compound 127-7 (9.7 g).

DMF (17 µL, 0.22 mmol) was added to a solution of compound 127-7 (750 mg, 2.19 mmol) in dichloromethane (10.0 mL) under ice bath conditions, and oxalyl chloride (0.77 mL, 8.76 mmol) was then added dropwise to the reaction solution. The ice bath was removed, and the mixture was stirred for 1 h at room temperature. The solvent was removed using a rotary-evaporator to give acyl chloride crude product, which was used directly in the next reaction step.

The above obtained acyl chloride crude product was dissolved in 10.0 mL of 1,2-dichloroethane, and then compound 127-8 (693 mg, 4.38 mmol) was added to the reaction solution. The mixture was stirred at room temperature until the substrate was reacted completely. The solvent was removed using a rotary-evaporator. The crude was purified by silica gel column to give compound 127-9 (800 mg).

Compound 127-9 (800 mg, 1.29 mmol) was dissolved in 5.0 mL of methanol and sodium borohydride (146 mg, 3.87 mmol) was added to the reaction system. The mixture was reacted at room temperature. The complete disappearance of the reactants was monitored by TLC. The reaction system was quenched with saturated aqueous sodium chloride solution and extracted with dichloromethane. The organic phases were combined, dried over anhydrous sodium sulfate, and filtered. The filtrate was concentrated to dryness to give crude compound 127-10 (800 mg), which was used directly in the next reaction without purification.

Crude compound 127-10 (300 mg, 0.48 mmol), 4-dimethylaminobutyric acid (94.4 mg, 0.72 mmol), EDCI (276 mg, 1.44 mmol), triethylamine (0.21 mL, 1.44 mmol) and DMAP (59 mg, 0.48 mmol) were dissolved in 5.0 mL of dichloromethane, and the reaction solution was stirred to react at room temperature for 12 h. The reaction solution was quenched with saturated aqueous sodium chloride and extracted with dichloromethane. The organic phases were combined, dried over anhydrous sodium sulfate, and filtered. The organic phase was collected, and the organic solvent was removed using a rotary-evaporator. The crude product was purified by preparative high performance liquid chromatography to give the compound 127 (43.6 mg)

¹H NMR (400 MHz, CD₃OD): δ ppm 0.77-0.93 (m, 28H), 1.08-1.74 (m, 38H), 1.76-1.84 (m, 2H), 1.22-2.27 (m, 10H), 2.33-2.37 (m, 4H), 4.03-4.12 (m, 4H), 4.92-4.97 (m, 1H); ESI-MS m/z: 738.6 [M+H]⁺.

### Example 128: Synthesis of compound 128

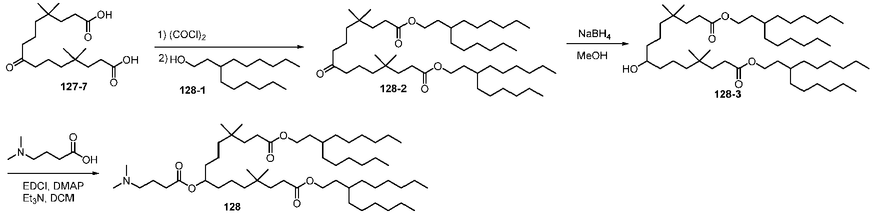

Referring to the method of Example 127, compound **128** was prepared as an oily product: 64.2 mg.

¹H NMR (400 MHz, CD₃OD): δ ppm 0.86 (s, 12 H), 0.91 (t, J = 6.8 Hz, 12H), 1.22-1.37 (m, 48 H), 1.51-1.61 (m, 14 H), 1.78-1.86 (m, 2 H), 2.24 (t, J = 8.0 Hz, 4H), 2.30 (s, 6H), 2.37 (t, J = 7.2 Hz, 2H), 2.43 (t, *J* = 8.0 Hz, 2 H), 4.10 (t, J = 6.8 Hz, 4H), 4.92-4.97 (m, 1 H); ESI-MS m/z: 878.7 [M+H]⁺.

### Example 129: Synthesis of compound 129

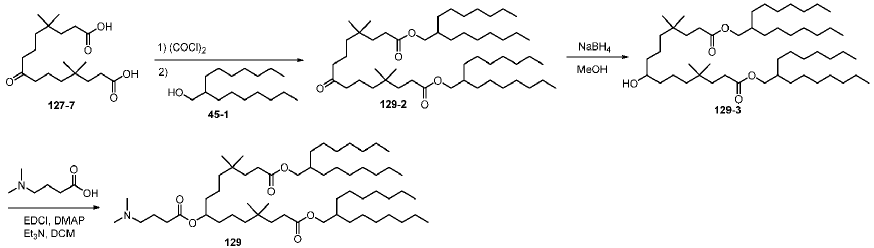

Referring to the method of Example 127, compound **129** was prepared as an oily product: 67.0 mg.

¹H NMR (400 MHz, CD₃OD): δ ppm 0.86 (s, 12 H), 0.88-0.93 (m, 12H), 1.12-1.40 (m, 52 H), 1.49-1.56 (m, 8 H), 1.59-1.66 (m, 4 H), 1.77-1.84 (m, 4 H), 2.22-2.27 (m, 10H), 2.34-2.38 (m, 4H), 4.01 (t, *J* = 6.8 Hz, 4 H), 4.91-4.97 (m, 1 H); ESI-MS m/z: 906.8 [M+H]⁺.

Example 130: Referring to the method of Example 20, compound 130 was prepared.

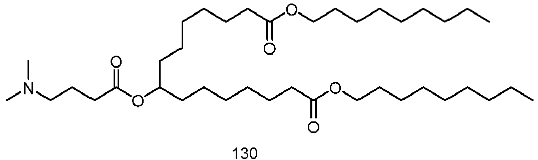

[M+H]+: 654.6. ¹H NMR (400 MHz, CDCl₃): δ ppm 0.77-0.86 (t, *J* = 7.2 Hz, 6H), 1.15-1.30 (m, 38H), 1.40-1.59 (m, 12H), 1.87-1.96 (m, 2H), 2.21 (t, *J* = 7.2 Hz, 4H), 2.28-2.37 (m, 2H), 2.40-2.50 (m, 5H), 2.56-2.67 (m, 2H), 3.92-4.07 (m, 4H), 4.72-4.90 (m, 1H).

Example 131: Referring to the method of Example 46, compound 131 was prepared.

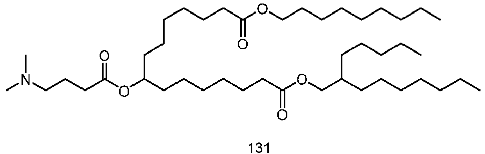

[M+H]+: 724.6. ¹H NMR (400 MHz, CDCl₃): δ ppm 0.89 (t, *J* = 7.2 Hz, 9H), 1.21-1.30 (m, 44H), 1.50-1.63 (m, 11H), 1.77-1.92 (m, 2H), 2.27-2.36 (m, 14H), 3.97 (d, *J* = 5.6 Hz, 2H), 4.06 (t, *J* = 6.8 Hz, 2H), 4.85-4.92 (m, 1H).

### Example 132: Synthesis of compound 132

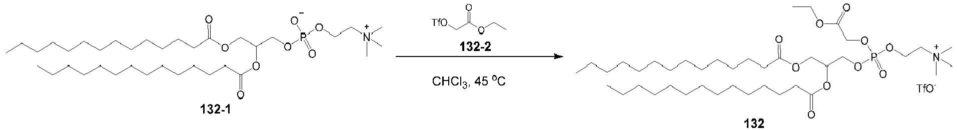

Compound 132-1 (101.7 mg, 0.15 mmol) and compound 132-2 (40.1 mg, 0.17 mmol) were added to 5.0 mL of chloroform solution, and the reaction solution was heated to 45 °C and reacted with stirring. The reactant 132-1 was consumed completely by LC-MS monitoring. The organic solvent was purged with nitrogen gas to dryness to give a crude product. The crude product was added to hexane and slurried (5.0 mL*3), then filtered by suction and dried to give 35.9 mg of product as a white solid.

¹H NMR: (400 MHz, CDCl₃): *δ* 0.89 (t, *J* = 7.2 Hz, 6H), 1.26-1.32 (m, 43H), 1.54-1.67 (m, 4H), 2.30-2.36 (m, 4H), 3.36 (s, 9H), 4.03 (br d, *J* = 2.8 Hz, 2H), 4.23-4.32 (m, 6H), 4.57-4.74 (m, 4H), 5.25-5.29 (m, 1H); ESI-MS m/z: 764.5 [M]⁺.

### Example 133: Synthesis of compound 133

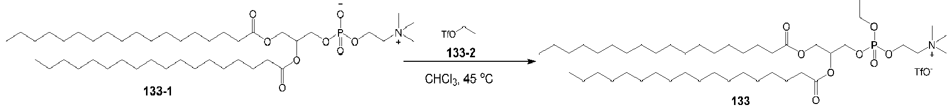

Compound 133-1(250 mg, 0.32 mmol) and compound 133-2(62.3 mg, 0.35 mmol) were added to 10.0 mL of chloroform solution, and the reaction solution was heated to 45 °C and reacted with stirring for 2 h. The reactant 133-1 was consumed completely by LC-MS monitoring. The organic solvent was purged with nitrogen gas to dryness to give a crude product. The crude product was added to hexane and slurried (8.0 mL*3), then filtered by suction and dried to give 165 mg of product as a white solid.

¹H NMR: (400 MHz, CDCl₃): *δ* 0.89 (t, *J* = 7.2 Hz, 6H), 1.26-1.38 (m, 58H), 1.52-1.67 (m, 5H), 2.31-2.37 (m, 4H), 3.31 (s, 9H), 3.92 (br d, *J* = 2.8 Hz, 2H), 4.16-4.34 (m, 6H), 4.47-4.63 (m, 2H), 5.24-5.28 (m, 1H); ESI-MS m/z: 818.7 [M]⁺.

### Pharmacological assay

### Assay example 1: Preparation of nanoparticles

Materials used for lipid nanoparticle assembly include: (1) ionizable lipid compound: e.g., ionizable lipid designed and synthesized in the present disclosure or DLin-MC3-DMA (purchased from AVT) as a control; (2) structured lipid: e.g., Cholesterol (purchased from Sigma-Aldrich); (3) phospholipid: e.g., DSPC, which is 1,2-distearoyl-SN-glycero-3-phosphocholine (distearoylphosphatidylcholine, purchased from AVT); (4) pegylated lipid: e.g. DMG-PEG2000, which is dimyristoylglycero-polyethylene glycol 2000 (1,2-dimyristoyl-rac-glycero-3-methoxypolyethylene glycol-2000, purchased from AVT); (5) active ingredient of nucleic acid fragments: e.g. Luciferase mRNA, siRNA, CRISPR Cas 9 mRNA, etc. (manufactured in-house).

Lipid nanoparticles were prepared by (1) dissolving and mixing ionizable lipid compound, cholesterol, phospholipid and pegylated lipid in ethanol at (molar percentages) 50%, 38.5%, 10% and 1.5%, respectively; (2) dissolving the mRNA active ingredient in 25 mM sodium acetate solution (pH = 4.5); (3) using an automated high-throughput microfluidic system to mix the organic phase containing the lipid mixture and the aqueous phase containing the mRNA component in the flow ratio range of 1:1 to 1:4 at a mixing speed of 10 mL/min to 18 mL/min; (4) the prepared lipid nanoparticles (N/P ratio of 6) were diluted with phosphate buffer solution and the nanoparticle solutions were ultrafiltered to the original preparation volume using ultrafiltration tubes (purchased from Millipore) with a cut-off molecular weight of 30 kDa; and (5) the obtained nanoparticles were filtered through a 0.2 µm sterile filter membrane and then stored in a sealed glass vial at low temperature.

The preparation method of lipid nanoparticles includes microfluidic mixing systems, but is not limited to this method, which also includes T-type mixers and ethanol injection method, and the like.

### Assay example 2: Characterization of physical properties of lipid nanoparticles

The particle size and particle size dispersity index (polydispersity index, PDI) of the prepared lipid nanoparticles were measured using a Zetasizer Pro (purchased from Malvern Instruments Ltd) and a DynaPro NanoStar (purchased from Wyatt) dynamic light scattering instrument. The degree of RNA encapsulation by lipid nanoparticles was characterized by the Encapsulation Efficiency%, which reflects the degree of binding of lipid nanoparticles to RNA fragments. This parameter was measured by the method of Quant-it^{™} RiboGreen RNA Assay (purchased from Invitrogen). Lipid nanoparticle samples were diluted in TE buffer (10 mM Tris-HCl, 1 mM EDTA, pH = 7.5). A portion of the sample solution was removed, to which 0.5% Triton (Triton X-100) was added, and then allowed to stand at 37° C for 30 minutes. Immediately after the addition of RIBOGREEN^{®} reaction solution, the fluorescence values were read on a Varioskan LUX multifunctional microplate reader (purchased from Thermofisher) at 485 nm for absorption and 528 nm for emission to give the encapsulation efficiency values.

### Assay example 3: Animal experiment

The delivery effect and safety of nanoparticles encapsulating luciferase mRNA (Trilink, L-7202) in mice were evaluated. The test mice were SPF-grade C57BL/6 mice, female, 6-8 weeks old, weighing 18-22 g, and were purchased from SPF (Beijing) Biotechnology Co., Ltd. All animals were acclimatized for more than 7 days prior to the experiment, and had free access to food and water during the experiment. The conditions include alternating light and dark for 12/12 h, the indoor temperature of 20-26°C and the humidity of 40-70%. The mice were randomly grouped. The lipid nanoparticles encapsulating luciferase mRNA prepared above were injected into mice by intravenous administration at a single dose of 0.5 mg/kg mRNA, and the mice were subjected to in vivo bioluminescence assay using a Small Animal In Vivo Imaging System (IVIS LUMINA III, purchased from PerkinElmer) at 6 h after administration. The assay was performed as follows: D-luciferin solution was prepared in saline at a concentration of 15 mg/mL, and each mouse was given the substrate by intraperitoneal injection. At ten minutes after administration of the substrate, the mice were anesthetized in an anesthesia chamber with isoflurane at a concentration of 2.5%. The anesthetized mice were placed in IVIS for fluorescence imaging, and the result showed that fluorescence was concentrated in the liver. Data acquisition and analysis were performed on the liver.

The in vivo delivery efficiency of lipid nanoparticle carriers was expressed as the mean values of fluorescence intensity and total photon count in different animals within the same subject group, as shown in Table 4. Higher values of fluorescence intensity and total photon count indicate higher in vivo delivery efficiency of this mRNA fragment by lipid nanoparticles. The lipid nanoparticles containing the cationic lipids of the present disclosure have good in vivo delivery efficiency and mainly target the liver.

**Table 4**

| **Cationic lipid compound** | **Particle size (nm)** | **Particle size dispersity (PDI)** | **Encaps ulation efficien cy (%)** | **Total photon count in vivo at 6 hours after administration (Total Flux)** |
|---|---|---|---|---|
| 1 | 80.07 | 0.06 | 91.54 | 6.43E+08 |
| 2 | 229.35 | 0.03 | 79.14 | 4.23E+08 |
| 3 | 133.80 | 0.04 | 86.88 | 1.75E+09 |
| 6 | 106.18 | 0.05 | 90.26 | 2.25E+09 |
| 7 | 203.67 | 0.04 | 69.51 | 1.16E+08 |
| 8 | 127.82 | 0.07 | 60.36 | 3.26E+07 |
| 9 | 352.82 | 0.07 | 19.41 | 6.05E+06 |
| 10 | 118.59 | 0.07 | 53.83 | 1.26E+09 |
| 11 | 46.49 | 0.03 | 95.61 | 4.42E+06 |
| 12 | 89.34 | 0.07 | 85.02 | 1.10E+09 |
| 13 | 157.10 | 0.08 | 56.65 | 1.08E+06 |
| 14 | 306.07 | 0.08 | 45.66 | 6.34E+07 |
| 15 | 91.68 | 0.06 | 84.78 | 4.89E+08 |
| 16 | 55.39 | 0.05 | 96.52 | 3.97E+07 |
| 17 | 81.43 | 0.12 | 30.95 | 6.77E+05 |
| 18 | 161.29 | 0.05 | 87.86 | 1.14E+10 |
| 19 | 135.15 | 0.19 | 67.96 | 3.20E+09 |
| 20 | 99.61 | 0.10 | 71.93 | 2.40E+10 |
| 23 | 151.35 | 0.07 | 70.22 | 1.43E+10 |
| 24 | 133.72 | 0.04 | 60.98 | 5.56E+09 |
| 25 | 236.90 | 0.06 | 32.43 | 1.89E+08 |
| 26 | 96.69 | 0.05 | 81.29 | >4.00E+10 |
| 27 | 105.70 | 0.05 | 88.99 | 2.20E+10 |
| 28 | 138.16 | 0.06 | 80.36 | 3.21E+09 |
| 32 | 116.61 | 0.07 | 99.02 | 7.30E+09 |
| 33 | 105.84 | 0.04 | 88.67 | 2.12E+08 |
| 34 | 104.71 | 0.04 | 90.29 | 1.26E+10 |
| 36 | 88.33 | 0.07 | 97.52 | 4.84E+09 |
| 37 | 92.18 | 0.03 | 89.07 | 7.88E+09 |
| 40 | 83.90 | 0.05 | 97.06 | 4.22E+09 |
| 41 | 104.27 | 0.05 | 93.91 | 2.19E+10 |
| 42 | 152.04 | 0.07 | 71.95 | 5.32E+09 |
| 46 | 97.30 | 0.09 | 95.68 | >4.00E+10 |
| 90 | 18.17 | 0.05 | 83.21 | 1.04E+07 |
| 91 | 32.61 | 0.05 | 102.99 | 1.29E+06 |
| 92 | 107.56 | 0.05 | 91.66 | 2.13E+09 |
| 97 | 157.00 | 0.25 | 93.72 | 3.37E+09 |
| 98 | 107.87 | 0.13 | 93.28 | 2.12E+10 |
| 99 | 139.99 | 0.05 | 90.69 | 5.30E+09 |
| 100 | 118.19 | 0.07 | 83.29 | 1.18E+10 |
| 101 | 152.45 | 0.06 | 76.09 | 5.19E+09 |
| 102 | 102.18 | 0.03 | 90.03 | 2.03E+10 |
| 103 | 148.67 | 0.04 | 90.97 | 5.69E+09 |
| 104 | 71.14 | 0.06 | 85.54 | 2.61E+10 |
| 105 | 78.80 | 0.05 | 94.67 | 3.33E+09 |
| 106 | 83.09 | 0.05 | 85.65 | 1.85E+10 |
| 107 | 99.54 | 0.05 | 83.74 | 1.36E+10 |
| 108 | 121.41 | 0.05 | 92.63 | 1.21E+10 |
| 109 | 101.04 | 0.06 | 87.11 | 1.32E+10 |
| 110 | 97.59 | 0.01 | 92.65 | 2.06E+08 |
| 111 | 141.30 | 0.06 | 95.12 | 1.79E+10 |
| 112 | 121.05 | 0.08 | 94.41 | 3.53E+10 |
| 113 | 112.65 | 0.03 | 94.77 | 3.59E+08 |
| 114 | 72.32 | 0.04 | 93.41 | 1.06E+08 |
| 115 | 77.12 | 0.04 | 91.76 | 2.56E+10 |
| 116 | 88.62 | 0.05 | 89.36 | >4.00E+10 |
| 117 | 127.31 | 0.06 | 95.29 | 4.48E+09 |
| 118 | 88.33 | 0.07 | 93.87 | 3.14E+10 |
| 119 | 80.98 | 0.05 | 86.92 | 3.25E+10 |
| 120 | 98.99 | 0.07 | 95.55 | 3.15E+10 |
| 121 | 103.57 | 0.06 | 93.62 | >4.00E+10 |
| 122 | 96.72 | 0.07 | 91.76 | >4.00E+10 |
| 123 | 84.98 | 0.05 | 92.27 | >4.00E+10 |
| 125 | 95.69 | 0.07 | 89.45 | 1.99E+10 |
| 126 | 133.76 | 0.06 | 94.35 | 1.77E+10 |
| 127 | 83.01 | 0.05 | 62.35 | 3.59E+10 |
| 128 | 83.10 | 0.05 | 79.88 | 1.00E+ 10 |
| 129 | 112.08 | 0.06 | 57.87 | 7.82E+09 |
| 130 | 131.2 | 0.18 | 97.08 | 4.45E+07 |
| 131 | 245.33 | 0.20 | 79.63 | 1.57E+09 |
| DLin-MC3-D MA | 96.83 | 0.04 | 95.62 | 8.04E+09 |

### Assay example 4

Materials used for lipid nanoparticle assembly includes: (1) ionizable lipid compound: e.g., ionizable lipid designed and synthesized in the present disclosure or ALC-0315 (purchased from AVT) as a control group; (2) structured lipid: e.g., cholesterol (purchased from Sigma-Aldrich); (3) phospholipid: e.g., DSPC, which is 1,2-distearoyl-SN-glycero-3-phosphocholine (distearoylphosphatidylcholine, purchased from AVT); (4) pegylated lipid compound: e.g., DMG-PEG2000, which is dimyristoylglycerol-polyethylene glycol 2000 (1,2-dimyristoyl-rac-glycero-3-methoxy-polyethylene glycol-2000, purchased from AVT); (5) active ingredient of nucleic acid fragments: e.g. Luciferase mRNA, siRNA, CRISPR Cas 9 mRNA, etc. (manufactured in-house); (6) permanently cationic lipid. The names of the materials for lipid nanoparticle assembly and their structural formulae are detailed in Table 5.

**Table 5**

| Sequence or lipid name | Detailed information |
|---|---|
| Luciferase mRNA | The sequence is a commercially available sequence fragment purchased from the Trilink website |
| Lipid 5 | 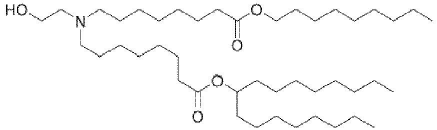 |
| SM102 | 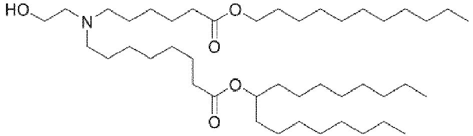 |
| Compound 20 | 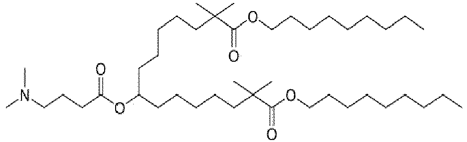 |
| Compound 26 | 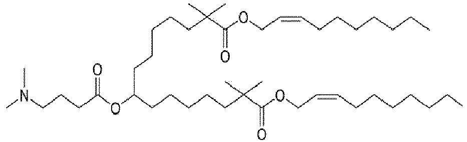 |
| Compound 46 | 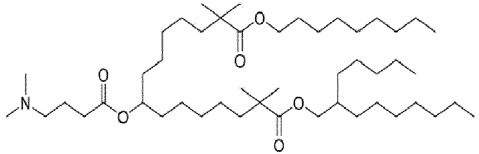 |
| Compound 108 | 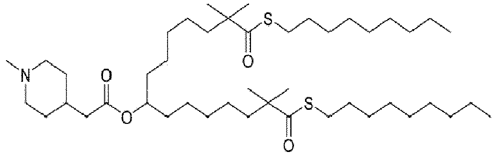 |
| Compound 133 | 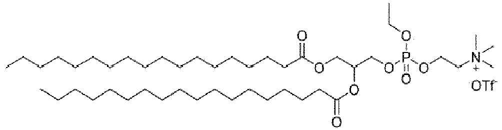 |
| 18:1 EPC | 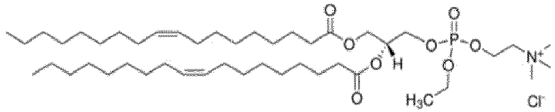 |
| Compound 132 | 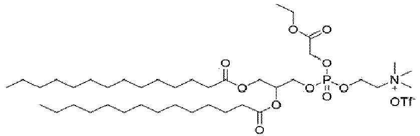 |
| DOTAP | 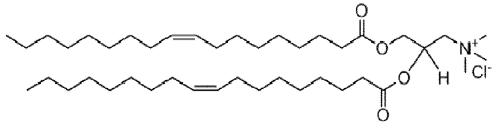 |
| DOTMA | 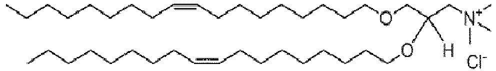 |
| DLin-MC3-DMA | 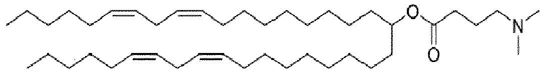 |
| ALC-0315 | 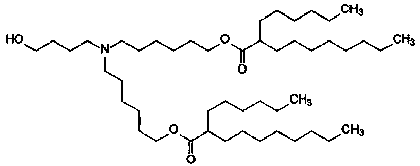 |
| Cholesterol | 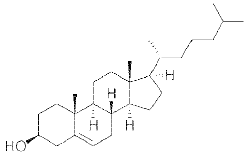 |
| DSPC | 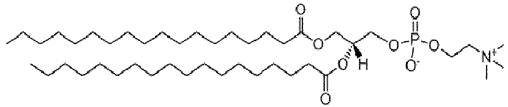 |
| DMG-PEG (PEG2K-DMG) | 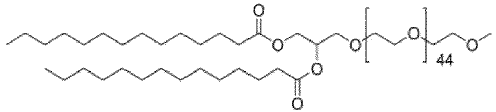 |
| ALC-0159 | 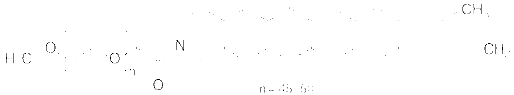 |

Lipid nanoparticles were prepared by (1) sequentially dissolving and mixing ionizable lipid compound, permanently cationic lipid, cholesterol, phospholipid, and pegylated lipid in ethanol respectively at the molar percentages provided; (2) dissolving the mRNA active ingredient in 25 mM sodium acetate solution (pH = 4.5); (3) using an automated high-throughput microfluidic system to mix the organic phase containing the lipid mixture and the aqueous phase containing the mRNA component in the flow ratio range of 1:1 to 1:4 at a mixing speed of 10 mL/min to 18 mL/min; (4) the prepared lipid nanoparticles were diluted with phosphate buffer solution and the nanoparticle solutions were ultrafiltered to the original preparation volume using ultrafiltration tubes with a cut-off molecular weight of 30 kDa (5) the obtained nanoparticles were filtered through a 0.2 µm sterile filter membrane and then stored in a sealed glass vial at low temperature. The preparation of lipid nanoparticles includes microfluidic mixing system, but is not limited to this method, which also includes T-type mixer and ethanol injection method, etc.

The produced lipid nanoparticles were characterized using the same method as in Assay example 2, and the results are shown in Table 6 (the percentage content of each component is represented by molar percentage).

**Table 6**

| **Form ulatio n No.** | **N/ P** | **Ioniz able catio** n (%) | **Choles terol (%)** | **Permanen t cation (%)** | **PEG2 K-DM G (%)** | **Ionizabl e cation** | **Permanen t cation** | **Particl e size (nm)** | **PDI** | **EE%** |
|---|---|---|---|---|---|---|---|---|---|---|
| **1** | **7** | 40 | 58.5 | 0 | 1.5 | Compou nd 26 | 18:1EPC | 96.43 | 0.09 | 90.21 |
| | | 40 | 53.5 | 5 | 1.5 | Compou nd 26 | 18:1EPC | 93.8 | 0.05 | 90.95 |
| | | 40 | 48.5 | 10 | 1.5 | Compou nd 26 | 18:1EPC | 85 | 0.07 | 95.11 |
| | | 40 | 38.5 | 20 | 1.5 | Compou nd 26 | 18:1EPC | 92.67 | 0.07 | 96.2 |
| **2** | **4** | 47.5 | 40.5 | 10 | 2 | Compou nd 46 | Compound 133 | 94.51 | 0.09 | 95.65 |
| **3** | **6** | 40 | 58.5 | 0 | 1.5 | Compou nd 108 | DOTAP | 97.95 | 0.07 | 91.54 |
| | | 40 | 38.5 | 20 | 1.5 | Compou nd 108 | DOTAP | 84.18 | 0.05 | 99.02 |

### Assay example 5

This assay example showed that after adding different permanent positively charged lipids to the above different ionizable lipid formulations, the fluorescent protein was highly expressed in situ in the muscle after intramuscular injection of LNP encapsulating luciferase mRNA.

The delivery effect and safety of nanoparticles encapsulating luciferase mRNA in mice were evaluated. The test mice were SPF-grade C57BL/6 mice, female, 6-8 weeks old, weighing 18-22 g, and were purchased from SPF (Beijing) Biotechnology Co., Ltd. All animals were acclimatized for more than 7 days prior to the experiment, and had free access to food and water during the experiment. The conditions include alternating light and dark for 12/12 h, the indoor temperature of 20-26°C and the humidity of 40-70%. The mice were randomly grouped. The lipid nanoparticles encapsulating luciferase mRNA prepared above were injected into mice by intramuscular injection administration in the leg at a single dose of 0.5 mg/kg mRNA, and the mice were subjected to in vivo bioluminescence assay using a Small Animal In Vivo Imaging System (IVIS LUMINA III, purchased from PerkinElmer) at 6, 24, 48, 72 and 120 h, etc. after administration. The assay was performed as follows: D-luciferin solution was prepared in saline at a concentration of 15 mg/mL, and each mouse was given the substrate by intraperitoneal injection. At ten minutes after administration of the substrate, the mice were anesthetized in an anesthesia chamber with isoflurane at a concentration of 2.5%. The anesthetized mice were placed in IVIS for fluorescence imaging, and data acquisition and analysis were performed on the concentrated distribution area of fluorescence.

The in vivo delivery efficiency of lipid nanoparticle carriers was expressed as the mean values of fluorescence intensity and total photon count in different animals within the same subject group. Higher values of fluorescence intensity and total photon count indicate higher in vivo delivery efficiency of this mRNA fragment by lipid nanoparticles. Lipid nanoparticles containing the cationic lipid of the present disclosure have good in vivo delivery efficiency.

### Formulation 1:

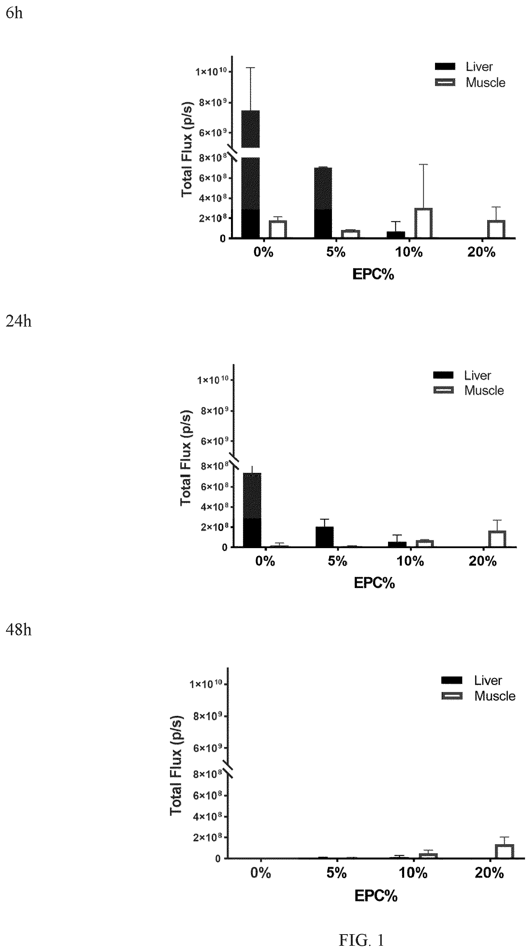

Different ratios of 18:1 EPC were added to the ionizable lipid compound 26 to prepare lipid nanoparticles, and then the lipid nanoparticles were introduced into mice by in situ injection into leg muscle. The results are shown in FIG. 1 and Table 7.

FIG. 1 shows the luminescence intensity of fluorescent proteins obtained by living imaging of the liver and muscle parts of mice on the IVIS instrument at 6h, 24h and 48h, respectively, after injection of formulation 1. The results of the fluorescent light intensity of the liver and muscle parts at different time points are shown, respectively. Each bar represents the mean of the fluorescence expression of three mice at the corresponding organ site (n=3).

It can be seen from FIG. 1 and Table 7 that after adding EPC, the expression of fluorescent protein in the liver decreased significantly. For the formula with the addition of 5% of 18:1 EPC, the expression of fluorescent protein in the liver was reduced to 9.4% of that of the assay group without EPC at 6 hours. As the amount of EPC% increased, the expression of fluorescent protein in the liver decreased significantly, and almost no signal expression in the liver was observed at 20%, as low as 0.03% of that of the assay group without EPC. At the same time, the ratio of fluorescence expression of fluorescent protein at in situ muscle site/liver site gradually increased with the increase of the added EPC%. From 6 hours to 48 hours after injection, with the prolongation of time after injection, the signal in the liver had a rapid and large attenuation, but the signal in the muscle of the assay group containing EPC attenuated slowly. At 48h, the signal intensity in the muscle of the assay group containing EPC was 33-662 times that of the assay group without EPC. The formulation group with the addition of 20% of EPC showed a very stable expression of fluorescent signal in the muscle site and no significant signal decay was found at 48h.

**Table 7**

| | 6h | | 24h | | 48h | |
|---|---|---|---|---|---|---|
| | Liver | Muscle | Liver | Muscle | Liver | Muscle |
| 0% | 7.49E+09 | 1.81E+08 | 7.33E+08 | 2.12E+07 | 3.03E+06 | 2.07E+05 |
| 5% | 7.05E+08 | 8.49E+07 | 2.07E+08 | 1.25E+07 | 1.11E+07 | 6.82E+06 |
| 10% | 6.97E+07 | 3.07E+08 | 5.72E+07 | 7.09E+07 | 1.36E+07 | 5.09E+07 |
| 20% | 2.34E+06 | 1.82E+08 | 2.53E+06 | 1.69E+08 | 3.75E+05 | 1.37E+08 |

### Formulation 3:

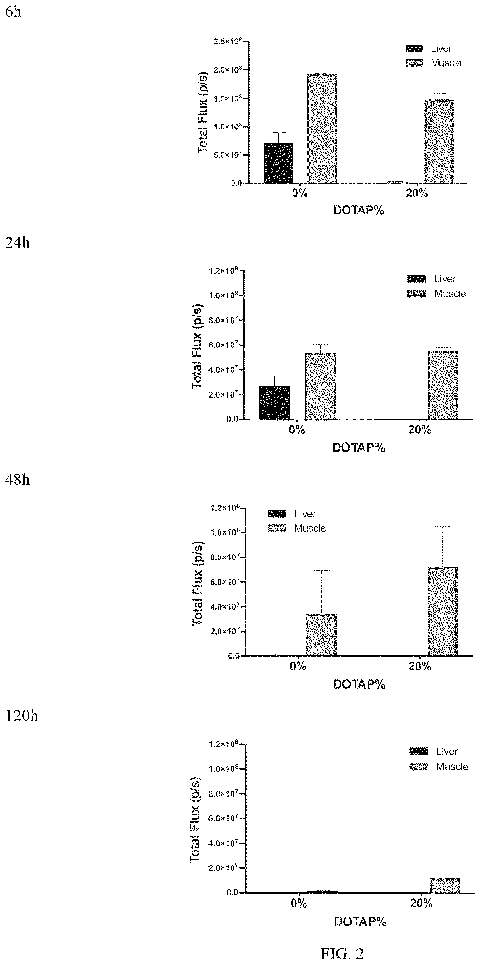

Formulation 3 is a formulation formed by formulating compound 108 with 0% or 20% of DOTAP, cholesterol, and PEGylated lipid. The same assay method as above was used to further verify the in situ expression in the muscle and the fluorescence expression at different time points of different combinations of ionizable lipids and permanent positively charged lipids. The results are shown in FIG. 2 and Table 8. FIG. 2 shows the results of the fluorescence intensity at the muscle site at different time points for the formulae with the addition of different proportions of DOTAP in formulation 3. Each point represents the mean of the fluorescence expression of four mice at the corresponding organ site and at different time points (n=4).

Because the lipid compound 108 itself has good targeting in situ to muscle, in the fluorescence imaging at 6 hours, the in situ expression of the formula with 0% of DOTAP in muscle was higher than that of the formula with 20% of DOTAP, but the expression of the formula with 0% of DOTAP was still relatively high in the liver. At 6 hours, the ratio of fluorescence expression in muscle in situ to liver was 2.7 for the formula with 0% of DOTAP, while for the formula with 20% of DOTAP, the ratio increased to 85.1, a 22.7-fold increase. This indicates that the addition of DOTAP enhanced the enrichment of the overall formulation of LNP in muscle in situ.

Over time, up to 24 h, the in situ expression of the formula with 0% of DOTAP in the muscle site was comparable to that of the formula with 20% of DOTAP. At 48 h, the in situ expression intensity of the formula with 0% of DOTAP in the muscle site was significantly lower than that of the formula with 20% of DOTAP in the muscle site. By 120 h, the in situ expression of the formula with 0% of DOTAP in the muscle site was essentially undetectable, but the formula with 20% of DOTAP still showed significant expression fluorescence value in the muscle site.

**Table 8**

| | 6h | | 24h | | 48h | | 120h | |
|---|---|---|---|---|---|---|---|---|
| | liver | muscle | liver | muscle | liver | muscle | liver | muscle |
| 0% | 7.14E+07 | 1.93E+08 | 2.72E+07 | 5.37E+07 | 1.44E+06 | 3.45E+07 | 6.44E+04 | 1.34E+06 |
| 20% | 1.74E+06 | 1.48E+08 | 8.43E+04 | 5.57E+07 | 6.79E+04 | 7.25E+07 | 5.60E+04 | 1.21E+07 |

### Assay example 6

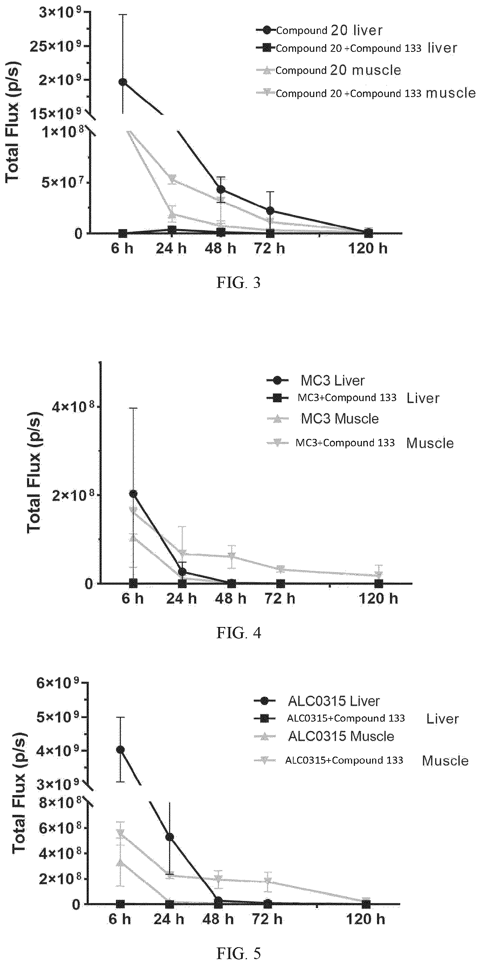

Formulations 5-1, 5-2, 6-1, 6-2, 7-1, 7-2, 8-1, 8-2, 8-3, 8-4, 8-5, 9-1, 9-2, 10-1, and 10-2 were prepared by the same method as in assay example 4 and assayed for delivery effect by the method of assay example 5. The formulation information and assay results of each formulation are shown in Tables 9-26 and FIGs. 3-11.

**Table 9**

| Formulation 5-1 | | Formulation 5-2 | |
|---|---|---|---|
| Lipid | Molar percentage % | Lipid | Molar percentage % |
| N/P | 6 | N/P | 6 |
| Compound 20 | 49.5 | Compound 20 | 49.5 |
| cholesterol | 46.5 | cholesterol | 46.5 |
| DSPC | 2.5 | Compound 133 | 2.5 |
| PEG2K-DMG | 1.5 | PEG2K-DMG | 1.5 |

**Table 10. Fluorescence light intensity (p/s) in muscle in situ and liver sites in mice after injection of formulation 5-1/5-2**

| | **Formulation 5-1 Liver** | **Formulation 5-2 Liver** | **Formulation 5-1 Muscle** | **Formulation** 5-2 **Muscle** |
|---|---|---|---|---|
| 6h | 1.97E+09 | 2.18E+04 | 2.73E+08 | 1.74E+08 |
| 24 h | 6.61E+08 | 3.81E+06 | 1.91E+07 | 5.29E+07 |
| 48 h | 4.30E+07 | 1.27E+06 | 7.61E+06 | 3.17E+07 |
| 72 h | 2.22E+07 | 2.29E+04 | 3.09E+06 | 1.12E+07 |
| 120 h | 9.47E+05 | 3.33E+04 | 1.38E+06 | 2.15E+06 |

According to FIG. 3 and Table 10, it can be seen that the expression level of formulation 5-2 with the addition of permanent cationic compound 133 in the liver decreased significantly, and was significantly lower than that of formulation 5-1 without the addition of permanent cation at any one time point. With the prolongation of time after injection, formulation 5-2 showed almost no detectable protein expression signal in the liver, but its signal in muscle decayed slowly, and maintained a significant fluorescent expression value in the muscle site.

**Table 11**

| Formulation 6-1 | | Formulation 6-2 | |
|---|---|---|---|
| Lipid | Molar percentage % | Lipid | Molar percentage % |
| N/P | 6 | N/P | 6 |
| DLin-MC3-DMA | 50 | DLin-MC3-DMA | 50 |
| cholesterol | 38.5 | cholesterol | 38.5 |
| DSPC | 10 | Compound 133 | 10 |
| PEG2K-DMG | 1.5 | PEG2K-DMG | 1.5 |

**Table 12. Fluorescence light intensity (p/s) in muscle in situ and liver sites in mice after injection of formulation 6-1/6-2**

| | **Formulation 6-1 Liver** | **Formulation 6-2 Liver** | **Formulation 6-1 Muscle** | **Formulation 6-2 Muscle** |
|---|---|---|---|---|
| 6h | 2.04E+08 | 1.72E+05 | 1.05E+08 | 1.62E+08 |
| 24h | 2.70E+07 | 1.24E+05 | 1.23E+07 | 6.68E+07 |
| 48h | 1.20E+07 | 2.42E+05 | 2.58E+06 | 6.06E+07 |
| 72h | 4.27E+05 | 6.56E+04 | 9.89E+05 | 3.19E+07 |
| 120h | 6.52E+04 | 1.30E+04 | 4.58E+05 | 1.75E+07 |

According to FIG. 4 and Table 12, it can be seen that the expression level of formulation 6-2 with the addition of permanent cationic compound 133 in the liver decreased significantly. At 6 hours, the expression level of formulation 6-2 with the addition of permanent cationic compound 133 in the liver was only 0.08% of that of formulation 6-1 without the addition of permanent cation. Moreover, at any time point, the expression level of formulation 6-2 in muscle was significantly higher than that of formulation 6-1. Formulation 6-2 always maintained hundreds of times the ratio of fluorescence expression in muscle in situ/liver, showing good muscle targeting.

**Table 13**

| Formulation 7-1 | | Formulation 7-2 | |
|---|---|---|---|
| Lipid | Molar percentage % | Lipid | Molar percentage % |
| N/P | 6 | N/P | 6 |
| ALC-0315 | 46.3 | ALC-0315 | 46.3 |
| cholesterol | 42.7 | cholesterol | 42.7 |
| DSPC | 9.4 | Compound 133 | 9.4 |
| ALC0159 | 1.6 | ALC0159 | 1.6 |

**Table 14. Fluorescence light intensity (p/s) in muscle in situ and liver sites in mice after injection of formulation 7-1/7-2**

| | **Formulation 7-1 Liver** | **Formulation 7-2 Liver** | **Formulation 7-1 Muscle** | **Formulation 7-2 Muscle** |
|---|---|---|---|---|
| 6 h | 4.04E+09 | 2.73E+06 | 3.33E+08 | 5.57E+08 |
| 24 h | 5.32E+08 | 6.62E+05 | 2.03E+07 | 2.27E+08 |
| 48 h | 3.09E+07 | 1.36E+05 | 7.61E+06 | 1.94E+08 |
| 72 h | 1.10E+07 | 2.02E+05 | 2.75E+06 | 1.76E+08 |
| 120 h | 1.02E+06 | 4.01E+04 | 8.55E+05 | 2.28E+07 |

According to FIG. 5 and Table 14, it can be seen that the expression level of formulation 7-2 with the addition of permanent cationic compound 133 in the liver decreased significantly. At 6 hours, the expression level of formulation 7-2 with the addition of permanent cationic compound 133 in the liver was only 0.07% of that of formulation 7-1 without the addition of permanent cation. Moreover, at any time point, the expression level of formulation 7-2 in muscle was significantly higher than that of formulation 7-1. Formulation 7-2 always maintained hundreds or even thousands of times the ratio of fluorescence expression in muscle in situ/liver, showing good muscle targeting.

**Table 15**

| Formulation 8-1 | | Formulation 8-2 | |
|---|---|---|---|
| Lipid | Molar percentage % | Lipid | Molar percentage % |
| N/P | 4 | N/P | 4 |
| Compound 46 | 47.5 | Compound 46 | 47.5 |
| cholesterol | 40.5 | cholesterol | 40.5 |
| DSPC | 10 | Compound 133 | 10 |
| PEG2K-DMG | 2 | PEG2K-DMG | 2 |

**Table 16. Fluorescence light intensity (p/s) in muscle in situ and liver sites in mice after injection of formulation 8-1/8-2**

| | **Formulation 8-1 Liver** | **Formulation 8-2 Liver** | **Formulation 8-1 Muscle** | **Formulation 8-2 Muscle** |
|---|---|---|---|---|
| 6 h | 2.00E+09 | 1.33E+07 | 2.17E+08 | 2.54E+08 |
| 24 h | 4.05E+08 | 4.73E+04 | 1.43E+07 | 3.36E+07 |
| 48 h | 1.82E+07 | 3.38E+04 | 4.15E+06 | 1.81E+07 |
| 72 h | 6.32E+06 | 4.16E+05 | 2.11E+06 | 1.96E+07 |
| 120 h | 6.56E+05 | 5.63E+04 | 7.90E+05 | 1.12E+07 |

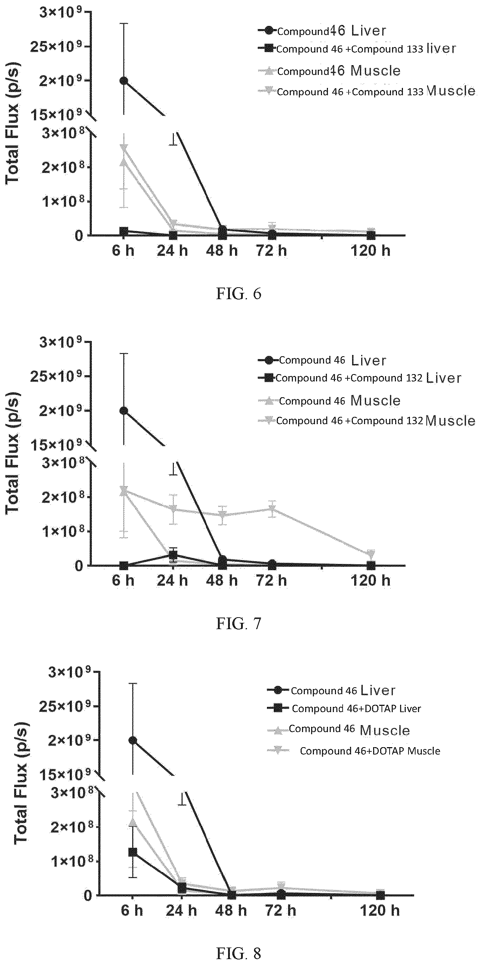

According to FIG. 6 and Table 16, it can be seen that the expression level of formulation 8-2 with the addition of permanent cationic compound 133 in the liver decreased significantly. At 6 hours, the expression level of formulation 8-2 with the addition of permanent cationic compound 133 in the liver was only 0.6% of that of formulation 8-1 without the addition of permanent cation. After 24 hours, formulation 8-2 showed almost no detectable protein expression signal in the liver. Moreover, at any one time point, the expression level of formulation 8-2 in muscle was significantly higher than that of formulation 8-1, showing good muscle targeting.

**Table 17**

| Formulation 8-3 | |
|---|---|
| Lipid | Molar percentage % |
| N/P | 4 |
| Compound 46 | 47.5 |
| cholesterol | 40.5 |
| Compound 132 | 10 |
| PEG2K-DMG | 2 |

**Table 18. Fluorescence light intensity (p/s) in muscle in situ and liver sites in mice after injection of formulation 8-1/8-3**

| | **Formulation 8-1 Liver** | **Formulation 8-3 Liver** | **Formulation 8-1 Muscle** | **Formulation 8-3 Muscle** |
|---|---|---|---|---|
| 6 h | 2.00E+09 | 3.21E+04 | 2.17E+08 | 2.21E+08 |
| 24 h | 4.05E+08 | 3.18E+07 | 1.43E+07 | 1.64E+08 |
| 48 h | 1.82E+07 | 1.30E+06 | 4.15E+06 | 1.46E+08 |
| 72 h | 6.31E+06 | 4.23E+05 | 2.11E+06 | 1.65E+08 |
| 120 h | 6.56E+05 | 2.75E+04 | 7.90E+05 | 3.03E+07 |

According to FIG. 7 and Table 18, it can be seen that the expression level of formulation 8-3 with the addition of permanent cationic compound 132 in the liver decreased significantly. At 6 hours, the expression level of formulation 8-3 with the addition of permanent cationic compound 132 in the liver was only 0.02‰ of that of formulation 8-1 without the addition of permanent cation. Moreover, at any one time point, the expression level of formulation 8-3 in muscle was significantly higher than that of formulation 8-1. From 72h to 120h after administration, the fluorescence expression ratio of formulation 8-3 in muscle in situ/liver continued to be hundreds or even thousands of times, showing good muscle targeting.

**Table 19**

| Formulation 8-4 | |
|---|---|
| Lipid | Molar percentage % |
| N/P | 4 |
| Compound 46 | 47.5 |
| cholesterol | 40.5 |
| DOTAP | 10 |
| PEG2K-DMG | 2 |

**Table 20. Fluorescence light intensity (p/s) in muscle in situ and liver sites in mice after injection of formulation 8-1/8-4**

| | **Formulation 8-1 Liver** | **Formulation 8-4 Liver** | **Formulation 8-1 Muscle** | **Formulation 8-4 Muscle** |
|---|---|---|---|---|
| 6 h | 2.00E+09 | 1.27E+08 | 2.17E+08 | 3.02E+08 |
| 24 h | 4.05E+08 | 2.18E+07 | 1.43E+07 | 3.37E+07 |
| 48 h | 9.40E+05 | 1.30E+06 | 4.15E+06 | 1.29E+07 |
| 72 h | 6.32E+06 | 5.36E+05 | 2.11E+06 | 2.22E+07 |
| 120 h | 6.56E+05 | 5.47E+04 | 7.90E+05 | 5.84E+06 |

According to FIG. 8 and Table 20, it can be seen that the expression level of formulation 8-4 with the addition of permanent cationic DOTAP in the liver decreased significantly. At 6 hours, the expression level of formulation 8-4 with the addition of permanent cationic DOTAP in the liver was only 6% of that of formulation 8-1 without the addition of permanent cation. Moreover, at any one time point, the expression level of formulation 8-4 in muscle was significantly higher than that of formulation 8-1. At the same time, the expression level of formulation 8-4 in muscle was also significantly higher than that in the liver, showing good muscle targeting.

**Table 21**

| Formulation 8-5 | |
|---|---|
| Lipid | Molar percentage % |
| N/P | 4 |
| Compound 46 | 47.5 |
| cholesterol | 40.5 |
| DOTMA | 10 |
| PEG2K-DMG | 2 |

**Table 22. Fluorescence light intensity (p/s) in muscle in situ and liver sites in mice after injection of formulation 8-1/8-5**

| | **Formulation 8-1 Liver** | **Formulation 8-5 Liver** | **Formulation 8-1 Muscle** | **Formulation 8-5 Muscle** |
|---|---|---|---|---|
| 6 h | 2.00E+09 | 1.28E+08 | 2.17E+08 | 2.28E+08 |
| 24 h | 4.05E+08 | 3.02E+05 | 1.43E+07 | 1.25E+08 |
| 48 h | 9.40E+05 | 6.70E+05 | 4.15E+06 | 1.39E+08 |
| 72 h | 6.31E+06 | 3.16E+05 | 2.11E+06 | 1.04E+07 |
| 120 h | 6.56E+05 | 2.91E+04 | 7.90E+05 | 2.08E+06 |

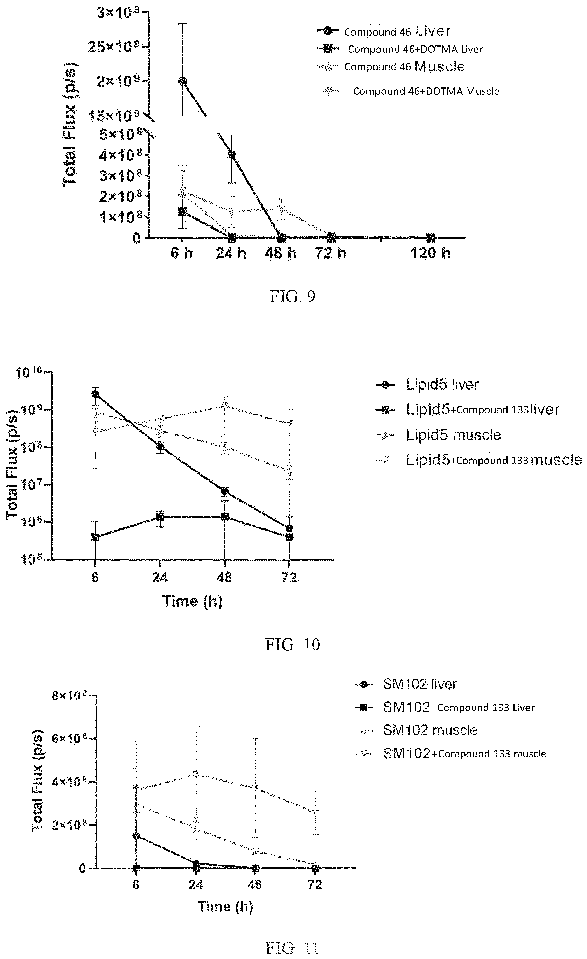

According to FIG. 9 and Table 22, it can be seen that the expression level of formulation 8-5 with the addition of permanent cationic DOTMA in the liver decreased significantly. At 6 hours, the expression level of formulation 8-5 with the addition of permanent cationic DOTMA in the liver was only 6% of that of formulation 8-1 without the addition of permanent cation. At 24 hours, the expression level of formulation 8-5 with the addition of permanent cationic DOTMA in the liver was reduced to only 0.08% of that of formulation 8-1, showing almost undetectable expression signal in the liver. Moreover, at any one time point, formulation 8-5 maintained an obvious expression fluorescence value in muscle, which was significantly higher than the expression level of formulation 8-1 in muscle, and was also significantly higher than the expression level of formulation 8-5 in the liver, showing good muscle targeting.

**Table 23**

| Formulation 9-1 | | Formulation 9-2 | |
|---|---|---|---|
| Lipid | Molar percentage % | Lipid | Molar percentage % |
| N/P | 6 | N/P | 6 |
| lipid5 | 50 | lipid5 | 50 |
| cholesterol | 38.5 | cholesterol | 38.5 |
| DSPC | 10 | Compound 133 | 10 |
| PEG2K-DMG | 1.5 | PEG2K-DMG | 1.5 |

**Table 24. Fluorescence light intensity (p/s) in muscle in situ and liver sites in mice after injection of formulation 9-1/9-2**

| | **Formulation 9-1 Liver** | **Formulation 9-2 Liver** | **Formulation 9-1 Muscle** | **Formulation 9-2 Muscle** |
|---|---|---|---|---|
| 6 h | 2.62E+09 | 3.90E+05 | 8.76E+08 | 2.64E+08 |
| 24 h | 1.06E+08 | 1.32E+06 | 2.82E+08 | 5.75E+08 |
| 48 h | 6.66E+06 | 1.36E+06 | 1.03E+08 | 1.26E+09 |
| 72 h | 6.70E+05 | 3.94E+05 | 2.30E+07 | 4.23E+08 |

According to FIG. 10 and Table 24, it can be seen that the expression level of formulation 9-2 with the addition of permanent cationic compound 133 in the liver decreased significantly. At any one time point, the ratio of fluorescence expression of formulation 9-2 in muscle in situ/liver was hundreds of times or higher, showing good muscle targeting.

**Table 25**

| Formulation 10-1 | | Formulation 10-2 | |
|---|---|---|---|
| Lipid | Molar percentage % | Lipid | Molar percentage % |
| N/P | 6 | N/P | 6 |
| SM102 | 50 | SM102 | 50 |
| cholesterol | 38.5 | cholesterol | 38.5 |
| DSPC | 10 | Compound 133 | 10 |
| PEG2K-DMG | 1.5 | PEG2K-DMG | 1.5 |

**Table 26. Fluorescence light intensity (p/s) in muscle in situ and liver sites in mice after injection of formulation 10-1/10-2**

| | **Formulation 10-1 Liver** | **Formulation 10-2 Liver** | **Formulation 10-1 Muscle** | **Formulation 10-2 Muscle** |
|---|---|---|---|---|
| 6 h | 1.51E+08 | 8.83E+05 | 2.96E+08 | 3.61E+08 |
| 24 h | 2.23E+07 | 8.66E+05 | 1.82E+08 | 4.36E+08 |
| 48 h | 1.93E+06 | 2.38E+05 | 7.79E+07 | 3.71E+08 |
| 72 h | 3.11E+05 | 2.23E+05 | 1.64E+07 | 2.57E+08 |

According to FIG. 11 and Table 26, it can be seen that the expression level of formulation 10-2 with the addition of permanent cationic compound 133 in the liver decreased significantly. At any one time point, formulation 10-2 maintained an obvious expression fluorescence value in the muscle site, which was significantly higher than the expression level of formulation 10-1 in the muscle site, and was also significantly higher than the expression level of formulation 10-2 in the liver, showing good muscle targeting.

In summary, the formulations obtained by combining different ionizable lipids and different cationic lipids in the present disclosure can significantly reduce the amount of drugs off-target to the liver, and have a good muscle targeting effect, so that the ratio of fluorescence expression of drugs in muscle in situ to in the liver can be as high as thousands of times; at the same time, the expression time of the drugs at the muscle injection site is prolonged, and the drugs are highly expressed within 72 hours and still expressed at 120 hours.

## Claims

1. A lipid nanoparticle for topical injection comprising a Long-Acting SusTained delivering lipid and an ionizable lipid, wherein the lipid nanoparticle acts at the injection site.

2. The lipid nanoparticle of claim 1, wherein the site of the topical injection is muscle or tumor tissue, alternatively muscle; alternatively, the lipid nanoparticle has an extended duration of action compared to a lipid nanoparticle without a Long-Acting SusTained delivering lipid.

3. The lipid nanoparticle of claim 1, which is capable of reducing off-target effects in tissues or organs at a non-injection site; alternatively, the tissue or organ at a non-injection site is the liver.

4. The lipid nanoparticle of any one of claims 1-3, which comprises the following components: a Long-Acting SusTained delivering lipid, a structured lipid, a polymer-conjugated lipid and an ionizable lipid, and comprises optionally a neutral phospholipid.

5. The lipid nanoparticle of any one of claims 1-4, wherein the Long-Acting SusTained delivering lipid is a permanently cationic lipid, alternatively a permanently cationic lipid with pKa > 10, or a permanently cationic lipid containing a quaternary ammonium structure.

6. The lipid nanoparticle of claim 5, wherein the permanently cationic lipid is selected from a pharmaceutically acceptable salt of the compound of formula (I): 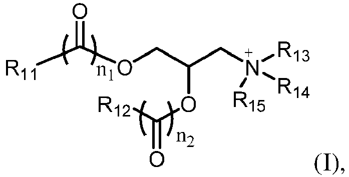 wherein
R₁₁ and R₁₂ are independently selected from C₆₋₃₀ alkyl, C₆₋₃₀ alkenyl and C₆₋₃₀ alkynyl, which are optionally substituted with 1, 2, 3, 4 or 5 substituents selected from: -OH, halogen, cyano, W₁₋₃₀ alkyl, C₁₋₃₀ haloalkyl, -O-C₁₋₃₀ alkyl, -S-C₁₋₃₀ alkyl, amino, -NH-C₁₋₃₀ alkyl and -N(C₁₋₃₀ alkyl)₂;
R₁₃, R₁₄ and R₁₅ are independently selected from C₁₋₆ alkyl, C₁₋₆ haloalkyl, C₂₋₆ alkenyl and C₂₋₆ alkynyl; or any two of them and the N atom to which they are attached together form 4- to 8-membered heterocycle;
n₁ and n₂ are independently selected from 0 and 1;
alternatively,
R₁₁ and R₁₂ are independently selected from C₁₀₋₂₅ alkyl, C₁₀₋₂₅ alkenyl and C₁₀₋₂₅ alkynyl, which are optionally substituted with 1, 2, 3, 4 or 5 substituents selected from: -OH, halogen, cyano, C₁₋₂₅ alkyl, C₁₋₂₅ haloalkyl, -O-C₁₋₂₅ alkyl, -S-C₁₋₂₅ alkyl, amino, -NH-C₁₋₂₅ alkyl and -N(C₁₋₂₅ alkyl)₂;
R₁₃, R₁₄ and R₁₅ are independently selected from C₁₋₆ alkyl, C₁₋₆ haloalkyl, C₂₋₆ alkenyl and C₂₋₆ alkynyl; or any two of them and the N atom to which they are attached together form 5- to 6-membered heterocycle;
n₁ and n₂ are independently selected from 0 and 1;
alternatively,
R₁₁ and R₁₂ are independently selected from C₁₃₋₂₀ alkyl, C₁₃₋₂₀ alkenyl and C₁₃₋₂₀ alkynyl, alternatively selected from C₁₃₋₁₈ alkyl, C₁₃₋₁₈ alkenyl and C₁₃₋₁₈ alkynyl, alternatively selected from C₁₅₋₁₈ alkyl, C₁₅₋₁₈ alkenyl and C₁₅₋₁₈ alkynyl, such as C₁₇₋₁₈ alkyl, C₁₇₋₁₈ alkenyl and C₁₇₋₁₈ alkynyl, which are optionally substituted with 1, 2, 3, 4 or 5 substituents selected from: -OH, halogen, cyano, C₁₋₂₀ alkyl, C₁₋₂₀ haloalkyl, -O-C₁₋₂₀ alkyl, -S-C₁₋₂₀ alkyl, amino, -NH-C₁₋₂₀ alkyl and -N(C₁₋₂₀ alkyl)₂;
R₁₃, R₁₄ and R₁₅ are independently selected from C₁₋₆ alkyl, C₁₋₆ haloalkyl, C₂₋₆ alkenyl and C₂₋₆ alkynyl, alternatively C₁₋₆ alkyl, such as Me;
n₁ and n₂ are independently selected from 0 and 1.

7. The lipid nanoparticle of claim 5, wherein the permanently cationic lipid is selected from a pharmaceutically acceptable salt of the compound of formula (II): 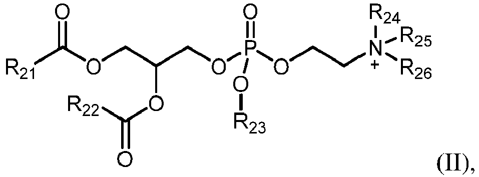 wherein
R₂₁ and R₂₂ are independently selected from C₆₋₃₀ alkyl, C₆₋₃₀ alkenyl and C₆₋₃₀ alkynyl, which are optionally substituted with 1, 2, 3, 4 or 5 substituents selected from: -OH, halogen, cyano, C₁₋₃₀ alkyl, C₁₋₃₀ haloalkyl, -O-C₁₋₃₀ alkyl, -S-C₁₋₃₀ alkyl, amino, -NH-C₁₋₃₀ alkyl and -N(C₁₋₃₀ alkyl)₂;
R₂₃ is selected from C₁₋₆ alkyl, C₁₋₆ haloalkyl, C₂₋₆ alkenyl and C₂₋₆ alkynyl, which is optionally substituted with 1, 2 or 3 R₂₃ₛ;
R₂₃ₛ is independently selected from C₁₋₆ alkyl, C₁₋₆ haloalkyl, -OC(O)R₂ₐ and -C(O)OR₂ₐ;
R₂ₐ is independently selected from H, C₁₋₆ alkyl and C₁₋₆ haloalkyl;
R₂₄, R₂₅ and R₂₆ are independently selected from C₁₋₆ alkyl, C₁₋₆ haloalkyl, C₂₋₆ alkenyl and C₂₋₆ alkynyl; or any two of them and the N atom to which they are attached together form 4- to 8-membered heterocycle;
alternatively,
R₂₁ and R₂₂ are independently selected from C₁₀₋₂₅ alkyl, C₁₀₋₂₅ alkenyl and C₁₀₋₂₅ alkynyl, which are optionally substituted with 1, 2, 3, 4 or 5 substituents selected from: -OH, halogen, cyano, C₁₋₂₅ alkyl, C₁₋₂₅ haloalkyl, -O-C₁₋₂₅ alkyl, -S-C₁₋₂₅ alkyl, amino, -NH-C₁₋₂₅ alkyl and -N(C₁₋₂₅ alkyl)₂;
R₂₃ is selected from C₁₋₆ alkyl, C₁₋₆ haloalkyl, C₂₋₆ alkenyl and C₂₋₆ alkynyl, which is optionally substituted with 1, 2 or 3 R₂₃ₛ;
R₂₃ₛ is independently selected from C₁₋₆ alkyl, C₁₋₆ haloalkyl, -OC(O)R₂ₐ and -C(O)OR₂ₐ;
R₂ₐ is independently selected from H, C₁₋₆ alkyl and C₁₋₆ haloalkyl;
R₂₄, R₂₅ and R₂₆ are independently selected from C₁₋₆ alkyl, C₁₋₆ haloalkyl, C₂₋₆ alkenyl and C₂₋₆ alkynyl; or any two of them and the N atom to which they are attached together form 5- to 6-membered heterocycle;
alternatively,
R₂₁ and R₂₂ are independently selected from C₁₃₋₂₀ alkyl, C₁₃₋₂₀ alkenyl and C₁₃₋₂₀ alkynyl,
alternatively selected from C₁₃₋₁₇ alkyl, C₁₃₋₁₇ alkenyl and C₁₃₋₁₇ alkynyl, which are optionally substituted with 1, 2, 3, 4 or 5 substituents selected from: -OH, halogen, cyano, C₁₋₂₀ alkyl, C₁₋₂₀ haloalkyl, -O-C₁₋₂₀ alkyl, -S-C₁₋₂₀ alkyl, amino, -NH-C₁₋₂₀ alkyl and -N(C₁₋₂₀ alkyl)₂; R₂₃ is selected from C₁₋₆ alkyl and C₁₋₆ haloalkyl, alternatively Me and Et, which is optionally substituted with 1, 2 or 3 R₂₃ₛ;
R₂₃ₛ is independently selected from C₁₋₆ alkyl, C₁₋₆ haloalkyl, -OC(O)R₂ₐ and -C(O)OR₂ₐ, alternatively selected from C₁₋₆ alkyl, C₁₋₆ haloalkyl and -C(O)OR₂ₐ;
R₂ₐ is independently selected from H, C₁₋₆ alkyl and C₁₋₆ haloalkyl, alternatively Et;
R₂₄, R₂₅ and R₂₆ are independently selected from C₁₋₆ alkyl, C₁₋₆ haloalkyl, C₂₋₆ alkenyl and C₂₋₆ alkynyl, alternatively C₁₋₆ alkyl, such as Me; or any two of them and the N atom to which they are attached together form 5- to 6-membered heterocycle;
alternatively, the permanently cationic lipid is selected from a pharmaceutically acceptable salt of the following compounds: 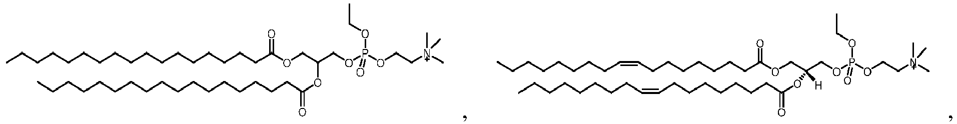 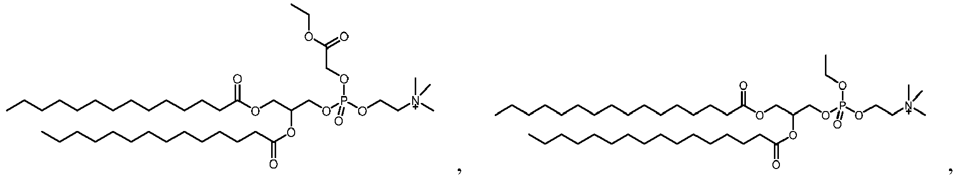 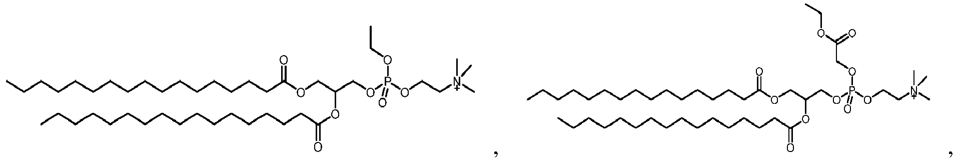 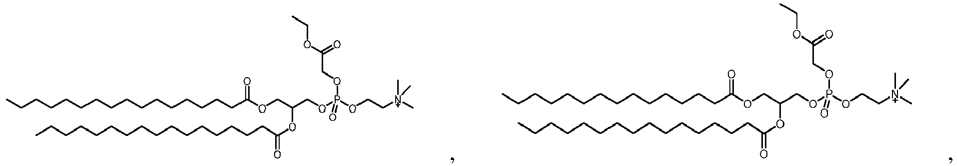 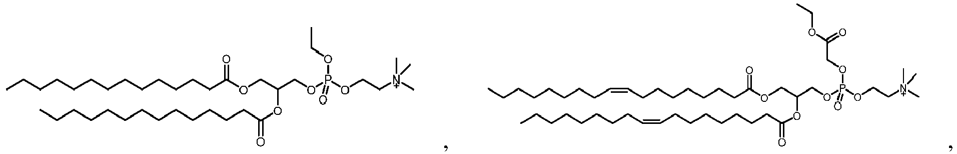 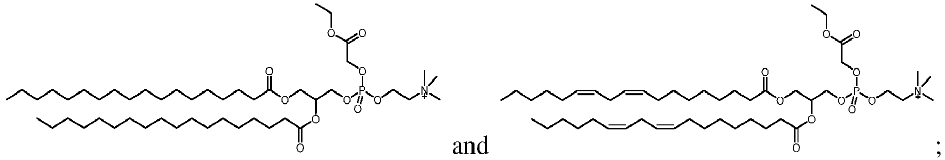
alternatively, the permanently cationic lipid is selected from one or more of: N-[1-(2,3-dioleyloxy)propyl]-N,N,N-trimethylammonium chloride (DOTMA), N-[1-(2,3-dioleoyloxy)propyl]-N,N,N-trimethylammonium chloride (DOTAP), ethylphosphatidylcholine (EPC) and derivatives thereof, N1-[2-((1S)-1-[(3-aminopropyl)amino]-4-[di(3-amino-propyl)amino]butylcarboxamido)ethyl ]-3,4-bis[oleyloxy]-benzamide (MVL5), dioctadecylamido-glycylspermine (DOGS), 3b-[N-(N',N'-dimethylaminoethyl)carbamoyl]cholesterol (DC-Chol) and dioctadecyldimethylammonium bromide (DDAB); alternatively selected from one or more of N-[1-(2,3-dioleyloxy)propyl]-N,N,N-trimethylammonium chloride (DOTMA), N-[1-(2,3-dioleoyloxy)propyl]-N,N,N-trimethylammonium chloride (DOTAP) and ethylphosphatidylcholine (EPC) and derivatives thereof; alternatively ethylphosphatidylcholine (EPC) and/or N-[1-(2,3-dioleoyloxy)propyl]-N,N,N-trimethylammonium chloride (DOTAP);
alternatively, the molar percentage content of the permanently cationic lipid is >0 mol%-30 mol%; alternatively 1.0 mol%-25 mol%; alternatively 2.5 mol%-20 mol%; alternatively 10 mol%-20 mol%; still alternatively 2.5 mol%, 5 mol%, 9.4 mol%, 10 mol% or 20 mol%.

8. The lipid nanoparticle of any one of claims 1-7, which does not contain a neutral phospholipid.

9. A lipid nanoparticle comprising the following components: a permanently cationic lipid, a structured lipid, a polymer-conjugated lipid and an ionizable lipid, and comprising no neutral phospholipid; alternatively, the permanently cationic lipid is as described in any one of claims 6-7.

10. The lipid nanoparticle of any one of claims 4-9, comprising the following components in molar percentages:
30 mol%-80 mol% of an ionizable lipid;
30 mol%-70 mol% of a structured lipid;
>0 mol%-30 mol% of a permanently cationic lipid;
>0 mol%-5 mol% of a polymer-conjugated lipid;
alternatively, comprising the following components in molar percentages:
35 mol%-65 mol% of an ionizable lipid;
30 mol%-60 mol% of a structured lipid;
1 mol%-25 mol% of a permanently cationic lipid;
0.5 mol%-3 mol% of a polymer-conjugated lipid;
alternatively, comprising the following components in molar percentages:
40 mol%-50 mol% of an ionizable lipid;
38.5 mol%-53.5 mol% of a structured lipid;
2.5 mol%-20 mol% of a permanently cationic lipid;
1.5 mol%-2 mol% of a polymer-conjugated lipid;
alternatively, the content of a permanently cationic lipid is 10 mol%-20 mol%;
alternatively, the content of a structured lipid is 38.5 mol%-48.5 mol%.

11. A lipid nanoparticle composition comprising the lipid nanoparticle of any one of claims 1-10, and a load;
alternatively, the load is selected from one or more of therapeutic agent, prophylactic agent and diagnostic agent;
alternatively, the therapeutic, prophylactic or diagnostic agent is a nucleic acid;
alternatively, the nucleic acid is selected from one or more of ASO, RNA and DNA;
alternatively, the RNA is selected from one or more of: interfering RNA (RNAi), small interfering RNA (siRNA), short hairpin RNA (shRNA), antisense RNA (aRNA), messenger RNA (mRNA), modified messenger RNA (mmRNA), long non-coding RNA (lncRNA), microRNA (miRNA), small activating RNA (saRNA), multimeric coding nucleic acid (MCNA), polymeric coding nucleic acid (PCNA), guide RNA (gRNA), CRISPRRNA (crRNA) and nucleases, alternatively mRNA, still alternatively, modified mRNA.

12. A method of preparing the lipid nanoparticle composition of claim 11, comprising: mixing various lipid components and then mixing the various lipid components with a load;
alternatively, comprising mixing a solution containing the various lipid components with a solution containing a load;
alternatively, mixing a solution containing the various lipid components with a solution containing a load by means of microfluidic or impingement jets;
alternatively, in the solution containing lipid components, the solvent is an organic solvent,
alternatively an alcoholic solvent, alternatively ethanol;
alternatively, the load is a nucleic acid, which is dissolved with sodium acetate solution, alternatively 20-30 mmol/L of sodium acetate solution.

13. A pharmaceutical composition comprising the lipid nanoparticle composition of claim 11, and pharmaceutically acceptable excipient (s).

14. The lipid nanoparticle composition of claim 11, or the pharmaceutical composition of claim 13, for use in treating, diagnosing, or preventing a disease.

15. The lipid nanoparticle composition of claim 11, or the pharmaceutical composition of claim 13, for use in delivering a load;
wherein the load is selected from one or more of therapeutic agent, prophylactic agent and diagnostic agent.
